# EUROPÄISCHE PATENTSCHRIFT

(11) **EP 0 680 469 B1**
(45) Veröffentlichungstag und Bekanntmachung des Hinweises auf die Patenterteilung: **26.04.2000**
(21) Anmeldenummer: 94905073.6
(22) Anmeldetag: 18.01.1994
(51) Int. Cl.: C07C 279/14, C07C 279/18, C07C 311/19, C07D 233/88, C07D 249/08, A61K 31/155, A61K 31/195, C07C 237/22

(54) **AMINOSÄUREDERIVATE, DIESE VERBINDUNGEN ENTHALTENDE ARZNEIMITTEL UND VERFAHREN ZU IHRER HERSTELLUNG**
AMINOACID DERIVATES, MEDICAMENTS CONTAINING THESE COMPOUNDS AND PROCESS FOR PREPARING THE SAME
DERIVES D'AMINO-ACIDES, MEDICAMENTS CONTENANT CES COMPOSES ET PROCEDE PERMETTANT DE LES PREPARER

(30) Priorität: 20.01.1993 DE 4301452; 06.08.1993 DE 4326465
(43) Veröffentlichungstag der Anmeldung: 08.11.1995
(73) Patentinhaber: Boehringer Ingelheim Pharma KG, 55216 Ingelheim am Rhein (DE)
(72) Erfinder: RUDOLF, Klaus, D-88400 Biberach (DE); EBERLEIN, Wolfgang, D-88400 Biberach (DE); ENGEL, Wolfhard, D-88400 Biberach (DE); MIHM, Gerhard, D-88400 Biberach (DE); DOODS, Henri, D-88447 Warthausen (DE); WIELAND, Heike-Andrea, D-88400 Biberach (DE); WILLIM, Klaus-Dieter, D-88454 Schweinhausen (DE); KRAUSE, Jürgen Dr., D-88444 Ummendorf (DE); DOLLINGER, Horst, D-55218 Ingelheim (DE); ESSER, Franz, D-55218 Ingelheim (DE); SCHNORRENBERG, Gerd, D-55435 Gau-Algehseim (DE); ENTZEROTH, Michael, D-88447 Warthausen (DE); WIENEN, Wolfgang, D-88437 Äpfingen (DE)
(74) Vertreter: Laudien, Dieter, Dr.
(86) Internationale Anmeldenummer: EP9400109
(87) Internationale Veröffentlichungsnummer: WO9417035

(56) Entgegenhaltungen:
- EP-A- 0 152 872
- EP-A- 0 383 690
- WO-A-92/08709
- GB-A- 1 538 207
- GB-A- 2 007 663
- US-A- 3 412 150
- US-A- 3 856 848
- "BIOCHEMISTRY", Band 10, Nr. , Seiten 4813 - 20

## Beschreibung

Gegenstand der vorliegenden Erfindung sind neue Aminosäurederivate der allgemeinen Formel

T - Z - NR¹ - (R²CR³) - CO - Y - (CH₂)n - R , (I)

deren Tautomere, deren Diastereomere, deren Enantiomere, deren Gemische und deren Salze, insbesondere deren physiologisch verträgliche Salze mit anorganischen oder organischen Säuren oder Basen, und Verfahren zu ihrer Herstellung.

In der WO-A-92/08709 werden bereits strukturverwandte N-α-(Naphthylsulfonyl)-3-amidinophenylalaninderivate beschrieben, die eine antithrombotische Wirksamkeit aufweisen. Ferner werden in der GB-A-1,538,207 antithrombotische N²-Arylsulfonyl-L-argininamide, insbesondere N²-Naphthylsulfonyl-Derivate, beschrieben. Des weiteren sind in Biochemistry 10 (25), 4813-4820 (1971) strukturverwandte Phenylalanin-alkylamide und Phenylalanin-phenylalkylamide im Zusammenhang mit der Untersuchung des aktiven Zentrums von Phe-tRNA Synthetase beschrieben.

In der obigen allgemeinen Formel I bedeuten
die R-(CH₂)ₙ-Gruppe eine Alkylgruppe mit 1 bis 3 Kohlenstoffatomen, die in ω-Stellung durch 2 Phenylgruppen substituiert sein kann,
eine geradkettige Alkylengruppe mit 2 bis 5 Kohlenstoffatomen, die endständig durch eine Hydroxygruppe substituiert ist,
eine Phenyl- oder Phenylalkylgruppe mit 1 bis 4 Kohlenstoffatomen im Alkylteil, wobei die Phenylgruppe jeweils durch ein Fluor-, Chlor- oder Bromatom, durch eine Alkylaminocarbonylgruppe mit 1 bis 4 Kohlenstoffatomen im Alkylteil, durch eine Ethylaminocarbonylaminogruppe, in der der Ethylteil durch ein oder zwei Phenylreste substituiert ist, durch eine Methyl-, Hydroxymethyl-, Phenyl-, Hydroxyphenyl-, Hydroxy-, Methoxy-, Ethoxy-, Dimethylaminosulfonyloxy-, Cyano-, Carboxy-, Methoxycarbonyl-, Acetyl-, Aminocarbonyl-, Dimethylaminocarbonyl-, Amino-, Methylamino-, Dimethylamino-, Acetylamino-, Methoxycarbonylamino-, Ethoxycarbonylamino-, Aminocarbonylamino-, Methylaminocarbonylamino-, Dimethylaminocarbonylamino-, Methylsulfonylamino-, Aminosulfonyl-, [Amino(imino)-methyl]-, [Amino(imino)methyl]amino-, [1,2-Dihydro-3,5(4H)-dioxo-1,2-diphenyl-3H-1,2,4-triazol-4-yl]methyl-, [1,5-Dihydro-2,4(3H)-dioxo-5,5-diphenyl-imidazol-3-yl]methyl-, Aminomethyl-, Aminosulfonylamino-, Hydroxyethyl-, Hydroxypropyl-, Hydroxybutyl-, 3-(Dimethylamino)propoxy- oder Ethoxycarbonyloxy-Gruppe substituiert sein kann,
eine Methylgruppe, die durch eine Hydroxycyclohexyl-, 4-Hydroxy-3-methyl-phenyl-, 3,4-Dimethoxyphenyl-, 3-Hydroxy-4-methoxy-phenyl-, 3-Methoxy-4-hydroxyphenyl-, 4-Amino-3,5-dibromphenyl-, 4-Amino-3,5-dichlorphenyl-, 4-Hydroxy-3,5-dibromphenyl-, 4-Hydroxy-3,5-dichlorphenyl-, 4-Amino-3-fluorphenyl-, 4-Hydroxy-3,5-dimethylphenyl-, Thienyl-, Pyridinyl-, Indolyl-, Benzimidazolyl-, Chinolinyl- oder 2-(Diphenylaminocarbonyl)-2,3-dihydro-1H-isoindolyl-Gruppe substituiert ist,
eine Ethylgruppe, die durch eine Indolyl-, 5-Methoxyindolyl-, 1,2-Diphenyl-3,5(4H)-dioxo-1,2,4-triazol-4-yl- oder Imidazolylgruppe substituiert ist,
eine 2-(2-Hydroxyphenyl)ethylgruppe, die in α-Stellung durch eine Aminocarbonylgruppe substituiert ist,
eine 3-[1-[2-[5,11-Dihydro-6(6H)-oxopyrido[2,3-b][1,4]benzodiazepin-5-yl]-2-oxoethyl]-4-piperidinyl]propyl- oder 3-Hydroxy-1-propin-1-yl-Gruppe,
die T-Z-Gruppe ein Wasserstoffatom,
eine Carbonylgruppe, die durch eine Alkylgruppe mit 1 bis 4 Kohlenstoffatomen, welche in α- oder β-Stellung durch eine Alkylgruppe mit 1 bis 3 Kohlenstoffatomen, durch ein oder zwei gegebenenfalls durch Methoxygruppen, Hydroxygruppen, Chlor- oder Bromatome mono- oder disubstituierte Phenylreste substituiert sein kann, substituiert ist,
eine Carbonylgruppe, die durch eine durch eine Benzyloxy- oder Phenylethoxygruppe substituierte Indolylgruppe, durch eine Methyl-, Tricyclo[3.3.1.1^{3.7}]dec-1-yl-, Benzyl-, (Hydroxyphenyl)methyl-, (Methylphenyl)methyl, (Biphenylyl)methyl-, (Dichlorphenyl)methyl-, Phenyl-, 3,5-Dichlorphenyl-, 3,4-Dichlorphenyl-, Naphthyl-, Naphthylmethyl-, α-Cyclopentyl-benzyl-, Diphenylamino-, Naphthylamino-, Hexamethylenimine-, 5-Phenylethoxy-indolyl-, 1,2,3,4-Tetrahydro-2-chinolinyl-, 2-Chinolinyl-, 1,2,3,4-Tetrahydro-3-chinolinyl-, (Dichlorphenoxy)methyl-, 9-Fluorenyl-, Triphenylmethyl-, 1-Piperidinyl-, (Diphenylmethyl)amino- oder 5,10-Dihydro-11(11H)-oxo-dibenzo[b,e][1,4]diazepin-5-yl-Gruppe substituiert ist, oder
eine 4-Amino-3,5-dichlor-phenylsulfonyl- oder Naphthylsulfonylgruppe,
R¹ das Wasserstoffatom,
R² einen unverzweigten Alkylrest mit 1 bis 5 Kohlenstoffatomen, der in ω-Stellung durch eine Guanidinogruppe, in der die Wasserstoffatome an den Stickstoffatomen jeweils durch Alkylgruppen mit 1 bis 3 Kohlenstoffatomen ersetzt sein können, durch eine Amino-, tert.Butoxycarbonylamino-, Dimethylamino-, N-Methyl-benzylamino-, Methylamino-, Aminocarbonyl-, Aminocarbonylamino-, Aminomethylidenimino-, Methylaminomethylidenimino-, [Amino(nitroimino)methyl]amino-, 1H-Imidazol-2-yl-amino-, 4,5-Dihydro-1H-imidazol-2-yl-amino-, (5-Amino-4H-1,2,4-triazol-3-yl)amino-, (3-Amino-1,2,4-oxadiazol-5-yl)amino- oder (5-Amino-1,2,4-oxadiazol-3-yl)amino-Gruppe substituiert ist,
eine Methylgruppe, die durch eine Phenyl-, Cyanophenyl-, Aminomethylphenyl-, Amidinophenyl-, Methylaminomethylideniminophenyl-, Cyaniminomethylaminophenyl-, Methyliminomethylaminophenyl-, (4,5-Dihydro-1H-imidazol-2-yl)phenyl- oder Imidazolylgruppe substituiert ist,
R³ das Wasserstoffatom oder die Methylgruppe und
Y eine gegebenenfalls durch eine Methyl- oder Ethylgruppe substituierte Iminogruppe mit den Maßgaben, daß
   (i)
      R¹ kein Wasserstoffatom darstellt, falls
      die Gruppe R-(CH₂)ₙ- eine Alkylgruppe mit 1 bis 3 Kohlenstoffatomen oder eine Phenylgruppe,
      R² eine Amidinophenylmethylgruppe,
      R³ ein Wasserstoffatom,
      T-Z eine 4-Amino-3,5-dichlorphenylsulfonylgruppe oder die Naphthylsulfonylgruppe und
      Y eine Iminogruppe bedeuten, sowie
   (ii)
      R² keinen unverzweigten Alkylrest mit 3 oder 4 Kohlenstoffatomen, der in ω-Stellung durch eine Guanidinogruppe, in der die Wasserstoffatome an den Stickstoffatomen jeweils durch eine Alkylgruppe mit 1 bis 3 Kohlenstoffatomen ersetzt sein können, durch eine Amino-, tert. Butoxycarbonylamino-, Dimethylamino-, N-Methylbenzylamino-, Methylamino-, Aminocarbonyl- oder Aminocarbonylaminogruppe substituiert ist, darstellt,
         falls
      R¹, R³ und T-Z jeweils ein Wasserstoffatom und
      Y eine gegebenenfalls durch eine Methyl- oder Ethylgruppe substituierte Iminogruppe bedeuten, sowie
   (iii)
      R² keine Phenylmethylgruppe darstellt, falls
      R¹, R³ und T-Z jeweils ein Wasserstoffatom,
      Y die Iminogruppe und
      die Gruppe R-(CH₂)ₙ- eine Alkylgruppe mit 1 bis 3 Kohlenstoffatomen oder eine Phenylalkylgruppe mit 1 bis 4 Kohlenstoffatomen im Alkylteil bedeuten,
   und folgende Verbindungen:
   (1) (R)-N-[[4-[(4,5-Dihydro-5,5-dimethyl-2,4(3H)-dioxo-1H-imidazol-3-yl)methyl]phenyl]methyl]-N²-(diphenylacetyl)-argininamid,
   (2) (R,S)-N²-(Diphenylacetyl)-N-[(4-hydroxyphenyl)methyl]-3-[(4-piperidinyl)methyl]-alaninamid,
   (3) (R,S)-3-[3-[Amino(hydroxyimino)methyl]phenyl]-N²-(diphenylacetyl)-N-[(4-hydroxyphenyl)methyl]-alaninamid,
   (4) (R,S)-3-[3-(Aminocarbonyl)phenyl]-N²-(diphenylacetyl)-N-[(4-hydroxyphenyl)methyl]-alaninamid,
   (5) (R)-N²-(2,2-Diphenylethyl)-N-[(4-hydroxyphenyl)methyl]-argininamid,
   (6) (R,S)-3-[(3-(Aminoiminomethyl)phenyl]-N²-(2,2-diphenylethyl)-N-[(4-hydroxyphenyl)methyl]-alaninamid,
   (7) (R)-N-[(4-Hydroxyphenyl)methyl]-N²-(5-phenyl-1-oxopentyl)-argininamid
   (8) (R)-N-[(4-Hydroxyphenyl)methyl]-N²-(4-phenyl-1-oxobutyl)-argininamid,
   (9) (R)-N-[(4-Hydroxyphenyl)methyl]-N²-(6-phenyl-1-oxohexyl)-argininamid,
   (10) (R,S)-N²-(2,2-Diphenyl-2-hydroxy-1-oxoethyl)-N-[(4-hydroxyphenyl)methyl]-argininamid,
   (11) N²-(Diphenylacetyl)-N-[(4-(methylamino)cyclohexyl)methyl]-argininamid,
   (12) (R)-N-[(4-Hydroxyphenyl)methyl]-N²-(tricyclo[3.3.1.1^{3.7}]-dec-1-ylacetyl)-argininamid,
   (13) (R)-N-[(4-Hydroxyphenyl)methyl]-N²-[3-(1-piperidinyl)-1-oxopropyl]-argininamid,
   (14) N²-(Diphenylacetyl)-N-[3-[1-[2-[5,11-dihydro-6(6H)-oxopyrido[2,3-b][1,4]benzodiazepin-5-yl]-2-oxoethyl]-4-piperidinyl]propyl]-argininamid,
   (15) (R)-N²-[(rac.-5,11-Dihydro-6-oxo-6H-dibenz[b,e]azepin-11-(15) (R)-N²-[(rac.-5,11-Dihydro-6-oxo-6H-dibenz[b,e]azepin-11-yl)carbonyl]-N-[(4-ethoxyphenyl)methyl]-argininamid und
   (16) N²-[N²-(Diphenylacetyl)-D-arginyl]-L-tyrosinamid,
   deren Tautomere, deren Diastereomere, deren Enantiomere, deren Gemische und deren Salze.

Die vorliegende Erfindung betrifft die Racemate, sofern in Verbindungen der allgemeinen Formel I das asymmetrische Kohlenstoffatom der zentralen Aminosäure das einzige Chiralitätselement ist. Die Anmeldung umfaßt aber auch die einzelnen Diastereomeren oder deren Gemische, die dann vorliegen, wenn eine unter die allgemeine Formel I fallende Verbindung zwei oder mehr als zwei Chiralitätselemente enthält. Besonders bevorzugt werden die unter die allgemeine Formel I fallenden Verbindungen, die hinsichtlich der Aminosäure-Partialatruktur

- NR¹ - (R²CR³) - CO -

D- bzw. (R)-konfiguriert sind.

Die Verbindungen der allgemeinen Formel I weisen wertvolle Eigenschaften auf. So stellen die Verbindungen der allgemeinen Formel I, in der T ein Wasserstoffatom und gleichzeitig Z eine Bindung bedeuten, wertvolle Zwischenprodukte zur Herstellung der restlichen Verbindungen der allgemeinen Formel I dar, welche wertvolle pharmakologische Eigenschaften aufweisen, insbesondere auf Grund ihrer NPY-antagonistischen Eigenschaften blutdrucksenkende Wirkungen. Ein weiterer Gegenstand der Erfindung sind diese Verbindungen enthaltende Arzneimittel, deren Verwendung und deren Herstellung.

Bevorzugte Verbindungen der obigen allgemeinen Formel I sind diejenigen, in denen
die R(CH₂)ₙ-Gruppe eine Phenylmethylgruppe, wobei die Phenylgruppe jeweils durch ein Fluor-, Chlor- oder Bromatom, durch eine Methyl-, Hydroxymethyl-, Hydroxyethyl-, Hydroxypropyl-, Hydroxy-, Methoxy-, Ethoxy-, Dimethylaminosulfonyloxy-, Ethoxycarbonyloxy-, Acetyl-, Methoxycarbonyl-, Aminocarbonyl-, Aminocarbonylamino-, Methylaminocarbonylamino-, Aminosulfonyl-, Dimethylamino-, [1,2-Dihydro-3,5(4H)-dioxo1,2-diphenyl-3H-1,2,4-triazol-4-yl]methyl- oder [1,5-Dihydro-2,4(3H)-dioxo-5,5-diphenyl-imidazol-3-yl]methyl-Gruppe substituiert ist,
eine Methylgruppe, die durch eine 3,4-Dimethoxyphenyl-, 3-Hydroxy-4-methoxyphenyl-, 3-Methoxy-4-hydroxyphenyl-, 4-Amino-3,5-di-bromphenyl-, 4-Amino-3,5-dichlorphenyl-, 4-Hydroxy-3,5-dimethyl-phenyl-, 4'-Hydroxy-4-biphenylyl-, Thienyl-, Pyridinyl-, Benz-imidazolyl- oder 1-[2-[5,11-Dihydro-6(6H)-oxopyrido[2,3-b][1,4]-benzodiazepin-5-yl]-2-oxo ethyl]-4-piperidinyl]- oder 4-Amino-3-fluorphenyl-Gruppe substituiert ist,
die T-Z-Gruppe eine Diphenylacetylgruppe, in der der Phenylkern jeweils durch ein Chlor- oder Bromatom substituiert sein kann,
eine Carbonylgruppe, die durch eine durch eine Benzyloxy- oder Phenylethoxygruppe substituierte Indolylgruppe, durch eine Benzyl-, (Dichlorphenyl)methyl-, Dichlorphenyl-, Naphthyl-, Naphthylmethyl-, α-Cyclopentylbenzyl-, 9-Fluorenyl- oder (Diphenylmethyl)amino-Gruppe substituiert ist,
R¹ das Wasserstoffatom,
R² eine geradkettige Alkylenkette mit 2 bis 5 Kohlenstoffatomen, welche endständig durch eine Amino-, Amidino-, Guanidino-, Aminocarbonyl-, Aminocarbonylamino- oder (1H-Imidazol-2-yl)amino-Gruppe substituiert ist, oder
eine Methylgruppe, die durch eine (Aminomethyl)phenyl-, Amidinophenyl- oder (4,5-Dihydro-1H-imidazol-2-yl)phenyl-Gruppe substituiert ist,
R³ das Wasserstoffatom oder die Methylgruppe und
Y eine gegebenenfalls durch eine Methylgruppe substituierte Iminogruppe bedeuten,
deren Tautomere, deren Diastereomere, deren Enantiomere, deren Gemische und deren Salze.

Als besonders bevorzugte Verbindungen seien beispielsweise folgende genannt:
(R)-N²-(Diphenylacetyl)-N-(phenylmethyl)-argininamid, 9-yl-, 5,11-Dihydro-6(6H)-oxo-dibenz[b,e]azepin-11-yl-, 5H-Dibenzo[a,d]cyclohepten-5-yl-, 10H-Phenothiazin-10-yl-, 2-Chlor-10H-phenothiazin-10-yl-, 5H-Dibenzo[b,f]azepin-5-yl-, 10,11-Dihydro-5H-dibenzo[b,f]azepin-5-yl-, 10H-Phenoxazin-10-yl-, 10H-Pyrido[3,2-b][1,4]benzothiazin-10-yl-, 9H-Xanthen-9-yl-, 9H-Thioxanthen-9-yl-, 5,11-Dihydro-6(6H)-oxo-pyrido[2,3-b][1,4]benzodiazepin-11-yl-,5,10-Dihydro-11(11H)-oxodibenzo[b,e][1,4]diazepin-5-yl-, 4,9-Dihydro-3-methyl-10(10H)-oxo-thieno[3,4-b][1,5]benzodiaze-pin-4-yl-, 1,3-Dimethyl-10-oxo-1,4,9,10-tetrahydropyrrolo[3,2-b][1,5]benzodiazepin-4-yl- oder 6,11-Dihydro-5(5H)-oxo-pyrido[2,3-b][1,5]-benzodiazepin-11-yl-Gruppe und
für R die Bedeutung der Phenyl-, 2-Pyridinyl-, 3-Pyridinyl-, 4-Pyridinyl-, 2-Thienyl-, 3-Thienyl-, 2-Furyl-, 3-Furyl-, 1H-Pyrrol-2-yl-, 1H-Pyrrol-3-yl-, 1-Methyl-1H-pyrrol-2-yl-, 1-Methyl-1H-pyrrol-3-yl-, 1-Naphthyl-, 2-Naphthyl-, 1H-Indol-2-yl-, 1H-Indol-3-yl-, 2,3-Dihydro-1H-isoindol-2-yl-, 2,3-Dihydro-1H-isoindol-3-yl-, 2,3-Dihydro-1H-isoindol-4-yl-, 2,3-Dihydro-1H-isoindol-5-yl-, 1H-Benzimidazol-2-yl-, 1H-Benzimidazol-4-yl-, 1H-Benzimidazol-5-yl-, Benzo[b]furan-2-yl-, Benzo[b]furan-3-yl-, Benzo[b]furan-5-yl-, Benzo[b]thiophen-2-yl-, Benzo[b]thiophen-3-yl-, Benzo[b]thiophen5-yl-, 2-Chinolinyl-, 3-Chinolinyl-, 4-Chinolinyl-, 6-Chinolinyl-, Benzo[c]thiophen-1-yl-, 1-Isochinolinyl-, 3-Isochinolinyl-, 4-Isochinolinyl-, Pyrazinyl-, 2-Pyrimidinyl-, 4-Pyrimidinyl-, 5-Pyrimidinyl-, 3-Pyridazinyl-, 4-Pyridazinyl-, 2-Imidazolyl-, 4-Imidazolyl-, 3-Pyrazolyl-, 4-Pyrazolyl-, 1,3-Oxazol-2-yl-, 1,3-Oxazol-4-yl-, 1,3-Oxazol-5-yl-, 3-Pyrazolyl-, 4-Pyrazolyl-, 3-Isoxazolyl-, 4-Isoxazolyl-, 5-Isoxazolyl-, 2-Chinazolinyl-, 4-Chinazolinyl- oder 2-Chinoxalinylgruppe, wobei diese zusätzlich durch die eingangs erwähnten Reste substituiert sein können,
für die bei der Definition der Reste T und R² erwähnten Schutzgruppen für eine Amino- oder Iminogruppe kommen die üblichen Schutzreste für eine Amino- oder Iminogruppe (siehe beispielsweise Houben-Weyl, Methoden der organischen Chemie Bd. 15/1) wie die p-Toluolsulfonyl-, Phenylmethoxycarbonyl-, tert.Butyloxycarbonyl-, (4-Methoxyphenyl)methoxycarbonyl-, Adamantyloxycarbonyl-, Biphenylylisopropyloxycarbonyl-, Isonicotinoyloxycarbonyl-, o-Nitrophenylsulfenyl-, Formyl-, o-Nitrophenylsulfenyl-, Biphenylylisopropyloxycarbonyl-, 9-Fluorenylmethoxycarbonyl-, Acetyl-, Trifluoracetyl-, (2-Chlorphenyl)methoxycarbonyl-, (4-Chlorphenyl)methoxycarbonyl-, (4-Nitrophenyl)methoxycarbonyl- oder Phthaloylgruppe in Betracht.

Die vorliegende Erfindung betrifft die Racemate, sofern in Verbindungen der allgemeinen Formel I das asymmetrische Kohlenstoffatom der zentralen Aminosäure das einzige Chiralitätselement ist. Die Anmeldung umfaßt aber auch die einzelnen Diastereomeren oder deren Gemische, die dann vorliegen, wenn eine unter die allgemeine Formel I fallende Verbindung zwei oder mehr als zwei Chiralitätselemente enthält. Besonders bevorzugt werden die unter die allgemeine Formel I fallenden Verbindungen, die hinsichtlich der Aminosäure-Partialstruktur

- NR¹ - (R²CR³) - CO -

D- bzw. (R)-konfiguriert sind.

Die Verbindungen der allgemeinen Formel I weisen wertvolle Eigenschaften auf. So stellen die Verbindungen der allgemeinen Formel I, in der T ein Wasserstoffatom und gleichzeitig Z eine Bindung bedeuten, wertvolle Zwischenprodukte zur Herstellung der restlichen Verbindungen der allgemeinen Formel I dar, welche wertvolle pharmakologische Eigenschaften aufweisen, insbesondere auf Grund ihrer NPY-antagonistischen Eigenschaften blutdrucksenkende Wirkungen. Ein weiterer Gegenstand der Erfindung sind diese Verbindungen enthaltende Arzneimittel, deren Verwendung und deren Herstellung.

Bevorzugte Verbindungen der obigen allgemeinen Formel I sind diejenigen, in denen
die R-(CH₂)ₙ-Gruppe eine Alkylgruppe mit 1 bis 3 Kohlenstoffatomen, die in ω-Stellung durch 2 Phenylgruppen substituiert sein kann,
eine geradkettige Alkylengruppe mit 2 bis 5 Kohlenstoffatomen, die endständig durch eine Hydroxygruppe substituiert ist,
eine Phenyl- oder Phenylalkylgruppe mit 1 bis 4 Kohlenstoffatomen im Alkylteil, wobei die Phenylgruppe jeweils durch ein Fluor-, Chlor- oder Bromatom, durch eine Alkylaminocarbonylgruppe mit 1 bis 4 Kohlenstoffatomen im Alkylteil, durch eine Ethylaminocarbonylaminogruppe, in der der Ethylteil durch ein oder zwei Phenylreste substituiert ist, durch eine Methyl-, Hydroxymethyl-, Phenyl-, Hydroxyphenyl-, Hydroxy-, Methoxy-, Ethoxy-, Dimethylaminosulfonyloxy-, Cyano-, Carboxy-, Methoxycarbonyl-, Acetyl-, Aminocarbonyl-, Dimethylaminocarbonyl-, Amino-, Methylamino-, Dimethylamino-, Acetylamino-, Methoxycarbonylamino-, Ethoxycarbonylamino-, Aminocarbonylamino-, Methylaminocarbonylamino-, Dimethylaminocarbonylamino-, Methylsulfonylamino-, Aminosulfonyl-, [Amino(imino)methyl]-, [Amino(imino)methyl]amino-, [1,2-Dihydro-3,5(4H)-dioxo-1,2-diphenyl-3H-1,2,4-triazol-4-yl]methyl-, [1,5-Dihydro-2,4(3H)-dioxo-5,5-diphenyl-imidazol-3-yl]methyl-, Aminomethyl-, Aminosulfonylamino-, Hydroxyethyl-, Hydroxypropyl-, Hydroxybutyl-, 3-(Dimethylamino)propoxy- oder Ethoxycarbonyloxy-Gruppe substituiert sein kann,
eine Methylgruppe, die durch eine Hydroxycyclohexyl-, 4-Hydroxy-3-methyl-phenyl-, 3,4-Dimethoxyphenyl-, 3-Hydroxy-4-methoxyphenyl-, 3-Methoxy-4-hydroxyphenyl-, 4-Amino-3,5-dibromphenyl-, 4-Amino-3,5-dichlorphenyl-, 4-Hydroxy-3,5-dibromphenyl-, 4-Hydroxy-3,5-dichlorphenyl-, 4-Amino-3-fluorphenyl-, 4-Hydroxy-3,5-dimethylphenyl-, Thienyl-, Pyridinyl-, Indolyl-, Benzimidazolyl-, Chinolinyl- oder 2-(Diphenylaminocarbonyl)-2,3-dihydro-1H-isoindolyl-Gruppe substituiert ist,
eine Ethylgruppe, die durch eine Indolyl-, 5-Methoxyindolyl-, 1,2-Diphenyl-3,5(4H)-dioxo-1,2,4-triazol-4-yl- oder Imidazolylgruppe substituiert ist,
eine 2-(2-Hydroxyphenyl)ethylgruppe, die in α-Stellung durch eine Aminocarbonylgruppe substituiert ist,
eine 3-[1-[2-[5,11-Dihydro-6(6H)-oxopyrido[2,3-b][1,4]benzodiazepin-5-yl]-2-oxoethyl]-4-piperidinyl]propyl- oder 3-Hydroxy-1-propin-1-yl-Gruppe,
die T-Z-Gruppe ein Wasserstoffatom,
eine Carbonylgruppe, die durch eine Alkylgruppe mit 1 bis 4 Kohlenstoffatomen, welche in α- oder β-Stellung durch eine Alkylgruppe mit 1 bis 3 Kohlenstoffatomen, durch ein oder zwei gegebenenfalls durch Methoxygruppen, Hydroxygruppen, Chlor- oder Bromatome mono- oder disubstituierte Phenylreste substituiert sein kann,
eine Carbonylgruppe, die durch eine durch eine Benzyloxy- oder Phenylethoxygruppe substituierte Indolylgruppe, durch eine Methyl-, Tricyclo[3.3.1.1^{3.7}]dec-1-yl-, Benzyl-, (Hydroxyphenyl)methyl-, (Methylphenyl)methyl, (Biphenylyl)methyl-, (Dichlorphenyl)methyl-, Phenyl-, 3,5-Dichlorphenyl-, 3,4-Dichlorphenyl-, Naphthyl-, Naphthylmethyl-, α-Cyclopentyl-benzyl-, Diphenylamino-, Naphthylamino-, Hexamethylenimino-, 5-Phenylethoxy-indolyl-, 1,2,3,4-Tetrahydro-2-chinolinyl-, 2-Chinolinyl-, 1,2,3,4-Tetrahydro-3-chinolinyl-, (Dichlorphenoxy)methyl-, 9-Fluorenyl-, Triphenylmethyl-, 1-Piperidinyl-, (Diphenylmethyl)amino- oder 5,10-Dihydro-11(11H)-oxo-dibenzo[b,e][1,4]diazepin-5-yl-Gruppe substituiert ist, oder
eine 4-Amino-3,5-dichlor-phenylsulfonyl- oder Naphthylsulfonylaminogruppe,
R¹ das Wasserstoffatom,
R² einen unverzweigten Alkylrest mit 1 bis 5 Kohlenstoffatomen, der in ω-Stellung durch eine Guanidinogruppe, in der die Wasserstoffatome an den Stickstoffatomen jeweils durch Alkylgruppen mit 1 bis 3 Kohlenstoffatomen ersetzt sein können, durch eine Amino-, tert.Butoxycarbonylamino-, Dimethylamino-, N-Methyl-benzylamino-, Methylamino-, Aminocarbonyl-, Aminocarbonylamino-, Aminomethylidenimino-, Methylaminomethylidenimino-, [Amino(nitroimino)methyl]amino-, 1H-Imidazol-2-yl-amino-, 4,5-Dihydro-1H-imidazol-2-yl-amino-, (5-Amino-4H-1,2,4-triazol-3-yl)amino-, (3-Amino-1,2,4-oxadiazol-5-yl)amino- oder (5-Amino-1,2,4-oxadiazol-3-yl)amino-Gruppe substituiert ist,
eine Methylgruppe, die durch eine Phenyl-, Cyanophenyl-, Aminomethylphenyl-, Amidinophenyl-, Methylaminomethylideniminophenyl-, Cyaniminomethylaminophenyl-, Methyliminomethylaminophenyl-, (4,5-Dihydro-1H-imidazol-2-yl)phenyl- oder Imidazolylgruppe substituiert ist,
R³ das Wasserstoffatom oder die Methylgruppe und
Y eine gegebenenfalls durch eine Methyl- oder Ethylgruppe substituierte Iminogruppe bedeuten,
deren Tautomere, deren Diastereomere, deren Enantiomere, deren Gemische und deren Salze.

Besonders bevorzugte Verbindungen der obigen allgemeinen Formel I sind diejenigen, in denen
die R-(CH₂)ₙ-Gruppe eine Alkylgruppe mit 1 bis 3 Kohlenstoffatomen, die in ω-Stellung durch 2 Phenylgruppen substituiert sein kann,
eine geradkettige Alkylengruppe mit 2 bis 5 Kohlenstoffatomen, die endständig durch eine Hydroxygruppe substituiert ist,
eine Phenyl- oder Phenylalkylgruppe mit 1 bis 3 Kohlenstoffatomen im Alkylteil, wobei die Phenylgruppe jeweils durch ein Fluor-, Chlor- oder Bromatom, durch eine Methyl-, Hydroxymethyl-, Hydroxyethyl-, Hydroxypropyl-, Hydroxy-, Methoxy-, Ethoxy-, Dimethylaminosulfonyloxy-, Acetyl-, Methoxycarbonyl-, Aminocarbonyl-, Aminocarbonylamino-, Methylaminocarbonylamino-, Aminosulfonyl-, Dimethylamino-, [1,2-Dihydro-3,5(4H)-dioxo-1,2-diphenyl-3H-1,2,4-triazol-4-yl]methyl-, [1,5-Dihydro-2,4(3H)-dioxo-5,5-diphenyl-imidazol-3-yl]methyl-oder Ethoxycarbonyloxy-Gruppe substituiert ist,
eine Methylgruppe, die durch eine 3,4-Dimethoxyphenyl-, 3-Hydroxy-4-methoxyphenyl-, 3-Methoxy-4-hydroxyphenyl-, 4-Amino-3,5-dibromphenyl-, 4-Amino-3,5-dichlorphenyl-, 4-Hydroxy-3,5-dimethylphenyl-, 4'-Hydroxy-4-biphenylyl-, Thienyl-, Pyridinyl-, Benzimidazolyl- oder 1-[2-[5,11-Dihydro-6(6H)-oxopyrido[2,3-b][1,4]benzodiazepin-5-yl]-2-oxoethyl]-4-piperidinyl]- oder 4-Amino-3-fluorphenyl-Gruppe substituiert ist,
die T-Z-Gruppe eine Diphenylacetylgruppe, in der der Phenylkern jeweils durch ein Chlor- oder Bromatom substituiert sein kann,
eine Carbonylgruppe, die durch eine durch eine Benzyloxy- oder Phenylethoxygruppe substituierte Indolylgruppe, durch eine Benzyl-, (Dichlorphenyl)methyl-, Dichlorphenyl-, Naphthyl-, Naphthylmethyl-, α-Cyclopentylbenzyl-, 9-Fluorenyl- oder (Diphenylmethyl)amino-Gruppe substituiert ist,
R¹ das Wasserstoffatom,
R² eine geradkettige Alkylenkette mit 2 bis 5 Kohlenstoffatomen, welche endständig durch eine Amino-, Amidino-, Guanidino-, Aminocarbonyl-, Aminocarbonylamino- oder (1H-Imidazol-2-yl)amino-Gruppe substituiert ist, oder
eine Methylgruppe, die durch eine (Aminomethyl)phenyl-, Amidinophenyl- oder (4,5-Dihydro-1H-imidazol-2-yl)phenyl-Gruppe substituiert ist,
R³ das Wasserstoffatom oder die Methylgruppe und
Y eine gegebenenfalls durch eine Methylgruppe substituierte Iminogruppe bedeuten,
deren Tautomere, deren Diastereomere, deren Enantiomere, deren Gemische und deren Salze.

Ganz besonders bevorzugte Verbindungen der obigen allgemeinen Formel I sind diejenigen, in denen
die R(CH₂)ₙ-Gruppe eine Phenylmethylgruppe, wobei die Phenylgruppe jeweils durch ein Fluor-, Chlor- oder Bromatom, durch eine Methyl-, Hydroxymethyl-, Hydroxyethyl-, Hydroxypropyl-, Hydroxy-, Methoxy-, Ethoxy-, Dimethylaminosulfonyloxy-, Ethoxycarbonyloxy-, Acetyl-, Methoxycarbonyl-, Aminocarbonyl-, Aminocarbonylamino-, Methylaminocarbonylamino-, Aminosulfonyl-, Dimethylamino-, [1,2-Dihydro-3,5(4H)-dioxo-1,2-diphenyl-3H-1,2,4-triazol-4-yl]methyl- oder [1,5-Dihydro-2,4(3H)-dioxo-5,5-diphenyl-imidazol-3-yl]methyl-Gruppe substituiert ist,
eine Methylgruppe, die durch eine 3,4-Dimethoxyphenyl-, 3-Hydroxy-4-methoxyphenyl-, 3-Methoxy-4-hydroxyphenyl-, 4-Amino-3,5-dibromphenyl-, 4-Amino-3,5-dichlorphenyl-, 4-Hydroxy-3,5-dimethylphenyl-, 4'-Hydroxy-4-biphenylyl-, Thienyl-, Pyridinyl-, Benzimidazolyl- oder 1-[2-[5,11-Dihydro-6(6H)-oxopyrido[2,3-b][1,4]benzodiazepin-5-yl]-2-oxoethyl]-4-piperidinyl]- oder 4-Amino-3-fluorphenyl-Gruppe substituiert ist,
die T-Z-Gruppe eine Diphenylacetylgruppe, in der der Phenylkern jeweils durch ein Chlor- oder Bromatom substituiert sein kann,
eine Carbonylgruppe, die durch eine durch eine Benzyloxy- oder Phenylethoxygruppe substituierte Indolylgruppe, durch eine Benzyl-, (Dichlorphenyl)methyl-, Dichlorphenyl-, Naphthyl-, Naphthylmethyl-, α-Cyclopentylbenzyl-, 9-Fluorenyl- oder (Diphenylmethyl)amino-Gruppe substituiert ist,
R¹ das Wasserstoffatom,
R² eine geradkettige Alkylenkette mit 2 bis 5 Kohlenstoffatomen, welche endständig durch eine Amino-, Amidino-, Guanidino-, Aminocarbonyl-, Aminocarbonylamino- oder (1H-Imidazol-2-yl)amino-Gruppe substituiert ist, oder
eine Methylgruppe, die durch eine (Aminomethyl)phenyl-, Amidinophenyl- oder (4,5-Dihydro-1H-imidazol-2-yl)phenyl-Gruppe substituiert ist,
R³ das Wasserstoffatom oder die Methylgruppe und
Y eine gegebenenfalls durch eine Methylgruppe substituierte Iminogruppe bedeuten,
deren Tautomere, deren Diastereomere, deren Enantiomere, deren Gemische und deren Salze.

Von speziellem Interesse sind solche Verbindungen der obigen allgemeinen Formel I, in denen die R(CH₂)ₙ-Gruppe eine Phenylmethylgruppe, wobei die Phenylgruppe jeweils durch ein Fluor-, Chlor- oder Bromatom, durch eine Methyl-, Hydroxymethyl-, Hydroxyethyl-, Hydroxypropyl-, Hydroxy-, Methoxy-, Ethoxy-, Dimethylaminosulfonyloxy-, Ethoxycarbonyloxy-, Acetyl-, Methoxycarbonyl-, Aminocarbonyl-, Aminocarbonylamino-, Methylaminocarbonylamino-, Aminosulfonyl-, Dimethylamino-, [1,2-Dihydro-3,5(4H)-dioxo-1,2-diphenyl-3H-1,2,4-triazol-4-yl] methyl- oder [1,5-Dihydro-2,4(3H)-dioxo-5,5-diphenyl-imidazol-3-yl]methyl-Gruppe substituiert ist,
eine Methylgruppe, die durch eine 3,4-Dimethoxyphenyl-, 3-Hydroxy-4-methoxyphenyl-, 3-Methoxy-4-hydroxyphenyl-, 4-Amino-3'5-dibromphenyl-, 4-Amino-3,5-dichlorphenyl-, 4-Hydroxy-3,5-dimethylphenyl-, 4'-Hydroxy-4-biphenylyl-, Thienyl-, Pyridinyl-, Benzimidazolyl- oder 1-[2-[5,11-Dihydro-6(6H)-oxopyrido[2,3-b][1,4]benzodiazepin-5-yl]-2-oxoethyl]-4-piperidinyl]- oder 4-Amino-3-fluorphenyl-Gruppe substituiert ist,
die T-Z-Gruppe eine Diphenylacetylgruppe, in der der Phenylkern jeweils durch ein Chlor- oder Bromatom substituiert sein kann,
eine Carbonylgruppe, die durch eine durch eine Benzyloxy- oder Phenylethoxygruppe substituierte Indolylgruppe, durch eine Benzyl-, (Dichlorphenyl)methyl-, Dichlorphenyl-, Naphthyl-, Naphthylmethyl-, α-Cyclopentylbenzyl-, 9-Fluorenyl- oder (Diphenylmethyl)amino-Gruppe substituiert ist,
R¹ das Wasserstoffatom,
R² eine geradkettige Alkylenkette mit 2 bis 5 Kohlenstoffatomen, welche endständig durch eine Amino-, Amidino-, Guanidino-, Aminocarbonyl-, Aminocarbonylamino- oder (1H-Imidazol-2-yl)amino-Gruppe substituiert ist, oder
eine Methylgruppe, die durch eine (Aminomethyl)phenyl-, Amidinophenyl- oder (4,5-Dihydro-1H-imidazol-2-yl)phenyl-Gruppe substituiert ist,
R³ das Wasserstoffatom und
Y eine gegebenenfalls durch eine Methylgruppe substituierte Iminogruppe bedeuten,
deren Tautomere, deren Diastereomere, deren Enantiomere, deren Gemische und deren Salze.

Als besonders bevorzugte Verbindungen seien beispielsweise folgende genannt:
(R)-N²-(Diphenylacetyl)-N-(phenylmethyl)-argininamid,
(R)-N²-(Diphenylacetyl)-N-[(4-methylphenyl)methyl]-argininamid,
(R)-N-[2-(4-Hydroxyphenyl)ethyl]-N²-[[5-(2-phenylethoxy)-1H-indol-2-yl)carbonyl]-argininamid,
N-[(4-Aminocarbonylaminophenyl)methyl]-N²-(diphenylacetyl)argininamid,
(R)-N-[(4-Hydroxyphenyl)methyl]-N²-[[5-(2-phenylethoxy)-1H-indol-2-yl]carbonyl]-argininamid,
(R)-N²-(Diphenylacetyl)-N-[(4-fluorphenyl)methyl]-argininamid,
(R)-N-[(4-Bromphenyl)methyl]-N²-(diphenylacetyl)-argininamid,
(R)-N²-(Diphenylacetyl)-N-(2-phenylethyl)-argininamid,
Optisch aktives Diastereomerengemisch von N²-(α-Cyclopentyl-phenylacetyl)-N-[(4-hydroxyphenyl)methyl]-D-argininamid,
N-[[4-(Dimethylamino)phenyl]methyl]-N²-(diphenylacetyl)argininamid,
(R)-N²-(Diphenylacetyl)-N-[[4-(hydroxymethyl)phenyl]methyl]argininamid,
(R)-N²-(Diphenylacetyl)-N-[[4-(1-oxoethyl)phenyl]methyl]-argininamid,
(R)-N-[(4-Chlorphenyl)methyl]-N²-(diphenylacetyl)-argininamid,
(R)-N²⁻(Diphenylacetyl)-N-[[4-[(methylaminocarbonyl)amino]-phenyl]methyl]-argininamid,
(R)-N-[(4-Amino-3,5-dichlorphenyl)methyl]-N²-(diphenylacetyl)-argininamid,
(R)-N-[(4-Amino-3,5-dichlorphenyl)methyl]-N²-(3,3-diphenyl-1-oxopropyl)-argininamid,
(R)-N-[(4-Amino-3,5-dichlorphenyl)methyl]-N²-(3,4-dichlorbenzoyl)-argininamid,
N²-(Diphenylacetyl)-N-[3-[1-[2-[5,11-dihydro-6(6H)-oxopyrido[2,3-b][1,4]benzodiazepin-5-yl]-2-oxoethyl]-4-piperidinyl]propyl]-argininamid,
N²⁻(Diphenylacetyl)-N-[(4-hydroxy-3-methoxyphenyl)methyl]argininamid,
N²-(Diphenylacetyl)-N-[(3-hydroxy-4-methoxyphenyl)methyl]-argininamid,
(R,S)-N-[(4-Amino-3,5-dibromphenyl)methyl]-N⁶-(aminoiminomethyl)-N²-(diphenylacetyl)-lysinamid,
N-[(3,5-Dimethyl-4-hydroxyphenyl)methyl]-N²-(diphenylacetyl)-argininamid,
N-[(1H-Benzimidazol-5-yl)methyl]-N²-(diphenylacetyl)-argininamid,
N²-(Diphenylacetyl)-N-[(4-hydroxy-3-methylphenyl)methyl]-argininamid,
N-[[4-(Aminocarbonyl)phenyl]methyl]-N²-(diphenylacetyl)argininamid,
(R,S)-N⁶-(Aminoiminomethyl)-N²-(diphenylacetyl)-N-[(4-hydroxyphenyl)methyl]-lysinamid,
(R)-N-[(4-Hydroxyphenyl)methyl]-N²-(phenylacetyl)-argininamid,
N²-(Diphenylacetyl)-N-[(1H-indol-5-yl)methyl]-argininamid,
(R)-N-[[4-(Aminosulfonyl)phenyl]methyl]-N²-(diphenylacetyl)-argininamid,
(R)-N²-(Diphenylacetyl)-N-[[4-(methoxycarbonyl)phenyl]methyl]-argininamid,
(R)-N²-(Diphenylacetyl)-N-[(4-pyridinyl)methyl]-argininamid,
(R)-N-[(4-Amino-3,5-dibromphenyl)methyl]-N²-(diphenylacetyl)-argininamid,
(R)-N²⁻(Diphenylacetyl)-N-[(2-thienyl)methyl]-argininamid,
(R)-N-[(4-Amino-3,5-dichlorphenyl)methyl]-N²-(2-naphthoyl)argininamid,
(R,S)-N⁵-(4,5-Dihydro-1H-imidazol-2-yl)-N²-(diphenylacetyl)-N-[(4-hydroxyphenyl)methyl]-ornithinamid,
(R,S)-N²-(Diphenylacetyl)-N-[(4-hydroxyphenyl)methyl]-N⁵-(1H-imidazol-2-yl)-ornithinamid,
(R)-N-[[3-[(1,2-Dihydro-3,5(4H)-dioxo-1,2-diphenyl-3H-1,2,4-triazol-4-yl)methyl]phenyl]methyl]-N²-(diphenylacetyl)-argininamid,
N²-(Diphenylacetyl)-N-[(3-hydroxyphenyl)methyl]-argininamid,
(R)-N-[[3-[(4,5-Dihydro-2,4(3H)-dioxo-5,5-diphenyl-1H-imidazol-3-yl)methyl]phenyl]methyl]-N²-(diphenylacetyl)-argininamid,
(R)-N²-(Diphenylacetyl)-N-[(4-hydroxyphenyl)methyl]-argininamid,
N²-(Diphenylacetyl)-N-[2-(4-hydroxyphenyl)ethyl]-argininamid,
N²-(Diphenylacetyl)-N-[(4'-hydroxy-[1,1'-biphenyl]-4-yl)methyl]-argininamid,
N-[[4-[(1,2-Dihydro-3,5(4H)-dioxo-1,2-diphenyl-3H-1,2,4-triazol-4-yl)methyl]phenyl]methyl]-N²-(diphenylacetyl)-argininamid,
N²-(Diphenylacetyl)-N-[(4-methoxyphenyl)methyl]-argininamid,
N²-(Diphenylacetyl)-N-[2-(4-methoxyphenyl)ethyl]-argininamid,
N²-(Diphenylacetyl)-N-[2-(3-methoxyphenyl)ethyl]-argininamid,
N²-(Diphenylacetyl)-N-[(3-methoxyphenyl)methyl]-argininamid,
(R,S)-3-[4-(Aminoiminomethyl)phenyl]-N²-(diphenylacetyl)-N-[(4-hydroxyphenyl)methyl]-alaninamid,
(R,S)-3-[3-(Aminoiminomethyl)phenyl]-N²-(diphenylacetyl)-N-[(4-methoxyphenyl)methyl]-alaninamid,
(R,S)-3-[3-(Aminoiminomethyl)phenyl]-N²-(diphenylacetyl)-N-[(4-hydroxyphenyl)methyl]-alaninamid,
(R)-N²-[Bis-(4-bromphenyl)acetyl]-N-[(4-hydroxyphenyl)methyl]-argininamid,
(R)-N²-(Diphenylacetyl)-N-[(4-ethoxyphenyl)methyl]-argininamid,
(R)-N²-(Diphenylacetyl)-N-[(4-hydroxyphenyl)methyl]-N-methyl-argininamid,
(R,S)-N-[(4-Amino-3,5-dibromphenyl)methyl]-3-[3-(aminoiminomethyl)phenyl]-N²-(diphenylacetyl)-alaninamind,
(R)-N-([4-[(Dimethylamino)sulfonyloxy]phenyl]methyl]-N²-(diphenylacetyl)-argininamid,
(R)-N-[(4-Hydroxyphenyl)methyl]-N²-(1-naphthoyl)-argininamid,
(R)-N-[(4-Hydroxyphenyl)methyl]-N²-(2-naphthoyl)-argininamid,
(R)-N²-(2,2-Diphenyl-2-hydroxyacetyl)-N-((4-hydroxyphenyl)methyl]-argininamid,
(R,S)-N²-(Diphenylacetyl)-N⁵-(1H-imidazol-2-yl)-N-[(4-methoxyphenyl)methyl]-ornithinamid,
(R,S)-3-[3-(Aminoiminomethyl)phenyl]-N²-[[(diphenylmethyl)amino]carbonyl]-N-[(4-hydroxyphenyl)methyl]-alaninamid,
(R,S)-N²-(Diphenylacetyl)-N⁵-(1H-imidazol-2-yl)-N-(phenylmethyl)-ornithinamid,
(R,S)-N-[(4-Amino-3,5-dichlorphenyl)methyl]-N²⁻(diphenylacetyl)-N⁵-(1H-imidazol-2-yl)-ornithinamid,
(R,S)-N-[(4-Hydroxyphenyl)methyl]-N⁵-(1H-imidazol-2-yl)-N²-(2-naphthoyl)-ornithinamid,
(R,S)-N²-[[(Diphenylmethyl)amino]carbonyl]-N-[(4-hydroxyphenyl)methyl]-N⁵-(1H-imidazol-2-yl)-ornithinamid,
(R,S)-N-[(4-Hydroxyphenyl)mehtyl]-N⁵-(1H-imidazol-2-yl)-N²-[(2-naphthyl)acetyl]-ornithinamid,
(R,S)-N-[(4-Hydroxyphenyl)methyl]-N⁵-(1H-imidazol-2-yl)-N²-[[(2-naphthyl)amino]carbonyl]-ornithinamid,
(R,S)-N-[(4-Hydroxyphenyl)methyl]-N⁵-(1H-imidazol-2-yl)-N²-[(1,2,3,4-tetrahydrochinolin-3-yl)carbonyl]-ornithinamid,
(R,S)-N-[(4-Hydroxyphenyl)methyl]-N⁵-(1H-imidazol-2-yl)-N²-[(1,2,3,4-tetrahydrochinolin-2-yl)carbonyl]-ornithinamid,
(R)-N-[(4-Aminosulfonylaminophenyl)methyl]-N²-(diphenylacetyl)-argininamid,
(R)-N-[(4-Aminophenyl)methyl]-N²-(diphenylacetyl)-argininamid,
(R)-N-[(6-Chinolinyl)methyl]-N²-(diphenylacetyl)-argininamid,
(R)-N²-[(3,4-Dichlorphenyl)acetyl]-N-[(4-hydroxyphenyl)methyl]-argininamid,
(R)-N²-(Diphenylacetyl)-N-[[4-(2-hydroxyethyl)phenyl]methyl]-argininamid,
(R,S)-N⁵-(3-Amino-1,2,4-oxadiazol-5-yl)-N²-(diphenylacetyl)-N-[(4-hydroxyphenyl)methyl]-ornithinamid,
(R)-N²-[(9-Fluorenyl)carbonyl]-N-[(4-hydroxyphenyl)methyl]-argininamid,
(R,S)-6-(Aminoiminomethyl)-N²-(diphenylacetyl)-N-[(4-hydroxyphenyl)methyl]-norleucinamid,
(R,S)-3-[4-(4,5-Dihydro-1H-imidazol-2-yl)phenyl]-N²-(diphenylacetyl)-N-[(4-hydroxyphenyl)methyl]-alaninamid,
(R,S)-3-[3-(Aminoiminomethyl)phenyl]-N²-(diphenylacetyl)-N-[(4-ethoxycarbonyloxyphenyl)methyl]-alaninamid,
(R,S)-N-[2-(1,2-Dihydro-1,2-diphenyl-3,5(4H)-dioxo-1,2,4-triazol-4-yl)ethyl]-N²-(diphenylacetyl)-argininamid,
(R,S)-N²-(Diphenylacetyl)-N-[(4-hydroxyphenyl)methyl]-2-methyl-argininamid,
(R)-N²-(Diphenylacetyl)-N-[[4-(3-hydroxypropyl)phenyl]methyl]-argininamid,
(R)-N-[[4-[(4,5-Dihydro-5,5-dimethyl-2,4(3H)-dioxo-1H-imidazol-3-yl)methyl]phenyl]methyl]-N²-(diphenylacetyl)-argininamid
und deren Salze.

Die Verbindungen der allgemeinen Formel I werden nach prinzipiell bekannten Methoden hergestellt, wobei besonders aus der Peptidchemie (siehe z. B. Houben-Weyl, Methoden der Organischen Chemie, Bd. 15/2) abgeleitete Verfahren angewandt werden. Als Aminoschutzgruppen können die in Houben-Weyl, Methoden der Organischen Chemie, Bd. 15/1, beschriebenen verwendet werden, wobei Urethanschutzgruppen, wie z. B. die Fluorenylmethoxycarbonyl-, Phenylmethoxycarbonyl- oder tert.-Butyloxycarbonylgruppe bevorzugt werden. Eventuell im Rest R² der Verbindungen der allgemeinen Formel I vorhandene funktionelle Gruppen werden zur Verhinderung von Nebenreaktionen durch geeignete Schutzgruppen (siehe z. B.: G.B. Fields et al., Int. J. Peptide Protein Res. 35, 161 (1990); T.W. Greene, Protective Groups in Organic Synthesis) zusätzlich geschützt. Als derartige seitenketten-geschützte Aminosäuren seien besonders Arg(NO₂), Arg(Mtr), Arg(di-Z), Arg(Pmc), Lys(Boc), Lys(Z), Orn(Boc), Orn(Z), Lys(Cl-Z) erwähnt, die, eventuell in Form von Derivaten, in der Regel käuflich sind. Die Seitenkette von [(Aminomethyl)phenyl]alanin läßt sich prinzipiell analog derjenigen von Ornithin oder Lysin schützen (siehe auch: M. Bodanszky, "Principles of Peptide Synthesis", Springer-Verlag 1984, S. 122-126), wobei besonders darauf zu achten ist, daß für den Schutz der α-Amino- und der Seitenketten-Aminogruppe sogenannte orthogonale Kombinationen von Schutzgruppen verwendet werden, z. B.:

| Schutz des N (Seitenkette) | N^{α}-Schutz |
|---|---|
| p-Toluolsulfonyl | Phenylmethoxycarbonyl |
| | tert.Butyloxycarbonyl |
| Phenylmethoxycarbonyl | (4-Methoxyphenyl)methoxycarbonyl |
| | tert. Butoxycarbonyl |
| | Adamantyloxycarbonyl |
| | Biphenylylisopropyloxycarbonyl |
| | Isonicotinoyloxycarbonyl |
| | o-Nitrophenylsulfenyl |
| | Formyl |
| tert. Butoxycarbonyl | Phenylmethoxycarbonyl |
| | p-Toluolsulfonyl |
| | o-Nitrophenylsulfenyl |
| | Biphenylylisopropyloxycarbonyl |
| | 9-Fluorenylmethoxycarbonyl |
| Acetyl, Trifluoracetyl, Formyl, (2-Chlorphenyl)methoxycarbonyl, (4-Chlorphenyl)methoxycarbonyl, 4-(Nitrophenyl)methoxycarbonyl, Phthaloyl | tert.Butyloxycarbonyl |

Statt Aminogruppen im Rest R² zu schützen, können auch Präcursor-Funktionen tragende, in der Seitenkette insbesondere durch Nitro oder Cyan substituierte Aminosäuren bzw. deren Derivate eingesetzt werden, beispielsweise 6-Cyan-norleucin, 2-(5-Nitro-pentyl)-glycin oder 3-(3-Cyanphenyl)-alanin.

Die basischen Funktionen in der Seitenkette von nicht käuflichen α-Aminosäuren, die beispielsweise durch (Aminoiminomethyl)-Gruppen charakterisiert sind, können in gleicher Weise geschützt werden, wie das für den Seitenkettenschutz von Arginin und seinen Derivaten bekannt ist (siehe auch M. Bodanszky, "Peptide Chemistry", Springer-Verlag, 1988, S. 94-97); als Schutzgruppen für die (Aminoiminomethyl)-Gruppe besonders geeignet sind die p-Toluolsulfonyl-, Mesitylensulfonyl(Mts-)-, Methoxytrimethylphenylsulfonyl(Mtr-)-, 2,2,5,7,8-Pentamethylchroman-6-sulfonyl(Pmc-)-, Pentachlorphenoxycarbonyl- und Nitro-Schutzgruppe.

Zur eigentlichen Kupplung werden die aus der Peptidchemie bekannten Methoden (siehe z. B. Houben-Weyl, Methoden der Organischen Chemie, Bd. 15/2) angewandt. Bevorzugt verwendet werden Carbodiimide, wie z. B. Dicyclohexylcarbodiimid (DCC), Diisopropylcarbodiimid (DIC) oder Ethyl-(3-dimethylaminopropyl)-carbodiimid, O-(1H-Benzotriazol-1-yl)-N,N-N',N'-tetramethyluroniumhexafluorophosphat (HBTU) oder -tetrafluoroborat (TBTU) oder 1H-Benzotriazol-1-yl-oxy-tris-(dimethylamino)-phosphoniumhexafluorophosphat (BOP). Durch Zugabe von 1-Hydroxybenzotriazol (HOBt) oder von 3-Hydroxy-4-oxo-3,4-dihydro-1,2,3-benzotriazin (HOObt) kann die Racemisierung gewünschtenfalls zusätzlich unterdrückt bzw. die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran, Acetonitril, Dimethylformamid (DMF), Dimethylacetamid (DMA), N-Methyl-pyrrolidon (NMP) oder Gemischen aus diesen und bei Temperaturen zwischen -30 und +30°C, bevorzugt -20 und +20°C, durchgeführt. Sofern erforderlich, wird als zusätzliche Hilfsbase N-Ethyl-diisopropylamin (DIEA) (Hünig-Base) bevorzugt.

Als weiteres Kupplungsverfahren zur Synthese von Verbindungen der allgemeinen Formel I wurde das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem. Soc. 73, 3547 (1951)), bei der unter Verwendung von Chlorkohlensäureisobutylester in Gegenwart von Besen, wie 4-Methylmorpholin oder 4-Ethylmorpholin, das gemischte Anhydrid aus der zu kuppelnden, gegebenenfalls N²-geschützten α-Aminosäure und dem Kohlensäuremonoisobutylester erhalten wird. Die Herstellung dieses gemischten Anhydrids und die Kupplung mit Aminen erfolgt im Eintopfverfahren, unter Verwendung der vorstehend genannten Lösemittel und bei Temperaturen zwiwchen -20 und +20°C, bevorzugt 0 und +20°C.

Eventuelle in der α-Aminosäureseitenkette vorhandene Schutzgruppen werden nach Aufbau des N- und C-terminal substituierten Aminosäurederivats abschließend mit geeigneten, im Prinzip gleichfalls literaturbekannten Reagenzien abgespalten, und zwar Arylsulfonyl- und Hetarylsulfonyl-Schutzgruppen bevorzugt acidolytisch, d. h. durch Einwirkung von starken Säuren, bevorzugt Trifluoressigsäure, Nitro- und Arylmethoxycarbonylschutzgruppen hydrogenolytisch, beispielsweise mit Wasserstoff in Gegenwart von Palladiummohr und unter Verwendung von Eisessig als Lösemittel. Enthält das Substrat gegen Hydrogenolyse empfindliche Funktionen, z. B. Halogenatome, wie Chlor, Brom oder Iod, eine Phenylmethanol- oder Hetarylmethanol-Funktion oder eine andere Benzylheteroatom-Bindung, insbesondere eine Benzyl-Sauerstoff-Bindung, so gelingt die Abspaltung der Nitrogruppe auch nichthydrogenolytisch, z. B. mit Zink/2 N Trifluoressigsäure (siehe auch; A. Turan, A. Patthy und S. Bajusz, Acta Chim. Acad. Sci. Hung, Tom. 85 (3), 327-332 [1975]; C.A. 83, 206526y [1975]), mit Zinn(II)-chlorid in 60%iger wässeriger Ameisensäure (siehe auch: SUNSTAR KK, JA-A-3271-299), mit Zink in Gegenwart von Essigsäure (s. auch: A. Malabarba, P. Ferrari, G. Cietto, R. Pallanza und M. Berti, J. Antibiot. 42 (12) 1800-1816 (1989)) oder überschüssigem wässerigem 20%igem Titan(III)-chlorid in wässerigem Methanol und in Gegenwart von wässerigem Ammoniumacetat-Puffer bei 24°C (siehe auch: R.M. Freidinger, R. Hirschmann und D.F. Veber, J. Org. Chem. 43 (25), 4800-4803 [1978]).

In der Seitenkette der α-Aminosäure gegebenenfalls vorhandene Präcursor-Funktionen können gleichfalls abschließend durch Hydrogenolyse in die gewünschten Aminofunktionen übergeführt werden; Nitroalkylgruppen ergeben dabei unter dem Chemiker geläufigen Bedingungen Aminoalkylgruppen, die Cyangruppe geht in die Aminomethyl-Gruppe über.

Nitrilfunktionen können stattdessen auch mit gegenüber sonstigen im Molekül enthaltenen kritischen Funktionen, insbesondere Amidgruppen, selektiven komplexen Hydriden reduziert werden (siehe auch: J. Seyden-Penne, "Reductions by the Alumino- and Borohydrides in Organic Synthesis", VCH Publishers Inc., 1991, S. 132ff.), z. B. mit Natriumborhydrid in Methanol und in Gegenwart von Cobalt(II)-chlorid, mit Natriumborhydrid in Tetrahydrofuran in Gegenwart von Trifluoressigsäure oder mit Tetrakis-(n-butyl)-ammoniumborhydrid in Dichlormethan; auch die Reduktion aliphatischer Nitrofunktionen zur primären Aminofunktion ist mit Natriumborhydrid in Gegenwart von Zinn(II)chlorid oder Kupfer(II)-acetylacetonat möglich, ohne daß die in Verbindungen vom Typ I vorhandenen Carboxamidgruppen angegriffen werden (siehe auch: J. Seyden-Penne, ibid. S. 137ff.).

Zur Herstellung der erfindungsgemäßen Verbindungen der allgemeinen Formel I sind die folgenden Verfahren besonders geeignet:
a) Zur Herstellung von Verbindungen der allgemeinen Formel I, in der T-Z die eingangs erwähnten Bedeutungen mit Ausnahme der eines Wasserstoffatoms besitzt:
   Kupplung von Verbindungen der allgemeinen Formel II,

   T^{A} - Z - X , (II)

   in der
   T^{A}-Z die eingangs für T-Z erwähnten Bedeutungen mit Ausnahme der eines Wasserstoffatoms besitzt und
   X die Hydroxygruppe, ein Halogenatom, wie das Chlor-, Brom- oder Iodatom, eine Alkylsulfonyloxygruppe mit 1 bis 10 Kohlenstoffatomen, eine gegebenenfalls durch Chlor- oder Bromatome, durch Methyl- oder Nitrogruppen mono-, di- oder trisubstituierte Phenylsulfonyloxy- oder Naphthylsulfonyloxygruppe bedeutet, wobei die Substituenten gleich oder verschieden sein können,
   mit α-Aminosäurederivaten der allgemeinen Formel III,

   H - NR¹ - (R^{2a}CR³) - CO - Y - (CH₂)ₙ-R , (III)

   in der
   n, R, R¹, R³ und Y wie eingangs definiert sind und R^{2a} die für R² eingangs erwähnten Bedeutungen besitzt oder einen durch die vorstehend erwähnten Schutzreste substituierten Rest R² oder einen Präcursorrest für den Rest R², z.B. einen Nitroalkyl- oder Cyanalkyl-Rest, darstellt,
   und, falls nötig, anschließende Abspaltung von Schutzgruppen oder Abwandlung von Präcursor-Funktionen nach den vorstehend beschriebenen Verfahren.
   Bedeutet in der allgemeinen Formel II X die Hydroxygruppe, dann werden die oben ausführlich diskutierten, aus der Peptidchemie bekannten Kupplungsmethoden verwendet, insbesondere unter Benutzung der erwähnten Kupplungsreagenzien DCC, DIC, HBTU, TBTU oder BOP, oder es wird nach der gemischten Anhydridmethode verfahren.
   Bedeutet in der allgemeinen Formel II X ein Halogenatom, eine Alkyl- oder Arylsulfonyloxygruppe, so wird die Umsetzung unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösemitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, N-Ethyl-diisopropylamin, N-Ethyl-dicyclohexylamin, 1,4-Diazabicyclo[2,2,2]octan oder 1,8-Diazabicyclo[5,4,0]undec-7-en, als Lösemittel beispielsweise Dichlormethan, Tetrahydrofuran, 1,4-Dioxan, Acetonitril, Dimethylformamid, Dimethylacetamid, N-Methyl-pyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.
b) Zur Herstellung von Verbindungen der allgemeinen Formel I, in der T-Z die eingangs erwähnten Bedeutungen mit Ausnahme der eines Wasserstoffatoms besitzt:
   Kupplung von Verbindungen der allgemeinen Formel IV,

   T^{A} - Z - NR¹- (R^{2a}CR³) - COOH , (IV)

   in der
   R¹ und R³ wie eingangs erwähnt definiert sind, T^{A}-Z die eingangs für T-Z erwähnten Bedeutungen mit Ausnahme der eines Wasserstoffatoms besitzt und R^{2a} die für R² eingangs erwähnten Bedeutungen besitzt oder einen durch Schutzreste substituierten Rest R² oder einen Präcursorrest für den Rest R² darstellt, mit Verbindungen der allgemeinen Formel V,

      H - Y - (CH₂)ₙ - R , (V)

      in der
   R-(CH₂)ₙ und Y die eingangs erwähnten Bedeutungen besitzen, und, falls nötig, anschließende Abspaltung von Schutzgruppen oder Abwandlung von Präcursor-Funktionen nach den vorstehend beschriebenen Verfahren.

   Die Kupplung wird unter Verwendung der aus der Peptidchemie bekannten und vorstehend beschriebenen Verfahren durchgeführt, insbesondere unter Benutzung von DCC, DIC, HBTU, TBTU oder BOP als Reagenzien oder nach der gemischten Anhydridmethode.
   Ist die verwendete Ausgangsverbindung IV enantiomerenrein, so muß, sofern Z eine nicht von einem Sauerstoffatom flankierte Carbonylgruppe darstellt, beim Kupplungsschritt mit einer partiellen, bei Verwendung von Triethylamin als Hilfsbase und von Dimethylformamid, Dimethylacetamid oder N-Methyl-pyrrolidon als Lösemittel mit einer quantitativen Racemisierung gerechnet werden.
c) Zur Herstellung von Verbindungen der allgemeinen Formel I, in der T-Z mit Ausnahme des Wasserstoffatoms die für T-Z eingangs erwähnten Bedeutungen besitzt und R² eine geradkettige Alkylgruppe mit 1 bis 5 Kohlenstoffatomen, die in ω-Stellung durch eine 4,5-Dihydro-1H-imidazol-2-yl-aminogruppe oder eine Guanidinogruppe, in der die Wasserstoffatome an den Stickstoffatomen jeweils durch Alkylgruppen mit 1 bis 3 Kohlenstoffatomen ersetzt sein können, substituiert ist, bedeutet:
   Umsetzung von Verbindungen der allgemeinen Formel VI,

   T^{A} - Z - NR¹ - (R^{2b}CR³) - CO - Y - (CH₂)ₙ - R , (VI)

   in der
   R-(CH₂)ₙ, R¹, R³ und Y wie eingangs erwähnt definiert sind, T^{A}-Z mit Ausnahme des Wasserstoffatoms die für T-Z eingangs erwähnten Bedeutungen besitzt und R^{2b} eine geradkettige Alkylgruppe mit 1 bis 5 Kohlenstoffatomen bedeutet, wobei die Alkylgruppe in ω-Stellung durch eine R⁶NH-Gruppe substituiert ist und R⁶ ein Wasserstoffatom oder eine Alkylgruppe mit 1 bis 3 Kohlenstoffatomen darstellt,
   mit Kohlensäurederivaten der allgemeinen Formel VII,

   X¹ - (C=NR⁷) - (R⁸NR⁹) , (VII)

   in der
   R⁷, R⁸ und R⁹, die gleich oder verschieden sein können, Wasserstoffatome, Alkylgruppen mit 1 bis 3 Kohlenstoffatomen oder R⁷ und R⁸ zusammen auch eine n-Alkylengruppe mit 2 Kohlenstoffatomen und X¹ eine Austrittsgruppe, wie eine Alkoxy-, Alkylthio-, Alkylsulfinyl- oder Alkylsulfonylgruppe mit jeweils 1 bis 10 Kohlenstoffatomen im Alkylteil, z.B. die Methoxy-, Ethoxy, Methylthio-, Ethylthio-, Methylsulfinyl-, Ethylsulfinyl-, Propylsulfinyl-, Isopropylsulfinyl-, Methylsulfonyl- oder Ethylsulfonylgruppe, das Chloratom, die SO₂H-, SO₃H- oder OPOCl₂-Gruppe oder den Rest der allgemeinen Formel XXIV, 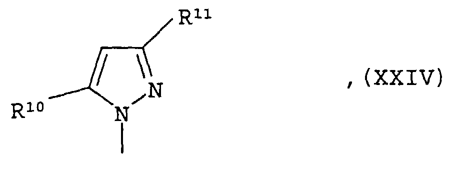 in der
      R¹⁰ und R¹¹, die gleich oder verschieden sein können, Wasserstoffatome oder Alkylreste mit 1 bis 3 Kohlenstoffatome darstellen,
   bedeuten.
   Die Umsetzungen werden nach literaturbekannten Verfahren (siehe G.B.L. Smith, J. Amer. Chem. Soc. 51, 476 [1929]; B. Rathke, Chem. Ber. 17, 297 [1884]; R. Phillips und H.T. Clarke, J. Amer. chem. Soc. 45, 1755 [1923]; S.J. Angyal und W.K. Warburton, J. Amer. Chem. Soc. 73, 2492 [1951]; H. Lecher und F. Graf, Chem. Ber. 56, 1326 [1923]; J. Wityak, S.J. Gould, S.J. Hein und D.A. Keszler, J. Org. Chem. 52, 2179 [1987]; T. Teraji, Y. Nakai, G.J. Durant, WO-A-81/00109, Chem. Abstr. 94, 192336z [1981]; C.A. Maryanoff, R.C. Stanzione, J.N. Plampin und J.E. Mills, J. Org. Chem. 51, 1882-1884 [1986]; A.E. Miller und J.J. Bischoff, Synthesis 1986, 777; R.A.B. Bannard, A.A. Casselman, W.F. Cockburn und G.M. Brown, Can. J. Chem. 36, 1541 [1958]; Aktieselskabet Grea, Kopenhagen, DE 28 26 452-C2; K. Kim. Y-T. Lin und H.S. Mosher, Tetrah. Letters, 29, 3183-3186 [1988]; H.B. Arzeno et al., Synth. Commun. 20, 3433-3437 [1990]; H. Bredereck und K. Bredereck, Chem. Ber. 94, 2278 [1961]; H. Eilingsfeld, G. Neubauer, M. Seefelder und H. Weidinger, Chem. Ber. 97, 1232 [1964]; P. Pruszynski, Can. J. Chem. 65, 626 [1987]; D.F. Gavin, W.J. Schnabel, E. Kober und M.A. Robinson, J. Org. Chem. 32, 2511 [1967]; N.K. Hart, S.R. Johns, J.A. Lamberton und R.I. Willing, Aust. J. Chem. 23, 1679 [1970]; CIBA Ltd., Belgisches Patent 655 403; Chem. Abstr. 64, 17481 [1966]; R.A.B. Bannard, A.A. Casselman, W.F. Cockburn und G.M. Brown, Can. J. Chem. 36, 1541 [1958]; J.P. Greenstein, J. Org. Chem. 2, 480 [1937]; F.L. Scott und J. Reilly, J. Amer. Chem. Soc. 74, 4562 [1952]; W.R. Roush und A.E. Walts, J. Amer. Chem. Soc. 106, 721 [1984], M.S. Bernatowicz, Y. Wu und G.R. Matsueda, J. Org. Chem. 57, 2497-2502 [1992]; H. Tsunematsu, T. Imamura und S. Makisumi, J. Biochem. 94, 123-128 [1983]) bei Temperaturen zwischen 0°C und +100°C, bevorzugt +40°C und +80°C, und unter Verwendung inerter Lösemittel, beispielsweise von Dichlormethan, Tetrahydrofuran, 1,4-Dioxan, Acetonitril, Dimethylformamid, Dimethylacetamid, N-Methyl-pyrrolidon oder Gemischen davon und in der Regel in Gegenwart von Hilfsbasen, insbesondere von Alkalicarbonaten wie Natrium- oder Kaliumcarbonat, oder tertiären Aminen, bevorzugt N-Ethyl-diisopropylamin oder Triethylamin, durchgeführt.
d) Zur Herstellung von Verbindungen der allgemeinen Formel I, in der T-Z mit Ausnahme des Wasserstoffatoms die für T-Z eingangs erwähnten Bedeutungen besitzt und R² eine geradkettige Alkylgruppe mit 1 bis 5 Kohlenstoffatomen darstellt, wobei die Alkylgruppe in ω-Stellung durch eine R^{8a}R⁹N-C(=NH)-NR⁶-Gruppe substituiert ist und R⁶, R^{8a} und R⁹, die gleich oder verschieden sein können, Wasserstoffatome oder Alkylgruppen mit 1 bis 3 Kohlenstoffatomen darstellen:
   Umsetzung von Verbindungen der allgemeinen Formel VI,

   T^{A} - Z - NR¹ - (R^{2b}CR³) - CO - Y - (CH₂)ₙ - R , (VI)

   in der
   R-(CH₂)ₙ, R¹, R³ und Y wie eingangs definiert sind, T^{A}-Z mit Ausnahme des Wasserstoffatoms die für T-Z eingangs erwähnten Bedeutungen besitzt und R^{2b} eine geradkettige Alkylgruppe mit 1 bis 5 Kohlenstoffatomen bedeutet, wobei die Alkylgruppe in ω-Stellung durch eine R⁶NH-Gruppe substituiert ist und R⁶ ein Wasserstoffatom oder eine Alkylgruppe mit 1 bis 3 Kohlenstoffatomen darstellt,
   mit Cyanamiden der allgemeinen Formel VIII,

   R^{8a}R⁹N - CN , (VIII)

   in der
   R^{8a} und R⁹, die gleich oder verschieden sein können, jeweils Wasserstoffatome oder Alkylgruppen mit jeweils 1 bis 3 Kohlenstoffatomen bedeuten.

   Die Umsetzungen werden bei Temperaturen zwischen Zimmertemperatur und der Siedetemperatur des benutzten Lösemittels bzw. Lösemittelgemisches durchgeführt. Als Lösemittel werden Alkohole wie Methanol, Ethanol oder n-Propanol, Ether wie Dioxan oder Ester wie Essigsäureethylester bevorzugt. Als weiteres Cosolvens kommt Wasser in Betracht. Obwohl die Reaktion auch ohne Zugabe von Säuren gelingt, wird die Umsetzung in Gegenwart organischer Säuren, z. B. Essigsäure, und besonders von starken Säuren, z. B. von Methansulfonsäure, Schwefelsäure, Bromwasserstoff, Chlorwasserstoff bzw. Salzsäure, bevorzugt. Die Verbindungen der allgemeinen Formel I fallen dabei in Form der entsprechenden Salze an (siehe auch: Houben-Weyl, Methoden der Organischen Chemie, 4. Auflage, Georg-Thieme-Verlag, Stuttgart, ab 1952, Band VIII, S. 98, S. 180; Ullmanns Encyclopädie der Technischen Chemie, Verlag Chemie, Weinheim, 1972-1977, Band VIII, S. 328; E.H. Sheers, Kirk-Othmer Encycl. Chem. Technol., 2nd ed., 10, 734 [1966]; A. Kämpf, Chem. Ber. 37, 1681 [1904]; R.A. Corral, O.O. Orazi und M.F. de Petruccelli, Chem. Commun. 1970, 556).
e) Zur Herstellung von Verbindungen der allgemeinen Formel I, in der T-Z mit Ausnahme des Wasserstoffatoms die für T-Z eingangs erwähnten Bedeutungen besitzt und R² eine geradkettige Alkylgruppe mit 1 bis 5 Kohlenstoffatomen darstellt, wobei die Alkylgruppe in ω-Stellung durch eine HR⁸N-C(=NR⁷)-NR⁶-Gruppe substituiert ist und R⁶, R⁷ und R⁸, die gleich oder verschieden sein können, Wasserstoffatome oder Alkylgruppen mit 1 bis 3 Kohlenstoffatomen darstellen:
   Umsetzung von Verbindungen der allgemeinen Formel VI,

   T^{A} - Z - NR¹ - (R^{2b}CR³) - CO - Y - (CH₂)ₙ - R , (VI)

   in der
   R-(CH₂)ₙ, R¹, R³ und Y wie eingangs definiert sind, T^{A}-Z mit Ausnahme des Wasserstoffatoms die für T-Z eingangs erwähnten Bedeutungen besitzt und R^{2b} eine geradkettige Alkylgruppe mit 1 bis 5 Kohlenstoffatomen bedeutet, die in ω-Stellung durch eine R⁶NH-Gruppe substituiert ist, wobei R⁶ ein Wasserstoffatom oder eine Alkylgruppe mit 1 bis 3 Kohlenstoffatomen darstellt,
   mit Carbodiimiden der allgemeinen Formel IX,

   R⁷ - N = C = N - R⁸ , (IX)

   in der
   R⁷ und R⁸, die gleich oder verschieden sein können, Wasserstoffatome oder Alkylgruppen mit 1 bis 3 Kohlenstoffatomen darstellen.

   Die Umsetzungen werden nach literaturbekannten Verfahren (siehe S.J.C. Snedker, J. Soc. Chem. Ind. (London) 45, 353T [1927],; F. Kurzer und K. Dourgahi-Zadeh, Chem. Rev. 67, 119 [1967]) durchgeführt. Setzt man dabei die Ausgangsverbindungen der allgemeinen Formel VI in Form ihrer Salze ein, so erhält man die Guanidiniumsalze der allgemeinen Formel I gleichfalls in Form der entsprechenden Salze.
f) Zur Herstellung von Verbindungen der allgemeinen Formel I, in der T-Z mit Ausnahme des Wasserstoffatoms die für T-Z eingangs erwähnten Bedeutungen besitzt und R² eine Amidinophenylmethylgruppe darstellt:
   Umsetzung von Verbindungen der allgemeinen Formel X,

   T^{A} - Z - NR¹ - (R^{2c}CR³) - CO - Y - (CH₂)ₙ - R , (X)

   in der
   R-(CH₂)ₙ, R¹, R³ und Y wie eingangs definiert sind, T^{A}-Z mit Ausnahme des Wasserstoffatoms die für T-Z eingangs erwähnten Bedeutungen besitzt und R^{2c} eine Cyanophenylmethylgruppe bedeutet,
   mit Alkoholen der allgemeinen Formel XI,

   R⁵ - OH , (XI)

   in der
   R⁵ eine Alkylgruppe mit 1 bis 3 Kohlenstoffatomen bedeutet, und
   anschließende Behandlung mit Ammmoniak.
   Die erste Stute der Umsetzung wird vorzugsweise in einem Alkohol der allgemeinen Formel XI als Lösungsmittel, z.B. in Methanol oder Ethanol, in Gegenwart von trockenem Chlorwasserstoff und in Abwesenheit von Wasser bei Temperaturen zwischen -30°C und +40°C, bevorzugt bei 0°C bis +20°C, durchgeführt, wobei die Iminoester, die bei dieser sauren Variante in Form ihrer Hydrochloride anfallen, in der Regel nicht gereinigt werden, sondern in der zweiten Stufe direkt durch Behandlung mit Ammoniak und bei Temperaturen zwischen -20°C und der Siedetemperatur des Lösemittels in die gesuchten Verbindungen der allgemeinen Formel I übergeführt werden (siehe auch: A. Pinner und F. Klein, Chem. Ber. 10, 1889 [1877]; A. Pinner, "Die Iminoäther und ihre Derivate", Oppenheim, Berlin, 1892; R. Roger und D.G. Neilson, Chem. Rev. 61, 179 [1961]; G. Wagner und J. Wunderlich, Pharmazie 31, 766 [1976]; G. Wagner, B. Voigt, D. Danicke und T. Liebermann, Pharmazie 31, 528 [1976]; R.R. Tidwell, L.L. Fox und J.D. Geratz, Biochim. Biophys. Acta 445, 729 [1976]; T. Pantev und R. Georgieva, Farmatsiya (Sofia) 29, 1 [1979]). Die Iminoester entstehen auch in Form ihrer freien Basen bei der basenkatalysierten Addition von Alkoholen der allgemeinen Formel XI an die Nitrile der Formel X. Als basische Katalysatoren dienen bevorzugt die den verwendeten Alkoholen entsprechenden Alkalialkoholate; ganz besonders bevorzugt wird die Kombination von Natriummethanolat und Methanol (siehe auch: C. Soula, A. Marsura und C. Luu-Duc, J. Pharm. Belg. 42, 293 [1987]; W.J. Haggerty und W.J. Rost, J. Pharm. Sci. 58, 50 [1969]).
   Bei der sauren Variante der Synthese von Amidinen der allgemeinen Formel I können in der ersten Stufe statt trockenem Chlorwasserstoff auch andere wasserfreie saure Agenzien eingesetzt werden, z. B. Bromwasserstoff, p-Toluolsulfonsäure oder Schwefelsäure. In der zweiten Stufe, der Umsetzung der erhaltenen Iminoester mit den Aminen der allgemeinen Formel XI verwendet man an Stelle des freien Ammoniaks häufig dessen Salze mit schwachen organischen Säuren, z. B. die entsprechenden Ammoniumcarbonate oder -acetate.
   Bei der alkalischen Variante wird in der zweiten Stufe Ammoniak in der Regel als mineralsaures Salz eingesetzt, z. B. als Hydrochlorid; als Lösemittel kann in der zweiten Stufe mit Vorteil auch Eisessig verwendet werden.
g) Zur Herstellung von Verbindungen der allgemeinen Formel I, in der T-Z mit Ausnahme des Wasserstoffatoms die für T-Z eingangs erwähnten Bedeutungen besitzt und R² eine Amidinophenylmethylgruppe darstellt:
   Hydrogenolyse einer Verbindung der allgemeinen Formel XII,

   T^{A} - Z - NR¹ - (R^{2d}CR³) - CO - Y - (CH₂)ₙ - R , (XII)

   in der
   R-(CH₂)ₙ, R¹, R³ und Y wie eingangs definiert sind, T^{A}-Z mit Ausnahme des Wasserstoffatoms die für T-Z eingangs erwähnten Bedeutungen besitzt und R^{2d} eine durch eine H₂N-C(=NOH)-Gruppe substituierte Phenylmethylgruppe darstellt.

   Die Hydrogenolyse wird unter Verwendung von Palladium- oder Nickelkatalysatoren, z. B. Palladium/Tierkohle, Palladiummohr, Palladium/Bariumsulfat oder Raney-Nickel, in geeigneten Lösemitteln, wie Ethanol, Methanol, Eisessig, 1,4-Dioxan oder Essigsäureethylester, bei Temperaturen zwischen 0 und +100°C, bevorzugt +50°C und +70°C, und einem Wasserstoffdruck von 0,5 bis 200 bar, bevorzugt 1 bis 5 bar, durchgeführt.
h) Zur Herstellung von Verbindungen der allgemeinen Formel I, in der T-Z mit Ausnahme des Wasserstoffatoms die für T-Z eingangs erwähnten Bedeutungen besitzt und R² eine Amidinophenylmethylgruppe darstellt:
   Überführung von Nitrilen der allgemeinen Formel X,

   T^{A} - Z - NR¹ - (R^{2c}CR³) - CO - Y - (CH₂)ₙ - R , (X)

   in der
   R-(CH₂)ₙ, R¹, R³ und Y wie eingangs definiert sind, T^{A}-Z mit Ausnahme des Wasserstoffatoms die für T-Z eingangs erwähnten Bedeutungen besitzt und R^{2c} eine Cyanophenylmethylgruppe darstellt,
   in Thioamide der allgemeinen Formel XIII,

   T^{A} - Z - NR¹ - (R^{2c'}CR³) - CO - Y - (CH₂)ₙ - R , (XIII)

   in der
   R-(CH₂)ₙ, R¹, R³, Y und T^{A}-Z wie oben erwähnt definiert sind und R^{2C'} eine durch eine Aminothiocarbonylgruppe substituierte Phenylmethylgruppe darstellt,
   anschließende Alkylierung mit Verbindungen der allgemeinen Formel XIV,

   R⁵ - X² , (XIV)

   in der
   R⁵ eine Alkylgruppe mit 1 bis 3 Kohlenstoffatomen und X² eine Austrittsgruppe wie ein Halogenatom, eine Alkylsulfonyloxy-, Alkoxysulfonyloxy- oder Arylsulfonyloxygruppe, z.B. ein Chlor-, Brom- oder Iodatom, eine Methansulfonyloxy-, Methoxysulfonyloxy- oder Toluolsulfonyloxygruppe, darstellt,
   oder mit einem Trialkyloxoniumtetrafluoroborat der allgemeinen Formel XV,

   (R⁵)₃OBF₄ , (XV)

   in der
   R⁵ eine Alkylgruppe mit 1 bis 3 Kohlenstoffatomen darstellt und
   anschließende Umsetzung mit Ammoniak.
   Die Umsetzungen werden nach literaturbekannten Verfahren durchgeführt (siehe auch: P. Chabrier und S.H. Renard, C.R. Acad. Sci. Paris 230, 1673 [1950]; Y. Nii, K. Okano, S. Kobayashi und M. Ohto, Tetrah. Lett. 1979, 2517; Hoffmann-La-Roche, EP-A-0 381 033).
   Zur Herstellung der Thiocarbonsäureamide der allgemeinen Formel XIII aus den Nitrilen der allgemeinen Formel X wird die Umsetzung mit Schwefelwasserstoff in Pyridin und in Gegenwart von gasförmigem Ammoniak oder von Triethylamin, gegebenenfalls auch im Druckautoklaven bevorzugt. Geeignete Reaktionstemperaturen liegen zwischen 0°C und +100°C, bevorzugt bei +50 bis +60°C (siehe auch: Houben-Weyl, Methoden der Organischen Chemie, 4. Auflage, Georg-Thieme Verlag, Stuttgart, ab 1952, Band IX, S. 762). Geeignet ist auch die Umsetzung mit Thioacetamid in mit trockenem Chlorwasserstoff gesättigtem Dimethylformamid bei Temperaturen zwischen 80 und 100°C (siehe auch: E.C. Taylor und J.A. Zoltewicz, J. Amer. Chem. Soc. 82, 2656 [1960]).
   Zur Herstellung der Thioimidsäureester bzw. ihrer Salze aus den Thioamiden der allgemeinen Formel XIII wird die Umsetzung mit Methyliodid bevorzugt. Als Lösemittel kommen Ketone, wie Aceton oder Cyclohexanon, und dipolare, aprotische Lösemittel vom Typ des Dimethylformamids, Dimethylacetamids, N-Methyl-pyrrolidons oder 1,3-Dimethyl-2-imidazolidinons oder Gemische davon in Betracht. Geeignete Reaktionstemperaturen liegen zwischen -20°C und +100°C, bevorzugt bei Raumtemperatur.
   Die Aminolyse erfolgt bei Temperaturen zwischen 0°C und +100°C, bevorzugt +40°C und +80°C, und unter Verwendung inerter Lösemittel, beispielsweise von Dichlormethan, Tetrahydrofuran, 1,4-Dioxan, Acetonitril, Dimethylformamid, Dimethylacetamid, N-Methyl-pyrrolidon oder Gemischen davon, und in der Regel in Gegenwart von Hilfsbasen, insbesondere von Alkalicarbonaten wie Natrium- oder Kaliumcarbonat, oder tertiären Aminen, bevorzugt N-Ethyl-diisopropylamin oder Triethylamin.
i) Zur Herstellung von Verbindungen der allgemeinen Formel I, in der T-Z mit Ausnahme des Wasserstoffatoms die für T-Z eingangs erwähnten Bedeutungen besitzt und R² Cyaniminomethylaminophenylmethylgruppe darstellt:
   Umsetzung von Verbindungen der allgemeinen Formel VI,

   T^{A} - Z - NR¹ - (R^{2b}CR³) - CO - Y - (CH₂)ₙ - R , (VI)

   R-(CH₂)ₙ, R¹, R³ und Y wie eingangs definiert sind, T^{A}-Z mit Ausnahme des Wasserstoffatoms die für T-Z eingangs erwähnten Bedeutungen besitzt und R^{2b} eine Aminophenylmethylgruppe bedeutet,
   mit N-Cyan-formimidsäureestern der allgemeinen Formel XVI,

   R¹³O-CH=N-CN , (XVI)

   in der
   R¹³ eine Alkylgruppe mit 1 bis 10 Kohlenstoffatomen, vorzugsweise jedoch die Methyl- oder Ethylgruppe, darstellt.

   Die Umsetzung wird vorzugsweise in Gegenwart von polaren Lösemitteln, beispielsweise von Aceton, Ethanol, Dimethylformamid, 1,4-Dioxan, Dimethylacetamid oder N-Methyl-pyrrolidon, und bei Temperaturen zwischen 0°C und +50°C, bevorzugt jedoch bei Zimmertemperatur, durchgeführt (siehe auch: C. Bazzano, C.P. Vanoni, M. Mondoni, A. Gallazzi, E. Cereda und A. Donetti, Eur. J. Med. Chem. 21, 27-33 [1986]).
j) Zur Herstellung von Verbindungen der allgemeinen Formel I, in der T-Z mit Ausnahme des Wasserstoffatoms die für T-Z eingangs erwähnten Bedeutungen besitzt und R² eine in ω-Stellung durch eine Aminomethylidenimino- oder Methylaminomethylideniminogruppe substituierte geradkettige Alkylgruppe mit 1 bis 5 Kohlenstoffatomen oder eine Methylaminomethylideniminophenylmethylgruppe darstellt:
   Umsetzung von Verbindungen der allgemeinen Formel XVII,

   T^{A} - Z - NR¹ - (R^{2d'}CR³) - CO - Y - (CH₂)ₙ - R , (XVII)

   in der
   R-(CH₂)ₙ, R¹, R³ und Y wie eingangs erwähnt definiert sind, T^{A}-Z mit Ausnahme der Bedeutung eines Wasserstoffatoms die eingangs für T-Z erwähnten Bedeutungen besitzt und R^{2d'} eine geradkettige Alkylgruppe mit 1 bis 5 Kohlenstoffatomen oder eine Phenylmethylgruppe darstellt, wobei die Alkylgruppe in ω-Stellung und der vorstehend erwähnte Aromat jeweils durch eine NC-N=CH-NH-Gruppe substituiert sind,
   mit Aminen der allgemeinen Formel XVIII,

   H₂NR^{8a} , (XVIII)

   in der
   R^{8a} ein Wasserstoffatom oder eine Methylgruppe darstellt.

   Die Umsetzung wird vorzugsweise unter Verwendung polarer Lösemittel, z. B. von Alkoholen wie Methanol oder Ethanol, von Ethern wie Tetrahydrofuran oder 1,4-Dioxan, von Dimethylformamid, Dimethylacetamid, N-Methyl-pyrrolidon, Dimethylsulfoxid, Wasser oder Gemischen davon, und bei Temperaturen zwischen 0°C und +70°C, bevorzugt bei Zimmertemperatur, durchgeführt (siehe auch: C. Bazzano, P.C. Vanoni, M. Mondani, A. Gallazzi, E. Cereda und A. Donetti, Eur. J. Med. Chem. 21, 27-33 [1986]; A. Donetti, E. Cereda, E. Bellora, A. Gallazzi, C. Bazzano, P.C. Vanoni, P. del Soldato, R. Micheletti, F. Pagani und A. Giachetti, J. Med. Chem. 27, 380 [1984]).
k) Zur Herstellung von Verbindungen der allgemeinen Formel I, in der T-Z eine Diphenylaminocarbonyl-, Naphthylaminocarbonyl-, Hexamethylenaminocarbonyl-, 1-Piperidinyl- oder (Diphenylmethyl)aminocarbonyl-Gruppe darstellt:
   Umsetzung von Isocyanaten der allgemeinen Formel XIX,

   O=C=N - (R^{2a}CR³)-CO-NR⁴-(CH₂)ₙ-R , (XIX)

   in der
   R-(CH₂)ₙ, R³ und R⁴ wie eingangs erwähnt definiert sind und R^{2a} die für R² eingangs erwähnten Bedeutungen besitzt oder ei-nen durch Schutzreste substituierten Rest R² oder einen Präcursorrest für den Rest R² darstellt,
   mit Aminen der allgemeinen Formel XX,

   T¹T²N-H , (XX)

   in der
   T¹ und T² jeweils eine Phenylgruppe oder
   T¹ ein Wasserstoffatom und T² eine Naphthyl- oder Diphenylmethylgruppe oder
   T¹ und T² zusammen eine n-Alkylengruppe mit 5 oder 6 Kohlenstoffatomen bedeuten.

   Die Umsetzung wird bei Temperaturen zwischen 0°C und 150°C, bevorzugt +20°C und 100°C, und gegebenenfalls in Gegenwart wasserfreier Lösemittel, z. B. von Tetrahydrofuran, 1,4-Dioxan, Dimethylformamid, Dimethylacetamid, N-Methyl-2-pyrrolidon oder 1,3-Dimethyl-2-imidazolidinon, durchgeführt.
l) Zur Herstellung von Verbindungen der allgemeinen Formel I, in der T-Z ein Wasserstoffatom darstellt:
   Abspaltung des Restes R¹⁴ von einer Verbindung der allgemeinen Formel XXI,

   R¹⁴-NR¹ - (R²CR³) - CO - Y - (CH₂)ₙ-R , (XXI)

   in der
   R-(CH₂)ₙ, R¹, R², R³ und Y wie eingangs erwähnt definiert sind und R¹⁴ eine tert.-Butoxycarbonyl- oder eine 9-Fluorenylmethoxycarbonylgruppe darstellt.

   Die Abspaltung der Boc-Gruppe erfolgt beispielsweise mit Trifluoressigsäure in Dichlormethan (siehe auch: M. Bodanszky, "Principles of Peptide Synthesic", Springer-Verlag, 1984, S. 64). Die Abspaltung der 9-Fluorenylmethoxycarbonyl-Gruppe erfolgt beispielsweise mit einer 20 bis 50%iger Lösung von Piperidin in Dimethylformamid (siehe auch: M. Bodanszky, "Principles of Peptide Synthesis", Springer-Verlag, 1984, S. 66).
m) Zur Herstellung von Verbindungen der allgemeinen Formel I, in der ein Wasserstoffatom einer im Rest R² vorhandenen HN<, HN= oder H₂N-Gruppe durch eine tert.-Butoxycarbonylgruppe ersetzt ist:
   Umsetzung einer Verbindung der allgemeinen Formel I,

   T - Z - NR¹ - (R²CR³) - CO - Y - (CH₂)ₙ - R , (I)

   in der
   R-(CH₂)ₙ, R¹ bis R³, T-Z und Y mit der Maßgabe wie eingangs definiert sind, daß R² mindestens eine freie HN<, HN= oder H₂N-Gruppe enthalten muß, mit einer Verbindung der allgemeinen Formel XXII,

      X³ - W , (XXII)

      in der
   W eine tert.-Butoxycarbonylgruppe darstellt und
   X³ eine Austrittsgruppe wie ein Halogenatom oder eine Aryloxygruppe, z. B. ein Chlor- oder Bromatom oder eine p-Nitrophenoxygruppe, bedeutet.

   Die Umsetzung wird zweckmäßigerweise in einem Lösungsmittel wie Tetrahydrofuran, Methylenchlorid, Chloroform, Essigester oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 60°C, durchgeführt.
n) Zur Herstellung von Verbindungen der allgemeinen Formel I, in der T-Z eine Naphthylaminocarbonyl- oder (Diphenylmethyl)aminocarbonyl-Gruppe darstellt:
   Umsetzung von Isocyanaten der allgemeinem Formel XXIII

   T'N=C=O , (XXIII)

   in der
   T' eine Naphthyl- oder Diphenylmethylgruppe bedeutet,
   mit Verbindungen der allgemeinen Formel III

   H - NR¹ - (R^{2a}CR³) - CO - Y - (CH₂)ₙ-R , (III)

   in der
   R-(CH₂)ₙ, R¹, R³ und Y wie eingangs erwähnt definiert sind und R^{2a} die für R² eingangs erwähnten Bedeutungen besitzt oder einen durch Schutzreste substituierten Rest R² oder einen Präcursorrest für den Rest R² darstellt.

Die Umsetzung wird bei Temperaturen zwischen O und 150°C, bevorzugt bei Temperaturen zwischen 20 und 100°C, und gegebenenfalls in Gegenwart wasserfreier Lösemittel, z.B. Tetrahydrofuran, 1,4-Dioxan, Dimethylformamid, Dimethylacetamid, N-Methyl-2-pyrrolidon oder 1,3-Dimethyl-2-imidazolidinon, durchgeführt.

Die erfindungsgemäßen Aminosäurederivate der allgemeinen Formel I enthalten wenigstens ein Chiralitätszentrum. Ist darüber hinaus noch der Rest T chiral, dann können die Verbindungen in Form zweier diastereomerer Antipodenpaare auftreten. Die Erfindung umfaßt die einzelnen Isomeren ebenso wie ihre Gemische.

Die Trennung der jeweiligen Diastereomeren gelingt auf Grund der unterschiedlichen physiko-chemischen Eigenschaften, z. B. durch fraktionierte Kristallisation aus geeigneten Lösemitteln, durch Hochdrucksflüssigkeits- oder Säulenchromatographie unter Verwendung chiraler oder bevorzugt achiraler stationärer Phasen.

Die Trennung von unter die allgemeine Formel I fallenden Racematen gelingt beispielsweise durch HPLC an geeigneten chiralen stationären Phasen (z. B. Chiral AGP, Chiralpak AD). Racemate, die eine basische Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)-Camphersulfonsäure, entstehen.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösungsmittel durchgeführt werden, solange dieses einen ausreichenden Unterschied hinsichtlich der Löslichkeit der Salze aufweist. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, neutralisiert und dadurch die entsprechende freie Verbindung in der (+)- oder (-)-Form erhalten.

Jeweils nur das (R)-Enantiomer bzw. ein Gemisch zweier optisch aktiver, unter die allgemeine Formel I fallender diastereomerer Verbindungen wird auch dadurch erhalten, daß man die oben beschriebenen Synthesen mit jeweils einer die entsprechende (R)-konfigurierte Aminosäure enthaltenen Reaktionskomponente durchführt.

Die zur Synthese der Verbindungen der allgemeinen Formel I erforderlichen Ausgangsmaterialien der allgemeinen Formeln II, V, VII, VIII, IX, XI, XV, XVI, XVIII, XX, XXIII und XXIV sowie die verwendeten Aminosäuren sind käuflich oder werden nach literaturbekannten Verfahren hergestellt. Die Säuren IV erhält man beispielsweise unter den Bedingungen einer Schotten-Baumann-Reaktion aus den entsprechenden α-Aminosäuren und Verbindungen der allgemeinen Formel II (siehe auch: M. Bodanszky und A. Bodanszky, "The Practice of Peptide Synthesis", Springer-Verlag, 1984, S. 9-30). Isocyanate der allgemeinen Formel XIX lassen sich leicht aus α-Aminosäurederivaten der allgemeinen Formel III, in der R¹ das Wasserstoffatom darstellt und die übrigen Reste wie oben definiert sind, bzw. aus deren Hydrochloriden durch Umsetzung mit Phosgen, Diphosgen oder Triphosgen in Gegenwart von Pyridin (siehe auch: J.S. Nowick, N.A. Powell, T.M. Nguyen und G. Noronha, J. Org. Chem. 57, 7364-7366 [1992]) herstellen.

Die erhaltenen Verbindungen der allgemeinen Formel I können, insbesondere für pharmazeutische Anwendungen, in ihre physiologisch verträglichen Salze mit anorganischen oder organischen Säuren, übergeführt werden. Als Säuren kommen hierfür beispielsweise Salzsäure, Bromwasserstoffsäure, Phosphorsäure, Salpetersäure, Schwefelsäure, Methansulfonsäure, p-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Dicyclohexylamin, Äthanolamin, Diäthanolamin und Triäthanolamin in Betracht.

Die neuen Verbindungen der allgemeinen Formel I mit Ausnahme derjenigen Verbindungen, in denen T-Z ein Wasserstoffatom darstellt, und deren physiologisch verträgliche Salze besitzen NPY-antagonistische Eigenschaften und zeigen gute Affinitäten in NPY-Rezeptorbindungsstudien. Die Verbindungen weisen in den nachstehend beschriebenen pharmakologischen Testsystemen sowohl in vivo als auch in vitro NPY-antagonistische Eigenschaften auf.

Zum Nachweis der Affinität von Verbindungen der allgemeinen Formel I zu humanen NPY-Rezeptoren und ihrer antagonistischen Eigenschaften werden die folgenden Versuche durchgeführt:

### A. Bindungsstudien mit (den humanen Y₁-Rezeptor exprimierenden) SK-N-MC-Zellen

Die Zellen werden durch ein Gemisch von 0,02% EDTA in PBS abgelöst und in 10 ml Inkubationsmedium (MEM/25 mM Hepes + 0,5% BSA, 50 mM PMSF, 0,1% Bacitracin, 3,75 mM CaCl₂) pro ca. 40 Mio. Zellen resuspendiert. Nach 5 min Zentrifugation (150 x g) wird das Pellet im gleichen Volumen und nach einem weiteren Waschschritt in 10 ml Inkubationsmedium resuspendiert, ausgezählt und auf 1,25 Mio. Zellen/ml verdünnt. Dann werden 200 ml einer Suspension von 1,25 Mio. Zellen/ml 3 Stunden bei Zimmertemperatur mit 25 ml einer 300 pM Lösung von [¹²⁵I]-Bolton-Hunter-NPY und steigenden Konzentrationen (10⁻¹¹ bis 10⁻⁶ M) der Testsubstanzen, unter Einhaltung eines Gesamtvolumens von jeweils 250 ml, inkubiert. Die Inkubation wird durch Zentrifugation (10 min bei 3000 x g und 4°C) beendet. Nach einmaligen Waschen mit PBS wird die Radioaktivität des Pellets im Gamma-Counter gemessen. Die so erhaltene Radioaktivität repräsentiert die Summe von spezifischer und unspezifischer Bindung von [¹²⁵I]-Bolton-Hunter-NPY. Der Anteil der unspezifischen Bindung wird als jene Radioaktivität definiert, die in Anwesenheit von 1 mM NPY gebunden wird. Die IC₅₀-Werte der nichtmarkierten Testsubstanzen werden graphisch ermittelt. Sie repräsentieren jene Konzentration der jeweiligen Testsubstanz, bei der die spezifische Bindung von [¹²⁵I]-Bolton-Hunter-NPY an den NPY-Y₁-Rezeptor um 50% gehemmt wird.

Die Verbindungen der allgemeinen Formel I mit Ausnahme derjenigen, in denen T ein Wasserstoffatom und Z gleichzeitig eine Bindung darstellt, zeigen in dem beschriebenen Test IC₅₀-Werte < 10.000 nM.

### B. In vitro NPY-Antagonismus

Männlichen Ratten (CHbb: THOM, 300 bis 350 g) wird Heparin verabreicht (100 IU, i.v.) und die Tiere werden anschließend durch einen Genickschlag getötet. Das Abdomen wird entlang der Körpermitte geöffnet und die linke Niere nach der Einführung von Kathetern in die renale Arterie, die renale Vene und den Harnleiter entnommen. Die isolierte Niere wird sofort mit einer modifizierten Krebs-Ringer-Lösung der folgenden Zusammensetzung perfundiert (4 ml/Minute):

| | |
|---|---|
| NaCl | 118.0 mmol/l |
| KH₂PO₄ | 1.2 mmol/l |
| KCl | 4.8 mmol/l |
| HgSO₄ | 1.2 mmol/l |
| CaCl₂ | 2.5 mmol/l |
| NaHCO₃ | 25.0 mmol/l |
| Glucose | 6.5 mmol/l |

Durch die auf 37°C temperierte Lösung wird eine Mischung von 95 % O₂/5 % CO₂ geleitet. Der Perfusionsdruck wird mit Hilfe eines Druckaufnehmers kontinuierlich gemessen. Nach einer 60-minütigen Stabilisierungsperiode wird die Perfusionsrate so eingestellt, daß ein Perfusionsdruck von ungefähr 100 mm Hg erreicht wird. Nach weiteren 30 Minuten wird das Experiment begonnen und NPY (1mN) als Bolus (0,1 ml) in 15-minütigen Intervallen verabreicht, bis die beobachtete Druckzunahme einen konstanten Wert erreicht. Die zu untersuchenden Verbindungen werden als kontinuierliche Infusion über einen Zeitraum von 5 Minuten verabreicht und anschließend NPY injiziert. Nach einer 30-minütigen Auswaschperiode wird die nächsthöhere Konzentration der Testsubstanz untersucht. Bei jeder Versuchsdurchführung werden 3 bis 5 verschiedene Konzentrationen der jeweiligen Verbindung getestet. Konzentrations-Wirkungs-Kurven können erstellt werden, indem die prozentuale Inhibierung der NPY-Wirkung gegen den Logarithums der Konzentration (mol/l) der Verbindung aufgetragen wird.

Die Verbindungen der allgemeinen Formel I mit Ausnahme derjenigen, in denen T-Z ein Wasserstoffatom darstellt, zeigen in dem beschriebenen in-vitro-Testmodell NPY-antagonistische Eigenschaften in einem Dosisbereich zwischen 10⁻⁸ bis 10⁻⁵ M.

### C. In-vivo-NPY-Antagonismus

Männliche normotensive Ratten (Chbb:THOM, 300 bis 350 g) werden mit Hexobarbital-Natrium (150 mg/kg, i.p.) anästhesiert. Nach Intubierung der Trachea werden die Tiere durch Einführung einer stumpfen Nadel durch das Auge in den Rückenmarkskanal despinalisiert. Die Tiere werden mit Hilfe einer Atmungspumpe (20 Pumphübe/Minute) mit sauerstoffreicher Raumluft beatmet. Eine Kanüle wird in die linke Karotis-Arterie eingeführt und der arterielle Blutdruck über ein Druckmeßgerät (Braun Melsungen Combitrans), welches mit einem Aufzeichnungsgerät verbunden ist, gemessen. Für Injektionszwecke wird ein Katheter in die linke Jugularvene gelegt, über welchen Heparin (200 IU/kg, i.v.) appliziert wird. Nach Stabilisierung des Blutdrucks erhalten die Tiere in einem Intervall von 15 Minuten 2 Bolus-Injektionen von NPY (10 mg/kg, i.v.). Die mittlere Zunahme des diastolischen Blutdrucks dient als Referenzwert (= 100 %). Die Testsubstanzen werden in steigender Dosierung (4 bis 6 Dosen) in Intervallen von 15 Minuten injiziert. Eine Minute nach Applikation der Testsubstanz wird NPY verabreicht.

Die antagonistische Wirksamkeit der Testsubstanzen wird bestimmt, indem die prozentuale Inhibierung der NPY-induzierten Blutdruckeffekte gegen den Logarithmus der Wirkstoffkonzentration aufgetragen wird.

Die Verbindungen der allgemeinen Formel I mit Ausnahme derjenigen, in denen T-Z ein Wasserstoffatom darstellt, zeigen in dem beschriebenen in vivo Testmodell nach intravenöser Gabe im Dosisbereich von 0,01 bis 10 mg/kg NPY-antagonistische Eigenschaften.

Auf Grund ihrer pharmakologischen Eigenschaften eignen sich die Verbindungen der allgemeinen Formel I mit Ausnahme derjenigen Verbindungen, in denen T-Z ein Wasserstoffatom darstellt, und deren physiologisch verträglichen Salze somit zur Behandlung von cardiovasculären Erkrankungen, z.B. zur Behandlung des Bluthochdrucks, der chronischen Herzinsuffizienz, von coronaren Herzerkrankungen, wie Angina pectoris, Myocardinfarkt und Syndrom X, ferner zur Behandlung von subarachnoidalen Blutungen, des chronischen Nierenversagens, von Tumorerkrankungen wie Phaeochromocytoma, von Hyperthyreoidismus sowie der Obesitas und des Diabetes.

Die zur Erzielung einer entsprechenden Wirkung erforderliche Dosierung beträgt zweckmäßigerweise bei intravenöser Gabe 0,01 bis 3 mg/kg Körpergewicht, vorzugsweise 0,1 bis 1 mg/kg Körpergewicht, und bei oraler Gabe 0,1 bis 10 mg/kg Körpergewicht, vorzugsweise 1 bis 10 mg/kg Körpergewicht, jeweils 1 bis 3 x täglich.

Hierzu lassen sich die erfindungsgemäß hergestellten Verbindungen der allgemeinen Formel I mit Ausnahme derjenigen Verbindungen, in denen T-Z ein Wasserstoffatom darstellt, gegebenenfalls in Kombination mit anderen Wirksubstanzen, wie z.B. Blutdrucksenkern, ACE-Hemmern, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorothiazid, Hydrochlorothiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorothiazid, 125 bis 2000 mg Chlorothiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:
nosäurederivaten der allgemeinen Formel III, in der R¹ das Wasserstoffatom darstellt und die übrigen Reste wie oben definiert sind, bzw. aus deren Hydrochloriden durch Umsetzung mit Phosgen, Diphosgen oder Triphosgen in Gegenwart von Pyridin (siehe auch: J.S. Nowick, N.A. Powell, T.M. Nguyen und G. Noronha, J. Org. Chem. 57, 7364-7366 [1992]) herstellen.

Die erhaltenen Verbindungen der allgemeinen Formel I können, insbesondere für pharmazeutische Anwendungen, in ihre physiologisch verträglichen Salze mit anorganischen oder organischen Säuren, übergeführt werden. Als Säuren kommen hierfür beispielsweise Salzsäure, Bromwasserstoffsäure, Phosphorsäure, Salpetersäure, Schwefelsäure, Methansulfonsäure, p-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Dicyclohexylamin, Äthanolamin, Diäthanolamin und Triäthanolamin in Betracht.

Die neuen Verbindungen der allgemeinen Formel I mit Ausnahme derjenigen Verbindungen, in denen T ein Wasserstoffatom und Z gleichzeitig eine Bindung darstellt, und deren physiologisch verträgliche Salze besitzen NPY-antagonistische Eigenschaften und zeigen gute Affinitäten in NPY-Rezeptorbindungsstudien. Die Verbindungen weisen in den nachstehend beschriebenen pharmakologischen Testsystemen sowohl in vivo als auch in vitro NPY-antagonistische Eigenschaften auf.

Zum Nachweis der Affinität von Verbindungen der allgemeinen Formel I zu humanen NPY-Rezeptoren und ihrer antagonistischen Eigenschaften werden die folgenden Versuche durchgeführt:

### A. Bindungsstudien mit (den humanen Y₁-Rezeptor exprimierenden) SK-N-MC-Zellen

Die Zellen werden durch ein Gemisch von 0,02% EDTA in PBS abgelöst und in 10 ml Inkubationsmedium (MEM/25 mM Hepes + 0,5% BSA, 50 µM PMSF, 0,1% Bacitracin, 3,75 mM CaCl₂) pro ca. 40 Mio. Zellen resuspendiert. Nach 5 min Zentrifugation (150 x g) wird das Pellet im gleichen Volumen und nach einem weiteren Waschschritt in 10 ml Inkubationsmedium resuspendiert, ausgezählt und auf 1,25 Mio. Zellen/ml verdünnt. Dann werden 200 µl einer Suspension von 1,25 Mio. Zellen/ml 3 Stunden bei Zimmertemperatur mit 25 µl einer 300 pM Lösung von [¹²⁵I]-Bolton-Hunter-NPY und steigenden Konzentrationen (10⁻¹¹ bis 10⁻⁶ M) der Testsubstanzen, unter Einhaltung eines Gesamtvolumens von jeweils 250 µl, inkubiert. Die Inkubation wird durch Zentrifugation (10 min bei 3000 x g und 4°C) beendet. Nach einmaligen Waschen mit PBS wird die Radioaktivität des Pellets im Gamma-Counter gemessen. Die so erhaltene Radioaktivität repräsentiert die Summe von spezifischer und unspezifischer Bindung von [¹²⁵I]-Bolton-Hunter-NPY. Der Anteil der unspezifischen Bindung wird als jene Radioaktivität definiert, die in Anwesenheit von 1 µM NPY gebunden wird. Die IC₅₀-Werte der nichtmarkierten Testsubstanzen werden graphisch ermittelt. Sie repräsentieren jene Konzentration der jeweiligen Testsubstanz, bei der die spezifische Bindung von [¹²⁵I]-Bolton-Hunter-NPY an den NPY-Y₁-Rezeptor um 50% gehemmt wird.

Die Verbindungen der allgemeinen Formel I mit Ausnahme derjenigen, in denen T ein Wasserstoffatom und Z gleichzeitig eine Bindung darstellt, zeigen in dem beschriebenen Test IC₅₀-Werte ≤ 10.000 nM.

### B. In vitro NPY-Antagonismus

Männlichen Ratten (CHbb: THOM, 300 bis 350 g) wird Heparin verabreicht (100 IU, i.v.) und die Tiere werden anschließend durch einen Genickachlag getötet. Das Abdomen wird entlang der Körpermitte geöffnet und die linke Niere nach der Einführung von Kathetern in die renale Arterie, die renale Vene und den Harnleiter entnommen. Die isolierte Niere wird sofort mit einer modifizierten Krebs-Ringer-Lösung der folgenden Zusammensetzung perfundiert (4 ml/Minute):

| | |
|---|---|
| NaCl | 118.0 mmol/l |
| KH₂PO₄ | 1.2 mmol/l |
| KCl | 4.8 mmol/l |
| HgSO₄ | 1.2 mmol/l |
| CaCl₂ | 2.5 mmol/l |
| NaHCO₃ | 25.0 mmol/l |
| Glucose | 6.5 mmol/l |

Durch die auf 37°C temperierte Lösung wird eine Mischung von 95 % O₂/5 % CO₂ geleitet. Der Perfusionsdruck wird mit Hilfe eines Druckaufnehmers kontinuierlich gemessen. Nach einer 60-minütigen Stabilisierungsperiode wird die Perfusionsrate so eingestellt, daß ein Perfusionsdruck von ungefähr 100 mm Hg erreicht wird. Nach weiteren 30 Minuten wird das Experiment begonnen und NPY (1µM) als Bolus (0,1 ml) in 15-minütigen Intervallen verabreicht, bis die beobachtete Druckzunahme einen konstanten Wert erreicht. Die zu untersuchenden Verbindungen werden als kontinuierliche Infusion über einen Zeitraum von 5 Minuten verabreicht und anschließend NPY injiziert. Nach einer 30-minütigen Auswaschperiode wird die nächathöhere Konzentration der Testsubstanz untersucht. Bei jeder Versuchsdurchführung werden 3 bis 5 verschiedene Konzentrationen der jeweiligen Verbindung getestet. Konzentrations-Wirkungs-Kurven können erstellt werden, indem die prozentuale Inhibierung der NPY-Wirkung gegen den Logarithums der Konzentration (mol/l) der Verbindung aufgetragen wird.

Die Verbindungen der allgemeinen Formel I mit Ausnahme derjenigen, in denen T ein Wasserstoffatom und Z gleichzeitig eine Bindung darstellt, zeigen in dem beschriebenen in-vitro-Testmodell NPY-antagonistische Eigenschaften in einem Dosisbereich zwischen 10⁻⁸ bis 10⁻⁵ M.

### C. In-vivo-NPY-Antagonismus

Männliche normotensive Ratten (Chbb:THOM, 300 bis 350 g) werden mit Hexobarbital-Natrium (150 mg/kg, i.p.) anästhesiert. Nach Intubierung der Trachea werden die Tiere durch Einführung einer stumpfen Nadel durch das Auge in den Rückenmarkskanal despinalisiert. Die Tiere werden mit Hilfe einer Atmungspumpe (20 Pumphübe/Minute) mit sauerstoffreicher Raumluft beatmet. Eine Kanüle wird in die linke Karotis-Arterie eingeführt und der arterielle Blutdruck über ein Druckmeßgerät (Braun Melsungen Combitrans), welches mit einem Aufzeichnungsgerät verbunden ist, gemessen. Für Injektionszwecke wird ein Katheter in die linke Jugularvene gelegt, über welchen Heparin (200 IU/kg, i.v.) appliziert wird. Nach Stabilisierung des Blutdrucks erhalten die Tiere in einem Intervall von 15 Minuten 2 Bolus-Injektionen von NPY (10 µg/kg, i.v.). Die mittlere Zunahme des diastolischen Blutdrucks dient als Referenzwert (= 100 %). Die Testsubstanzen werden in steigender Dosierung (4 bis 6 Dosen) in Intervallen von 15 Minuten injiziert. Eine Minute nach Applikation der Testsubstanz wird NPY verabreicht.

Die antagonistische Wirksamkeit der Testsubstanzen wird bestimmt, indem die prozentuale Inhibierung der NPY-induzierten Blutdruckeffekte gegen den Logarithmus der Wirkstoffkonzentration aufgetragen wird.

Die Verbindungen der allgemeinen Formel I mit Ausnahme derjenigen, in denen T ein Wasserstoffatom und Z gleichzeitig eine Bindung darstellt, zeigen in dem beschriebenen in vivo Testmodell nach intravenöser Gabe im Dosisbereich von 0,01 bis 10 mg/kg NPY-antagonistische Eigenschaften.

Auf Grund ihrer pharmakologischen Eigenschaften eignen sich die Verbindungen der allgemeinen Formel I mit Ausnahme derjenigen Verbindungen, in denen T ein Wasserstoffatom und Z gleichzeitig eine Bindung darstellt, und deren physiologisch verträglichen Salze somit zur Behandlung von cardiovasculären Erkrankungen, z.B. zur Behandlung des Bluthochdrucks, der chronischen Herzinsuffizienz, von coronaren Herzerkrankungen, wie Angina pectoris, Myocardinfarkt und Syndrom X, ferner zur Behandlung von subarachnoidalen Blutungen, des chronischen Nierenversagens, von Tumorerkrankungen wie Phaeochromocytoma, von Hyperthyreoidismus sowie der Obesitas und des Diabetes.

Die zur Erzielung einer entsprechenden Wirkung erforderliche Dosierung beträgt zweckmäßigerweise bei intravenöser Gabe 0,01 bis 3 mg/kg Körpergewicht, vorzugsweise 0,1 bis 1 mg/kg Körpergewicht, und bei oraler Gabe 0,1 bis 10 mg/kg Körpergewicht, vorzugsweise 1 bis 10 mg/kg Körpergewicht, jeweils 1 bis 3 x täglich.

Hierzu lassen sich die erfindungsgemäß hergestellten Verbindungen der allgemeinen Formel I, gegebenenfalls in Kombination mit anderen Wirksubstanzen, wie z.B. Blutdrucksenkern, ACE-Hemmern, Diuretika und/oder Kalzium-Antagonisten, zusammen mit einem oder mehreren inerten üblichen Trägerstoffen und/oder Verdünnungamitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorothiazid, Hydrochlorothiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorothiazid, 125 bis 2000 mg Chlorothiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Ein weiterer Gegenstand der Erfindung ist die Verwendung der Verbindungen der allgemeinen Formel I mit Ausnahme derjenigen Verbindungen, in denen T ein Wasserstoffatom und Z gleichzeitig eine Bindung darstellt, als wertvolle Hilfsmittel zur Erzeugung und Reinigung (Affinitätschromatographie) von Antikörpern sowie, nach geeigneter radioaktiver Markierung, beispielsweise durch direkte Markierung mit ¹²⁵I oder ¹³¹I oder durch Tritiierung geeigneter Vorstufen, beispielsweise durch Ersatz von Halogenatomen durch Tritium, in RIA- und ELISA-Assays und als diagnostische bzw. analytische Hilfsmittel in der Neutrotransmitter-Forschung.

Die nachfolgenden Beispiele sollen die Erfindung näher erläutern:

### Vorbemerkungen:

"Fp." bedeutet "Schmelzpunkt", "Z." bedeutet "Zersetzung". Für alle Verbindungen liegen befriedigende Elementaranalysen, IR-, UV-, ¹H-NMR-, in der Regel auch Massenspektren vor. Wenn nicht anderes angegeben, wurden R_{F}-Werte unter Verwendung von DC-Fertigplatten Kieselgel 60 F₂₅₄ (E. Merck, Darmstadt, Artikel-Nr. 5729) und eines Fließmittels aus n-Butanol/Eisessig/Wasser = 4/1/1 (v/v/v), ohne Kammersättigung bestimmt. Falls nähere Angaben zur Konfiguration fehlen, bleibt offen, ob es sich um das (R)-Enantiomer handelt oder ob partielle oder gar völlige Racemisierung eingetreten ist.

### Beispiel 1

### (R)-N-[[4-(Acetylamino)phenyl]methyl]-N²-(diphenylacetyl)argininamid-acetat

### a) (R)-N⁵-[Amino(nitroimino)methyl]-N²-(diphenylacetyl)ornithin

Zu der Aufschlämmung von 50 g (0,228 Mol) H-D-Arg(NO₂)-OH in 400 ml Tetrahydrofuran gab man die Lösung von 9,12 g (0,228 Mol) Natriumhydroxid in 100 ml Wasser. In diese Mischung tropfte man anschließend innerhalb von 30 Minuten gleichzeitig die Lösung 52,6 g (0,228 Mol) Diphenyacetylchlorid in 400 ml Tetrahydrofuran und die Lösung von 9,12 g (0,228 Mol) Natriumhydroxid in Wasser ohne äußere Kühlung ein, rührte den Ansatz noch 12 Stunden bei Raumtemperatur und destillierte anschließend die Lösemittel im Wasserstrahlvakuum ab. Der verbleibende ölige Rückstand wurde in 600 ml Wasser gelöst und die erhaltene wässerige Lösung dann mit 230 ml 1N wässeriger Salzsäure angesäuert. Der anfallende Niederschlag wurde in 500 ml Ethylacetat aufgenommen, die Essigesterlösung daraufhin mit Wasser gründlich gewaschen, über Natriumsulfat getrocknet und im Vakuum vom Lösemittel befreit. Nach dem Umkristallisieren aus Aceton erhielt man 80,0 g (85 % der Theorie) an farblosen Kristallen vom Fp. 80°C.
IR (KBr): 1710 (C=O), 1655 (C=O) cm⁻¹
ESI-MS: (M-H)⁻ = 412 (berechnet: 412)

### b) (R)-N-[[4-(Acetylamino)phenyl]methyl]-N⁵-[amino(nitroimino)methyl]-N²-(diphenylacetyl)-ornithinamid

Zu der mit gestoßenem Eis/Ethanol gekühlten Lösung von 0,82 g (1,98 mMol) (R)-N⁵-[Amino(nitroimino)methyl]-N²-(diphenylacetyl)-ornithin in 10 ml Tetrahydrofuran gab man nacheinander 0,202 g (2,0 mMol) N-Methyl-morpholin und 0,273 g (2,0 mMol) Chlorkohlensäureisobutylester und nach 15 minütigem Rühren unter äußerer Kühlung 0,328 g (2,0 mMol) 4-(Aminomethyl)-acetanilid (Fp.: 126-127°C, hergestellt in 77%iger Ausbeute durch katalytische Hydrierung von 4-Cyan-acetanilid in Gegenwart von Ammoniak und Raney-Nickel). Man ließ auf Zimmertemperatur erwärmen, saugte vom ausgefallenen Niederschlag ab, dampfte das Filtrat im Vakuum ein, nahm den Rückstand in warmem Essigsäureethylester/Methanol auf, filtrierte und wusch die erhaltenen farblosen Kristalle gründlich mit Methanol.
Ausbeute 700 mg (63 % der Theorie).
IR (KBr): 1640, 1665, 1690 cm⁻¹ (C=O)
   - EI-MS:: (M+H)⁺ = 560
   (M+Na)⁺ = 582

### c) (R)-N-[[4-(Acetylamino)phenyl]methyl]-N²-(diphenylacetyl)-argininamid-acetat

Die Lösung von 0,59 g (1,054 mMol) (R)-N-[[4-Acetylamino)phenyl]methyl]-N⁵-[amino(nitroimino)methyl]-N²-(diphenylacetyl)-ornithinamid in 150 ml 80%iger wässeriger Essigsäure wurde in Gegenwart von 0,25 g Palladiummohr bei 40°C und 5 bar Wasserstoffdruck bis zur Beendigung der Wasserstoffaufnahme hydriert. Der Katalysator wurde abfiltriert, das Filtrat im Vakuum eingedampft, zweimal mit je 10 ml Wasser versetzt und abermals eingedampft. Der Rückstand wurde mit Ether, dann mit Aceton ausgekocht, schließlich mit einer Mischung aus 95 % Aceton und 5 % EtOH (v/v) digeriert, wobei man die obige Verbindung in Form farbloser Kristalle erhielt.
Fp.: 175-177°C.
Ausbeute: 0,4 g (66 % der Theorie), R_{F} = 0,60.
IR (KBr): 1653-1680 cm⁻¹ (C=O)
ESI-MS: (M+H)⁺ = 515

### Beispiel 2

### (R)-N²-(Diphenylacetyl)-N-[[4-(ethoxycarbonylamino)phenyl]-methyl]-argininamid-acetat

### a) (R)-N⁵-[Amino(nitroimino)methyl]-N²-(diphenylacetyl)N-[[4-(ethoxycarbonylamino)phenyl]methyl]-ornithinamid 5

Hergestellt analog Beispiel 1b) aus (R)-N⁵-[Amino(nitroimino)methyl]-N²-(diphenylacetyl)-ornithin, [4-(Ethoxycarbonylamino)phenyl]methylamin (Fp.: 96-98°C, hergestellt in 64%iger Ausbeute aus 4-(Ethoxycarbonylamino)benzonitril durch katalytische Hydrierung in Gegenwart von 1N HCl und 5%iger Palladium/Tierkohle als Katalysator) und Chlorkohlensäureisobutylester in einer Ausbeute von 70 % der Theorie.
Fp.: 154-156°C (Diisopropylether).
IR (KBr): 1700, 1678, 1640-1660 cm⁻¹ (C=O, C=N)
   - ESI-MS:: (M+H)⁺ = 590.
   (M+Na)⁺ = 612
   (M+K)⁺ = 628

### b) (R)-N²-(Diphenylacetyl)-N-[[4-(ethoxycarbonylamino)phenyl]methyl]-argininamid-acetat

Hergestellt analog Beispiel 1c) aus (R)-N⁵-[Amino(nitroimino)methyl)-N²-(diphenylacetyl)-N-[[4-(ethoxycarbonylamino)phenyl]methyl]-ornithinamid durch katalytische Hydrierung in Gegenwart von Palladiummohr und 80%iger wässeriger Essigsäure in einer Ausbeute von 74 % der Theorie. Farblose Kristalle vom Fp. 152-154°C. R_{f} = 0,71.
IR (KBr): 1640, 1680, 1710, 1730 cm⁻¹ (C=O, C=N)
ESI-MS: (M+H)⁺ = 545

### Beispiel 3

### (R)-N²-(Diphenylacetyl)-N-(phenylmethyl)argininamid-acetat

### a) (R)-N⁵-[Amino(nitroimino)methyl]-N²-(diphenylacetyl)N-(phenylmethyl)-ornithinamid

Hergestellt analog Beispiel 1b) aus (R)-N⁵-[Amino(nitroimino)methyl]-N²-(diphenylacetyl)-ornithin, Benzylamin und Chlorkohlensäureisobutylester in einer Ausbeute von 89 % der Theorie.
Fp.: 195-197°C (Essigsäureethylester).
IR (KBr): 1640 cm⁻¹ (C=O, C=N)
   - ESI-MS:: (M+Na)⁺ = 525
   (M+K)⁺ = 541

### b) (R)-N²-(Diphenylacetyl)-N-(phenylmethyl)-argininamidacetat

Hergestellt analog Beispiel 1c) aus (R)-N⁵-[Amino(nitroimino)methyl]-N²-(diphenylacetyl)-N-(phenylmethyl)-ornithinamid durch katalytische Hydrierung in Gegenwart von Palladiummohr und 80%iger wässeriger Essigsäure in einer Ausbeute von 97 % der Theorie. Farblose, amorphe Substanz, R_{f} = 0,75.
IR (KBr): 1665 cm⁻¹ (C=O)
ESI-MS: (M+H)⁺ = 458

### Beispiel 4

### (R)-N²-(Diphenylacetyl)-N-[(4-methylphenyl)methyl]-argininamid-acetat

### a) (R)-N⁵-[Amino(nitroimino)methyl]-N²-(diphenylacetyl)N-[(4-methylphenyl)methyl]-ornithinamid

Hergestellt analog Beispiel 1b) aus (R)-N⁵-(Amino(nitroimino)methyl]-N²-(diphenylacetyl)-ornithin, (4-Methylphenyl)methanamin und Chlorkohlensäureisobutylester in einer Ausbeute von 77 % der Theorie.
Fp.: 202-204°C (Essigsäureethylester).
IR (KBr): 1640 cm⁻¹ (C=O)
   - ESI-MS:: (M+H)⁺ = 517
   (M+Na)⁺ = 539
   (M+K)⁺ = 555

### b) (R)-N²-(Diphenylacetyl)-N-[(4-methylphenyl)methyl]-argininamid-acetat

Hergestellt analog Beispiel 1c) aus (R)-N⁵-[Amino(nitroimino)methyl]-N²-(diphenylacetyl)-N-[(4-methylphenyl)-methyl]-ornithinamid durch katalytische Hydrierung in Gegenwart von Palladiummohr und 80%iger wässeriger Essigsäure in einer Ausbeute von 77 % der Theorie.
Farblose, amorphe Substanz, R_{f}= 0,76.
IR (KBr): 1655 cm⁻¹ (C=O)
ESI-MS: (M+H)⁺ = 472

### Beispiel 5

### (R)-N-[2-(4-Hydroxyphenyl)ethyl]-N²-[[5-(2-phenylethoxy)-1H-indol-2-yl]carbonyl]-argininamid-acetat

### a) 2-Nitro-5-(2-phenylethoxy)-toluol

Zu einer aus 15 g (0,652 Mol) Natrium und 500 ml wasserfreiem Ethanol bereiteten Natriumethanolat-Lösung gab man nacheinander 100 g (0,653 Mol) 5-Hydroxy-2-nitrotoluol und 90 ml (121,95 g = 0,659 Mol) 2-Phenylethylbromid und kochte 5 Stunden unter Rückfluß. Man gab nochmals 50 ml (0,366 Mol) 2-Phenylethylbromid zu und erhitzte abermals 10 Stunden unter Rückfluß. Das Lösemittel wurde im Vakuum abdestilliert, der Rückstand in Ether aufgenommen und mehrfach mit verdünnter Natronlauge extrahiert. Die etherische Phase wurde eingeengt, der Rückstand mit 400 ml Petrolether 35/60 gründlich verrührt. Die entstandenen Kristalle wurden abgenutscht und mit Petrolether gewaschen.
Ausbeute: 95,2 g (57 % der Theorie) an schwach gelblichen Kristallen vom Fp. 70-72°C.
IR (CH₂Cl₂): 1340, 1515 cm¹ (NO₂)

### b) [2-Nitro-5-(2-phenylethoxy)phenyl]brenztraubensäure

Zu der durch Eintragen von 43,7 g (0,389 Mol) Kalium-tert.-butylat in ein Gemisch aus 420 ml wasserfreiem Ether und 162 ml wasserfreiem Ethanol erhaltenen klaren Lösung gab man 50,6 ml (54,4 g = 0,373 Mol) Oxalsäurediethylester, wobei sich eine ockerfarbene trübe Mischung bildete, und 30 Minuten später die Lösung von 95 g (0,369 Mol) 2-Nitro-5-(2-phenylethoxy)-toluol in 100 ml wasserfreiem Ether. Anschließend kochte man 4 Stunden unter Rückfluß und hielt noch 36 Stunden bei Zimmertemperatur. Der schwarz-violette Niederschlag wurde abfiltriert, gründlich mit trockenem Ether gewaschen und an der Luft getrocknet. Ausbeute an [2-Nitro-5-(2-phenylethoxy)phenyl]brenztraubensäureethylester-Kaliumsalz: 101,5 g (70 % der Theorie).

98,0 g (0,248 Mol) dieses Kaliumsalzes wurden mit 800 ml Wasser verrührt, mit verdünnter Natronlauge auf pH 8-9 eingestellt und über Nacht bei Raumtemperatur gerührt. Die Lösung wurde filtriert, das Filtrat mit konzentrierter Salzsäure vorsichtig bis zur Beendigung der Fällungsreaktion versetzt. Die ausgefallene hellgelbe Säure wurde in Dichlormethan aufgenommen, die Lösung mit Wasser gewaschen, über Natriumsulfat getrocknet und im Vakuum eingedampft. Man erhielt 87,0 g (74 % der Theorie) an schwach gelblichen Kristallen vom Fp. 100-105°C.
IR (CH₂Cl₂): 1738, 1790 cm⁻¹ (C=O)
ESI-MS: M⁺ = 329

### c) 5-(2-Phenylethoxy)-1H-indol-2-carbonsäure

15,0 g (0,0456 Mol) [2-Nitro-5-(2-phenylethoxy)phenyl]brenztraubensäure wurden in einer Lösung aus 65 ml konz. Ammoniak und 28 ml Wasser gelöst. Dazu gab man rasch eine Lösung von 85 g (0,306 Mol) Eisen(II)-sulfat-heptahydrat in 93 ml Wasser, erhitzte 1 Stunde auf dem Dampfbad und kochte 30 Minuten unter Rückfluß. Man filtrierte noch heiß und wusch den Niederschlag gründlich mit 75 ml 5%igem wässerigem Ammoniak. Die vereinigten, noch heißen Filtrate wurden mit konz. Salzsäure gegen Kongorot angesäuert. Nach dem Erkalten wurde mit Essigsäureethylester erschöpfend extrahiert und wie üblich weiter aufgearbeitet. Man erhielt 9,0 g (70 % der Theorie) an farblosen Kristallen vom Fp. 184-187°C (wässeriges Ethanol).
IR (KBr): 1685 cm⁻¹ (C=O)
MS: M⁺ = 281

### d) (R)-N²-(tert.Butoxycarbonyl)-N-[2-(4-hydroxyphenyl)ethyl]-N⁵-[amino(nitroimino)methyl]-ornithinamid

Zu der Mischung aus 1,0 g (3,13 Mol) Boc-D-Arg(NO₂)-OH, 0,57 g (3,28 mMol) Tyramin-hydrochlorid, 0,91 ml (0,66 g = 6,53 mMol) Triethylamin und 20 ml wasserfreiem Acetonitril gab man unter Rühren und äußerer Kühlung mit Eiswasser 1,05 g (3,27 mMol) TBTU. Man ließ auf Raumtemperatur erwärmen und rührte noch über Nacht unter diesen Bedingungen. Vom Niederschlag wurde abfiltriert, das Filtrat im Vakuum eingedampft, der Rückstand zwischen Wasser und Essigsäureethylester verteilt. Die Essigesterphase wurde anschließend an Kieselgel (Macherey-Nagel, 35-70 mesh ASTM) und unter Verwendung von Dichlormethan/Methanol/Cyclohexan/konz. wässeriges Ammoniak = 68/15/15/2 (v/v/v/v) säulenchromatographisch gereinigt. Man erhielt 0,8 g (58 % der Theorie) der Titelverbindung in Form eines amorphen Schaums, R_{f} 0,49 (Macherey-Nagel Polygram^{(R)} SIL G/UV₂₅₄, Fertigfolien für die DC; Fließmittel: Dichlormethan/Methanol/Cyclohexan/konz. wässeriges Ammoniak = 68/15/15/2 (v/v/v/v)).
- ESI-MS:: (M+H)⁺ = 439
(M+Na)⁺ = 461

### e) (R)-N⁵-[Amino(nitroimino)methyl]-N-(2-(4-hydroxyphenyl)-ethyl]-ornithinamid-trifluoracetat

Zu der von außen mit gestoßenem Eis/Ethanol gekühlten Lösung von 0,8 g (1,82 mMol) (R)-N⁵-[Amino(nitroimino)methyl]-N²-(tert.-butoxycarbonyl)-N-[2-(4-hydroxyphenyl)ethyl]-ornithinamid in 20 ml Dichlormethan gab man 2,5 ml Trifluoressigsäure, ließ anschließend auf Raumtemperatur erwärmen und rührte die Mischung weitere 4 Stunden bei dieser Temperatur. Die erhaltene klare Lösung wurde im Wasserstrahlvakuum eingedampft und zweimal mit je 10 ml Wasser und einmal mit 10 ml Toluol versetzt und anschließend jeweils erneut zur Trockene gebracht. Das erhaltene, amorphe (R)-N⁵-(Amino(nitroimino)methyl]-N-[2-(4-hydroxyphenyl)ethyl]-ornithinamid-trifluoracet at (0,61 g, 74 % der Theorie) vom R_{f} 0,33 (Untersuchungsbedingungen wie im Beispiel 5d) wurde ohne weitere Reinigung weiterverarbeitet.
EI-MS: (M+H)⁺ = 339; (2M+H)⁺ = 677

### f) (R)-N⁵-[Amino(nitroimino)methyl]-N-[2-(4-hydroxyphenyl)-ethyl]-N²-[[5-(2-phenylethoxy)-1H-indol-2-yl]carbonyl]-ornithinamid

Zu der Mischung aus 0,61 g (1,35 mMol) (R)-N⁵-[Amino(nitroimino)methyl]-N-[2-(4-hydroxyphenyl)ethyl]-ornithinamid-trifluoracetat, 0,52 g (1,85 mMol) 5-(2-Phenylethoxy)-1H-indol2-carbonsäure, 0,56 ml (0,407 g = 4,02 mMol) Triethylamin und 9 ml wasserfreiem Acetonitril gab man unter Rühren und äußerer Kühlung in Eiswasser 0,65 g (2,024 mMol) TBTU. Man ließ auf Raumtemperatur erwärmen und rührte noch über Nacht bei der gleichen Temperatur. Man dampfte im Vakuum ein, verteilte den Rückstand nach Zugabe von wenig Methanol zwischen Dichlormethan und Wasser und säulenchromatographierte die Dichlormethan-Phase an Kieselgel (Macherey-Nagel, 35-70 mesh ASTM; Dichlormethan/Essigsäureethylester/Methanol/Cyclohexen/ konz. wässeriges Ammoniak = 59/25/7,5/7,5/1 (v/v/v/v)).
IR (KBr): 1630 cm⁻¹ (C=O)
ESI-MS: (M-H)⁻ = 680

### g) (R)-N-[2-(4-Hydroxyphenyl)ethyl]-N²-[[5-(2-phenylethoxy)-1H-indol-2-yl]carbonyl]-argininamid-acetat

Hergestellt analog Beispiel 1c) aus (R)-N⁵-[Amino(nitroimino)methyl]-N-[2-(4-hydroxyphenyl)ethyl]-N²-[[5-(2-phenyl-ethoxy)-1H-indol-2-yl]carbonyl]-ornithinamid durch katalytische Hydrierung in Gegenwart von Palladiummohr und 80%iger wässeriger Essigsäure in einer Ausbeute von 53 % der Theorie. Farbloser Schaum vom R_{f} 0,73.
IR (KBr): 1620-1670 cm⁻¹ (C=O, C=N)
ESI-MS: (M+H)⁺ = 557

### Beispiel 6

### (R)-N²-(Diphenylacetyl)-N-(3-hydroxypropyl)-argininamidacetat

### a) (R)-N⁵-[Amino(nitroimino)methyl]-N²-(diphenylacetyl)-N-(3-hydroxypropyl)-ornithinamid

Hergestellt analog Beispiel 5d) aus (R)-N⁵-[Amino(nitroimino)methyl]-N²-(diphenylacetyl)-ornithin und 3-Aminopropanol in Gegenwart von TBTU in einer Ausbeute von 46 % der Theorie.
Fp.: 178-181°C (Dichlormethan/Methanol/Wasser).
IR (KBr): 1640, 1660 cm⁻¹ (C=O, C=N)
EI-MS: (M-H)⁻ = 469

### b) (R)-N²-(Diphenylacetyl)-N-(3-hydroxypropyl)-argininamidacetat

Hergestellt analog Beispiel 1c) aus (R)-N⁵-[Amino(nitroimino)methyl]-N²-(diphenylacetyl)-N-(3-hydroxypropyl) ornithinamid durch katalytische Hydrierung in Gegenwart von Palladiummohr und 80%iger wässeriger Essigsäure in einer Ausbeute von 73 % der Theorie.
Farblose, amorphe Substanz vom R_{f} 0,44; löslich in Wasser.
IR (KBr): 1635-1690 cm⁻¹ (C=O, C=N)
ESI-MS: (M+H)⁺ = 426

### Beispiel 7

### (R)-N-[[4-[[(Dimethylamino)carbonyl]amino]phenyl]methyl]-N²-(diphenylacetyl)-argininamid-acetat

### a) (R)-N⁵-[Amino(nitroimino)methyl]-N-[[4-[[(dimethylamino)carbonyl]amino]phenyl]methyl]-N²-(diphenylacetyl)ornithinamid

Hergestellt analog Beispiel 1b) aus (R)-N⁵-[Amino(nitroimino)methyl]-N²-(diphenylacetyl)-ornithin und N,N-Dimethyl-N'-[4-(aminomethyl)phenyl]harnstoff (Fp.: 114-115°C, hergestellt in einer Ausbeute von 83 % der Theorie aus N,N-Dimethyl-N'-(4-cyanphenyl)-harnstoff durch katalytische Hydrierung in Gegenwart von Ammoniak und Raney-Nickel) und Chlorkohlensäureisobutylester in einer Ausbeute von 70 % der Theorie.
Farblose Kristalle vom Fp. 128-130°C.
IR (KBr): 1630-1690 cm⁻¹ (C=O, C=N)
   - ESI-MS:: (M+H)⁺ = 589
   (M+Na)⁺ = 611

### b) (R)-N-[[4-[[(Dimethylamino)carbonyl]amino]phenyl]methyl]-N²-(diphenylacetyl)-argininamid-acetat

Hergestellt analog Beispiel 1c) aus (R)-N⁵-[Amino(nitroimino)methyl]-N-[[4-[[(dimethylamino)carbonyl]amino]phenyl]-methyl]-N²-(diphenylacetyl)-ornithinamid durch katalytische Hydrierung in Gegenwart von Palladiummohr und 80%iger wässeriger Essigsäure in einer Ausbeute von 68 % der Theorie. Farblose Kristalle vom Fp. 162-164°C (Aceton/Ethanol = 10/1 (v/v)) und R_{f} 0,53.
IR (KBr): 1640, 1660 cm⁻¹ (C=N, C=O)
ESI-MS: (M+H)⁺ = 544

### Beispiel 8

### N-[(4-Aminocarbonylaminophenyl)methyl]-N²-(diphenylacetyl)argininamid-acetat

### a) N⁵-[Amino(nitroimino)methyl]-N-[(4-aminocarbonylaminophenyl)methyl]-N²-(diphenylacetyl)-ornithinamid

Zu der Lösung von 702 mg (1,698 mMol) (R)-N⁵-[Amino(nitroimino)methyl]-N²-(diphenylacetyl)-ornithin, 280 mg (1,695 mMol) [4-(Aminomethyl)phenyl]harnstoff (Fp.: 151-153°C), hergestellt aus 4-Cyanphenyl-harnstoff durch katalytische Hydrierung in Gegenwart von Ammoniak und von Raney-Nickel) und 0,17 g (1,68 mMol) Triethylamin in 20 ml wasserfreiem Dimethylformamid gab man 0,55 g (1,713 mMol) TBTU und rührte die Mischung 1 Stunde bei Zimmertemperatur. Das Lösemittel wurde im Wasserstrahlvakuum abdestilliert, der Rückstand gründlich mit Wasser gewaschen und schließlich aus heißem Essigsäureethylester umkristallisiert. Man erhielt 0,5 g (53 % der Theorie) an farblosen Kristallen vom Fp. 182-183°C. IR (KBr): 1650, 1670 cm⁻¹ (C=O, C=N)

### b) N-[(4-Aminocarbonylaminophenyl)methyl]-N²-(diphenylacetyl)-argininamid-acetat

Hergestellt analog Beispiel 1c) aus N⁵-[Amino(nitroimino)methyl]-N-[(4-aminocarbonylaminophenyl)methyl]-N²-(diphenylacetyl)-ornithinamid durch katalytische Hydrierung in Gegenwart von Palladiummohr und 80%iger wässeriger Essigsäure in einer Ausbeute von 77 % der Theorie. Farblose, amorphe Substanz vom R_{f} 0,57.
IR (KBr): 1630-1690 cm⁻¹ (C=O, C=N)
ESI-MS: (M+H)⁺ = 516

### Beispiel 9

### (R)-N-[(4-Hydroxyphenyl)methyl]-N²-[[5-(2-phenylethoxy)-1H-indol-2-yl]carbonyl]-argininamid-acetat

### a) (R)-N²-(tert.-Butoxycarbonyl)-N-[(4-hydroxyphenyl)methyl]-N⁵-[amino(nitroimino)methyl]-ornithinamid

Hergestellt analog Beispiel 5d) aus Boc-D-Arg(NO₂)-OH, (4-Hydroxyphenyl)methanamin und TBTU in einer Ausbeute von 63 % der Theorie. Farblose, amorphe Substanz vom R_{f} 0,54 (Macherey-Nagel, Polygram^{(R)} SIL G/UV₂₅₄, Fertigfolien für die DC; Fließmittel: Dichlormethan/Methanol/Cyclohexan/ konz. wässeriges Ammoniak = 68/15/15/2 (v/v/v/v)).
IR (KBr): 1620, 1640, 1690, 1725 cm⁻¹ (C=N, C=O)
   - ESI-MS:: (M+H)⁺ = 425
   (M+NH₄)⁺ = 442
   (M+Na)⁺ = 447
   (2M+H)⁺ = 849
   (2M+Na)⁺ = 871.

### b) (R)-N⁵-[Amino(nitroimino)methyl]-N-[(4-hydroxyphenyl)methyl]-ornithinamid-trifluoracetat

Hergestellt analog Beispiel 5e) aus (R)-N⁵-[Amino(nitroimino)methyl]-N²-(tert.-butoxycarbonyl)-N-[(4-hydroxyphenyl)methyl]-ornithinamid und Trifluoressigsäure in einer Ausbeute von 70 % der Theorie. Farblose, amorphe Substanz vom R_{f} 0,3 (Untersuchungsbedingungen wie in Beispiel 9a). EI-MS: (M+H)⁺ = 325

### c) (R)-N⁵-[Amino(nitroimino)methyl]-N-[(4-hydroxyphenyl)methyl]-N²-[[5-(2-phenylethoxy)-1H-indol-2-yl]carbonyl]ornithinamid

Hergestellt analog Beispiel 5f) aus 5-(2-Phenylethoxy)-1H-indol-2-carbonsäure, (R)-N⁵-[Amino(nitroimino)methyl]-N-[(4-hydroxyphenyl)methyl]-ornithinamid-trifluoracetat und TBTU in einer Ausbeute von 70 % der Theorie. Farbloser, amorpher Schaum.
IR (KBr): 1630-1690 cm⁻¹ (C=N, C=O)
EI-MS: (M-H)⁻ = 586

### d) (R)-N-[(4-Hydroxyphenyl)methyl]-N²-[[5-(2-phenylethoxy)-1H-indol-2-yl]carbonyl]-argininamid-acetat

Hergestellt analog Beispiel 1c) aus (R)-N⁵-[Amino(nitroimino)methyl]-N-[(4-hydroxyphenyl)methyl]-N²-[[5-(2-phenylethoxy)-1H-indol-2-yl]carbonyl]-ornithinamid durch katalytische Hydrierung in Gegenwart von Palladiummohr und 80%iger wässeriger Essigsäure in einer Ausbeute von 92 % der Theorie. Farblose Kristalle vom Fp. 106-109°C (Ethanol und Diisopropylether) und R_{f} 0,70.
IR (KBr): 1630-1690 cm⁻¹ (C=N, C=O)
ESI-MS: (M+H)⁺ = 543

### Beispiel 10

### (R)-N²-(Diphenylacetyl)-N-(4-hydroxybutyl)-argininamidacetat

### a) (R)-N⁵-[Amino(nitroimino)methyl]-N²-(diphenylacetyl)-N-(4-hydroxybutyl)-ornithinamid

Hergestellt analog Beispiel 1b) aus (R)-N⁵-[Amino(nitroimino)methyl]-N²-(diphenylacetyl)-ornithin, 4-Amino-1-butanol und Chlorkohlensäureisobutylester in einer Ausbeute von 80 % der Theorie. Farblose Kristalle vom Fp. 136-138°C (Dichlormethan).
IR (KBr): 1640 cm⁻¹ (C=O, C=N)
   - ESI-MS:: (M+H)⁺ = 485
   (M+Na)⁺ = 507
   (M+K)⁺ = 523
   (2M+Na)⁺ = 991

### b) (R)-N²-(Diphenylacetyl)-N-(4-hydroxybutyl)-argininamidacetat

Hergestellt analog Beispiel 1c) aus (R)-N⁵-[Amino(nitroimino)methyl]-N²-(diphenylacetyl)-N-(4-hydroxybutyl) ornithinamid durch katalytische Hydrierung in Gegenwart von Palladiummohr und 80%iger wässeriger Essigsäure in einer Ausbeute von 75 % der Theorie.
Farblose, amorphe Substanz vom R_{f} 0,47.
IR (KBr): 1630-1690 cm⁻¹ (C=N, C=O)
ESI-MS: (M+H)⁺ = 440

### Beispiel 11

### (R)-N²-(Diphenylacetyl)-N-(5-hydroxypentyl)-argininamidacetat

### a) (R)-N⁵-[Amino(nitroimino)methyl]-N²-(diphenylacetyl)N-(5-hydroxypentyl)-ornithinamid

Hergestellt analog Beispiel 1b) aus (R)-N⁵-[Amino(nitroimino)methyl]-N²-(diphenylacetyl)-ornithin, 5-Amino-1-pentanol und Chlorkohlensäureisobutylester in einer Ausbeute von 67 % der Theorie.
Farblose Kristalle vom Fp. 168-170°C.
IR (KBr): 1640 cm⁻¹ (C=N, C=O)
   - ESI-MS:: (M+H)⁺ = 499
   (M+Na)⁺ = 521
   (M+K)⁺ = 537

### b) (R)-N²-(Diphenylacetyl)-N-(5-hydroxypentyl)-argininamidacetat

Hergestellt analog Beispiel 1c) aus (R)-N⁵-[Amino(nitroimino)methyl]-N²-(diphenylacetyl)-N-(5-hydroxypentyl)ornithinamid durch katalytische Hydrierung in Gegenwart von Palladiummohr und 80%iger wässeriger Essigsäure in einer Ausbeute von 100 % der Theorie.
Farblose, amorphe Substanz vom R_{f} 0,52.
IR (KBr): 1640, 1690 cm⁻¹ (C=N, C=O)
ESI-MS: (M+H)⁺ = 454

### Beispiel 12

### (R)-N²-(Diphenylacetyl)-N-[(4-fluorphenyl)methyl]-argininamid-acetat

### a) (R)-N⁵-[Amino(nitroimino)methyl]-N²-(diphenylacetyl)-N-[(4-fluorphenyl)methyl]-ornithinamid

Hergestellt analog Beispiel 1b), jedoch unter Verwendung von Acetonitril als Lösemittel an Stelle von Tetrahydrofuran, aus (R)-N⁵-[Amino(nitroimino)methyl]-N²-(diphenylacetyl)ornithin, (4-Fluorphenyl)methanamin und Chlorkohlensäureisobutylester in einer Ausbeute von 68 % der Theorie.
Farblose Kristalle vom Fp. 124-126°C (Essigsäureethylester/ tert.-Butyl-methylether).
IR (KBr): 1635-1690 cm⁻¹ (C=N, C=O)
   - ESI-MS:: (M+H)⁺ = 521.
   (M+Na)⁺ = 543
   (M+K)⁺ = 559

### b) (R)-N²-(Diphenylacetyl)-N-[(4-fluorphenyl)methyl]argininamid-acetat

Hergestellt analog Beispiel 1c) aus (R)-N⁵-[Amino(nitroimino)methyl]-N²-(diphenylacetyl)-N-[(4-fluorphenyl)methyl]-ornithinamid durch katalytische Hydrierung in Gegenwart von Palladiummohr und 80%iger wässeriger Essigsäure in einer Ausbeute von 90 % der Theorie.
Farblose, amorphe Substanz vom R_{f} 0,73.
IR (CH₂Cl₂): 1630-1690 cm⁻¹ (C=N, C=O)
ESI-MS: (M+H)⁺ = 476

### Beispiel 13

### N²-(Diphenylacetyl)-N-[2-(1H-indol-3-yl)ethyl]-argininamidacetat

### a) N⁵-[Amino(nitroimino)methyl]-N²-(diphenylacetyl)-N-[2-(1H-indol-3-yl)ethyl]-ornithinamid

Zu der eisgekühlten Mischung aus 0,82 g (1,983 mMol) (R)-N⁵-[Amino(nitroimino)methyl]-N²-(diphenylacetyl)-ornithin, 0,39 g (1,993 mMol) Tryptaminhydrochlorid, 0,20 g (1,976 mMol) Triethylamin und 20 ml wasserfreiem Tetrahydrofuran gab man 0,41 g (1,987 mMol) N,N'-Dicyclohexylcarbodiimid und 0,27 g (1,998 mMol) HOBt und bewahrte die Mischung anschließend 14 Stunden bei einer Temperatur von 0°C bis +5°C auf. Vom kristallinen Niederschlag wurde abgenutscht, das Filtrat im Vakuum vom Lösemittel befreit, der verbleibende Rückstand zwischen Wasser und 10 ml Essigsäureethylester verteilt und die Essigester-Phase nach nochmaligen Waschen mit Wasser mit Natriumsulfat getrocknet und anschließend 8 Stunden bei Raumtemperatur belassen. Der ausgefallene kristalline Niederschlag wurde abgenutscht, mit wenig Essigsäureethylester gewaschen, dann mit 10 ml Methanol aufgekocht, schließlich abgenutscht und mit wenig Diethylether gewaschen. Man erhielt 0,40 g (36 % der Theorie) an farblosen Kristallen vom Fp. 176-178°C.
IR (KBr): 1640 cm⁻¹ (C=O, C=N)
   - ESI-MS:: (M+H)⁺ = 556
   (M+Na)⁺ = 578

### b) N²-(Diphenylacetyl)-N-[2-(1H-indOl-3-yl)ethyl]-argininamid-acetat

Hergestellt analog Beispiel 1c) aus N⁵-[Amino(nitroimino)methyl]-N²-(diphenylacetyl)-N-[2-(1H-indol-3-yl)ethyl]ornithinamid durch katalytische Hydrierung in Gegenwart von Palladiummohr und 80%iger wässeriger Essigsäure in einer Ausbeute von 67 % der Theorie.
Farblose, amorphe Substanz vom R_{f} 0,78.
IR (KBr): 1630-1690 cm⁻¹ (C=N, C=O)
ESI-MS: (M+H)⁺ = 511

### Beispiel 14

### (R)-N-[(4-Bromphenyl)methyl]-N²-(diphenylacetyl)-argininamid-hydrochlorid

### a) (R)-N⁵-[Amino(nitroimino)methyl]-N-[(4-bromphenyl)methyl-N²-(diphenylacetyl)-ornithinamid

Hergestellt analog Beispiel 12a) aus (R)-N⁵-[Amino(nitroimino)methyl]-N²-(diphenylacetyl)-ornithin, 4-Bromphenylmethanamin und Chlorkohlensäureisobutylester in einer Ausbeute von 73 % der Theorie.
Farblose Kristalle vom Fp. 226-228°C.
IR (KBr): 1645 cm⁻¹ (Amid-C=O)
   - ESI-MS:: (M+H)⁺ = 581/583 (Br)
   (M+Na)⁺ = 603/605 (Br)

### b) (R)-N-[(4-Bromphenyl)methyl]-N²-(diphenylacetyl)argininamid-hydrochlorid

0,76 g (1,307 mMol) (R)-N⁵-[Amino(nitroimino)methyl]-N-[(4-bromphenyl)methyl]-N²-(diphenylacetyl)-ornithinamid wurden in 23 ml 60%iger wässeriger Ameisensäure gelöst, mit 2,0 g (8,864 mMol) Zinn(II)-chlorid-dihydrat versetzt und 10 Minuten lang auf +50°C erwärmt. Man fügte 20 ml Ameisensäure zu, hielt 3 Stunden bei 50°C, gab nochmals 1,0 g (4,432 mMol) Zinn(II)-chlorid-dihydrat zu und erhitzte weitere 5 Stunden auf +50°C. Dann wurden Wasser und Ameisensäure im Vakuum und bei einer Badtemperatur von maximal +50°C abdestilliert. Der verbleibende zähviskose Rückstand wurde sorgfältig mit Wasser digeriert, dann abgenutscht und erneut mit Wasser gewaschen, dann an der Luft getrocknet. Das erhaltene feste Material wurde mit Acetonitril erschöpfend ausgekocht. Die vereinigten Acetonitril-Extrakte hinterließen nach dem Eindampfen 0,8 g einer porösen, fast farblosen Substanz, die in 3 ml eines Gemisches aus Butanol/Eisessig/Wasser (4/1/1 (v/v/v)) aufgenommen wurde. Der nach 1-stündigem Stehen bei Zimmertemperatur entstandene Brei wurde auf 0°C gekühlt, dann abgenutscht und nacheinander mit 1 ml eiskaltem Butanol/ Eisessig/Wasser (4/1/1 (v/v/v))-Gemisch, dann mit 2 ml Wasser sorgfältig gewaschen, zuletzt im Vakuum über Diphosphorpentoxid getrocknet. Man erhielt 0,28 g (37 % der Theorie) an farblosen Kristallen vom Fp. 135-138°C und R_{f} 0,73.
- IR (KBr):: 1680 cm⁻¹ (Amidin-C=N)
1635 cm⁻¹, 1655 cm⁻¹(Amid-C=O)
ESI-MS: (M+H)⁺ = 536/538 (Br)

### Beispiel 15

### (R)-N²-(Diphenylacetyl)-N-(2-phenylethyl)-argininamidacetat

### a) (R)-N⁵-[Amino(nitroimino)methyl]-N²-(diphenylacetyl)-N-(2-phenylethyl)-ornithinamid

Hergestellt analog Beispiel 12a) aus (R)-N⁵-(Amino(nitroimino)methyl]-N²-(diphenylacetyl)-ornithin, 2-Phenylethylamin und Chlorkohlensäureisobutylester in einer Ausbeute von 84 % der Theorie.
Farblose Kristalle vom Fp. 178-181°C (Essigsäureethylester).
IR (KBr): 1675 (Amidin-C=N), 1660, 1625 cm⁻¹ (Amid-C=O)
   - ESI-MS:: (M+H)⁺ = 517
   (M+Na)⁺ = 539
   (M+K)⁺ = 555

### b) (R)-N²-(Diphenylacetyl)-N-(2-phenylethyl)-argininamidacetat

Hergestellt analog Beispiel 1c) aus (R)-N⁵-[Amino(nitroimino)methyl]-N²-(diphenylacetyl)-N-(2-phenylethyl)-orni thinamid durch katalytische Hydrierung in Gegenwart von Palladiummohr und 80%iger wässeriger Essigsäure in einer Ausbeute von 100 % der Theorie.
Farblose, amorphe Substanz vom R_{f} 0,75.
IR (CH₂Cl₂): 1630-1690 cm⁻¹ (C=O, C=N)
ESI-MS: (M+H)⁺ = 472

### Beispiel 16

### (R)-N²-([1,1'-Biphenyl]-4-ylacetyl)-N-[(4-hydroxyphenyl)methyl]-argininamid-acetat

### a) (R)-N⁵-[Amino(nitroimino)methyl]-N²-([1,1'-biphenyl]-4-ylacetyl)-N-[(4-hydroxyphenyl)methyl]-ornithinamid

Hergestellt analog Beispiel 13a) aus [1,1'-Biphenyl]-4-essigsäure und (R)-N⁵-[Amino(nitroimino)methyl]-N-[(4-hydroxyphenyl)methyl]-ornithinamid in Gegenwart von N,N'-Dicyclohexylcarbodiimid und HOBt in einer Ausbeute von 50 % der Theorie.
Farblose Kristalle vom Fp. 205-210°C (Z.).
IR (KBr): 1615, 1635, 1625 cm⁻¹ (Amid-C=O)
   - ESI-MS:: (M+H)⁺ = 519
   (M+Na)⁺ = 541
   (M+K)⁺ = 557

### b) (R)-N²-([1,1'-Biphenyl]-4-ylacetyl)-N-[(4-hydroxyphenyl)methyl]-argininamid-acetat

Hergestellt analog Beispiel 1c) aus (R)-N⁵-[Amino(nitroimino)methyl]-N²-([1,1'-biphenyl]-4-ylacetyl)-N-[(4-hydroxyphenyl)methyl)-ornithinamid durch katalytische Hydrierung in Gegenwart von Palladiummohr und 80%iger wässeriger Essigsäure in einer Ausbeute von 46 % der Theorie.
Farblose Kristalle vom Fp. 159-163°C (Isopropanol) und R_{f} 0,70.
IR (KBr): 1640 cm⁻¹ (C=O)
ESI-MS: (M+H)⁺ = 474

### Beispiel 17

### N²-(Diphenylacetyl)-N-[(4-methansulfonylaminophenyl)methyl]-argininamid-acetat

### a) N⁵-[Amino(nitroimino)methyl]-N²-(diphenylacetyl)-N-[(4-methansulfonylaminophenyl)methyl]-ornithinamid

Hergestellt analog Beispiel 8a) aus (R)-N⁵-(Amino(nitroimino)methyl]-N²-(diphenylacetyl)-ornithin und (4-Methansulfonylaminophenyl)methanamin (Fp.: 258°C (Z.), hergestellt aus 4-Aminobenzonitril über 4-Cyan-methansulfonanilid vom Fp. 195-196°C) und in Gegenwart von TBTU in einer Ausbeute von 44 % der Theorie. Fp.: 178-180°C (Ethanol).
IR (KBr): 1642 cm⁻¹ (Amid-C=O)
   - ESI-MS:: (M+H)⁺ = 596
   (M+Na)⁺ = 618
   (M+K)⁺ = 634

### b) N²-(Diphenylacetyl)-N-[(4-methansulfonylaminophenyl)methyl]-argininamid-acetat

Hergestellt analog Beispiel 1c) aus N⁵-[Amino(nitroimino)methyl]-N²-(diphenylacetyl)-N-[(4-methansulfonylaminophenyl)methyl]-ornithinamid durch katalytische Hydrierung in Gegenwart von Palladiummohr und 80%iger wässeriger Essigsäure in einer Ausbeute von 54 % der Theorie.
Farblose, amorphe Substanz vom R_{f} 0,60.
IR (KBr): 1630-1690 cm⁻¹ (C=O, C=N)
ESI-MS: (M+H)⁺ = 551

### Beispiel 18

### Optisch aktives Diastereomerengemisch von N²-(α-Cyclopentyl-phenylacetyl)-N-[(4-hydroxyphenyl)methyl]-D-argininamid-acetat

### a) N⁵-[Amino(nitroimino)methyl]-N²-(α-cyclopentyl-phenylacetyl)-N-[(4-hydroxyphenyl)methyl]-D-ornithinamid (Diastereomerengemisch)

Hergestellt analog Beispiel 8a) aus (R)-N-[(4-Hydroxyphenyl)methyl]-N⁵-[amino(nitroimino)methyl]-ornithinamid und racemischer α-Cyclopentyl-phenylessigsäure und in Gegenwart von TBTU in einer Ausbeute von 69 % der Theorie. Farblose, amorphe Substanz.
IR (KBr): 1630-1690 cm⁻¹ (C=O, C=N)
   - ESI-MS:: (M+H)⁺ = 511
   (M+Na)⁺ = 533

### b) N²-(α-Cyclopentyl-phenylacetyl)-N-[(4-hydroxyphenyl)methyl]-D-argininamid-acetat (Diastereomerengemisch)

Hergestellt analog Beispiel 1c) aus N⁵-[Amino(nitroimino)methyl]-N²-(α-cyclopentyl-phenylacetyl)-N-[(4-hydroxyphenyl)methyl]-D-ornithinamid (Diastereomerengemisch) durch katalytische Hydrierung in Gegenwart von Palladiummohr und 80%iger wässeriger Essigsäure in einer Ausbeute von 100 % der Theorie.
Farblose, amorphe Substanz vom R_{f} 0,76.
IR (KBr): 1630-1690 cm⁻¹ (C=O, C=N)
ESI-MS: (M+H)⁺ = 466

### Beispiel 19

### (R)-N-[(4-Hydroxyphenyl)methyl]-N²-(tricyclo[3.3.1.1³'⁷]-dec-1-ylacetyl)-argininamid-acetat

### a) (R)-N⁵-[Amino(nitroimino)methyl]-N-[(4-hydroxyphenyl)methyl]-N²-(tricyclo[3.3.1.1^{3,7}]dec-1-ylacetyl)-ornithinamid

Hergestellt analog Beispiel 8a) aus (R)-N-[(4-Hydroxyphenyl)methyl]-N⁵-[amino(nitroimino)methyl]-ornithinamid, Tricyclo[3.3.1.1³'⁷]decan-1-essigsäure und TBTU in einer Ausbeute von 85 % der Theorie.
Farblose Kristalle vom Fp. 100-106°C.
IR (KBr): 1620-1690 cm⁻¹ (C=O, C=N)
   - ESI-MS:: (M+Na)⁺ = 509
   (M+K)⁺ = 525

### b) (R)-N-[(4-Hydroxyphenyl)methyl]-N²-(tricyclo[3.3.1.1³'⁷]-dec-1-ylacetyl)-argininamid-acetat

Hergestellt analog Beispiel 1c) aus (R)-N⁵-[Amino(nitroimino)methyl]-N-[(4-hydroxyphenyl)methyl]-N²-(tricyclo[3.3.1.1^{3,7}]dec-1-ylacetyl)-ornithinamid durch katalytische Hydrierung in Gegenwart von Palladiummohr und 80%iger wässeriger Essigsäure in einer Ausbeute von 90 % der Theorie. Farblose, amorphe Substanz vom R_{f} 0,72.
IR (KBr): 1620-1690 cm⁻¹ (C=O, C=N)
ESI-MS: (M+H)⁺ = 456

### Beispiel 20

### N-[[4-(Dimethylamino)phenyl]methyl]-N²-(diphenylacetyl)argininamid-diacetat und N²-(Diphenylacetyl)-N-[(4-(methylamino)cyclohexyl]methyl]argininamid-diacetat (Diastereomerengemisch)

### a) N⁵-[Amino(nitroimino)methyl]-N²-(diphenylacetyl)-N-[[4-(dimethylamino)phenyl]methyl]-ornithinamid

Hergestellt analog Beispiel 13a) aus (R)-N⁵-[Amino(nitroimino)methyl]-N²-(diphenylacetyl)-ornithin, [4-(Dimethylamino)phenyl]methanamin, N,N'-Dicyclohexylcarbodiimid und HOBt in einer Ausbeute von 76 % der Theorie.
Farblose Kristalle vom Fp. 221-223°C.
IR (KBr): 1640 cm⁻¹ (Amid-C=O)
   - ESI-MS:: (M+H)⁺ = 546
   (M+Na)⁺ = 568
   (2M+Na)⁺ = 1113

### b) N-[[4-(Dimethylamino)phenyl]methyl]-N²-(diphenylacetyl)argininamid-diacetat und N²-(Diphenylacetyl)-N-[[4-(methylamino)cyclohexyl]methyl]-argininamid-diacetat

Hergestellt analog Beispiel 1c) aus N⁵-[Amino(nitroimino)methyl]-N²-(diphenylacetyl)-N-[[4-(dimethylamino)phenyl]methyl]-ornithinamid durch katalytische Hydrierung in Gegenwart von Palladiummohr und 80%iger wässeriger Essigsäure.

Das rohe Produkt wurde säulenchromatographisch (MN-Kieselgel 60, Macherey-Nagel, 70-230 mesh ASTM; Fließmittel: Butanol/ Eisessig/Wasser = 4/1/1 (v/v/v)) in 2 Fraktionen zerlegt. Das Produkt mit dem höheren R_{f}-Wert wurde als N²-(Diphenylacetyl)-N-[[4-(methylamino)cyclohexyl]methyl]-argininamid-diacetat (Diastereomerengemisch) identifiziert und mit einer Ausbeute von 15 % der Theorie erhalten.
Farblose, amorphe Substanz vom R_{f} 0,63.
IR (KBr): 1630-1690 cm⁻¹ (C=O, C=N)
EI-MS: (M+H)⁺ = 493

Das Hauptprodukt, N-[(4-(Dimethylamino)phenyl]methyl]-N²-(diphenylacetyl)-argininamid-diacetat, wurde in einer Ausbeute von 22 % der Theorie isoliert.
Farblose, amorphe Substanz vom R_{f} 0,50.
IR (KBr): 1630-1690 cm⁻¹ (C=O, C=N)
EI-MS: (M+H)⁺ = 501

### Beispiel 21

### (R)-N-([1,1'-Biphenyl]-4-ylmethyl)-N²-(diphenylacetyl)argininamid-acetat

### a) (R)-N⁵-[Amino(nitroimino)methyl]-N-([1,1'-biphenyl]-4-ylmethyl)-N²-(diphenylacetyl)-ornithinamid

Hergestellt analog Beispiel 12a) aus (R)-N⁵-[Amino(nitroimino)methyl]-N²-(diphenylacetyl)-ornithin, [1,1'-Biphenyl]-4-methanamin (hergestellt aus [1,1'-Biphenyl]-4-carbonitril durch katalytische Hydrierung in Gegenwart von Raney-Nickel und Ammoniak) und Chlorkohlensäureisobutylester in einer Ausbeute von 73 % der Theorie. Farblose Kristalle vom Fp. 100-102°C (Essigsäureethylester/tert.-Butyl-methylether).
IR (KBr): 1640 cm⁻¹ (Amid-C=O)
   - ESI-MS:: (M+H)⁺ = 579
   (M+Na)⁺ = 601

### b) (R)-N⁵-([1,1'-Biphenyl]-4-ylmethyl)-N²-(diphenylacetyl)-argininamid-acetat

Hergestellt analog Beispiel 1c) aus (R)-N⁵-[Amino(nitroimino)methyl]-N-([1,1'-biphenyl]-4-ylmethyl)-N²-(diphenylacetyl)-ornithinamid durch katalytische Hydrierung in Gegenwart von Palladiummohr und 80%iger wässeriger Essigsäure in einer Ausbeute von 83 % der Theorie.
Farbloses, amorphes Produkt vom R_{f} 0,76.
IR (CH₂Cl₂): 1660 cm⁻¹ (C=O)
ESI-MS: (M+H)⁺ = 534

### Beispiel 22

### (R)-N²-(Diphenylacetyl)-N-[[4-(hydroxymethyl)phenyl]methyl]-argininamid-acetat

### a) (R)-N⁵-[Amino(nitroimino)methyl]-N²-(diphenylacetyl)-N-[[4-(hydroxymethyl)phenyl]methyl]-ornithinamid

Hergestellt analog Beispiel 12a) aus (R)-N⁵-[Amino(nitroimino)methyl]-N²-(diphenylacetyl)-ornithin, [4-(Hydroxymethyl)phenyl]methanamin (Fp.: 75-77°C, hergestellt aus 4-Cyan-benzaldehyd durch Reduktion mit Lithiumaluminiumhydrid) und Chlorkohlensäureisobutylester in einer Ausbeute von 77 % der Theorie.
Farblose, amorphe Substanz.
IR (KBr): 1620-1690 cm⁻¹ (C=O, C=N)
   - EI-MS:: (M+H)⁺ = 533
   (M+Na)⁺ = 555

### b) (R)-N²-(Diphenylacetyl)-N-[[4-(hydroxymethyl)phenyl]methyl]-argininamid-acetat

Hergestellt analog Beispiel 14b) aus (R)-N⁵-[Amino(nitroimino)methyl]-N²-(diphenylacetyl)-N-[[4-hydroxymethyl)phenyl]methyl]-ornithinamid durch Reduktion mit Zinn(II)-chlorid-dihydrat in Gegenwart von 60%iger wässeriger Ameisensäure in einer Ausbeute von 10 % der Theorie.
Farblose, amorphe Substanz vom R_{f} 0,62.
IR (KBr): 1630-1690 cm⁻¹ (C=O, C=N)
ESI-MS: (M+H)⁺ = 488

### Beispiel 23

### (R)-N²-(Diphenylacetyl)-N-[[4-(1-oxoethyl)phenyl]methyl]argininamid-hydrochlorid

### a) (R)-N-[[4-(1-Oxoethyl)phenyl]methyl]-N⁵-[amino(nitroimino)methyl]-N²-(tert.-butoxycarbonyl)-ornithinamid

Hergestellt analog Beispiel 5d) aus Boc-D-Arg(NO₂)-OH, [4-(1-Oxoethyl)phenyl]methanamin-hydrochlorid (W. Korytnyk, N. Angelino, C. Dave und L. Caballas, J. Med. Chem. 21, 507-513 [1978]) und TBTU in einer Ausbeute von 79 % der Theorie.
Farblose, amorphe Substanz.
IR (CH₂Cl₂): 1680 cm⁻¹ (C=N, C=O)
Schulter bei 1715 cm¹ (Ester-C=O)

### b) (R)-N⁵-[Amino(nitroimino)methyl]-N-[[4-(1-oxoethyl)phenyl]methyl]-ornithinamid

Hergestellt analog Beispiel 5e) aus (R)-N⁵-[Amino(nitroimino)methyl]-N²-(tert.-butoxycarbonyl)-N-[[4-(1-oxoethyl)phenyl]methyl]-ornithinamid durch Behandlung mit Trifluoressigsäure in Dichlormethan. Das so erhaltene Salz wurde in Wasser gelöst, diese Lösung ammoniakalisch gestellt und die gesuchte Base schließlich aus der wässerigen Lösung durch Sättigen mit Kochsalz ausgefällt. Das erhaltene Produkt wurde im Vakuum über di-Phosphorpentoxid getrocknet und ohne weitere Reinigung in der folgenden Stufe verwendet.

### c) (R)-N⁵-[Amino(nitroimino)methyl]-N²-(diphenylacetyl)-N-[[4-(1-oxoethyl)phenyl]methyl]-ornithinamid

Hergestellt analog Beispiel 5f) aus (R)-N⁵-[Amino(nitroimino)methyl]-N-[[4-(1-oxoethyl)phenyl]methyl]-ornithinamid, Diphenylessigsäure und TBTU in einer Ausbeute von 48 % der Theorie.
Farblose Kristalle vom Fp. 208-210°C (Z.).
IR (KBr): 1640, 1660, 1680 cm⁻¹ (C=O, C=N)

### d) (R)-N²-(Diphenylacetyl)-N-[[4-(1-oxoethyl)phenyl]methyl]-argininamid-hydrochlorid

Hergestellt analog Beispiel 14b) aus (R)-N⁵-[Amino(nitroimino)methyl]-N²-(diphenylacetyl)-N-[[4-(1-oxoethyl)phenyl]methyl]-ornithinamid durch Reduktion mit Zinn(II)-chlorid-dihydrat in Gegenwart von 60%iger wässeriger Ameisensäure in einer Ausbeute von 23 % der Theorie.
Farblose, amorphe Substanz vom R_{f} 0,67.
IR (KBr): 1630-1700 cm⁻¹ (C=O, C=N)
EI-MS: (M+H)⁺ = 500

### Beispiel 24

### (R)-N-[(4-Chlorphenyl)methyl]-N²-(diphenylacetyl)-argininamid-hydrochlorid

### a) (R)-N-[(4-Chlorphenyl)methyl]-N⁵-[amino(nitroimino)methyl]-N²-(tert.-butoxycarbonyl)-ornithinamid

Hergestellt analog Beispiel 5d) aus Boc-D-Arg(NO₂)-OH, (4-Chlorphenyl)methanamin und TBTU in einer Ausbeute von 87 % der Theorie.
Farblose, amorphe Substanz.
- IR (CH₂Cl₂):: 1630 (C=O), 1675 (C=O oder C=N),
1715 (Schulter, Ester-C=O) cm⁻¹

### b) (R)-N⁵-[Amino(nitroimino)methyl]-N-[(4-chlorphenyl)methyl]-ornithinamid

Hergestellt analog Beispiel 23b) aus (R)-N⁵-[Amino(nitroimino)methyl]-N²-(tert.-butoxycarbonyl)-N-[(4-chlorphenyl)methyl]-ornithinamid durch Behandlung mit Trifluoressigsäure in Dichlormethan.
Ausbeute: 83 % der Theorie.
   Farblose, amorphe Verbindung, die ohne völlige Reinigung weiterverarbeitet wurde.

### c) (R)-N⁵-[Amino(nitroimino)methyl]-N-[(4-chlorphenyl)methyl]-N²-(diphenylacetyl)-ornithinamid

Hergestellt analog Beispiel 5f) aus (R)-N⁵-[Amino(nitroimino)methyl]-N-[(4-chlorphenyl)methyl]-ornithinamid, Diphenylessigsäure und TBTU in einer Ausbeute von 64 % der Theorie.
Farblose Kristalle vom Fp. 212-215°C (Essigsäureethylester).
IR (KBr): 1645 cm⁻¹ (C=O)

### d) (R)-N-[(4-Chlorphenyl)methyl]-N²-(diphenylacetyl)argininamid-hydrochlorid

Hergestellt analog Beispiel 14b) aus (R)-N⁵-[Amino(nitroimino)methyl]-N-[(4-chlorphenyl)methyl]-N²-(diphenylacetyl)-ornithinamid durch Reduktion mit Zinn(II)-chlorid-dihydrat in Gegenwart von 60%iger wässeriger Ameisensäure in einer Ausbeute von 72 % der Theorie.
Farblose, amorphe Substanz vom R_{f} 0,74.
IR (KBr): 1620-1690 cm⁻¹ (C=O, C=N)
ESI-MS: (M+H)⁺ = 492/494 (Cl)

### Beispiel 25

### (R)-N²-(Diphenylacetyl)-N-[4-(hydroxymethyl)phenyl]-argininamid-hydrochlorid-hydrat

### a) (R)-N⁵-[Amino(nitroimino)methyl]-N²-(diphenylacetyl)N-[4-(hydroxymethyl)phenyl]-ornithinamid

Hergestellt analog Beispiel 12a) aus (R)-N⁵-[Amino(nitroimino)methyl]-N²-(diphenylacetyl)-ornithin, (4-Aminophenyl)methanol und Chlorkohlensäureisobutylester in einer Ausbeute von 86 % der Theorie.
Farblose Kristalle vom Fp. 239-240°C und R_{f} 0,32 (Macherey-Nagel, Polygram^{(R)} SIL G/UV₂₅₄, Fertigfolien für die DC; Fließmittel: Dichlormethan/Methanol/Cyclohexan/Essigsäureethylester/konz. wässeriges Ammoniak = 66/13/13/6/2, (v/v/v/v/v).

### b) (R)-N²-(Diphenylacetyl)-N-[4-(hydroxymethyl)phenyl]argininamid-hydrochlorid-hydrat

Die Mischung von 1,2 g (2,34 mMol) (R)-N⁵-[Amino(nitroimino)methyl]-N²-(diphenylacetyl)-N-[4-(hydroxymethyl)phenyl]-ornithinamid, 200 ml Methanol und 1,6 g 10%igem Palladium auf Tierkohle wurde bei 40°C und 5 bar Wasserstoffdruck bis zur Beendigung der Wasserstoffaufnahme hydriert. Der Katalysator wurde abfiltriert, das Filtrat im Vakuum eingedampft, der Rückstand in Wasser aufgenommen und abgenutscht. Der gallertartige Filterrückstand wurde erneut in 30 ml Wasser suspendiert, mit 2N Salzsäure angesäuert und rach Zugabe von nochmals 30 ml Wasser 1 Stunde gerührt. Der zwischenzeitlich entstandene Niederschlag wurde gesammelt und aus Acetonitril umkristallisiert. Man erhielt 0,2 g (16 % der Theorie) an farblosen Kristallen vom Fp. 138-141°C und R_{f} 0,62.
R (KBr): 1655 cm⁻¹ (C=O)
EI-MS: (M+H)⁺ = 474

### Beispiel 26

### (R)-N²-(Diphenylacetyl)-N-(4-hydroxy-2-butin-1-yl)-argininamid-acetat-hydrat

### a) (R)-N⁵-(Amino(nitroimino)methyl]-N²-(diphenylacetyl)N-(4-hydroxy-2-butin-1-yl)-ornithinamid

Hergestellt analog Beispiel 12a) aus (R)-N⁵-[Amino(nitroimino)methyl]-N²-(diphenylacetyl)-ornithin und 4-Amino-2-butin-1-ol (Fp.: 167-169°C, hergestellt durch Umsetzung von 4-Chlor-2-butin-1-ol mit konz. wässerigem Ammoniak bei 100°C) in Gegenwart von Chlorkohlensäureisobutylester in einer Ausbeute von 17 % der Theorie.
Farblose Kristalle vom Fp. 151-153°C (Acetonitril).
IR (KBr): 3390, 3290 (NH), 1645 (C=O) cm⁻¹
   - ESI-MS:: (M+H)⁺ = 481
   (M+Na)⁺ = 503
   (M+K)⁺ = 519

### b) (R)-N²-(Diphenylacetyl)-N-(4-hydroxy-2-butin-1-yl)argininamid-acetat-hydrat

Hergestellt analog Beispiel 14b) aus (R)-N⁵-[Amino(nitroimino)methyl]-N²-(diphenylacetyl)-N-(4-hydroxy-2-butin- 1-yl)-ornithinamid durch Reduktion mit Zinn(II)-chlorid-dihydrat in Gegenwart von 60%iger wässeriger Ameisensäure in einer Ausbeute von 9 % der Theorie.
Farblose, amorphe Substanz vom R_{f} 0,54.
IR (KBr): 1655 cm⁻¹ (C=O)
ESI-MS: (M+H)⁺ = 436

### Beispiel 27

### (R,S)-N²-(Diphenylacetyl)-N⁶-[ethylamino(ethylimino)methyl]-N-[(4-hydroxyphenyl)methyl]-lysinamid-dihydroiodid

### a) (R,S)-N²-(Diphenylacetyl)-N⁶-[(phenylmethoxy)carbonyl]-lysin

Hergestellt analog Beispiel 1a) aus Diphenylacetylchlorid und rac. N⁶-(Phenylmethoxycarbonyl)-lysin in Gegenwart von Natronlauge. Man erhielt 81 % der Theorie an farblosen Kristallen vom Fp. 98-100°C (Diisopropylether).
- IR (KBr):: 3235 (NH), 1735 (Ester-C=O), 1715 (Säure-C=O),
1650 (Amid-C=O) cm⁻¹

### b) (R,S)-N²-(Diphenylacetyl)-N-[(4-hydroxyphenyl)methyl]-N⁶-[(phenylmethoxy)carbonyl]-lysinamid

Hergestellt analog Beispiel 8a) aus (R,S)-N²-(Diphenylacetyl)-N⁶-[(phenylmethoxy)carbonyl]-lysin und (4-Hydroxyphenyl)methanamin in Gegenwart von TBTU in einer Ausbeute von 88 % der Theorie.
Farblose Kristalle vom Fp. 86-92°C (Dichlormethan/Methanol = 95/5 (v/v)) und R_{f} 0,78 (Macherey-Nagel, Polygram^{(R)} SIL G/UV₂₅₄, Fertigfolien für die DC; Fließmitttel: Dichlormethan/Methanol/Cyclohexan/konz. wässeriges Ammoniak = 68/15/15/2 (v/v/v/v)).
IR (KBr): 1695, 1645 cm⁻¹ (C=N, C=O)

### c) (R,S)-N²-(Diphenylacetyl)-N-[(4-hydroxyphenyl)methyl]lysin-acetat

3,0 g (5,175 mMol) (R,S)-N²-(Diphenylacetyl)-N-[(4-hydroxyphenyl)methyl]-N⁶-[(phenylmethoxy)carbonyl]-lysinamid wurden in einem Gemisch von 70 ml Methanol, 30 ml Eisessig und 10 ml Wasser suspendiert und nach Zugabe von 0,8 g 10%ige Palladium/Tierkohle bei Raumtemperatur und einem Druck von 5 bar bis zur Beendigung der Wasserstoffaufnahme hydriert. Nach Entfernung von Katalysator und Lösemitteln wurde der verbliebene Rückstand aus wenig Diisopropylether umkristallisiert. Man erhielt 2,0 g (76 % der Theorie) an farblosen Kristallen vom Fp. 113-116°C und R_{f} 0,24 (Untersuchungsbedingungen wie im Beispiel 27b)).
IR (KBr): 1640 cm⁻¹ (Amid-C=O)
MS: M⁺ = 445

### d) (R,S)-N²-(Diphenylacetyl)-N⁶-[ethylamino(ethylimino)methyl]-N-[(4-hydroxyphenyl)methyl]-lysinamid-dihydroiodid

Das Gemisch aus 1,2 g (2,373 mMol) (R,S)-N²-(Diphenylacetyl)N-[(4-hydroxyphenyl)methyl]-lysin-acetat, 0,8 g (3,052 mMol) N,N'-Diethyl-S-methyl-thiuroniumiodid, 0,91 g (8,58 mMol) wasserfreiem Natriumcarbonat und 12 ml Dimethylformamid wurde unter Rühren 12 Stunden auf 65°C, dann noch 2 Stunden auf 80°C erhitzt. Anschließend wurde das Gemisch mit Eiswasser gekühlt und filtriert, der Filterkuchen noch gründlich mit Dimethylformamid gewaschen. Die Filtrate wurden im Vakuum eingedampft, der verbliebene Rückstand an Kieselgel (Macherey-Nagel, 35-70 mesh ASTM; mobile Phase: Ethylacetat/Methanol/Eisessig = 50/25/0,5 (v/v/v)) säulenchromatographisch gereinigt. Man erhielt 0,7 g (37 % der Theorie) einer farblosen, amorphen Substanz vom R_{F} 0,73.
IR (KBr): 1622, 1659 cm⁻¹ (C=O, C=N)
EI-MS: (M+H)⁺ = 544

### Beispiel 28

### (R)-N²-(Diphenylacetyl)-N-[[4-(hydroxymethyl)phenyl]methyl]-argininamid-hydrat

### a) (R)-N⁵-[Amino(nitroimino)methyl]-N²-(tert.-butoxycarbonyl)-N-[[4-(hydroxymethyl)phenyl]methyl]-ornithinamid

Hergestellt analog Beispiel 5d) aus Boc-D-Arg(NO₂)-OH, [4-Hydroxymethyl)phenyl]methanamin und TBTU in einer Ausbeute von 80 % der Theorie.
Farblose, amorphe Substanz.
IR (KBr): 1715, 1695, 1655, 1630 (C=O, C=N)
   - ESI-MS:: (M+Na)⁺ = 461
   (M+K)⁺ = 477
   (2M+Na)⁺ = 899

### b) (R)-N⁵-[Amino(nitroimino)methyl]-N-[[4-(hydroxymethyl)phenyl]methyl]-ornithinamid

Hergestellt analog Beispiel 23b) aus (R)-N⁵-[Amino(nitroimino)methyl]-N²-(tert.-butoxycarbonyl)-N-[[4-(hydroxymethyl)phenyl]methyl]-ornithinamid durch Einwirkung von Trifluoressigsäure in einer Ausbeute von 83 % der Theorie.
Farblose, amorphe Substanz, die ohne völlige Reinigung weiterverarbeitet wurde.

### c) (R)-N⁵-[Amino(nitroimino)methyl]-N²-(diphenylacetyl)N-[[4-(hydroxymethyl)phenyl]methyl]-ornithinamid

Hergestellt analog Beispiel 5f) aus (R)-N⁵-[Amino(nitroimino)methyl]-N-[[4-(hydroxymethyl)phenyl]methyl]-ornithinamid, Diphenylessigsäure und TBTU in einer Ausbeute von 79 % der Theorie.
Farblose, amorphe Substanz, hinsichtlich ihres dünnschichtchromatographischen Verhaltens völlig identisch mit einem nach Beispiel 22a) hergestellten Präparat.

### d) (R)-N²-(Diphenylacetyl)-N-[[4-(hydroxymethyl)phenyl]methyl]-argininamid-hydrat

Hergestellt in völliger Analogie zu Beispiel 22b; die erhaltene Substanz wurde jedoch anschließend zusätzlich säulenchromatographisch (Kieselgel, Macherey-Nagel, Typ 60, 70-230 mesh ASTM; mobile Phase: Essigsäureethylester/Methanol/Eisessig = 70/30/1 (v/v/v)) gereinigt; die geeigneten Eluate wurden im Vakuum eingedampft, der Rückstand wurde in wenig Wasser aufgenommen und mit 1N NaOH alkalisch gestellt. Die ausgefallenen Kristalle wurden abgenutscht, gründlich mit Wasser gewaschen und über di-Phosphorpentoxid im Vakuum getrocknet.
Ausbeute: 13 % der Theorie.
   Farblose Kristalle vom Fp. 140-142°C und R_{f} 0,62.
IR (KBr): 1641 cm⁻¹ (C=O)
ESI-MS: (M+H)⁺ = 488

### Beispiel 29

### (R,S)-α-[(Diphenylacetyl)amino]-N-[(4-hydroxyphenyl)methyl]-1H-imidazol-4-propanamid

### a) (R,S)-α-Amino-N-[(4-hydroxyphenyl)methyl]-1-(phenylmethyl)-1H-imidazol-4-propanamid

Hergestellt analog Beispiel 23b) aus (R,S)-α-[(tert.-Butoxycarbonyl)amino]-N-[(4-hydroxyphenyl)methyl]-1-(phenylmethyl)-1H-imidazol-4-propanamid durch Einwirkung von Trifluoressigsäure in einer Ausbeute von 100 % der Theorie. Das Produkt wurde ohne weitere Reinigung umgesetzt.

### b) (R,S)-α-[(Diphenylacetyl)amino]-N-[(4-hydroxyphenyl)methyl]-1-(phenylmethyl)-1H-imidazol-4-propanamid und (R,S)-α-[(Diphenylacetyl)amino]-N-[[4-(diphenylacetoxy)phenyl]methyl]-1-(phenylmethyl)-1H-imidazol-4-propanamid

Das unter den Reaktonsbedingungen von Beispiel 5f) aus (R,S)-α-Amino-N-[(4-hydroxyphenyl)methyl]-1-(phenylmethyl)1H-imidazol-4-propanamid, Diphenylessigsäure und TBTU erhaltene rohe Gemisch wurde säulenchromatographisch (Kieselgel MN 60, Macherey-Nagel, 70-230 mesh ASTM; mobile Phase: Essigsäureethylester/Methanol = 9/1 (v/v)) in zwei Produkte getrennt.
b1) (R,S)-α-[(Diphenylacetyl)amino]-N-[[4-(diphenylacetoxy) phenyl]methyl]-1H-imidazol-4-propanamid
   Ausbeute: 30 % der Theorie;
      farblose Kristalle vom Fp. 168°C und R_{f} 0,44 (Merck-DC-Fertigplatten, Kieselgel 60 F₂₅₄, Schichtdicke 0,25 mm; Fließmittel: Essigsäureethylester/Methanol 9/1 (v/v)).
   IR (KBr): 1747 (Ester-C=O), 1643 (Amid-C=O) cm⁻¹
   MS: M⁺ = 738
b2) (R,S)-α-[(Diphenylacetyl)amino]-N-[(4-hydroxyphenyl)methyl]-1-(phenylmethyl)-1H-imidazol-4-propanamid
   Ausbeute: 8 % der Theorie;
      farblose Kristalle vom Fp. 212-214°C und R_{f} 0,34 (Untersuchungsbedingungen wie vorstehend).
   IR (KBr): 1643 cm⁻¹ (Amid-C=O)
      - ESI-MS:: (M+H)⁺ = 545
      (M+Na)⁺ = 567

### c) (R,S)-α-[(Diphenylacetyl)amino]-N-[(4-hydroxyphenyl)methyl]-1H-imidazol-4-propanamid

1,0 g (1,836 mMol) (R,S)-α-[(Diphenylacetyl)amino]-N-[(4-hydroxyphenyl)methyl]-1-(phenylmethyl)-1H-imidazol-4-propanamid wurden in 100 ml Methanol gelöst und in Gegenwart von 500 mg Palladium/Tierkohle (10%ig) und 2 ml 1N Salzsäure bei 50°C und einem Wasserstoffdruck von 5 bar hydriert. Es wurde vom Katalysator abfiltriert, das Filtrat eingedampft, der Rückstand in Wasser aufgenommen, mit Pottasche alkalisch gestellt und mit Essigsäureethylester erschöpfend extrahiert. Die vereinigten und über Natriumsulfat getrockneten Essigesterextrakte wurden vom Lösemittel im Vakuum befreit und säulenchromatographisch (Kieselgel MN 60, Macherey-Nagel, 70-230 mesh ASTM; mobile Phase: Essigsäureethylester/Methanol/konz. wässeriges Ammoniak = 90/10/1 (v/v/v)) gereinigt. Man erhielt 0,2 g (24 % der Theorie) einer farblosen, amorphen Substanz vom R_{f} 0,65.
IR (KBr): 1647 cm⁻¹ (Amid-C=O)
MS: M⁺ = 454

### Beispiel 30

### (R)-N²-(Diphenylacetyl)-N-[[4-[(methylaminocarbonyl)amino]phenyl]methyl]-argininamid-diacetat

### a) (R)-N⁵-[Amino(nitroimino)methyl]-N²-(diphenylacetyl)N-[[4-[(methylaminocarbonyl)amino]phenyl]methyl]-ornithinamid

Hergestellt analog Beispiel 5d), jedoch unter Verwendung von N,N-Diisopropyl-ethylamin (Hünig-Base) an Stelle von Triethylamin, aus (R)-N²-(Diphenylacetyl)-N⁵-[amino(nitroimino)methyl]-ornithin und N-Methyl-N'-[4-(aminomethyl)phenyl]-harnstoff (Fp.: 144-145°C, hergestellt aus N-Methyl-N'-(4-cyanophenyl)-harnstoff durch katalytische Hydrierung in Gegenwart von Raney-Nickel und Ammoniak) in Gegenwart von TBTU.
Ausbeute: 61 % der Theorie.
   Farblose, amorphe Substanz vom R_{f} 0,29 (Untersuchungsbedingungen wie im Beispiel 29b).
IR (KBr): 1639 cm⁻¹ (Carboxamid-C=O)
   - ESI-MS:: (M+H)⁺ = 575
   (M+Na)⁺ = 597

### b) (R)-N²-(Diphenylacetyl)-N-[[4-[(methylaminocarbonyl)amino]phenyl]methyl]-argininamid-diacetat

Hergestellt analog Beispiel 1c) aus (R)-N⁵-[Amino(nitroimino)methyl]-N²-(diphenylacetyl)-N-[[4-[(methylaminocarbonyl)amino]phenyl]methyl]-ornithinamid durch katalytische Hydrierung in Gegenwart von Palladiummohr und 80%iger wässeriger Essigsäure in einer Ausbeute von 83 % der Theorie.
Farblose, amorphe Substanz vom R_{f} 0,58.
IR (KBr): 1647 cm⁻¹ (Amid-C=O)
ESI-MS: (M+H)⁺ = 530

### Beispiel 31

### (R,S)-N²-(Diphenylacetyl)-N-[(4-hydroxyphenyl)methyl]-N⁵-methyl-N⁵-(phenylmethyl)-ornithinamid

### a) α-(Acetylamino)-α-[3-[(phenylmethyl)methylamino]propyl]-malonsäurediethylester

Eine aus 5,8 g (0,252 Mol) Natrium und 250 ml wasserfreiem Ethanol frisch bereitete Natriumethylatlösung wurde bei Raumtemperatur und innerhalb von etwa 15 Minuten zu der aus 49,4 g (0,25 Mol) 3-Chlor-N-methyl-N-(phenylmethyl)-propanamin, 60 g (0,268 Mol) 97%igem Acetamido-malonsäurediethylester, 11,3 g (0,075 Mol) Natriumiodid und 800 ml trockenem Dioxan erhaltenen Mischung zugetropft. Man rührte 30 Minuten bei Zimmertemperatur und erhitzte dann 5 Stunden lang unter Rückfluß. Man ließ über Nacht bei Raumtemperatur stehen, filtrierte vom Unlöslichen ab, befreite das Filtrat vom Lösemittel und verteilte den verbliebenen Rückstand zwischen Essigsäureethylester und Wasser. Die Essigesterphase wurde über Natriumsulfat getrocknet und eingedampft, das erhaltene Öl schließlich säulenchromatographisch (Kieselgel MN 60, Macherey-Nagel, 70-230 mesh ASTM; mobile Phase: Dichlormethan/Methanol/konz. wässeriges Ammoniak = 90/10/0,25 (v/v/v)) gereinigt. Man erhielt 53 g (56 % der Theorie) eines farblosen, viskosen Öls.
IR (KBr): 1741.6 (Ester-C=O), 1683.8 (Amid-C=O) cm⁻¹

### b) (R,S)-N⁵-Methyl-N⁵-(phenylmethyl)-ornithin-dihyarochlorid

20,4 g (0,0539 Mol) α-(Acetylamino)-α-[3-[(phenylmethyl)methylamino]propyl]-malonsäurediethylester wurden in 50 ml Eisessig gelöst und nach Zugabe von 100 ml 3N wässeriger Salzsäure 6 Stunden lang unter Rückfluß gekocht. Die nach dem Abdampfen des Lösemittels verbleibende und in quantitativer Ausbeute erhaltene hochviskose, schwach gelbliche Masse wurde ohne weitere Reinigung weiter umgesetzt.

### c) (R,S)-N²-(Diphenylacetyl)-N⁵-methyl-N⁵-(phenylmethyl)ornithin-hydrochlorid

Diphenylacetylchlorid und (R,S)-N⁵-Methyl-N⁵-(phenylmethyl)ornithin-dihydrochlorid wurden analog Beispiel 1a) umgesetzt. Die erhaltene Mischung wurde im Wasserstrahlvakuum eingeengt, bis das als Lösemittel verwendete Tetrahydrofuran entfernt war, dann mit 3N wässeriger Salzsäure sauer gestellt und mit Diethylether sorgfältig extrahiert. Die wässerige Phase wurde anschließend bei vermindertem Druck und einer Badtemperatur von maximal +40°C eingedampft. Die Ausbeute an farblosen Kristallen vom Fp. 125-130°C, die ohne Reinigung in der nächsten Stufe eingesetzt wurden, betrug 27 % der Theorie. IR (KBr): 1715 (Carbonbäure-C=O), 1664 (Amid-C=O) cm⁻¹

### d) (R,S)-N²-(Diphenylacetyl)-N-[(4-hydroxyphenyl)methyl]N⁵-methyl-N⁵-(phenylmethyl)-ornithinamid

Hergestellt analog Beispiel 8a) aus (R,S)-N²-(Diphenylacetyl)-N⁵-methyl-N⁵-(phenylmethyl)-ornithin-hydrochlo rid, (4-Hydroxyphenyl)methanamin und TBTU in einer Ausbeute von 28 % der Theorie.
Farblose Kristalle vom Fp. 160-162°C (Essigsäureethylester) und R_{f} 0,75.
IR (KBr): 1679.9 und 1633.6 (Amid-C=O) cm⁻¹

### Beispiel 32

### (R)-N²-(Diphenylacetyl)-N-[(4-hydroxycyclohexyl)methyl]argininamid-acetat (Diastereomeren-Gemisch)

### a) (4-Hydroxycyclohexyl)methanamin (cis/trans-Gemisch)

Die Lösung von 1,0 g (8,12 mMol) (4-Hydroxyphenyl)methanamin in einer aus 0,34 g (8,50 mMol) Natriumhydroxid und 10 ml Wasser bereiteten Lauge wurde nach Zusatz von 0,3 g Rhodium/ Tierkohle (5 %ig) 20 Stunden bei 70°C und einem Wasserstoffdruck von 50 psi hydriert. Der Katalysator wurde abfiltriert, das Filtrat im Vakuum eingedampft. Der verbliebene Rückstand wurde in wenig Wasser gelöst, diese Lösung mit einigen Tropfen 40%iger Natronlauge auf pH 14 gebracht, mit Kochsalz gesättigt und unter Verwendung eines Rotationsperforators 3 Tage lang mit Diethylether extrahiert. Die Diethyletherextrakte wurden mit Natriumsulfat getrocknet und vom Lösemittel befreit und ergaben 0,24 g (23 % der Theorie) eines farblosen, viskosen Öls.
IR (CH₂Cl₂): 3610 cm⁻¹ (OH)

### b) (R)-N⁵-[Amino(nitroimino)methyl]-N²-[(tert.-butyloxy)carbonyl]-N-[(4-hydroxycyclohexyl)methyl]-ornithinamid (Diastereomerengemisch)

Hergestellt analog Beispiel 8a) aus Boc-D-Arg(NO₂)-OH und (4-Hydroxycyclohexyl)methanamin in Gegenwart von TBTU in einer Ausbeute von 41 % der Theorie.
Farblose, amorphe Substanz.
IR (KBr): 1650 (Amid-C=O), 1700 (Ester-C=O) cm⁻¹
   - EI-MS:: (M+H)⁺ = 431
   (M+Na)⁺ = 453
   (2M+H)⁺ = 861
   (2M+Na)⁺ = 883

### c) (R)-N⁵-[Amino(nitroimino)methyl]-N-[(4-hydroxycyclohexyl)methyl]-ornithinamid (Diastereomerengemisch)

Hergestellt analog Beispiel 23b) aus (R)-N⁵-[Amino(nitroimino)methyl]-N²-(tert.-butyloxycarbonyl)-N-[(4-hydroxycyclohexyl)methyl]-ornithinamid (Diastereomerengemisch) durch Einwirkung von Trifluoressigsäure in einer Ausbeute von 71 % der Theorie.
Farblose, amorphe Substanz.
IR (KBr): 1681.8 (C=O) cm⁻¹

### d) (R)-N⁵-[Amino(nitroimino)methyl]-N²-(diphenylacetyl)N-[(4-hydroxycyclohexyl)methyl]-ornithinamid (Diastereomerengemisch)

Zu der Lösung von 1,6 g (4,84 mMol) (R)-N⁵-[Amino(nitroimino)methyl]-N-[(4-hydroxycyclohexyl)methyl]-ornithinamid (Diastereomerengemisch) in 20 ml wasserfreiem Tetrahydrofuran tropfte man zunächst 1,5 g (14,8 mMol) Triethylamin, dann die Lösung von 0,7 g (3,034 mMol) Diphenylacetylchlorid, gelöst in 5 ml trockenem Tetrahydrofuran. Nach 20-minütigem Rühren bei Raumtemperatur wurde im Vakuum eingedampft, der Rückstand zwischen Wasser und Essigsäureethylester verteilt, die Essigesterphase über Natriumsulfat getrocknet und eingedampft. Der Rückstand wurde an Kieselgel (Macherey-Nagel, 70-230 mesh ASTM) unter Verwendung von Dichlormethan/Methanol/konz. wässerigem Ammoniak = 90/10/0,25 (v/v/v)) säulenchromatographisch gereinigt. Man erhielt 0,15 g (9 % der Theorie) eines farblosen, amorphen Produkts.
IR (KBr): 1649 cm⁻¹ (Amid-C=O)
ESI-MS: (M-H)⁻ = 523

### e) (R)-N²-(Diphenylacetyl)-N-[(4-hydroxycyclohexyl)methyl]argininamid-acetat (Diastereomerengemisch)

Hergestellt analog Beispiel 1c) aus (R)-N⁵-[Amino(nitroimino)methyl]-N²-(diphenylacetyl)-N-[(4-hydroxycyclohexyl)methyl]-ornithinamid (Diastereomerengemisch) durch katalytische Hydrierung in Gegenwart von Palladiummohr und 80%iger wässeriger Essigsäure.
Ausbeute: 84 % der Theorie.
   Farblose, amorphe, wasserlösliche Substanz vom R_{f} 0,63.
IR (KBr): 1652.9 cm⁻¹ (Amid-C=O)
ESI-MS: (M+H)⁺ = 480

### Beispiel 33

### (R,S)-N⁵,N⁵-Dimethyl-N²-(diphenylacetyl)-N-[(4-hydroxyphenyl)methyl]-ornithinamid

### a) α-(Acetylamino)-α-[3-(dimethylamino)propyl]-malonsäurediethylester

Hergestellt analog Beispiel 31a) aus Acetamidomalonsäurediethylester und 3-Chlor-N,N-dimethyl-propanamin in Gegenwart von Natriumethanolat.
Ausbeute: 27 % der Theorie.
   Farbloses, viskoses Öl.
IR (KBr): 1741.6 (Ester-C=O), 1679.9 (Amid-C=O) cm⁻¹

### b) (R,S)-N⁵,N⁵-Dimethyl-ornithin-dihydrochlorid

Hergestellt analog Beispiel 31b) aus α-(Acetylamino)-α-[3-(dimethylamino)propyl]-malonsäurediethylester und Salzsäure in einer Ausbeute von 100 % der Theorie.
Farblose, hochviskose Substanz, die ohne Reinigung in der nächsten Stufe verwendet wurde

### c) (R,S)-N⁵,N⁵-Dimethyl-N²-(diphenylacetyl)-ornithinhydrochlorid

Hergestellt analog Beispiel 31c) aus (R,S)-N⁵,N⁵-Dimethylornithin-dihydrochlorid und Diphenylacetylchlorid in einer Ausbeute von 3 % der Theorie. Farblose Kristalle, die ohne Reinigung in der nächsten Stufe umgesetzt wurden.

### d) (R,S)-N⁵,N⁵-Dimethyl-N²-(diphenylacetyl)-N-[(4-hydroxyphenyl)methyl]-ornithinamid

Hergestellt analog Beispiel 8a) aus (R,S)-N⁵,N⁵-Dimethyl-N²-(diphenylacetyl)-ornithin-hydrochlorid, (4-Hydroxyphenyl)methanamin und TBTU in einer Ausbeute von 33 % der Theorie. Farblose, amorphe Substanz.
MS: M⁺ = 459
IR (KBr): 1652,9 cm⁻¹ (Amid-C=O)

### Beispiel 34

### (R,S)-N²-(Diphenylacetyl)-N⁵-[ethylamino(ethylimino)methyl]-N-[(4-hydroxyphenyl)methyl]-ornithinamid-acetathydroiodid

### a) (R,S)-N²-(Diphenylacetyl)-N⁵-[(phenylmethoxy)carbonyl]-ornithin

Hergestellt analog Beispiel 1a) aus Diphenyacetylchlorid und D,L-N⁵-[(Phenylmethoxy)carbonyl]-ornithin in Gegenwart von Natronlauge. Man erhielt 96 % der Theorie an farblosen Kristallen vom Fp. 120-122.
IR (KBr): 3320 (NH), 1715, 1685, 1665, 1645 cm⁻¹ (C=O)

### b) (R,S)-N²-(Diphenylacetyl)-N-[(4-hydroxyphenyl)methyl]-N⁵-[(phenylmethoxy)carbonyl]-ornithinamid

Hergestellt analog Beispiel 8a) aus (R,S)-N²-(Diphenylacetyl)-N⁵-[(phenylmethoxy)carbonyl]-ornithin und 4-Hydroxybenzenmethanamin in Gegenwart von TBTU in einer Ausbeute von 50 % der Theorie.
Farblose Kristalle vom Fp. 118-121°C (Essigsäureethylester).
IR (KBr): 1740 (Ester-C=O), 1695, 1645 cm⁻¹ (Amid-C=O).
MS: M⁺ = 565

### c) (R,S)-N²-(Diphenylacetyl)-N-[(4-hydroxyphenyl)methyl]ornithinamid-acetat

Hergestellt analog Beispiel 27c) aus (R,S)-N²-(Diphenylacetyl)-N-[(4-hydroxyphenyl)methyl]-N⁵-[(phenylmethoxy)carbonyl]-ornithinamid durch katalytische Hydrierung in Gegenwart von Palladium/Tierkohle.
Ausbeute von 95 % der Theorie.
Farblose Kristalle vom Fp. 185-186°C und R_{f} 0,42 (Macherey-Nagel, Polygram^{(R)} SIL G/UV₂₅₄, Fertigfolien für die DC; Fließmittel: Essigsäureethylester/Methanol/Eisessig = 50/50/1 (v/v/v)).
IR (KBr): 1640 cm⁻¹ (Amid-C=O).
ESI-MS: (M+H)⁺ = 432

### d) (R,S)-N²-(Diphenylacetyl)-N⁵-[ethylamino(ethylimino)methyl]-N-[(4-hydroxyphenyl)methyl]-ornithinamid-acetathydroiodid

Hergestellt analog Beispiel 27d) aus (R,S)-N²-(Diphenylacetyl)-N-[(4-hydroxyphenyl)methyl]-ornithin-acetat und N,N'-Diethyl-S-methyl-thiuroniumiodid (Fp.: 73-74°C, hergestellt aus N,N'-Diethylthioharnstoff und Methyliodid) in einer Ausbeute von 47 % der Theorie.
Farblose, amorphe Substanz vom R_{f} 0,70.
IR (KBr): 1654.8, 1627.8 cm⁻¹ (C=O, C=N).
EI-MS: (M+H)⁺ = 530

### Beispiel 35

### (R)-N-[(4-Amino-3,5-dichlorphenyl)methyl]-N²-(diphenylacetyl)-argininamid

### a) (R)-N-[(4-Amino-3,5-dichlorphenyl)methyl]-N⁵-[amino-(nitroimino)methyl]-N²-(diphenylacetyl)-ornithinamid

Hergestellt analog Beispiel 5f), jedoch unter Verwendung von N-Ethyl-diisoproylamin an Stelle von Triethylamin, aus (R)-N-[(4-Amino-3,5-dichlorphenyl)methyl]-N⁵-[amino-(nitroimino)methyl]-ornithinamid, Diphenylessigsäure und TBTU in einer Ausbeute von 71 % der Theorie.
Farblose Kristalle vom Fp. 224-225°C (Ethanol).
IR (KBr): 1633.6 cm⁻¹ (C=O)

### b) (R)-N-[(4-Amino-3,5-dichlorphenyl)methyl]-N²-(diphenylacetyl)-argininamid

Hergestellt analog Beispiel 14b) aus (R)-N-[(4-Amino-3,5-dichlorphenyl)methyl]-N⁵-[amino(nitroimino)methyl]-N²-(diphenylacetyl)-ornithinamid durch Reduktion mit Zinn(II)-chlorid-dihydrat in Gegenwart von 60%iger wässeriger Ameisensäure.
Ausbeute: 65 % der Theorie.
   Farblose Kristalle vom Fp. 148-152°C und R_{f} 0,62.
IR (KBr): 1656.8 cm⁻¹ (Amid-C=O).
EI-MS: (M+H)⁺ = 541/543/545 (Cl₂)

Die Substanz ist nach MS- und NMR-Untersuchungen durch (R)-N²-(Diphenylacetyl-N-[(3,5-dichlor-4-(formylamino)-phenyl)methyl]-argininamid verunreinigt.

### Beispiel 36

### (R)-N-[(4-Amino-3,5-dichlorphenyl)methyl]-N²-(3,3-diphenyl-1-oxopropyl)-argininamid-acetat-hydrochlorid

### a) (R)-N-[(4-Amino-3,5-dichlorphenyl)methyl]-N⁵-[amino-(nitroimino)methyl]-N²-[(tert.-butyloxy)carbonyl]ornithinamid

Zu der Mischung aus 21,7 g (0,068 Mol) Boc-D-Arg(NO₂)-OH, 12,2 ml (0,07 Mol) N,N-Diisopropyl-ethylamin und 13,0 g (0,068 Mol) (4-Amino-3,5-dichlorphenyl)methanamin (hergestellt aus 4-Amino-3,5-dichlorbenzaldehyd und α-Aminoisobuttersäure in Analogie zu G.P. Rizzi, J. Org. Chem. 36, 1710-1711 (1971]) in 225 ml wasserfreiem Dimethylformamid gab man unter Rühren und äußerer Kühlung mit Eiswasser 22,4 g (0,0698 Mol) TBTU und hielt anschließend 2 Stunden bei Raumtemperatur. Die Mischung wurde in reichlich Wasser eingerührt, dann mit Essigsäureethylester erschöpfend extrahiert. Die vereinigten Essigesterauszüge wurden nacheinander mit 10%iger wässeriger Zitronensäure, Wasser, gesättigter wässeriger Natriumhydrogencarbonatlösung und Wasser geweschen, über Magnesiumsulfat getrocknet und eingedampft. Man erhielt 27,9 g (83 % der Theorie) an farblosen Kristallen vom Fp. 105-107°C.
IR (KBr): 1630, 1660, 1700 cm⁻¹ (C=O)

### b) (R)-N-[(4-Amino-3,5-dichlorphenyl)methyl]-N⁵-[amino-(nitroimino)methyl]-ornithinamid

Die Lösung von 27,9 g (0,0567 Mol) (R)-N-[(4-Amino-3,5-dichlorphenyl)methyl]-N⁵-[amino(nitroimino)methyl]-N²-[(tert.-butyloxy)carbonyl]-ornithinamid in 200 ml wasserfreiem Methanol wurde zu 150 ml einer Lösung von trockenem Chlorwasserstoff in absolutem Methanol zugetropft. Nach Beendigung der Gasentwicklung (nach ca. 30 Minuten) wurde das Lösemittel im Vakuum abgezogen, der Rückstand in Wasser aufgenommen, die Mischung filtriert und das Filtrat ammonialkalisch gestellt. Man extrahierte mit Essigsäureethylester, trocknete die vereinigten Auszüge über Magnesiumsulfat und dampfte im Vakuum ein. Man erhielt 200 g (90 % der Theorie) eines farblosen Öls, das nach einigen Tagen kristallin erstarrte.
IR (KBr): 1624.0 cm⁻¹ (C=O)

### c) (R)-N-[(4-Amino-3,5-dichlorphenyl)methyl]-N⁵-[amino-(nitroimino)methyl]-N²-(3,3-diphenyl-1-oxopropyl)ornithinamid

Hergestellt analog Beispiel 35a) aus 3,3-Diphenylpropansäure, (R)-N-[(4-Amino-3,5-dichlorphenyl)methyl]-N⁵-[amino-(nitroimino)methyl]-ornithinamid und TBTU in einer Ausbeute von 58 % der Theorie.
Farblose Kristalle vom Fp. 218-219°C (Essigsäureethylester).
IN (KBr): 1631.7 cm⁻¹ (C=O)

### d) (R)-N-[(4-Amino-3,5-dichlorphenyl)methyl]-N²-(3,3-diphenyl-1-oxopropyl)-argininamid-acetat-hydrochlorid

Hergestellt analog Beispiel 14b) aus (R)-N-[(4-Amino-3,5-dichlorphenyl)methyl]-N⁵-[amino(nitroimino)methyl]-N²-(3,3-diphenyl-1-oxopropyl)-ornithinamid durch Reduktion mit Zinn(II)-chlorid-dihydrat in Gegenwart von 60%iger wässeriger Ameisensäure in einer Ausbeute von 40 % der Theorie.
Farblose, amorphe Substanz vom R_{f} 0,62.
IR (KBr): 1656.8 cm⁻¹ (Carboxamid-C=O)
ESI-MS: (M+H)⁺ = 555/556/559 (Cl₂)

Die Substanz ist nach MS- und NMR-Untersuchungen durch (R)-N-[[3,5-Dichlor-4-(formylamino)phenyl)methyl]-N²-(3,3-diphenyl-1-oxopropyl)-argininamid bzw. dessen Salz verunreinigt.

### Beispiel 37

### (R)-N-[(4-Aminophenyl)methyl]-argininamid-acetat-dihydrochlorid

Hergestellt analog Beispiel 1c aus (R)-N-[(4-Amino-3,5-dichlorphenyl)methyl]-N⁵-[amino(nitromimino)methyl]-ornithinamid durch katalytische Hydrierung in Gegenwart von Palladiummohr und 80%iger wässeriger Essigsäure.
Ausbeute 57 % der Theorie.
Farblose, amorphe Substanz vom R_{f} 0,07.
IR (KBr): 1668.3 cm⁻¹ (Amid-C=O)
ESI-MS: (M+H)⁺ = 279

### Beispiel 38

### (R)-N²-[(4-Amino-3,5-dichlorphenyl)sulfonyl]-N-[(4-hydroxyphenyl)methyl]-argininamid-hydrochlorid

### a) (R)-N²-[(4-Amino-3,5-dichlorphenyl)sulfonyl]-N⁵-[amino(nitroimino)methyl]-ornithin

Die Suspension von 2,6 g (10,55 mMol) 4-Amino-3,5-dichlorbenbenzolsulfonylchlorid in 31 ml Wasser wurde nacheinander mit 2,6 g Natriumhydrogencarbonat und der Lösung von 2,02 g (9,2 mMol) H-D-Arg(NO₂)-OH in 15 ml Aceton versetzt und die Mischung anschließend durch Zutropfen von 40%iger Natronlauge bei pH 10,5-10,8 gehalten. Nach ca. 45 Minuten war die Mischung pH-stabil, wurde noch 1 Stunde bei Zimmertemperatur gerührt, dann mit verdünnter Salzsäure auf pH 3 gebracht und schließlich mit 3 g fester Zitronensäure versetzt. Die Mischung wurde mit Essigsäureethylester erschöpfend extrahiert, die Extrakte wurden über Natriumsulfat getrocknet und eingedampft. Der Rückstand wurde mit Diethylether verrieben und ergab 3,0 g (74 % der Theorie) an farblosen Kristallen vom Fp. 196-200°C.
IR (KBr): 1728.1 cm⁻¹ (Carbonsäure-C=O), 1625.9 (Amid-C=O), 1340 und 1170 (SO₂N) cm⁻¹

### b) (R)-N²-[(4-Amino-3,5-dichlorphenyl)sulfonyl]-N⁵-[amino(nitroimino)methyl]-N-[(4-hydroxyphenyl)methyl]ornithinamid

Hergestellt analog Beispiel 8a), jedoch unter Verwendung von N,N-Diisopropyl-ethylamin an Stelle von Triethylamin, aus (R)-N²-[(4-Amino-3,5-dichlorphenyl)sulfonyl]-N⁵-[amino-(nitroimino)methyl]-ornithin und (4-Hydroxyphenyl)methanamin sowie TBTU in einer Ausbeute von 58 % der Theorie. Farblose Kristalle vom Fp. 241-242°C.
IR (KBr): 1639.4 (Amid-C=O), 1336.6 und 1159.2 (SO₂N) cm⁻¹ .

### c) (R)-N²-[(4-Amino-3,5-dichlorphenyl)sulfonyl]-N-[(4-hydroxyphenyl)methyl]-argininamid-hydrochlorid

Hergestellt analog Beispiel 14b) aus (R)-N²-[(4-Amino-3,5-dichlorphenyl)sulfonyl]-N⁵-[amino(nitroimino)methyl]-N-[(4-hydroxyphenyl)methyl]-ornithinamid durch Reduktion mit Zinn(II)-chlorid-dihydrat in Gegenwart von 60%iger wässeriger Ameisensäure.
Ausbeute: 80 % der Theorie.
   Farblose Kristalle vom Fp. 245-249°C und R_{f} 0,65.
   - IR (KBr):: 1662.5, 1639.4 (C=O, C=N) cm⁻¹
   1334.7, 1159.2 (SO₂N) cm⁻¹.
ESI-MS: (M+H)⁺ = 503/505/507 (Cl₂)

### Beispiel 39

### (R)-N-[(4-Amino-3,5-dichlorphenyl)methyl]-N²-benzoyl-argininamid-hydrochlorid

### a) (R)-N-[(4-Amino-3,5-dichlorphenyl)methyl]-N⁵-[amino-(nitroimino)methyl]-N²-benzoyl-ornithinamid

Hergestellt analog Beispiel 38b) aus Benzoesäure und (R)-N-[(4-Amino-3,5-dichlorphenyl)methyl]-N⁵-[amino(nitroimino)methyl]-ornithinamid sowie TBTU in einer Ausbeute von 91 % der Theorie.
Farblose Kristalle vom Fp. 235-237°C.
IR (KBr): 1625.9 cm⁻¹ (Amid-C=O)

### b) (R)-N-[(4-Amino-3,5-dichlorphenyl)methyl]-N²-benzoylargininamid-hydrochlorid

Hergestellt analog Beispiel 14b) aus (R)-N-[(4-Amino-3,5-dichlorphenyl)methyl]-N⁵-[amino(nitroimino)methyl]-N²-benzoyl-ornithinamid durch Reduktion mit Zinn(II)-chlorid-dihydrat in Gegenwart von 60%iger wässeriger Ameisensäure. Ausbeute: 46 % der Theorie.
Farblose Kristalle vom Fp. 212-214°C (Diisopropylether) und R_{f} 0,65.
IR (KBr): 1652.9 cm⁻¹ (Amid-C=O).
ESI-MS: (M+H)⁺ = 451/453/455 (Cl₂)

### Beispiel 40

### (R)-N-[(4-Amino-3,5-dichlorphenyl)methyl]-N²-(2,2-diphenyl-1-oxopropyl)-argininamid-hydrochlorid

### a) (R)-N-[(4-Amino-3,5-dichlorphenyl)methyl]-N⁵-[amino-(nitroimino)methyl]-N²-(2,2-diphenyl-1-oxopropyl)ornithinamid

Hergestellt analog Beispiel 38b) aus 2,2-Diphenyl-propansäure und (R)-N-[(4-Amino-3,5-dichlorphenyl)methyl]-N⁵-[amino-(nitroimino)methyl]-ornithinamid sowie TBTU in einer Ausbeute von 75 % der Theorie.
Farblose Kristalle vom Fp. 221-223°C.
IR (KBr): 1624.0, 1666.4 cm⁻¹ (C=O, C=N)

### b) (R)-N-[(4-Amino-3,5-dichlorphenyl)methyl]-N²-(2,2-diphenyl-1-oxopropyl)-argininamid-hydrochlorid

Hergestellt analog Beispiel 14b) aus (R)-N-[(4-Amino-3,5-dichlorphenyl)methyl]-N⁵-[amino(nitroimino)methyl]-N²-(2,2-diphenyl-1-oxopropyl)-ornithinamid durch Reduktion mit Zinn-(II)-chlorid-dihydrat in Gegenwart von 60%iger wässeriger Ameisensäure.
Ausbeute: 95 % der Theorie.
   Farblose, amorphe Substanz vom R_{f} 0,63.
IR (KBr): 1652.9 cm⁻¹ (Amid-C=O).
ESI-MS: (M+H)⁺ = 555/557/559 (Cl₂)
Die Substanz ist nach MS- und NMR-Untersuchungen durch (R)-N-[[3,5-Dichlor-4-(formylamino)phenyl]methyl]-N²-(2,2-diphenyl-1-oxopropyl)-argininamid bzw. dessen Salz verunreinigt.

### Beispiel 41

### (R)-N-[(4-Amino-3,5-dichlorphenyl)methyl]-N²-(3,4-dichlorbenzoyl)-argininamid-hydrochlorid-hydrat

### a) (R)-N-[(4-Amino-3,5-dichlorphenyl)methyl]-N⁵-[amino-(nitroimino)methyl]-N²-(3,4-dichlorbenzoyl)-ornithinamid

Hergestellt analog Beispiel 38b) aus 3,4-Dichlorbenzoesäure und (R)-N-[(4-Amino-3,5-dichlorphenyl)methyl]-N⁵-[amino-(nitroimino)methyl]-ornithin sowie TBTU in einer Ausbeute von 88 % der Theorie.
Farblose Kristalle vom Fp. >260°C.
IR (KBr): 1668.3, 1627.8 cm⁻¹ (C=O)

### b) (R)-N-[(4-Amino-3,5-dichlorphenyl)methyl]-N²-(3,4-dichlorbenzoyl)-argininamid-hydrochlorid-hydrat

Hergestellt analog Beispiel 14b) aus (R)-N-[(4-Amino-3,5-dichlorphenyl)methyl]-N⁵-[amino(nitroimino)methyl]-N²-(3,4-dichlorbenzoyl)-ornithinamid durch Reduktion mit Zinn(II)-chlorid-dihydrat in Gegenwart von 60%iger wässeriger Ameisensäure.
Ausbeute: 90 % der Theorie.
   Farblose Kristalle vom Fp. 252-255°C und R_{f} 0,69.
IR (KBr): 1691.5, 1649.0 cm⁻¹ (C=O)
ESI-MS: (M+H)⁺ = 519/521/523/525/527 (Cl₄)
Die Substanz ist nach MS- und NMR-Untersuchungen durch (R)-N-[3,5-Dichlor-4-(formylamino)phenyl]methyl]-N²-(3,4-dichlorbenzoyl)-argininamid bzw. dessen Salze verunreinigt.

### Beispiel 42

### (R,S)-N⁶-(Aminoiminomethyl)-N²-(diphenylacetyl)-N-[[3-[(1,2-dihydro-3,5(4H)dioxo-1,2-diphenyl-3H-1,2,4-triazol-4-yl)-methyl]phenyl]methyl]-lysinamid

### a) 1,2-Dihydro-1,2-diphenyl-3H-1,2,4-triazol-3,5(4H)-dion

50 g (0,378 Mol) Ethylallophanat und 76,6 g (0,416 Mol) 1,2-Diphenylhydrazin wurden in 100 ml wasserfreiem Xylol suspendiert und unter Rühren 6 Stunden unter Rückfluß gekocht, wobei sich Ammoniak entwickelte. Das Lösemittel wurde abdestilliert, der Rückstand erneut mit 150 ml Xylol versetzt und 1 Stunde unter Rückfluß gekocht. Man ließ erkalten, nutschte das Reaktionsprodukt ab, wusch gründlich mit kaltem Xylol nach. Nach dem Trocknen im Vakuum erhielt man 60,0 g (63 % der Theorie) an farblosen Kristallen vom Fp. 216-217°C.

### b) 3-[(1,2-Dihydro-3,5(4H)-dioxo-1,2-diphenyl-3H-1,2,4-triazol-4-yl)methyl]-benzonitril

Zu der Lösung von 10,5 g (41,5 mMol) 1,2-Dihydro-1,2-diphenyl-3H-1,2,4-triazol-3,5(4H)-dion in 40 ml wasserfreiem Dimethylformamid gab man unter äußerer Kühlung mit Eis 4,5 g (40,1 mMol) Kalium-tert.-butylat und nach halbstündigem Rühren bei dieser Temperatur die Lösung von 8,0 g (40,8 mMol) 3-(Brommethyl)-benzonitril in 10 ml absolutem Dimethylformamid und ließ anschließend über Nacht auf Zimmertemperatur erwärmen. Abschließend wurde 1 Stunde auf 70°C erwärmt, das Lösemittel im Vakuum entfernt, der verbliebene Rückstand in Wasser aufgenommen und abgenutscht. Nach dem Waschen mit Diethylether und Trocknen an der Luft erhielt man 13,6 g (92 % der Theo- rie) an farblosen Kristallen.

### c) 3-[(1,2-Dihydro-3,5(4H)-dioxo-1,2-diphenyl-3H-1,2,4-triazol-4-yl)methyl]-benzenmethanamin

12,0 g (32,6 mMol) 3-[(1,2-Dihydro-3,5(4H)-dioxo-1,2-diphenyl-3H-1,2,4-triazol-4-yl)methyl]-benzonitril wurden in 500 ml mit Ammoniak gesättigtem Methanol gelöst und nach Zugabe von 3 g Raney-Nickel bis zur Beendigung der Wasserstoffaufnahme bei Raumtemperatur und 5 bar Wasserstoffdruck hydriert. Nach Aufarbeitung erhielt man 8,0 g (66 % der Theorie) einer farblosen, kristallinen Substanz.

### d) (R,S)-N²-(Diphenylacetyl)-N⁶-[(phenylmethoxy)carbonyl]-lysin

Hergestellt analog Beispiel 1a) aus Diphenylacetylchlorid und (R,S)-N⁶-[(Phenylmethoxy)carbonyl]-lysin in einer Ausbeute von 84 % der Theorie.
Farblose Kristalle vom Fp. 99-100°C.
IR (KBr): 1735 (Ester-C=O), 1715 (Säure-C=O), 1650 (Amid-C=O) cm⁻¹

### e) (R,S)-N²-(Diphenylacetyl)-N-[[3-[(1,2-dihydro-3,5(4H)-dioxo-1,2-diphenyl-3H-1,2,4-triazol-4-yl)methyl]phenyl]methyl]-N⁶-[(phenylmethoxy)carbonyl]-lysinamid

Hergestellt analog Beispiel 5d) aus (R,S)-N²-(Diphenylacetyl)-N⁶-[(phenylmethoxy)carbonyl]-lysin und 3-[(1,2-Dihydro-3,5(4H)-dioxo-1,2-diphenyl-3H-1,2,4-triazol-4-yl)methyl]-benzenmethanamin sowie TBTU in einer Ausbeute von 91 % der Theorie.
Farblose Kristalle.
IR (KBr): 1720, 1685, 1640 cm⁻¹ (C=O).
ESI-MS: (M+Na)⁺ = 851

### f) (R,S)-N²-(Diphenylacetyl)-N-[[3-[(1,2-dihydro-3,5(4H)dioxo-1,2-diphenyl-3H-1,2,4-triazol-4-yl)methyl]phenyl]methyl]-lysinamid

Die Mischung aus 3,0 g (3,619 mMol) (R,S)-N²-(Diphenylacetyl)-N-[[3-[(1,2-dihydro-3,5(4H)-dioxo-1,2-diphenyl-3H-1,2,4-triazol-4-yl)methyl]phenyl]methyl]-N⁶-[(phenylmethoxy) carbonyl]-lysinamid, 50 ml Methanol, 40 ml Tetrahydrofuran, 0,5 ml Trifluoressigsäure und 0,3 g Palladium/Tierkohle (10%ig) wurde 72 Stunden bei Zimmertemperatur und einem Druck von 50 psi in einer Parr-Apparatur geschüttelt. Der nach Entfernen von Katalysator und Lösemitteln verbliebene Rückstand wurde säulenehromatographisch gereinigt (Kieselgel Baker 30-60 µm; mobile Phase: Essigsäureethylester/Methanol/Cyclohexan/konz. wässeriges Ammoniak = 8/1/1/0,1 (v/v/v/v)). Man erhielt 1,2 g (40 % der Theorie) einer farb- losen, hochviskosen, amorphen Substanz.

### g) (R,S)-N⁶-(Aminoiminomethyl)-N²-(diphenylacetyl)-N-[[3-[(1,2-dihydro-3,5(4H)-dioxo-1,2-diphenyl-3H-1,2,4-triazol-4-yl)methyl]phenyl]methyl]-lysinamid

Die Mischung aus 1,1 g (1,583 mMol) (R,S)-N²-(Diphenylacetyl)-N-[[3-[(1,2-dihydro-3,5(4H)-dioxo-1,2-diphenyl-3H-1,2,4-triazol-4-yl)methyl]phenyl]methyl]-lysinamid, 600 mg (2,982 mMol) 3,5-Dimethylpyrazol-1-carbonsäureamidiniumnitrat, 0,8 ml (5,74 mMol) Triethylamin und 25 ml Tetrahydrofuran wurde 30 Stunden bei Raumtemperatur gerührt, anschließend in reichlich Wasser eingegossen. Das nach Abdekantieren der wässerigen Phase verbleibende Öl wurde säulenchromatographisch (Kieselgel Baker 30-60 µm) unter Verwendung von anfangs Essigsäureethylester, dann Methanol, zuletzt Methanol/ Eisessig = 95/5 (v/v) gereinigt. Die geeigneten Eluate wurden vereinigt, im Vakuum eingedampft, in Methanol aufgenommen und natronalkalisch gestellt. Man erhielt 800 mg (69 % der Theorie) einer farblosen, amorphen Substanz vom R_{f} 0,75.
IR (KBr): 1620-1690 cm⁻¹ (C=O).
ESI-MS: (M+H)⁺ = 737

### Beispiel 43

### N²-(Diphenylacetyl)-N-(3,3-diphenylpropyl)-argininamid

### a) N⁵-[Amino(nitroimino)methyl]-N²-(diphenylacetyl)-N-(3,3-diphenylpropyl)-ornithinamid

Hergestellt analog Beispiel 5d) aus (R)-N⁵-[Amino(nitroimino)methyl]-N²-(diphenylacetyl)-ornithin, 3,3-Diphenylpropanamin und TBTU in einer Ausbeute von 52 % der Theorie. Farblose, kristalline Substanz. IR (CH₂Cl₂): 1620, 1655 cm⁻¹ (C=O, C=N)

### b) N²-(Diphenylacetyl)-N-(3,3-diphenylpropyl)-argininamid

Hergestellt analog Beispiel 1c) aus N⁵-[Amino(nitroimino)methyl]-N²-(diphenylacetyl)-N-(3,3-diphenylpropyl)-ornithinamid durch katalytische Hydrierung in Gegenwart von Palladiummohr und 80%iger wässeriger Essigsäure in einer Ausbeute von 53 % der Theorie.
Farblose, amorphe Substanz vom R_{f} 0,72.
IR (CH₂Cl₂): 1630-1690 cm⁻¹ (C=O, C=N)
EI-MS: (M+H)⁺ = 562

### Beispiel 44

### N²-(Diphenylacetyl)-N-(2,2--diphenylethyl)-argininamid

### a) N⁵-[Amino(nitroimino)methyl]-N²-(diphenylacetyl)-N-(2,2-diphenylethyl)-ornithinamid

Hergestellt analog Beispiel 5d) aus (R)-N⁵-[Amino(nitroimino)methyl]-N²-(diphenylacetyl)-ornithin, 2,2-Diphenylethanamin und TBTU in einer Ausbeute von 98 % der Theorie. Farblose, amorphe Substanz.
IR (KBr): 1635 cm⁻¹ (C=O)

### b) N²-(Diphenylacetyl)-N-(2,2-diphenylethyl)-argininamid

Hergestellt analog Beispiel 1c) aus N⁵-[Amino(nitroimino)-methyl]-N²-(diphenylacetyl)-N-(2,2-diphenylethyl)-ornithinamid durch katalytische Hydrierung in Gegenwart von Palladiummohr und 80%iger wässeriger Essigsäure in einer Ausbeute von 100 % der Theorie.
Farblose, amorphe Substanz vom R_{f} 0,77.
IR (CH₂Cl₂): 1630-1690 cm⁻¹ (C=O)
ESI-MS: (M+H)⁺ = 548

### Beispiel 45

### N²-(Diphenylacetyl-N-[2-(5-methoxy-1H-indol-3-yl)ethyl]-argininamid

### a) N⁵-[Amino(nitorimino)methyl]-N²-(diphenylacetyl)-N-[2-(5-methoxy-1H-indol-3-yl)ethyl]-ornithinamid

Hergestellt analog Beispiel 5d) aus (R)-N⁵-[Amino(nitroimino)methyl]-N²-(diphenylacetyl)-ornithin, 5-Methoxytryptamin und TBTU in einer Ausbeute von 36 % der Theorie. Farblose Kristalle.

### b) N²-(Diphenylacetyl)-N-[2-(5-methoxy-1H-indol-3-yl) ethyl]-argininamid

Hergestellt analog Beispiel 1c) aus N⁵-[Amino(nitroimino)methyl]-N²-(diphenylacetyl)-N-[2-(5-methoxy-1H-indol-3-yl)-ethyl]-ornithinamid durch katalytische Hydrierung in Gegenwart von Palladiummohr und 80%iger wässeriger Essigsäure in einer Ausbeute von 68 % der Theorie.
Farblose, amorphe Substanz vom R_{f} 0,70.
IR (KBr): 1620-1690 cm⁻¹ (C=O)
EI-MS: (M+H)⁺ = 541

### Beispiel 46

### N-[[2,3-Dihydro-2-[(diphenylamino)carbonyl]-1H-isoindol-5-yl]methyl]-N²-(diphenylacetyl)-argininamid

### a) N⁵-[Amino(nitroimino)methyl]-N-[[2,3-dihydro-2-[(diphenylamino)carbonyl]-1H-isoindol-5-yl]methyl]-N²-(diphenylacetyl)-ornithinamid

Hergestellt analog Beispiel 5d) aus (R)-N⁵-[Amino(nitroimino)methyl]-N²-(diphenylacetyl)-ornithin, 2,3-Dihydro-2-[(diphenylamino)carbonyl]-1H-isoindol-5-methanamin (Fp.: 129-130°C; hergestellt aus 3,4-Dimethylbenzoesäuremethylester über 3,4-Bis(brommethyl)benzoesäuremethylester[N-Bromsuccinimid/Azoisobutyronitril]; 2,3-Dihydro-2-(phenylmethyl)-1H-isoindol-5-carbonsäuremethylester [Benzenmethanamin], Fp.: 72°C (tert.-Butylmethylether); 2,3-Dihydro-2-(phenylmethyl)-1H-isoindol-5-carbonsäure [wässerig-ethanolische Natronlauge]; 2,3-Dihydro-2-(phenylmethyl)-1H-isoindol-5-carbonsäurechlorid-hydrochlorid [Thionylchlorid], Fp.: 226-228°C (Z.); 2,3-Dihydro-2-(phenylmethyl)-1H-isoindol-5-carboxamid (konz. wässeriges Ammoniak]; 2,3-Dihydro-2-(phenylmethyl)-1H-isoindol-5-methanamin [Lithiumaluminiumhydrid]; N-[(tert.-butyloxy)carbonyl]-2,3-dihydro-2-(phenylmethyl)-1H-isoindol-5-methanamin [Pyrokohlensäure-di-tert.-butylester], Fp.: 114-115°C; N-[(tert.-Butyloxy)carbonyl]-2,3-dihydro-1H-isoindol-5-methanamin [Wasserstoff/Palladium/Tierkohle], Fp.: 114-115°C; N-[(tert.-Butyloxy)carbonyl]-2,3-dihydro-2-[(diphenylamino)carbonyl]-1H-isoindol-5-methanamin [Diphenylcarbamoylchlorid], zuletzt durch Umsetzung mit methanolischer Chlorwasserstofflösung, dann mit Natronlauge) und TBTU in einer Ausbeute von 34 % der Theorie.
Farblose Kristalle.
IR (CH₂Cl₂): 1630, 1655 cm⁻¹ (C=O)

### b) N-[[2,3-Dihydro-2-[(diphenylamino)carbonyl]-1H-isoindol-5-yl]methyl]-N²-(diphenylacetyl)-argininamid

Hergestellt analog Beispiel 1c) aus N⁵-[Amino(nitroimino)methyl]-N-[[2,3-dihydro-2-[(diphenylamino)carbonyl]-1H-isoindol-5-yl]methyl]-N²-(diphenylacetyl)-ornithinamid durch katalytische Hydrierung in Gegenwart von Palladiummohr und 80%iger wässeriger Essigsäure in einer Ausbeute von 60 % der Theorie.
Farblose, amorphe Substanz vom R_{f} 0,68.
IR (KBr): 1630-1690 cm⁻¹ (C=O)
EI-MS: (M+H)⁺ = 694

### Beispiel 47

### N²-(Diphenylacetyl)-N-[3-[1-[2-[5,11-dihydro-6(6H)-oxopyrido[2,3-b][1,4]benzodiazepin-5-yl]-2-oxoethyl]-4-piperidinyl]propyl]-argininamid

### a) N⁵-[Amino(nitroimino)methyl]-N²-(diphenylacetyl)-N-[3-[1-[2-[5,11-dihydro-6(6H)oxo-pyrido[2,3-b][1,4]-benzodiazepin-5-yl]-2-oxoethyl]-4-piperidinyl]propyl]ornithinamid

Hergestellt analog Beispiel 8a) aus (R)-N⁵-[Amino(nitroimino)methyl]-N²-(diphenylacetyl)-ornithin, [3-[1-[2-[5,11-Dihydro-6(6H)oxo-pyrido[2,3-b][1,4]benzodiazepin-5-yl]-2-oxo ethyl]-4-piperidinyl]-propanamin (Fp.: 232-234°C; hergestellt aus 2-Brom-N-[(tert.-butyloxy)carbonyl]-ethanamin über N-[(tert.-Butyloxy)carbonyl]-3-(4-pyridinyl)-propanamin [4-Picolin/n-Butyllithium]; N-[(tert.-Butyloxy)carbonyl]-3-(4-piperidinyl)propanamin-3-[1-[2-[5,11-dihydro-6(6H)oxo-pyrido[2,3-b][1,4]benzodiazepin-5-yl]-2-oxoethyl]-4-piperidinyl]propanamin [11-(Chloracetyl)-5,11-dihydro-6H-pyrido[2,3-b]-[1,4]benzodiazpin-6-on] und Behandlung mit einer Lösung von Bromwasserstoff in Eisessig) und TBTU in einer Ausbeute von 53 % der Theorie.
Farblose, amorphe Substanz, die roh weiterverarbeitet wurde.

### b) N²-(Diphenylacetyl)-N-[3-[1-[2-[5,11-dihydro-6(6H)oxopyrido[2,3-b][1,4]benzodiazepin-5-yl]-2-oxoethyl]-4-piperidinyl]propyl]-argininamid

Hergestellt analog Beispiel 1c) aus N⁵-[Amino(nitroimino)methyl]-N²-(diphenylacetyl)-N-[3-[1-[2-[5,11-dihydro-6(6H)-oxo-pyrido[2,3-b][1,4]benzodiazepin-5-yl]-2-oxoethyl]-4-piperidinyl]propyl]-ornithinamid durch katalytische Hydrierung in Gegenwart von Palladiummohr und 80%iger wässeriger Essigsäure in einer Ausbeute von 60 % der Theorie.
Farblose Kristalle vom Fp. 172-174°C und R_{f} 0,23.
IR (KBr): 1630-1690 cm⁻¹ (C=O)
ESI-MS: (M+H)⁺ = 744

### Beispiel 48

### N-[(1,1'-Biphenyl)-2-ylmethyl]-N²-(diphenylacetyl)-argininamid-acetat

### a) N⁵-[Amino(nitroimino)methyl]-N-([1,1'-biphenyl]-2-yl-methyl)-N²-(diphenylacetyl)-ornithinamid

Hergestellt analog Beispiel 5d) aus (R)-N⁵-[Amino(nitroimino)methyl]-N²-(diphenylacetyl)-ornithin, [1,1-Biphenyl]-2-methanamin und TBTU in einer Ausbeute von 42 % der Theorie.
Farblose Kristalle vom Fp. 205-207°C.
IR (KBr): 1635, 1655, 1680 cm⁻¹ (C=O, C=N)
   - ESI-MS:: (M+H)⁺ = 579
   (M+Na)⁺ = 601
   (2M+Na)⁺ = 1179

### b) N-([1,1'-Biphenyl]-2-ylmethyl)-N²-(diphenylacetyl)-argininamid-acetat

Hergestellt analog Beispiel 1c) aus N⁵-[Amino(nitroimino)-methyl]-N-([1,1'-biphenyl]-2-ylmethyl)-N²-(diphenylacetyl)-ornithinamid durch katalytische Hydrierung in Gegenwart von Palladiummohr und 80%iger wässeriger Essigsäure in einer Ausbeute von 89 % der Theorie.
Farblose Kristalle vom Fp. 106-108°C und R_{f} 0,75.
IR (CH₂Cl₂): 1630-1690 cm⁻¹ (C=O)
EI-MS: (M+H)⁺ = 534

### Beispiel 49

### N²-(Diphenylacetyl)-N-[(4-hydroxy-3-methoxyphenyl)methyl]-argininamid-acetat

### a) N⁵-[Amino(nitroimino)methyl]-N²-(diphenylacetyl)-N-[(4-hydroxy-3-methoxyphenyl)methyl]-ornithinamid

Hergestellt analog Beispiel 5d) aus (R)-N⁵-[Amino(nitroimino)methyl]-N²-(diphenylacetyl)-ornithin, 4-Hydroxy-3-methoxybenzenmethanamin und TBTU in einer Ausbeute von 14 % der Theorie.
Farblose Kristalle vom Fp. 197-198°C (Essigsäureethylester).

### b) N²-(Diphenylacetyl)-N-[(4-hydroxy-3-methoxyphenyl)-methyl]-argininamid-acetat

Hergestellt analog Beispiel 1c) aus N⁵-[Amino(nitroimino)-methyl]-N²-(diphenylacetyl)-N-[(4-hydroxy-3-methoxyphenyl)- methyl]-ornithinamid durch katalytische Hydrierung in Gegenwart von Palladiummohr und 80%iger wässeriger Essigsäure in einer Ausbeute von 97 % der Theorie.
Farblose amorphe Substanz vom R_{f} 0,65.
EI-MS: (M+H)⁺ = 504

### Beispiel 50

### N-[(3,4-Dimethoxyphenyl)methyl]-N²-(diphenylacetyl)-argininamid

### a) N⁵-[Amino(nitroimino)methyl]-N-[(3,4-dimethoxyphenyl)-methyl]-N²-(diphenylacetyl)-ornithinamid

Hergestellt analog Beispiel 5d) aus (R)-N⁵-[Amino(nitroimino)methyl]-N²-(diphenylacetyl)-ornithin, 3,4-Dimethoxybenzenmethanamin und TBTU in einer Ausbeute von 68 % der Theorie.
Farblose Kristalle vom Fp. 135-137°C (Methanol).
IR (KBr): 1640 cm⁻¹ (C=O)
   - ESI-MS:: (M+H)⁺ = 563
   (M+Na)⁺ = 585
   (M+K)⁺ = 601

### b) N-[(3,4-Dimethoxyphenyl)methyl]-N²-(diphenylacetyl)-argininamid

Hergestellt analog Beispiel 1c) aus N⁵-[Amino(nitroimino)-methyl]-N-[(3,4-dimethoxyphenyl)methyl]-N²-(diphenylacetyl)-ornithinamid durch katalytische Hydrierung in Gegenwart von Palladiummohr und 80%iger wässeriger Essigsäure in einer Ausbeute von 58 % der Theorie.
Farblose, amorphe Substanz R_{f} 0,62.
ESI-MS: (M+H)⁺ = 518

### Beispiel 51

### N-(Diphenylacetyl)-N-[(3-hydroxy-4-methoxyphenyl)methyl]-argininamid-acetat

### a) N⁵-[Amino(nitroimino)methyl]-N²-(diphenylacetyl)-N-[(3-hydroxy-4-methoxyphenyl)methyl]-ornithinamid

Hergestellt analog Beispiel 5d) aus (R)-N⁵-[Amino(nitroimino)methyl]-N²-(diphenylacetyl)-ornithin, 3-Hydroxy-4-methoxybenzenmethanamin (Fp.: 162-163°C [Methanol], hergestellt aus 3-Hydroxy-4-methoxybenzaldehyd durch katalytische Hydrierung in Gegenwart von Ammoniak und Raney-Nickel) und TBTU in einer Ausbeute von 66 % der Theorie.
Farblose Kristalle vom Fp. 183-185°C (Essigsäureethylester).
IR (KBr): 1675, 1655, 1630 cm⁻¹ (C=O, C=N)
   - ESI-MS:: (M+H)⁺ = 549
   (M+Na)⁺ = 571
   (M+K)⁺ = 587
   (2M+Na)⁺ = 1119

### b) N²-(Diphenylacetyl)-N-[(3-hydroxy-4-methoxyphenyl)-methyl]-argininamid-acetat

Hergestellt analog Beispiel 1c) aus N⁵-[Amino(nitroimino)-methyl]-N²-(diphenylacetyl)-N-[(3-hydroxy-4-methoxyphenyl)-methyl]-ornithinamid durch katalytische Hydrierung in Gegenwart von Palladiummohr und 80%iger wässeriger Essigsäure in einer Ausbeute von 90 % der Theorie.
Farblose Kristalle vom Fp. 152°C und R_{f} 0,67.
IR (KBr): 1620-1690 cm⁻¹ (C=O)
ESI-MS: (M+H)⁺ = 504

### Beispiel 52

### (R,S)-N-[(4-Amino-3,5-dibromphenyl)methyl]-N⁶-(aminoiminomethyl)-N²-(diphenylacetyl)-lysinamid

### a) (R,S)-N²-(Diphenylacetyl)-lysin

Hergestellt analog Beispiel 27c) aus (R,S)-N²-(Diphenylacetyl)-N⁶-[(phenylmethoxy)carbonyl]-lysin durch katalytische Hydrierung in Gegenwart von Palladium/Tierkohle.
Ausbeute: 68 % der Theorie.
   Farblose Kristalle.
IR (KBr): 1660 cm⁻¹ (C=O)

### b) (R,S)-N⁶-[(tert.-Butyloxy)carbonyl]-N²-(diphenylacetyl)-lysin

Zu der Lösung von 1,0 g (2,938 mMol) (R,S)-N²-(Diphenylacetyl)-lysin in 6 ml (6 mMol) 1N Natronlauge und 10 ml Tetrahydrofuran gab man 0,66 g (3,024 mMol) Di-tert.-butyl-dicarbonat und rührte anschließend 1 Stunde lang bei Zimmertemperatur. Man verdünnte mit 20 ml Wasser, schüttelte einmal mit 30 ml tert.-Butyl-methylether aus und befreite anschließend die wässerige Phase von organischen Lösemitteln durch Destillation im Vakuum. Die wässerige Phase wurde dann mit 20%iger wässeriger Zitronensäure-Lösung angesäuert, worauf man vom Niederschlag abdekandierte und den Rückstand in Aceton aufnahm. Die Acetonlösung wurde über Natriumsulfat getrocknet und im Vakuum eingedampft. Man erhielt 1,1 g (85 % der Theorie) einer nach einigen Tagen langsam kristallisierenden Substanz.
IR (CH₂Cl₂): 1715 (Ester-C=O), 1675 (Amid-C=O) cm⁻¹

### c) (R,S)-N-[(4-Amino-3,5-dibromphenyl)methyl]-N⁶-[(tert.-butyloxy)carbonyl]-N²-(diphenylacetyl)-lysinamid

Zu der Lösung von 0,75 g (1,7 mMol) (R,S)-N⁶-[(tert.-Butyloxy)carbonyl]-N²-(diphenylacetyl)-lysin in 10 ml Tetrahydrofuran gab man zunächst 0,325 g (2,0 mMol) N,N'-Carbonyldiimidazol, dann nach 30-minütigem Rühren bei 40°C 0,56 g (2,0 mMol) 4-Amino-3,5-dibrombenzenmethanamin und rührte noch 1 Stunde ohne äußere Erwärmung. Man goß das Reaktionsgemisch in 30 ml Wasser ein, versetzte mit 15 ml tert.-Butylmethylether und rührte die wasserunlösliche Masse, bis sie durchkristallisiert war. Man nutschte ab, wusch den Niederschlag gründlich mit Wasser und tert.-Butyl-methylether, trocknete im Vakuum und erhielt 0,8 g (67 % der Theorie) an farblosen Kristallen vom Fp. 169-171°C.
IR (KBr): 1685, 1640 cm⁻¹ (C=O)

### d) (R,S)-N-[(4-Amino-3,5-dibromphenyl)methyl]-N²-(diphenylacetyl)-lysinamid

Hergestellt analog Beispiel 36b) aus (R,S)-N-[(4-Amino-3,5-dibromphenyl)methyl]-N⁶-[(tert.-butyloxy)carbonyl]-N²-(diphenylacetyl)-lysinamid durch Behandlung mit methanolischer Chlorwasserstofflösung in einer Ausbeute von 99 % der Theorie.
Farblose Kristalle vom Fp. 162-163°C.
IR (KBr): 1640 cm⁻¹ (C=O).
MS: M⁺ = 600/602/604 (Br₂)

### e) (R,S)-N-[(4-Amino-3,5-dibromphenyl)methyl]-N⁶-(aminoiminomethyl)-N²-(diphenylacetyl)-lysinamid

Hergestellt analog Beispiel 42g) aus (R,S)-N-[(4-Amino-3,5-dibromphenyl)methyl]-N²-(diphenylacetyl)-lysinamid und 3,5-Dimethylpyrazol-1-carbonsäureamidiniumnitrat in einer Ausbeute von 78 % der Theorie.
Farblose Kristalle vom Fp. 139-140°C (Aceton).
IR (KBr): 1640 cm⁻¹
ESI-MS: (M+H)⁺ = 643/645/647 (Br₂)

### Beispiel 53

### N²-(Diphenylacetyl)-N-[2-(4-imidazolyl)ethyl]-argininamid

### a) N⁵-[Amino(nitroimino)methyl]-N²-(diphenylacetyl)-N-[2-(4-imidazolyl)ethyl]-ornithinamid

Hergestellt analog Beispiel 5d) aus (R)-N⁵-[Amino(nitroimino)methyl]-N²-(diphenylacetyl)-ornithin, Histamin und TBTU in einer Ausbeute von 45 % der Theorie.
Farblose, amorphe Substanz vom R_{f} 0,45 (Merck DC-Fertigplatten, Kieselgel 60 F₂₅₄; Fließmittel: Ethylacetat).

### b) N²-(Diphenylacetyl)-N-[2-(4-imidazolyl)ethyl]-argininamid

Hergestellt analog Beispiel 1c) aus N⁵-[Amino(nitroimino)methyl]-N²-(diphenylacetyl)-N-[2-(4-imidazolyl)ethyl]-ornithinamid durch katalytische Hydrierung in Gegenwart von Palladiummohr und 80%iger wässeriger Essigsäure in einer Ausbeute von 88 % der Theorie.
Farblose, amorphe Substanz vom R_{f} 0,34.
IR (KBr): 1630-1690 cm⁻¹ (C=O)
   - ESI-MS:: (M+H)⁺ = 462
   (M+2H)⁺⁺ = 231

### Beispiel 54

### (R,S)-N²-(Diphenylacetyl)-N-[(4-hydroxyphenyl)methyl]-phenylalaninamid

### a) (R,S)-N²-(Diphenylacetyl)-phenylalanin

Hergestellt analog Beispiel 1a) aus Diphenylacetylchlorid und (R,S)-Phenylalanin in einer Ausbeute von 70 % der Theorie. Farblose Kristalle vom Fp. 154°C (Methanol/Wasser = 4/1 (v/v)).
IR (KBr): 1710 (Carbonsäure-C=O), 1660 (Amid-C=O) cm⁻¹

### b) (R,S)-N²-(Diphenylacetyl)-N-[(4-hydroxyphenyl)methyl]phenylalaninamid

Hergestellt analog Beispiel 8a) aus (R,S)-N²-(Diphenylacetyl)-phenylalanin, 4-Hydroxybenzenmethanamin und TBTU in einer Ausbeute von 43 % der Theorie.
Farblose Kristalle vom Fp. 117-119°C und R_{f} 0,95.
IR (KBr): 1645, 1630 cm⁻¹ (C=O)

### Beispiel 55

### N-[(3,5-Dimethyl-4-hydroxyphenyl)methyl]-N²-(diphenylacetyl)-argininamid-acetat

### a) N⁵-[Amino(nitroimino)methyl]-N-[(3,5-dimethyl-4-hydroxyphenyl)methyl]-N²-(diphenylacetyl)-ornithinamid

Hergestellt analog Beispiel 8a) aus (R)-N⁵-[Amino(nitroimino)methyl]-N²-(diphenylacetyl)-ornithin, 3,5-Dimethyl-4-hydroxybenzenmethanamin (hergestellt aus 3,5-Dimethyl-4-hydroxybenzaldehyd und Ammoniak durch katalytische Hydrierung in Gegenwart von Raney-Nickel) und TBTU.
Ausbeute: 50 % der Theorie.
   Farblose Kristalle vom Fp. 194°C (Essigsäureethylester).
   - ESI-MS:: (M+H)⁺ = 547
   (M+Na)⁺ = 569
   (2M+Na)⁺ = 1115
   (M+K)⁺ = 585

### b) N-[(3,5-Dimethyl-4-hydroxyphenyl)methyl]-N²-(diphenylacetyl)-argininamid-acetat

Hergestellt analog Beispiel 1c) aus N⁵-[Amino(nitroimino)methyl]-N-[(3,5-dimethyl-4-hydroxyphenyl)methyl]-N²-(di phenylacetyl)-ornithinamid durch katalytische Hydrierung in Gegenwart von Palladiummohr und 80%iger wässeriger Essigsäure in einer Ausbeute von 90 % der Theorie.
Farblose, amorphe Substanz vom R_{f} 0,75.
IR (KBr): 1620-1690 cm⁻¹ (C=O)
ESI-MS: (M+H)⁺ = 502

### Beispiel 56

### N-[(1H-Benzimidazol-5-yl)methyl]-N²-(diphenylacetyl)argininamid

### a) N⁵-[Amino(nitroimino)methyl]-N-[(1H-benzimidazol-5-yl)methyl]-N²-(diphenylacetyl)-ornithinamid

Hergestellt analog Beispiel 8a) aus (R)-N⁵-[Amino(nitroimino)methyl]-N²-(diphenylacetyl)-ornithin, 1H-Benzimidazol-5-methanamin (Fp.: 134°C; hergestellt aus 1H-Benzimidazol-5-carbonsäure über 1H-Benzimidazol-5-carbonamid [Ammoniumcarbonat], Fp.: 266°C; 1H-Benzimidazol-5-carbonitril [Phosphoroxidchlorid/Piperidin], Fp.: 230-231°C; dann katalytische Hydrierung in Gegenwart von Ammoniak und Raney-Nickel) und TBTU.
Ausbeute: 36 % der Theorie.
   Farblose Kristalle vom Fp. 158-160°C.
IR (KBr): 1635 cm⁻¹ (C=O)
   - ESI-MS:: (M+H)⁺ = 543
   (M+Na)⁺ = 565

### b) N-[(1H-Benzimidazol-5-yl)methyl]-N²-(diphenylacetyl)argininamid

Hergestellt analog Beispiel 1c) aus N⁵-[Amino(nitroimino)methyl]-N-[(1H-benzimidazol-1-yl)methyl]-N²-(diphenylacetyl)-ornithinamid durch katalytische Hydrierung in Gegenwart von Palladiummohr und 80%iger wässeriger Essigsäure in einer Ausbeute von 21 % der Theorie.
Farblose, amorphe Substanz vom R_{f} 0,45.
IR (KBr): 1620-1690 cm⁻¹ (C=O).
ESI-MS: (M+H)⁺ = 498

### Beispiel 57

### N²-(Diphenylacetyl)-N-[(4-hydroxy-3-methylphenyl)methyl]argininamid-acetat

### a) N⁵-[Amino(nitroimino)methyl]-N²-(diphenylacetyl)-N-[(4-hydroxy-3-methylphenyl)methyl]-ornithinamid

Hergestellt analog Beispiel 8a) aus (R)-N⁵-[Amino(nitroimino)methyl]-N²-(diphenylacetyl)-ornithin, 4-Hydroxy-3-methylbenzenmethanamin (Fp.: 108°C; hergestellt aus 4-Hydroxy-3-methylbenzaldehyd und Ammoniak durch katalytische Hydrierung in Gegenwart von Raney-Nickel) und TBTU in einer Ausbeute von 71 % der Theorie.
Farblose, amorphe Substanz.
IR (KBr): 1620-1690 cm⁻¹ (C=O).
   - ESI-MS:: (M+H)⁺ = 533.
   (M+Na)⁺ = 555

### b) N²-(Diphenylacetyl)-N-[(4-hydroxy-3-methylphenyl)methyl]-argininamid-acetat

Hergestellt analog Beispiel 1c) aus N⁵-[Amino(nitroimino)methyl]-N²-(diphenylacetyl)-N-[(4-hydroxy-3-methylphenyl)methyl]-ornithinamid durch katalytische Hydrierung in Gegenwart von Palladiumwohr und 80%iger wässeriger Essigsäure in einer Ausbeute von 83 % der Theorie.
Farblose, amorphe Substanz vom R_{f} 0,72.
IR (KBr): 1620-1690 cm⁻¹ (C=O)
ESI-MS: (M+H)⁺ = 488

### Beispiel 58

### N-[[4-(Aminocarbonyl)phenyl]methyl]-N²-(diphenylacetyl)argininamid-acetat

### a) N-[[4-(Aminocarbonyl)phenyl]methyl]-N⁵-[amino(nitroimino)methyl]-N²-(diphenylacetyl)-ornithinamid

Hergestellt analog Beispiel 8a) aus (R)-N⁵-[Amino(nitroimino)methyl]-N²-(diphenylacetyl)-ornithin, 4-(Aminocarbonyl)-benzenmethanamin (Fp.: 146°C; hergestellt aus 4-Cyanbenzoylchlorid über 4-Cyanbenzamid (Fp.: 227°C) durch katalytische Hydrierung in Gegenwart von Ammoniak und Raney-Nickel) und TBTU in einer Ausbeute von 76 % der Theorie. Farblose Kristalle vom Fp. 215-216°C (Z.) Essigsäureethylester).
IR (KBr): 1665, 1640 cm⁻¹ (C=O, C=N)

### b) N-[[4-(Aminocarbonyl)phenyl]methyl]-N²-(diphenylacetyl)argininamid-acetat

Hergestellt analog Beispiel 1c) aus N-[[4-(Aminocarbonyl)phenyl]methyl]-N⁵-[amino(nitroimino)methyl]-N²-(diphenylacetyl)-ornithinamid durch katalytische Hydrierung in Gegenwart von Palladiummohr und 80%iger wässeriger Essigsäure in einer Ausbeute von 85 % der Theorie.
Farblose, amorphe Substanz vom R_{f} 0,60.
IR (KBr): 1630-1690 cm⁻¹ (C=O)
ESI-MS: (M+H)⁺ = 501

### Beispiel 59

### (R,S)-N⁶-(Aminoiminomethyl)-N-[(3,5-dibrom-4-hydroxyphenyl)methyl]-N²-(diphenylacetyl)-lysinamid

### a) (R,S)-N⁶-[(tert.-Butyloxy)carbonyl]-N-[(3,5-dibrom-4-hydroxyphenyl)methyl]-N²-(diphenylacetyl)-lysinamid

Hergestellt analog Beispiel 52c) aus (R,S)-N⁶-[(tert.-Butyloxy)carbonyl]-N²-(diphenylacetyl)-lysin, N,N'-Carbonyl-diimidazol und 3,5-Dibrom-4-hydroxybenzenmethanamin Fp.: 179-181°C; hergestellt aus 3,5-Dibrom-4-hydroxybenzonitril durch Reduktion mit Lithiumaluminiumhydrid) in einer Ausbeute von 67 % der Theorie.
Farblose Kristalle.
IR (KBr): 1690, 1635 cm⁻¹ (C=O)

### b) (R,S)-N-[(3,5-Dibrom-4-hydroxyphenyl)methyl]-N²-(diphenylacetyl)-lysinamid

Hergestellt analog Beispiel 23b) aus (R,S)-N⁶-[(tert.-Butyloxy)carbonyl]-N-[(3,5-dibrom-4-hydroxyphenyl)methyl]-N²-(diphenylacetyl)-lysinamid durch Einwirkung von Trifluoressigsäure in einer Ausbeute von 73 % der Theorie (Fp.: 244-246°C).
IR (KBr): 1680, 1640 cm⁻¹ (C=O)
MS: M⁺ = 601/603/605 (Br₂)

### c) (R,S)-N⁶-(Aminoiminomethyl)-N-[(3,5-dibrom-4-hydroxyphenyl)methyl]-N²-(diphenylacetyl)-lysinamid

Hergestellt analog Beispiel 42g), jedoch unter Verwendung von Dimethylformamid als Lösemittel an Stelle von Tetrahydrofuran, aus (R,S)-N-[(3,5-Dibrom-4-hydroxyphenyl)methyl]-N²-(diphenylacetyl)-lysinamid und 3,5-Dimethylpyrazol-1-carbonsäureamidiniumnitrat in einer Ausbeute von 47 % der Theorie. Farblose Kristalle vom Fp. 186-188°C und R_{f} 0,72.
IR (KBr): 1655 cm⁻¹ (C=O)
EI-MS: (M+H)⁺ = 644/646/648 (BR₂)

### Beispiel 60

### (R,S)-N⁶-(Aminoiminomethyl)-N²-(diphenylacetyl)-N-[(4-hydroxyphenyl)methyl]-lysinamid

### a) (R,S)-N⁶-[(tert.-Butyloxy)carbonyl]-N²-(diphenylacetyl)-N-[(4-hydroxyphenyl)methyl]-lysinamid

Hergestellt analog Beispiel 8a) aus (R,S)-N⁶-[(tert.-Butyloxy)carbonyl]-N²-diphenylacetyl)-lysin, 4-Hydroxybenzenmethanamin und TBTU in einer Ausbeute von 51 % der Theorie. Farblose Kristalle vom Fp. 161-162°C.
- IR (KBr):: 3280 (OH, NH), 1695, 1680, 1635 (C=O),
1520 (Amid-II) cm⁻¹

### b) (R,S)-N²-(Diphenylacetyl)-N-[(4-hydroxyphenyl)methyl]lysinamid

Hergestellt analog Beispiel 23b) aus (R,S)-N⁶-[(tert.-Butyloxy)carbonyl]-N²-(diphenylacetyl)-N-[(4-hydroxyphenyl)methyl]-lysinamid durch Einwirkung von Trifluoressigsäure in einer Ausbeute von 78 % der Theorie.
Farblose Kristalle vom Fp. 198-200°C (Aceton).
IR (KBr): 1650, 1680 cm⁻¹ (C=O)

### c) (R,S)-N⁶-(Aminoiminomethyl)-N²-(diphenylacetyl)-N-[(4-hydroxyphenyl)methyl]-lysinamid

Hergestellt analog Beispiel 59c) aus (R,S)-N²-Diphenylacetyl)-N-[(4-hydroxyphenyl)methyl]-lysinamid und 3,5-Dimethylpyrazol-1-carbonsäureamidiniumnitrat in einer Ausbeute von 37 % der Theorie.
Farblose Kristalle vom Fp. 194-197°C und R_{f} 0,72.
IR (KBr): 1640 cm⁻¹ (C=O).
EI-MS: (M+H)⁺ = 488

### Beispiel 61

### (R,S)-N⁶-(Aminoiminomethyl)-N²-(diphenylacetyl)-N-[(4-methoxyphenyl)methyl]-lysinamid

### a) (R,S)-N⁶-[(tert.-Butyloxy)carbonyl]-N²-(diphenylacetyl)-N-[(4-methoxyphenyl)methyl]-lysinamid

Hergestellt analog Beispiel 8a) aus (R,S)-N⁶-[(tert.-Butyloxy)carbonyl]-N²-(diphenylacetyl)-lysin, 4-Methoxybenzenmethanamin und TBTU in einer Ausbeute von 54 % der Theorie. Farblose Kristalle vom Fp. 130-131°C (Acetonitril/Diethylether)
IR (CH₂Cl₂): 3430, 3310 cm⁻¹ (NH), 1710, 1665 cm⁻¹ (C=O), 1510 cm⁻¹ (Amid-II).

### b) (R,S)-N²-(Diphenylacetyl)-N-[(4-methoxyphenyl)methyl]lysinamid-trifluoracetat

Hergestellt analog Beispiel 5e) aus (R,S)-N⁶-[(tert.-Butyloxy)carbonyl]-N²-(diphenylacetyl)-N-[(4-methoxyphenyl)methyl]-lysinamid durch Einwirkung von Trifluoressigsäure in einer Ausbeute von 87 % der Theorie.
Farblose Kristalle vom Fp. 193°C (Essigsäureethylester).
- IR (KBr):: 3280 cm⁻¹ (NH)
1685, 1670, 1640 cm⁻¹ (C=O)

### c) (R,S)-N⁶-(Aminoiminomethyl)-N²-(diphenylacetyl)-N-[(4-methoxyphenyl)methyl]-lysinamid

Hergestellt analog Beispiel 59c) aus (R,S)-N²-(Diphenylacetyl)-N-[(4-methoxyphenyl)methyl]-lysinamid-trifluoracetat und 3,5-Dimethylpyrazol-1-carbonsäureamidiniumnitrat in einer Ausbeute von 66 % der Theorie.
Farblose Kristalle vom Fp. 140-143°C und R_{f} 0,62.
IR (KBr): 1645 cm⁻¹ (C=O)
ESI-MS: (M+H)⁺ = 502

### Beispiel 62

### (R)-N-[(4-Hydroxyphenyl)methyl]-N²-(phenylacetyl)-argininamid-acetat

### a) (R)-N⁵-[Amino(nitroimino)methyl]-N-[(4-hydroxyphenyl)methyl]-N²-(phenylacetyl)-ornithinamid

Hergestellt analog Beispiel 5f) aus (R)-N⁵-[Amino(nitroimino)methyl]-N-[(4-hydroxyphenyl)methyl]-ornithinamid, Phenylessigsäure und TBTU in einer Ausbeute von 61 % der Theorie.
Wurde ohne Reinigung weiterverarbeitet.

### b) (R)-N-[(4-Hydroxyphenyl)methyl]-N²-(phenylacetyl)argininamid-acetat

Hergestellt analog Beispiel 1c) aus (R)-N⁵-[Amino(nitroimino)methyl]-N-[(4-hydroxyphenyl)methyl]-N²-(phenylacetyl)-ornithinamid durch katalytische Hydrierung in Gegenwart von Palladiummohr und 80%iger wässeriger Essigsäure in einer Ausbeute von 76 % der Theorie.
Farblose, amorphe Substanz vom R_{f} 0,65.
IR (KBr): 1650 cm⁻¹ (C=O)
ESI-MS: (M+H)⁺ = 398

### Beispiel 63

### (R)-N²-Acetyl-N-[(4-hydroxyphenyl)methyl]-argininamidacetat

### a) (R)-N²-Acetyl-N⁵-[amino(nitroimino)methyl]-N-[(4-hydroxyphenyl)methyl]-ornithinamid

0,3 g (0,925 mMol) (R)-N⁵-[Amino(nitroimino)methyl]-N-[(4-hydroxyphenyl)methyl]-ornithinamid wurden in 3 ml Eisessig gelöst und nach Zugabe von 3 ml (0,032 Mol) Acetanhydrid 30 Minuten auf eine Reaktionstemperatur von 50°C erwärmt. Das nach dem Abdampfen der flüchtigen Komponenten verbleibende Produkt wurde ohne Reinigung weiterverarbeitet.

### b) (R)-N²-Acetyl-N-[(4-hydroxyphenyl)methyl]-argininamidacetat

Hergestellt analog Beispiel 1c) aus (R)-N²-Acetyl-N⁵-[Amino(nitroimino)methyl]-N-[(4-hydroxyphenyl)methyl]-ornithinamid durch katalytische Hydrierung in Gegenwart von Palladiummohr und 80%iger wässeriger Essigsäure in einer Ausbeute von 65 % der Theorie.
Farblose, amorphe Substanz vom R_{f} 0,45.
IR (KBr): 1655 cm⁻¹ (C=O)
ESI-MS: (M+H)⁺ = 322

### Beispiel 64

### N²-[Diphenylacetyl)-N-[(1H-indol-5-yl)methyl]-argininamidacetat

### a) N⁵-[Amino(nitroimino)methyl]-N²-(diphenylacetyl)-N-[(1H-indol-5-yl)methyl]-ornithinamid

Hergestellt analog Beispiel 5d) aus (R)-N⁵-[Amino(nitroimino)methyl]-N²-(diphenylacetyl)-ornithin, 1H-Indol-5-methanamin (Fp.: 123-124°C; erhalten aus 1H-Indol-5-carbonitril durch katalytische Hydrierung in Gegenwart von Raney-Nickel und Ammoniak) und TBTU in einer Ausbeute von 62 % der Theorie.
Farblose Kristalle vom Fp. 203-204°C.
IR (KBr): 1640 cm⁻¹ (C=O)

### b) N²-(Diphenylacetyl)-N-[(1H-indol-5-yl)methyl]-argininamid-acetat

Hergestellt analog Beispiel 1c) aus N⁵-[Amino(nitroimino)methyl]-N²-(diphenylacetyl)-N-[(1H-indol-5-yl)methyl]-ornithinamid durch katalytische Hydrierung in Gegenwart von Palladiummohr und 80%iger wässeriger Essigsäure in einer Ausbeute von 54 % der Theorie.
Farblose, amorphe Substanz vom R_{f} 0,74.
ESI-MS: (M+H)⁺ = 497

### Beispiel 65

### (R)-N-[[4-(Aminosulfonyl)phenyl]methyl]-N²-(diphenylacetyl)-argininamid

### a) (R)-N⁵-[Amino(nitroimino)methyl]-N-[[4-(aminosulfonyl)phenyl]methyl]-N²-(diphenylacetyl)-ornithinamid

Hergestellt analog Beispiel 1b) aus (R)-N⁵-[Amino(nitroimino)methyl]-N²-(diphenylacetyl)-ornithin, 4-(Aminosulfonyl)benzenmethanamin und Chlorkohlensäureisobutylester in einer Ausbeute von 30 % der Theorie.
Farblose Kristalle vom Fp. 162°C (Ethanol).
IR (KBr): 1685, 1645 cm⁻¹ (C=O)
   - ES-MS:: (M+H)⁺ = 582
   (M+Na)⁺ = 604
   (M+K)⁺ = 620

### b) (R)-N-[[4-(Aminosulfonyl)phenyl]methyl]-N²-(diphenylacetyl)-argininamid

Hergestellt analog Beispiel 1c) aus (R)-N⁵-[Amino(nitroimino)methyl]-N-[[4-(aminosulfonyl)phenyl]methyl]-N²-(diphenylacetyl)-ornithinamid durch katalytische Hydrierung in Gegenwart von Palladiummohr und 80%iger wässeriger Essigsäure in einer Ausbeute von 30 % der Theorie.
Farblose, amorphe Substanz vom R_{f} 0,60.
IR (KBr): 1630-1690 cm⁻¹ (C=O)
ESI-MS: (M+H)⁺ = 537

### Beispiel 66

### N-[[4-[(Dimethylamino)carbonyl]phenyl]methyl]-N²-(diphenylacetyl)-argininamid-acetat

### a) N⁵-[Amino(nitroimino)methyl]-N-[[4-[(dimethylamino)carbonyl]phenyl]methyl]-N²-(diphenylacetyl)-ornithinamid

Hergestellt analog Beispiel 5d) aus (R)-N⁵-[Amino(nitroimino)methyl]-N²-(diphenylacetyl)-ornithin, 4-(Dimethylaminocarbonyl)benzenmethanamin und TBTU in einer Ausbeute von 36 % der Theorie.
Farblose Kristalle vom Fp. 192°C.
IR (KBr): 1680, 1640 cm⁻¹ (C=O)

### b) N-[[4-[(Dimethylamino)carbonyl]phenyl]methyl]-N²-(diphenylacetyl)-argininamid-acetat

Hergestellt analog Beispiel 1c) aus N⁵-[Amino(nitroimino)methyl]-N-[[4-[(dimethylamino)carbonyl]phenyl]methyl]-N²-(diphenylacetyl)-ornithinamid durch katalytische Hydrierung in Gegenwart von Palladiummohr und 80%iger wässeriger Essigsäure in einer Ausbeute von 97 % der Theorie.
Farblose, amorphe Substanz vom R_{f} 0,55.
IR (KBr): 1660 cm⁻¹ (C=O)
ESI-MS: (M+H)⁺ = 529

### Beispiel 67

### (R)-N²-(Diphenylacetyl)-N-[[4-(methoxycarbonyl)phenyl]methyl]-argininamid-acetat

### a) (R)-N⁵-[Amino(nitroimino)methyl]-N²-(diphenylacetyl)N-[[4-(methoxycarbonyl)phenyl]methyl]-ornithinamid

Zu der Mischung von 2,1 g (5,08 mMol) (R)-N⁵-[Amino(nitroimino)methyl-N²-(diphenylacetyl)-ornithin, 1,61 g (5,014 mMol) TBTU und 0,7 g (5,18 mMol) HOBt in 50 ml wasserfreiem Dimethylformamid gab man unter Rühren und äußerer Kühlung mit Eiswasser die Mischung aus 2,6 ml (14,9 mMol) N,N-Diisopropyl-ethylamin und 1,1 g (5,45 mMol) 4-(Methoxycarbonyl)-benzenmethanamin (hergestellt aus 4-(Methoxycarbonyl)benzonitril durch katalytische Hydrierung unter Verwendung von Palladium/Tierkohle als Katalysator und in Gegenwart von methanolischer Salzsäure), ließ auf Zimmertemperatur erwärmen und rührte eine Stunde lang unter diesen Bedingungen. Das Reaktionsgemisch wurde in 500 ml Eiswasser eingerührt, mit 200 ml tert.-Butyl-methylether überschichtet und über Nacht durchgerührt. Man nutschte ab, wusch den Niederschlag gründlich mit Wasser durch und trocknete ihn an der Luft. Das Produkt wurde in Tetrahydrofuran/Methanol-Gemisch (1/1 (v/v)) heiß gelöst, die Lösung warm filtriert, das Filtrat im Vakuum eingedampft. Man erhielt 2,1 g (73 % der Theorie) einer farblosen kristallinen Substanz. IR (KBr): 1724.3 (Ester-C=O), 1639.4 (Amid-C=O) cm⁻¹

### b) (R)-N²-(Diphenylacetyl)-N-[[4-(methoxycarbonyl)phenyl]methyl]-argininamid-acetat

Hergestellt analog Beispiel 1c) aus (R)-N⁵-[Amino(nitroimino)methyl]-N²-(diphenylacetyl)-N-[[4-(methoxycarbonyl) phenyl]methyl]-ornithinamid durch katalytische Hydrierung in Gegenwart von Palladiummohr und 80%iger wässeriger Essigsäure in einer Ausbeute von 67 % der Theorie.
Farblose, amorphe Substanz vom R_{f} 0,68.
IR (CH₂Cl₂): 1720, 1665 cm⁻¹ (C=O)
ESI-MS: (M+H)⁺ = 516

### Beispiel 68

### (R)-N²-(Diphenylacetyl)-N-[(4-pyridinyl)methyl]-argininamid-formiat

### a) (R)-N⁵-[Amino(nitroimino)methyl]-N²-(diphenylacetyl)N-[(4-pyridinyl)methyl]-ornithinamid

Hergestellt analog Beispiel 1b) aus (R)-N⁵-[Amino(nitroimino)methyl]-N²-(diphenylacetyl)-ornithin, 4-Pyridinmethanamin und Chlorkohlensäureisobutylester in einer Ausbeute von 70 % der Theorie.
Farblose Kristalle.
IR (KBr): 1665, 1655, 1630, 1610 cm⁻¹ (C=O), C=N)
   - ESI-MS:: (M+H)⁺ = 504
   (M+Na)⁺ = 526

### b) (R)-N²-(Diphenylacetyl)-N-[[4-pyridinyl)methyl]argininamid-formiat

Hergestellt analog Beispiel 14b) aus (R)-N⁵-[Amino(nitroimino)methyl]-N²-(diphenylacetyl)-N-[(4-pyridinyl)methyl]ornithinamid durch Reduktion mit Zinn(II)-chlorid-dihydrat in Gegenwart von 60%iger wässeriger Ameisensäure in einer Ausbeute von 91 % der Theorie.
Farblose, amorphe Substanz vom R_{f} 0,39.
IR (KBr): 1650 cm⁻¹ (C=O)
ESI-MS: (M+H)⁺ = 459

### Beispiel 69

### (R)-N-[(4-Amino-3,5-dibromphenyl)methyl]-N²-(diphenylacetyl)-argininamid-hydrochlorid

### a) (R)-N-[(4-Amino-3,5-dibromphenyl)methyl]-N⁵-[amino-(nitroimino)methyl]-N²-[(tert.-butyloxy)carbonyl]ornithinamid

Hergestellt analog Beispiel 1b) aus (R)-N⁵-[Amino(nitroimino)methyl]-N²-[(tert.-butyloxy)carbonyl]-ornithin, 4-Amino-3,5-dibrombenzenmethanamin und Chlorkohlensäureisobutylester in einer Ausbeute von 80 % der Theorie.
Farblose, amorphe Substanz.
IR (KBr): 1700, 1660, 1625 cm⁻¹ (C=O, C=N)

### b) (R)-N-[(4-Amino-3,5-dibromphenyl)methyl]-N⁵-[amino-(nitroimino)methyl]-ornithinamid-hydrochlorid

Hergestellt analog Beispiel 36b) aus (R)-N-[(4-Amino-3,5-dibromphenyl)methyl]-N⁵-[amino(nitroimino)methyl]-N²-[(tert.-butyloxy)carbonyl]-ornithinamid durch Behandlung mit methanolischem Chlorwasserstoff in quantitativer Ausbeute. Farblose, amorphe Substanz, die ohne Reinigung weiterverarbeitet wurde.

### c) (R)-[(4-Amino-3,5-dibromphenyl)methyl]-N⁵-[amino(nitroimino)methyl]-N²-(diphenylacetyl)-ornithinamid

Zu der eisgekühlten Lösung von 2,518 g (5 mMol) (R)-N-[(4-Amino-3,5-dibromphenyl)methyl]-N⁵-[amino(nitroimino)methyl]ornithinamid-hydrochlorid und 2,8 ml (20,1 mMol) Triethylamin in einem Gemisch aus 80 ml Tetrahydrofuran und 50 ml Dimethylformamid tropfte man die Lösung von 1,3 g (5,64 mMol) Diphenylacetylchlorid in 20 ml Tetrahydrofuran und rührte anschließend, nach Fortnahme der Kühlung, 2 Stunden bei einer Reaktionstemperatur von 40-50°C. Nach üblicher Aufarbeitung erhielt man 2,3 g (68 % der Theorie) einer farblosen, amorphen Substanz.

### d) (R)-N-[(4-Amino-3,5-dibromphenyl)methyl]-N²-(diphenylacetyl)-argininamid-hydrochlorid

Hergestellt analog Beispiel 14b) aus (R)-[(4-Amino-3,5-dibromphenyl)methyl]-N⁵-[amino(nitroimino)methyl]-N²-(diphenylacetyl)-ornithinamid durch Reduktion mit Zinn(II)-chlorid-dihydrat in Gegenwart von 60%iger wässeriger Ameisensäure in einer Ausbeute von 17 % der Theorie.
Farblose Kristalle von R_{f} 0,65.
IR (KBr): 1645 cm⁻¹ (C=O)
EI-MS: (M+H)⁺ = 629/631/633 (Br₂)

### Beispiel 70

### (R,S)-3-[4-[(Aminoiminomethyl)amino]phenyl]-N²-(diphenylacetyl)-N-[(4-methoxyphenyl)methyl]-alaninamid-hydrochlorid

### a) (R,S)-N²-(Diphenylacetyl)-3-(4-nitrophenyl)-alanin

Hergestellt analog Beispiel 1a) aus Diphenylacetylchlorid und (R,S)-3-(4-Nitrophenyl)-alanin in Gegenwart von Natronlauge in einer Ausbeute von 100 %. Farblose Kristalle, die ohne weitere Reinigung umgesetzt wurden.

### b) (R,S)-N²-(Diphenylacetyl)-N-[(4-methoxyphenyl)methyl]-3-(4-nitrophenyl)-alaninamid

Hergestellt analog Beispiel 52c) aus (R,S)-N²-(Diphenylacetyl)-3-(4-nitrophenyl)-alanin, 4-Methoxybenzenmethanamin und N,N'-Carbonyldiimidazol in einer Ausbeute von 34 % der Theorie.
Farblose Kristalle vom Fp. 220-222°C.
IR (KBr): 3310 (N-H), 1640 (C=O) cm⁻¹
MS: M⁺ = 523

### c) (R,S)-3-(4-Aminophenyl)-N²-(diphenylacetyl)-N-[(4-methoxyphenyl)methyl]-alaninamid

Hergestellt analog Beispiel 25b) aus (R,S)-N²-(Diphenylacetyl)-N-[(4-methoxyphenyl)methyl]-3-(4-nitrophenyl)-alaninamid durch katalytische Hydrierung in Gegenwart von Palladium/Tierkohle in einer Ausbeute von 95 % der Theorie.
Farblose Kristalle vom Fp. 206-207°C.
- IR (KBr):: 3470, 3380, 3300 cm⁻¹ (N-H, NH₂)
1680, 1635 cm⁻¹ (C=O)
MS: M⁺ = 493

### d) (R,S)-3-[4-[(Aminoiminomethyl)amino]phenyl]-N²-(diphenylacetyl)-N-[(4-methoxyphenyl)methyl]-alaninamid-hydrochlorid

2,0 g (4,05 mMol) (R,S)-3-(4-Aminophenyl)-N²-(diphenylacetyl)-N-[(4-methoxyphenyl)methyl]-alaninamid wurden in dem Gemisch aus 50 ml Methanol und 50 ml Dioxan gelöst und mit 4 ml 1N wässeriger Salzsäure versetzt. Das erhaltene Gemisch wurde im Vakuum eingedampft und der verbleibende Rückstand von noch enthaltenem Wasser durch zweimaliges azeotropes Abdestillieren unter Verwendung von Toluol als Schleppmittel befreit. Das so erhaltene Hydrochlorid wurde in 50 ml Dioxan aufgeschlämmt, worauf man so viel Methanol (ca. 50 ml) zusetzte, daß die Substanz in der Wärme (ca. 60°C) vollständig in Lösung ging. Man gab 0,42 g (10 mMol) Cyanamid zu und kochte 3 Stunden unter Rückfluß. Nach nochmaliger Zugabe von 1,0 g (23,8 mMol) Cyanamid wurde weitere 15 Stunden unter Rückfluß erhitzt. Das Reaktionsgemisch wurde im Vakuum von den Lösemitteln befreit, der Rückstand in wenig Wasser suspendiert und bis zur kompletten Lösung mit Tetrahydrofuran versetzt. Dann wurde mit Kochsalz gesättigt, die Tetrahydrofuran-Phase abgetrennt und im Vakuum eingedampft, der Rückstand an Kieselgel (200-500 µm) unter Verwendung von anfangs Dichlormethan/Methanol = 9/1 (v/v), dann Dichlormethan/ Methanol/konz. wässeriges Ammoniak = 8/2/0,3 (v/v/v) chromatographisch gereinigt. Man erhielt 100 mg (4,3 % der Theorie) an farblosen Kristallen vom Fp. 149-150°C und R_{f} 0,77.
IR (KBr): 1660, 1640 cm⁻¹ (C=O)
ESI-MS: (M+H)⁺ = 536

### Beispiel 71

### (R,S)-3-[4-[[(Cyanimino)methyl)amino]phenyl]-N²-(diphenylacetyl)-N-[(4-methoxyphenyl)methyl]-alaninamid

10,0 g (20,25 mMol) (R,S)-3-(4-Aminophenyl)-N²-(diphenylacetyl)-N-[(4-methoxyphenyl)methyl]-alaninamid wurden in einem Gemisch aus 50 ml Methanol und 250 ml Tetrahydrofuran in der Wärme gelöst. Nach Zugabe von 4,0 g (40,77 mMol) N-Cyan-formimidsäureethylester wurde die Mischung 15 Stunden bei Zimmertemperatur gerührt. Der nach dem Abdestillieren der Lösemittel verbleibende Rückstand wurde an Kieselgel (200-500 µm; mobile Phase: Dichlormethan/Methanol = 9/1 (v/v) chromatographisch gereinigt. Man erhielt 6,6 g (60 % der Theorie) an farblosen Kristallen vom R_{f} 0,9.
IR (KBr): 1685, 1665, 1645 cm⁻¹ (C=O, C=N)
ESI-MS: (M+H)⁺ = 546

### Beispiel 72

### (R,S)-N²-(Diphenylacetyl)-N-[(4-methoxyphenyl)methyl]-3-[4-[[(methylamino)methyliden]amino]phenyl]-alaninamid

1,0 g (1,834 mMol) (R,S)-3-[4-[[(Cyanimino)methyl]amino]phenyl]-N²-(diphenylacetyl)-N-[(4-methoxyphenyl)methyl]alaninamid wurden in 20 ml Dimethylformamid gelöst, mit 1,7 ml (0,6 g = 18 mMol) einer wässerigen 40%igen Methylamin-Lösung versetzt und 2 Tage bei Raumtemperatur gerührt. Das Reaktionsgemisch wurde in reichlich Wasser eingerührt, der entstandene Feststoff abgenutscht und an Kieselgel (200-500 µm; mobile Phase: Dichlormethan/Methanol = 9/1 (v/v)) chromatographisch gereinigt. Man erhielt 25 mg (2,5 % der Theorie) an farblosen Kristallen vom R_{f} 0,9.
IR (KBr): 1690, 1660, 1640 cm⁻¹ (C=O, C=N)
ESI-MS: (M+H)⁺ = 535

### Beispiel 73

### (R)-N²-(Diphenylacetyl)-N-[(2-thienyl)methyl]-argininamidacetat

### a) (R)-N⁵-[Amino(nitroimino)methyl]-N-[(2-thienyl)methyl]ornithinamid

Zunächst wurde Boc-Arg(NO₂)-OH mit Thiophen-2-methanamin und TBTU entsprechend Beispiel 8a) umgesetzt, das so erhaltene (R)-N⁵-[Amino(nitroimino)methyl]-N²-[(tert.-butyloxy)carbonyl]-N-[(2-thienyl)methyl]-ornithinamid ohne Reinigung anschließend mit Trifluoressigsäure entsprechend Beispiel 23b) zur Reaktion gebracht. Das in quantitativer Ausbeute erhaltene Rohprodukt wurde ohne Reinigung weiterverarbeitet.

### b) (R)-N⁵-[Amino(nitroimino)methyl]-N²-(diphenylacetyl)-N-[(2-thienyl)methyl]-ornithinamid

Hergestellt analog Beispiel 69c) aus (R)-N⁵-[Amino(nitroimino)methyl]-N-[(2-thienyl)methyl]-ornithinamid und Diphenylacetylchlorid in einer Ausbeute von 50 % der Theorie. Farblose Kristalle vom Fp. 192°C.
- IR (KBr):: 3430, 3300 cm⁻¹ (NH)
1640 cm⁻¹ (C=O)

### c) (R)-N²-(Diphenylacetyl)-N-[(2-thienyl)methyl]-argininamid-acetat

Hergestellt analog Beispiel 1c) aus (R)-N⁵-[Amino(nitroimino)methyl]-N²-(diphenylacetyl)-N-[(2-thienyl)methyl] ornithinamid durch katalytische Hydrierung in Gegenwart von Palladiummohr und 80%iger wässeriger Essigsäure in einer Ausbeute von 12 % der Theorie. Farblose, amorphe Substanz vom R_{f} 0,72.
IR (KBr): 1660 cm⁻¹ (C=O)
EI-MS: (M+H)⁺ = 464

### Beispiel 74

### (R)-N-[(4-Hydroxyphenyl)methyl]-N²-(1-oxo-2-propylpentyl)argininamid-acetat

### a) (R)-N⁵-(Amino(nitroimino)methyl]-N-[(4-hydroxyphenyl)methyl]-N²-(1-oxo-2-propylpentyl)-ornithinamid

Hergestellt analog Beispiel 5d), jedoch unter Verwendung von Tetrahydrofuran an Stelle von Acetonitril, aus (R)-N⁵-[Amino(nitroimino)methyl]-N-[(4-hydroxyphenyl)methyl]-ornithinamid, 2-Propyl-pentansäure und TBTU in einer Ausbeute von 65 % der Theorie. Farblose, kristalline Substanz, die ohne Reinigung weiterverarbeitet wurde.

### b) (R)-N-[(4-Hydroxyphenyl)methyl]-N²-(1-oxo-2-propylpentyl)-argininamid-acetat

Hergestellt analog Beispiel 1c) aus (R)-N⁵-[Amino(nitroimino)methyl]-N-[(4-hydroxyphenyl)methyl]-N²-(1-oxo-2-propylpentyl)-ornithinamid durch katalytische Hydrierung in Gegenwart von Palladiummohr und 80%iger wässeriger Essigsäure in einer Ausbeute von 10 % der Theorie.
Farblose, amorphe Substanz vom R_{f} 0,7.
IR (KBr): 1650 cm⁻¹ (C=O)
EI-MS: (M+H)⁺ = 406

### Beispiel 75

### (R,S)-N⁶-(Iminomethyl)-N²-(diphenylacetyl)-N-[(4-methoxyphenyl)methyl]-lysinamid-hydrochlorid

Die Mischung aus 0,69 g (1,2 mMol) (R,S)-N²-(Diphenylacetyl)-N-[(4-methoxyphenyl)methyl]-lysinamid-trifluoracetat, 40 ml Acetonitril und 0,2 g (1,826 mMol) Formimidsäureethylester-hydrochlorid wurde 3 Tage bei Zimmertemperatur gerührt. Ungelöste Anteile wurden abfiltriert und das Filtrat im Vakuum eingedampft, der Rückstand an Kieselgel (J. T. Baker, Kieselgel für flash-Chromatographie, 30-60 µm, mobile Phase: Essigsäureethylester/Methanol/Eisessig = 7/3/0,3 (v/v/v)) chromatographisch gereinigt. Man erhielt 0,15 g (24 % der Theorie) einer farblosen, amorphen Substanz vom R_{f} 0,68.
IR (KBr): 1640 cm⁻¹ (C=O)

### Beispiel 76

### (R,S)-3-[3-[(Aminoiminomethyl)amino]phenyl]-N²-(diphenylacetyl)-N-[(4-methoxyphenyl)methyl]-alaninamid-hydrochlorid

### a) (R,S)-N²-(Diphenylacetyl)-3-(3-nitrophenyl)-alanin

Hergestellt analog Beispiel 1a) aus Diphenylacetylchlorid und (R,S)-3-(3-Nitrophenyl)-alanin-hydrochlorid (Fp.: 251-252°C; erhalten aus 3-Nitrobenzylchlorid über α-Acetamido-α-(3-nitrobenzyl)malonsäurediethylester [Acetamidomalonester/ Natriumethylat], Fp.: 153-154°C, durch abschließendes Kochen mit einem Gemisch aus Eisessig und 6N wässeriger Salzsäure) in Gegenwart von Natronlauge in einer Ausbeute von 58 % der Theorie.
Farblose Kristalle vom Fp. 157-158°C.
IR (KBr): 1650, 1715, 1740 cm⁻¹ (C=O)

### b) (R,S)-N²-(Diphenylacetyl)-N-[(4-methoxyphenyl)methyl]-3-(3-nitrophenyl)-alaninamid

Hergestellt analog Beispiel 1b) aus (R,S)-N²-(Diphenylacetyl)-3-(3-nitrophenyl)-alanin, 4-Methoxybenzenmethanamin und Chlorkohlensäureisobutylester in einer Ausbeute von 93 % der Theorie.
Farblose Kristalle vom Fp. 220-222.
- IR (KBr):: 1676, 1637.5 cm⁻¹ (C=O)
1352, 1527.5 cm⁻¹ (NO₂)

### c) (R,S)-3-(3-Aminophenyl)-N²-(diphenylacetyl)-N-[(4-methoxyphenyl)methyl]-alaninamid

Hergestellt analog Beispiel 25b) aus (R,S)-N²-(Diphenylacetyl)-N-[(4-methoxyphenyl)methyl]-3-(3-nitrophenyl)-alaninamid durch katalytische Hydrierung in Gegenwart von Palladium/Tierkohle in einer Ausbeute von 82 % der Theorie.
Farblose Kristalle vom Fp. 184-185°C.
IR (KBr): 1641.3 cm⁻¹ (C=O)

### d) (R,S)-3-[3-[(Aminoiminomethyl)amino]-phenyl]-N²-(diphenylacetyl)-N-[(4-methoxyphenyl)methyl]-alaninamid-hydrochlorid-dihydrat

Hergestellt analog Beispiel 70d) aus (R,S)-3-(3-Aminophenyl)-N²-(diphenylacetyl)-N-[(4-methoxyphenyl)methyl]-alaninamid und Cyanamid in einer Ausbeute von 84 % der Theorie.
Farblose Kristalle vom Fp. 127-128°C (Z.) und R_{f} 0,8.
IR (KBr): 1660.6 cm⁻¹ (C=O)
EI-MS: (M+H)⁺ = 536

### Beispiel 77

### (R,S)-N²-(Diphenylacetyl)-N-[(4-methoxyphenyl)methyl]-3-[3-[[(methylamino)methyliden]amino]phenyl]-alaninamid

### a) (R,S)-3-[3-[[(Cyanimino)methyl]amino]phenyl]-N²-(diphenylacetyl)-N-[(4-methoxyphenyl)methyl]-alaninamid

Hergestellt analog Beispiel 71 aus (R,S)-3-(3-Aminophenyl)-N²⁻(diphenylacetyl)-N-[(4-methoxyphenyl)methyl]-alaninamid und N-Cyan-formimidsäureethylester in einer Ausbeute von 86 % der Theorie.
Farblose, amorphe Substanz, die ohne Reinigung weiterverarbeitet wurde.

### b) (R,S)-N²-(Diphenylacetyl)-N-[(4-methoxyphenyl)methyl]-3-[3-[[(methylamino)methyliden]amino]phenyl]-alaninamid

Hergestellt analog Beispiel 72 aus (R,S)-3-[3-[[(Cyanimino)methyl]amino]phenyl]-N²-(diphenylacetyl)-N-[(4-methoxyphenyl)methyl]-alaninamid und Methylamin in einer Ausbeute von 26 % der Theorie.
Farblose, amorphe Substanz vom R_{f} 0,95.
IR (KBr): 1643.3 cm⁻¹ (C=O)
MS: M⁺ = 534

### Beispiel 78

### (R,S)-N²-(Diphenylacetyl)-N⁵-(iminomethyl)-N-[(4-methoxyphenyl)methyl]-ornithinamid-hydrochlorid

### a) (R,S)-N²-(Diphenylacetyl)-N-[(4-methoxyphenyl)methyl]-N⁵-[(phenylmethoxy)carbonyl]-ornithinamid

Hergestellt analog Beispiel 5d) aus (R,S)-N²-(Diphenylacetyl)-N⁵-[(phenylmethoxy)carbonyl]-ornithin und 4-Methoxybenzenmethanamin in Gegenwart von TBTU in quantitativer Ausbeute.
Farblose Kristalle, die ohne Reinigung weiterverarbeitet wurden.

### b) (R,S)-N²-(Diphenylacetyl)-N-[(4-methoxyphenyl)methyl]ornithinamid-acetat

Hergestellt analog Beispiel 27c) aus (R,S)-N²-(Diphenylacetyl)-N-[(4-methoxyphenyl)methyl]-N⁵-[(phenylmethoxy)carbonyl]-ornithinamid durch katalytische Hydrierung in Gegenwart von Palladium/Tierkohle. Ausbeute: 75 % der Theorie.
Farblose Kristalle vom Fp. 141-143°C
IR (KBr): 1649.0 cm⁻¹ (C=O)

### c) (R,S)-N²-(Diphenylacetyl)-N⁵-(iminomethyl)-N-[(4-methoxyphenyl)methyl]-ornithinamid-hydrochlorid

Hergestellt analog Beispiel 75 aus (R,S)-N²-(Diphenylacetyl)-N-[(4-methoxyphenyl)methyl]-ornithinamid-acetat und Formimidsäureethylester-hydrochlorid in Gegenwart von 4-Methylmorpholin.
Farblose Kristalle vom Fp. 145-147°C und R_{f} 0,65. Ausbeute: 20 % der Theorie.
IR (KBr): 1714.6, 1652.9 cm⁻¹ (C=N, C=O)

### Beispiel 79

### (R,S)-3-[3-(Aminoiminomethyl)phenyl]-N²-[(diphenylamino)carbonyl]-N-[(4-hydroxyphenyl)methyl]-alaninamid-hydrochlorid

### a) (R,S)-3-(3-Cyanphenyl)-alaninmethylester-hydrochlorid

Die Mischung aus 33,0 g (0,146 Mol) 3-(3-Cyanphenyl)-alaninhydrochlorid, 11 wasserfreiem Methanol und 37,5 g (0,345 Mol) Chlortrimethylsilan wurde 3 Tage bei Zimmertemperatur gerührt. Der nach Abdestillieren des Lösemittels verbleibende Rückstand wurde in 200 ml Dichlormethan aufgenommen und gründlich mit 10%iger wässeriger Natriumhydrogencarbonat-Lösung gewaschen. Das nach Trocknen und Eindampfen der Dichlormethan-Lösung verbleibende ölige Produkt wurde in trockenem Ethylacetat aufgenommen und mit etherischer Chlorwasserstofflösung ins Hydrochlorid übergeführt. Man erhielt 23,0 g (65 % der Theorie) an farblosen Kristallen vom Fp. 157-159°C.
- IR (KBr):: 1733.9 cm⁻¹ (Ester-C=O)
2229.6 cm⁻¹ (C=N)

### b) (R,S)-2-(3-Cyanphenyl)-1-(methoxycarbonyl)-ethylisocyanat

Zu der Mischung aus 9,2 g (0,038 Mol) (R,S)-3-(3-Cyanphenyl)-alaninmethylester-hydrochlorid, 150 ml wasserfreiem Dichlormethan und 14,7 ml (0,182 Mol) trockenem Pyridin tropfte man bei einer Reaktionstemperatur von 0 bis 5°C und unter Rühren 30 ml (0,0585 Mol) einer 1,95 M Lösung von Phosgen in Toluol. Man ließ weitere 2 Stunden bei 0°C rühren, filtrierte vom salzartigen Niederschlag ab und engte das Filtrat im Vakuum ein. Der verbleibende ölige Rückstand wurde in 150 ml trockenem Dichlormethan gelöst. Aliquote Teile davon wurden ohne Reinigung in den folgenden Reaktionen verwendet.

### c) (R,S)-3-(3-Cyanphenyl)-N²-[(diphenylamino)carbonyl]alaninmethylester

Die Mischung aus 50 ml (0,0127 Mol) der vorstehend beschriebenen Lösung von (R,S)-2-(3-Cyanphenyl)-1-(methoxycarbonyl)ethylisocyanat in Dichlormethan wurde mit 3,0 g (0,0177 Mol) Diphenylamin und 1,8 g (0,0147 Mol) 4-Dimethylaminopyridin 2 Stunden lang unter Rückfluß gekocht. Man verdünnte mit weiteren 50 ml Dichlormethan und wusch die organische Phase zweimal mit 5%iger wässeriger Salzsäure und einmal mit Wasser, trocknete sie mit Magnesiumsulfat und dampfte sie im Vakuum ein. Der ölige Rückstand (1,5 g, d.s. 30 % der Theorie) wurde ohne weitere Reinigung in der nächsten Stufe verwendet.
MS: M⁺ = 399

### d) (R,S)-3-(3-Cyanphenyl)-N²-[(diphenylamino)carbonyl]alanin

Die Lösung von 5,78 g (0,0145 Mol) (R,S)-3-(3-Cyanphenyl)-N²-[(diphenylamino)carbonyl]-alaninmethylester in 60 ml Tetrahydrofuran wurde unter Rühren tropfenweise mit der Lösung von 2,5 g (0,60 Mol) Lithiumhydroxid-hydrat in 10 ml Wasser versetzt. Nach einer Stunde wurde das Tetrahydrofuran im Vakuum abdestilliert und der Rückstand zwischen Ether und Wasser verteilt. Die etherische Phase wurde verworfen, die wässerige mit verdünnter Salzsäure angesäuert und mit Essigsäureethylester erschöpfend extrahiert. Die vereinigten Essigesterextrakte ergaben nach üblicher Aufarbeitung 5,4 g (97 % der Theorie) eines farblosen, langsam kristallisierenden Öls, das ohne weitere Reinigung in den folgenden Stufen umgesetzt wurde.
- IR (KBr):: 2229.6 cm⁻¹ (C=N)
1733.9, 1672.2 cm⁻¹ (C=O)

### e) (R,S)-3-(3-Cyanphenyl)-N²-[(diphenylamino)carbonyl]-N-[(4-hydroxyphenyl)methyl]-alaninamid

Hergestellt analog Beispiel 8a) aus (R,S)-3-(3-Cyanphenyl)-N²-[(diphenylamino)-carbonyl]-alanin, 4-Hydroxybenzenmethanamin und TBTU in einer Ausbeute von 53 % der Theorie. Farblose, amorphe Substanz, die ohne weitere Reinigung verwendet wurde.

### f) (R,S)-3-[3-(Aminoiminomethyl)phenyl]-N²-[(diphenylamino)carbonyl]-N-[(4-hydroxyphenyl)methyl]-alaninamidhydrochlorid

3,9 g (7,95 mMol) (R,S)-3-(3-Cyanphenyl)-N²-[(diphenylamino)carbonyl]-N-[(4-hydroxyphenyl)methyl]-alaninamid wurden in 40 ml mit trockenem Chlorwasserstoff gesättigtem wasserfreiem Ethanol gelöst und 24 Stunden lang bei Zimmertemperatur gerührt. Man destillierte das Lösemittel im Vakuum und bei einer Badtemperatur unter 30°C ab, nahm den Rückstand in 30 ml absolutem Ethanol auf und fügte 5 g (52 mMol) Ammoniumcarbonat zu. Nach abermals 24-stündigem Rühren bei Raumtemperatur wurden die ungelösten Anteile abfiltriert, das Filtrat im Vakuum eingedampft und der Rückstand zwischen Wasser und Essigsäureethylester verteilt. Die wässerige Phase wurde im Vakuum eingedampft und der Rückstand mehrmals mit wenig Wasser und Methanol sowie zuletzt mit Diethylether verrieben. Man erhielt 2,1 g (49 % der Theorie) einer farblosen, amorphen Substanz vom R_{f} 0,75.
IR (KBr): 1652.9 cm⁻¹ (C=O)
ESI-MS: (M+H)⁺ = 508

### Beispiel 80

### (R)-N-[(4-Amino-3,5-dichlorphenyl)methyl]-N²-(2-naphthoyl)argininamid-acetat

### a) (R)-N-[(4-Amino-3,5-dichlorphenyl)methyl]-N⁵-[amino-(nitroimino)methyl]-N²-(2-naphthoyl)-ornithinamid

Hergestellt analog Beispiel 38b) aus (R)-N-[(4-Amino-3,5-dichlorphenyl)methyl]-N⁵-[amino(nitroimino)methyl]-ornithinamid, 2-Naphthoesäure und TBTU in einer Ausbeute von 59 % der Theorie.
Farblose Kristalle vom Fp. 232-233°C (Methanol).
IR (KBr): 1662.5, 1633.6 cm⁻¹ (C=O)

### b) (R)-N-[(4-Amino-3,5-dichlorphenyl)methyl]-N²-(2-naphthoyl)-argininamid-acetat

Hergestellt analog Beispiel 14b) aus (R)-N-[(4-Amino-3,5-dichlorphenyl)methyl]-N⁵-[amino(nitroimino)methyl]-N²-(2-naphthoyl)-ornithinamid durch Reduktion mit Zinn(II)-chlorid-dihydrat in 60%iger wässeriger Ameisensäure in einer Ausbeute von 62 % der Theorie.
Farblose, amorphe Substanz vom R_{f} 0,65.
IR (KBr): 1652.9 cm⁻¹ (C=O)
ESI-MS: (M+H)⁺ = 501/503/505 (Cl₂)
Die Substanz ist nach MS- und NMR-Untersuchungen verunreinigt durch (R)-N-[(3,5-Dichlor-4-(formylamino)phenyl]methyl]-N²-(2-naphthyl)-argininamid-acetat.

### Beispiel 81

### (R)-N²-(Diphenylacetyl)-N-[[4-[(methylamino)carbonyl]phenyl]methyl]-argininamid-acetat

### a) (R)-N⁵-[Amino(nitroimino)methyl]-N²-(diphenylacetyl)-N-[[4-[(methylamino)carbonyl]phenyl]methyl]-ornithinamid

Hergestellt analog Beispiel 67a), jedoch unter Verwendung von Acetonitril als Lösemittel anstelle von Dimethylformamid, aus 2 (R)-N⁵-[Amnino(nitroimino)methyl]-N²-(diphenylacetyl)ornithin, 4-[(Methylamino)carbonyl]benzenmethanamin (hergestellt aus 4-Cyan-N-methylbenzamid durch katalytische Hydrierung in Gegenwart von Palladium/Tierkohle) und TBTU in einer Ausbeute von 22 % der Theorie.
Farblose, amorphe Substanz, die ohne Reinigung weiterverarbeitet wurde.

### b) (R)-N²-(Diphenylacetyl)-N-[[4-[(methylamino)carbonyl]phenyl]methyl]-argininamid-acetat

Hergestellt analog Beispiel 1c) aus (R)-N⁵-[Amino(nitroimino)methyl]-N²-(diphenylacetyl)-N-[[4-[(methylamino)carbonyl]phenyl]methyl]-ornithinamid durch katalytische Hydrierung in Gegenwart von Palladiummohr und von 80%iger wässeriger Essigsäure in einer Ausbeute von 32 % der Theorie.
Farblose, amorphe Substanz vom R_{f} 0,73.
IR (KBr): 1652.9 cm⁻¹ (C=O)
ESI-MS: (M+H)⁺ = 515

### Beispiel 82

### (R)-N-[[4-[(Butylamino)carbonyl]phenyl]methyl]-N²-diphenylacetyl)-argininamid-acetat

### a) (R)-N⁵-[Amino(nitroimino)methyl]-N-[[4-[(butylamino)carbonyl]phenyl]methyl]-N²-(diphenylacetyl)-ornithinamid

Hergestellt analog Beispiel 81a) aus (R)-N⁵-[Amino(nitroimino)methyl]-N²-(diphenylacetyl)-ornithin, 4-[(Butylamino)carbonyl]-benzenmethanamin und TBTU in einer Ausbeute von 62 % der Theorie.
Farblose Kristalle vom Fp. 182°C (Z.).
- IR (KBr):: 3377.2, 3273.0 cm⁻¹ (NH)
1668.3, 1631.7 cm⁻¹ (C=O)

### b) (R)-N-[[4-[(Butylamino)carbonyl]phenyl]methyl]-N²-(diphenylacetyl)-argininamid-acetat

Hergestellt analog Beispiel 1c) aus (R)-N⁵-[Amino(nitroimino)methyl]-N-[[4-[(butylamino)carbonyl]phenyl]methyl]-N²-(diphenylacetyl)-ornithinamid durch katalytische Hydrierung in Gegenwart von Palladiummohr und 80%iger wässeriger Essigsäure in einer Ausbeute von 45 % der Theorie.
Farblose, amorphe Substanz vom R_{f} 0,80.
IR (KBr): 1649 cm⁻¹ (C=O)
ESI-MS: (M+H)⁺ = 557

### Beispiel 83

### (R,S)-N⁵-(4,5-Dihydro-1H-imidazol-2-yl)-N²-(diphenylacetyl)-N-[(4-hydroxyphenyl)methyl]-ornithinamid

Die Mischung aus 0,5 g (1,159 mMol) (R,S)-N²-(diphenylacetyl)-N-[(4-hydroxyphenyl)methyl]-ornithinamid, 317,3 mg (1,3 mMol) 2-(Methylmercapto)-2-imidazolin-hydroiodid, 0,43 ml (2,47 mMol) N,N-Diisopropyl-ethylamin und 20 ml wasserfreiem Ethanol wurde 5 Stunden unter Rückfluß gekocht. Das Reaktionsgemisch wurde zwischen Essigsäurethylester und reichlich Wasser verteilt, die organische Phase über Natriumsulfat getrocknet, mit Aktivkohle behandelt und im Vakuum eingedampft. Chromatographische Reinigung (neutrales Aluminiumoxid, Aktivitätsstufe IV [Firma ICN Biomedicals]; mobile Phase: Dichlormethan/Methanol = 9/1 (v/v)) ergab 50 mg (6,3 % der Theorie) einer farblosen, amorphen Substanz.
MS: M⁺ = 499

### Beispiel 84

### (R)-N²-(Diphenylacetyl)-N-[[4-(hydroxycarbonyl)phenyl]methyl]-argininamid

### a) (R)-N⁵-[Amino(nitroimino)methyl]-N²-(diphenylacetyl)-N-[[4-(hydroxycarbonyl)phenyl]methyl]-ornithinamid

Hergestellt analog Beispiel 79d), jedoch unter Verwendung von Methanol als Lösemittel statt Tetrahydrofuran, aus (R)-N⁵-[Amino(nitroimino)methyl]-N²-(diphenylacetyl)-N-[[4-(methoxy)carbonyl]phenyl]-methyl]-ornithinamid und Lithiumhydroxid-hydrat in einer Ausbeute von 17 % der Theorie.
Farblose Kristalle vom Fp. 157-158°C (Z.)
IR (KBr): 1637.5 cm⁻¹ (C=O)

### b) (R)-N²-(Diphenylacetyl)-N-[[4-(hydroxycarbonyl)phenyl]methyl]-argininamid

Hergestellt analog Beispiel 1c) aus (R)-N⁵-[Amino(nitroimino)methyl]-N²-(diphenylacetyl)-N-[[4-(hydroxycarbonyl)phenyl]methyl]-argininamid durch katalytische Hydrierung in Gegenwart von Palladiummohr und 80%iger wässeriger Essigsäure in einer Ausbeute von 58 % der Theorie.
Farblose Kristalle vom Fp. 164-165°C.
IR (KBr): 1652.9 cm⁻¹ (C=O)
   - ESI-MS:: (M+H)⁺ = 502
   (M+Na)⁺ = 524

### Beispiel 85

### (R,S)-N²-(Diphenylacetyl)-N-[(4-hydroxyphenyl)methyl]-N⁵-(1H-imidazol-2-yl)-ornithinamid

Die Mischung aus 1,0 g (2,317 mMol) (R,S)-N²-(Diphenylacetyl)-N-[(4-hydroxyphenyl)methyl]-ornithinamid, 1,9 g (5,685 mMol) N-[(2,2-Diethoxy)ethyl]-S-methylthiuroniumiodid, 1 ml (7,17 mMol) Triethylamin und 20 ml Ethanol wurde 2 Stunden lang unter Rückfluß gekocht, wobei Methanthiol freigesetzt wurde. Man dampfte die Reaktionsmischung im Vakuum ein, nahm den Rückstand in 5 ml Methanol auf, setzte 5 ml konzentrierte Salzsäure zu und rührte eine Stunde bei Zimmertemperatur. Dann wurde das Methanol im Vakuum entfernt, die wässerige Phase einmal mit Essigsäureethylester (20 ml) ausgeschüttelt und dann deutlich ammoniakalisch gestellt. Man rührte 15 Minuten kräftig durch, nutschte die entstandenen Kristalle ab und kristallisierte sie je einmal aus der erforderlichen Menge Dioxan/Methanol (9/1 (v/v)) und Tetrahydrofuran/Methanol (9/1 (v/v)) um und erhielt 430 mg (37 % der Theorie) an farblosen Kristallen vom Fp. 244-245°C (Z.)
IR (KBr): 1647.1 cm⁻¹ (C=O)
MS: M⁺ = 497

### Beispiel 86

### (R,S)-3-[3-(Aminoiminomethyl)phenyl]-N²-[(hexahydro-1H-azepin-1-yl)carbonyl]-N-[(4-hydroxyphenyl)methyl]-alaninamid-hydrochlorid

### a) (R,S)-3-(3-Cyanphenyl)-N²-[(hexahydro-1H-azepin-1-yl)carbonyl]-alaninmethylester

Hergestellt analog Beispiel 79c) aus (R,S)-2-(3-Cyanphenyl)-1-(methoxycarbonyl)ethylisocyanat und Hexamethylenimin in einer Ausbeute von 100 % der Theorie.
Farblose Kristalle vom Fp. 111-112°C (Dichlormethan).
- IR (KBr):: 2227.5 cm⁻¹ (C≡N)
1743.5 cm⁻¹ (Ester-C=O)
1624.0 cm⁻¹ (Amid-C=O)

### b) (R,S)-3-(3-Cyanphenyl)-N²-[(hexahydro-1H-azepin-1-yl)carbonyl]-alanin

Hergestellt analog Beispiel 79d) aus (R,S)-3-(3-Cyanphenyl)-N²-[(hexahydro-1H-azepin-1-yl)-carbonyl]-alaninmethylester und Lithiumhydroxid-hydrat in einer Ausbeute von 67 % der Theorie.
Farblose Kristalle, die ohne Reinigung weiterverarbeitet wurden.

### c) (R,S)-3-(3-Cyanphenyl)-N²-[(hexahydro-1H-azepin-1-yl)carbonyl]-N-[(4-hydroxyphenyl)methyl]-alaninamid

Hergestellt analog Beispiel 8a) aus (R,S)-3-(3-Cyanphenyl)-N²-[(hexahydro-1H-azepin-1-yl)carbonyl]-N-[(4-hydroxyphenyl)methyl]-alanin, 4-Hydroxybenzenmethanamin und TBTU in einer Ausbeute von 64 % der Theorie.
Farblose Kristalle vom Fp. 155-157°C.
- IR (KBr):: 3294.2 cm⁻¹ (NH)
2227.7 cm⁻¹ (C≡N)
1664.5 cm⁻¹ (Amid-C=O)

### d) (R,S)-3-[3-(Aminoiminomethyl)phenyl]-N²-[(hexahydro-1H-azepin-1-yl)carbonyl]-N-[(4-hydroxyphenyl)methyl]-alaninamid-hydrochlorid

Hergestellt analog Beispiel 79f)) aus (R,S)-3-(3-Cyanphenyl)-N²-[(hexahydro-1H-azepin-1-yl)-carbonyl]-N-[(4-hydroxyphenyl)methyl]-alaninamid unter Verwendung von zunächst ethanolischer Chlorwasserstofflösung und anschließend Ammoniumcarbonat.
Ausbeute: 11 % der Theorie.
   Farblose Kristalle vom Fp. 164-166°C.
ESI-MS: (M+H)⁺ = 438

### Beispiel 87

### (R)-N-[(4-Amino-3,5-dichlorphenyl)methyl]-N²-[(4-amino-3,5-dichlorphenyl)sulfonyl]-argininamid

### a) (R)-N-[(4-Amino-3,5-dichlorphenyl)methyl]-N²-[(4-amino-3,5-dichlorphenyl)sulfonyl]-N⁵-[amino(nitroimino)methyl]-ornithinamid

Hergestellt analog Beispiel 8a), jedoch unter Verwendung von 4-Methylmorpholin anstelle von Triethylamin, aus (R)-N²-[(4-Amino-3,5-dichlorphenyl)sulfonyl]-N⁵-[amino(nitroimino)methyl]-ornithin und 4-Amino-3,5-dichlor-benzenmnethanamin in einer Ausbeute von 67 % der Theorie.
Farblose Kristalle vom Fp. 186-188°C.
- IR (KBr):: 1643.3, 1621.4 cm⁻¹ (Amid-C=O, NO₂)
1336.6, 1161.1 cm⁻¹ (SO₂-N)

### b) (R)-N-[(4-Amino-3,5-dichlorphenyl)methyl]-N²-[(4-amino-3'5-dichlorphenyl)sulfonyl]-argininamid

Hergestellt analog Beispiel 14b) aus (R)-N-[(4-Amino-3,5-dichlorphenyl)methyl]-N²-[(4-amino-3,5-dichlorphenyl)sulfonyl]-N⁵-[amino(nitroimino)methyl]-ornithinamid durch Reduktion mit Zinn(II)-chlorid-dihydrat in Gegenwart von 60%iger wässeriger Ameisensäure und in einer Ausbeute von 67 % der Theorie.
Farblose Kristalle vom Fp. 254-256 und R_{f} 0,78.
- IR (KBr):: 1672.2, 1625.9 cm⁻¹ (C=O)
1342.4, 1137.9 cm⁻¹ (SO₂-N)
ESI-MS: (M+H)⁺ = 570/572/574/576/578 (Cl₄)

### Beispiel 88

### (R)-N-[[3-[(1,2-Dihydro-3,5(4H)-dioxo-1,2-diphenyl-3H-1,2,4-triazol-4-yl)methyl]phenyl]methyl]-N²-(diphenylacetyl)argininamid

### a) (R)-N⁵-[Amino(nitroimino)methyl]-N²-[(tert.-butyloxy)carbonyl]-N-[[3-[(1,2-dihydro-3,5(4H)-dioxo-1,2-diphenyl-3H-1,2,4-triazol-4-yl)methyl]phenyl]methyl]-ornithinamid

Hergestellt analog Beispiel 13a), jedoch unter Verwendung eines Gemisches aus Tetrahydrofuran/Dimethylformamid (1/1 (v/v) als Lösemittel an Stelle von Tetrahydrofuran, aus Boc-D-Arg(NO₂)-OH und 3-[(1,2-Dihydro-3,5(4H)-dioxo-1,2-diphenyl-3H-1,2,4-triazol-4-yl)methyl]-benzenmethanamin in einer Ausbeute von 28 % der Theorie.
Farblose, amorphe Substanz.
- IR (KBr):: 1785 cm⁻¹ (Fünfring-C=O)
1725 cm⁻¹ (Ester-C=O)
1660, 1630 cm⁻¹ (Amid-C=O)
- ESI-MS:: (M+H)⁺ = 674
(M+Na)⁺ = 696
(M+K)⁺ = 712

### b) (R)-N⁵-[Amino(nitroimino)methyl]-N-[[3-[(1,2-dihydro-3,5-(4H)-dioxo-1,2-diphenyl-3H-1,2,4-triazol-4-yl)methyl]phenyl]methyl]-ornithinamid

Hergestellt analog Beispiel 23b), jedoch unter Verwendung von gesättigter wässeriger Natriumcarbonat-Lösung anstelle von Ammoniak, aus (R)-N⁵-[Amino(nitroimino)methyl]-N²-[(tert.-butyloxy)carbonyl-N-[[3-[(1,2-dihydro-3,5(4H)-dioxo-1,2-diphenyl-3H-1,2,4-triazol-4-yl)methyl]phenyl]methyl]ornithinamid durch Einwirkung von Trifluoressigsäure in einer Ausbeute von 80 % der Theorie.
Farblose, amorphe Substanz, die ohne Reinigung weiterverarbeitet wurde.

### c) (R)-N⁵-[Amino(nitroimino)methyl]-N-[[3-[(1,2-dihydro-3,5(4H)-dioxo-1,2-diphenyl-3H-1,2,4-triazol-4-yl)methyl]phenyl]methyl]-N²-(diphenylacetyl)-ornithinamid

Hergestellt analog Beispiel 69c), jedoch unter Verwendung von 4-Methylmorpholin anstelle von Triethylamin und von Tetrahydrofuran als Lösemittel anstelle von Dimethylformamid, aus (R)-N⁵-[Amino(nitroimino)methyl]-N-[[3-[(1,2-dihydro-3,5-(4H)-dioxo-1,2-diphenyl-3H-1,2,4-triazol-4-yl)methyl]phenyl]methyl]-ornithinamid und Diphenylacetylchlorid in einer Ausbeute von 40 % der Theorie.
Farblose, amorphe Substanz, die ohne Reinigung weiterverarbeit wurde.
- IR (KBr):: 1785 cm⁻¹ (Triazolidindion)
1725 cm⁻¹ (Triazolidindion)
1650 cm⁻¹ (Amid-C=O)
- ESI-MS:: (M+H)⁺ = 768
(M+Na)⁺ = 790
(M+K)⁺ = 806

### d) (R)-N-[[3-[(1,2-Dihydro-3,5(4H)-dioxo-1,2-diphenyl-3H-1,2,4-triazol-4-yl)methyl]phenyl]methyl]-N²-(diphenylacetyl)-argininamid

Hergestellt analog Beispiel 1c) aus (R)-N⁵-[Amino(nitroimino)methyl]-N-[[3-[(1,2-dihydro-3,5(4H)-dioxo-1,2-diphenyl-3H-1,2,4-triazol-4-yl)methyl]phenyl]methyl]-N²-(diphenylacetyl)-ornithinamid durch katalytische Hydrierung in Gegenwart von Palladiummohr und 80%iger wässeriger Essigsäure in einer Ausbeute von 48 % der Theorie.
Farblose, amorphe Substanz von R_{f} 0,78.

### Beispiel 89

### (R,S)-N-[[3-[(1,2-Dihydro-3,5(4H)-dioxo-1,2-diphenyl-3H-1,2,4-triazol-4-yl)methyl]phenyl]methyl]-N²-(diphenylacetyl)-2-methyl-argininamid

### a) (R,S)-2-(2-Cyanethyl)-N²-(phenylmethylen)-alaninmethylester

Zu der Lösung von 10,0 g (0,052 Mol) (R,S)-N²-(Phenylmethylen)-alaninmethylester (hergestellt aus Benzaldehyd und D,L-Alaninmethylester) in 200 ml Acetonitril gab man nacheinander 14 g (0,101 Mol) Kaliumcarbonat, 1,1 g (0,00483 Mol) Benzyl-triethylammoniumchlorid und 3,3 ml (0,05 Mol) Acrylnitril und rührte die Mischung anschließend 4 Stunden bei Zimmertemperatur. Die Mischung wurde vom Lösemittel befreit, der Rückstand zwischen Wasser und Petrolether/Ethylacetat (1/1 (v/v)) verteilt, die organische Phase getrocknet, mit Aktivkohle behandelt und eingedampft. Das in einer Ausbeute von 10,0 g (82 % der Theorie) anfallende farblose Öl wurde ohne Reinigung weiterverarbeitet.
- IR (CH₂Cl₂):: 1735 cm⁻¹ (Ester-C=O)
2240 cm⁻¹ (C≡N)
MS: M⁺ = 244

### b) (R,S)-2-(2-Cyanethyl)-alaninmethylester-hydrochlorid

Die Mischung aus 10,0 g (0,041 Mol) (R,S)-2-(2-Cyanethyl)-N²-(phenylmethylen)-alaninmethylester, 25 ml Methanol und 30 ml 1N wässeriger Salzsäure wurde eine Stunde bei Zimmertemperatur gerührt. Nach Zugabe von 15 ml 4N Salzsäure und Überschichten mit 100 ml Diethylether wurde die Mischung abermals 14 Stunden gerührt. Die Etherphase wurde noch einmal mit 20 ml 1N Salzsäure ausgeschüttelt, die wässerigen Phasen danach vereint und im Vakuum eingedampft. Der verbleibende viskose Brei wurde in 50 ml Methanol aufgenommen und abermals eingedampft und dieser Vorgang ein weiteres Mal wiederholt. Man erhielt 6,5 g (82 % der Theorie) eines farblosen Kristallisats, das ohne Reinigung weiterverarbeitet wurde.

### c) (R,S)-2-(2-Cyanethyl)-N²-(diphenylacetyl)-alaninmethylester

Hergestellt analog Beispiel 69c), jedoch unter Verwendung von 4-Methylmorpholin anstelle von Triethylamin und von Acetonitril ab Lösemittel anstelle von Dimethylformamid, aus Diphenylacetylchlorid und (R,S)-2-(2-Cyanethyl)-alaninmethylester-hydrochlorid in einer Ausbeute von 42 % der Theorie.
Farblose Kristalle vom Fp. 177-179°C.
- IR (CH₂Cl₂):: 2240 cm⁻¹ (C≡N)
1740 cm⁻¹ (Ester-C=O)
1680 cm⁻¹ (Amid-C=O)
MS: M⁺ = 350

### d) (R,S)-2-(2-Cyanethyl)-N²-(diphenylacetyl)-alanin

Hergestellt analog Beispiel 79d) aus (R,S)-2-(2-Cyanethyl)-N²-(diphenylacetyl)-alaninmethylester und Lithiumhydroxid-hydrat in einer Ausbeute von 73 % der Theorie.
Farblose Kristalle vom Fp. 167-169°C (Aceton).
- IR (KBr):: 2250 cm⁻¹ (C≡N)
1750, 1715 cm⁻¹ (Carbonsäure-C=O)
1645 cm⁻¹ (Amid-C=O)

### e) (R,S)-2-(2-Cyanethyl)-N-[[3-[(1,2-dihydro-3,5(4H)-dioxo-1,2-diphenyl-3H-1,2,4-triazol-4-yl)methyl]phenyl]methyl]-N²-(diphenylacetyl)-alaninamid

Hergestellt analog Beispiel 1b) aus (R,S)-2-(2-Cyanethyl)-N²-(diphenylacetyl)-alanin, 3-[(1,2-Dihydro-3,5(4H)-dioxo-1,2-diphenyl-3H-1,2,4-triazol-4-yl)methyl]-benzenmethanamin und Chlorkohlensäureisobutylester in einer Ausbeute von 48 % der Theorie.
Farblose, amorphe Substanz, die ohne Reinigung weiterverarbeitet wurde.
- IR (KBr):: 3440, 3270 cm⁻¹ (NH, zum Teil assoziiert)
2250 cm⁻¹ (C≡N)
1780, 1720 cm⁻¹ (Triazolidindion-C=O)
1680 cm⁻¹ (Amid-C=O)
MS: M⁺ = 690

### f) (R,S)-N-[[3-[(1,2-Dihydro-3,5(4H)-dioxo-1,2-diphenyl-3H-1,2,4-triazol-4-yl)methyl]phenyl]methyl]-N²-(diphenylacetyl)-2-methyl-ornithinamid

0,69 g (1 mMol) (R,S)-2-(2-Cyanethyl)-N-[[3-[(1,2-dihydro-3,5-(4H)-dioxo-1,2-diphenyl-3H-1,2,4-triazol-4-yl)methyl]phenyl]-methyl]-N²-(diphenylacetyl)-alaninamid wurden in einer Mischung aus 10 ml Tetrahydrofuran und 50 ml mit Ammoniak gesättigtem Methanol gelöst und nach Zusatz von 0,6 g Raney-Nickel bei einem Druck von 5 bar und bei Raumtemperatur bis zur Beendigung der Wasserstoffaufnahme hydriert. Nach üblicher Aufarbeitung erhielt man 0,5 g (72 % der Theorie) einer farblosen, amorphen Substanz, die ohne Reinigung weiterverarbeitet wurde.
IR (CH₂Cl₂): 1680, 1630 cm⁻¹ (Amid-C=O)
MS: M⁺ = 694

### g) (R,S)-N-[[3-[(1,2-Dihydro-3,5(4H)-dioxo-1,2-diphenyl-3H-1,2,4-triazol-4-yl)methyl]phenyl]methyl]-N²-(diphenylacetyl)-2-methyl-argininamid

Hergestellt analog Beispiel 42g) aus (R,S)-N-[[3-[(1,2-Dihydro-3,5(4H)-dioxo-1,2-diphenyl-3H-1,2,4-triazol-4-yl)methyl]phenyl]methyl]-N²-(diphenylacetyl)-2-methyl-ornithinamid und 3,5-Dimethylpyrazol-1-carbonsäureamidiniumnitrat in einer Ausbeute von 41 % der Theorie.
Farblose, amorphe Substanz von R_{f} 0,75.
IR (KBr): 1685, 1630 cm⁻¹ (Amid-C=O)
EI-MS: (M+H)⁺ = 737

### Beispiel 90

### N⁵-[Amino(nitroimino)methyl]-N²-(diphenylacetyl)-N-[(4-hydroxyphenyl)methyl]-ornithinamid

Hergestellt analog Beispiel 8a) aus (R)-N⁵-[Amino(nitroimino)methyl]-N²-(diphenylacetyl)-ornithin, 4-Hydroxybenzenmethanamin und TBTU in einer Ausbeute von 45 % der Theorie.
Farblose Kristalle vom Fp. 172-175°C (Aceton/Diethylether).
- IR (KBr):: 3600 cm⁻¹ (O-H)
1645 cm⁻¹ (Amid-C=O)
1550, 1275 cm⁻¹ (N-NO₂)
- ESI-MS:: (M+H)⁺ = 519
(M+Na)⁺ = 541

### Beispiel 91

### N²-(Diphenylacetyl)-N-[(3-hydroxyphenyl)methyl]-argininamid-acetat

### a) N⁵-[Amino(nitroimino)methyl]-N²-(diphenylacetyl)-N-[(3-hydroxyphenyl)methyl]-ornithinamid

Hergestellt analog Beispiel 8a) aus (R)-N⁵-[Amino(nitroimino)methyl]-N²-(diphenylacetyl)-ornithin, 3-Hydroxybenzenmethanamin und TBTU in einer Ausbeute von 38 % der Theorie.
Farblose, amorphe Substanz.
- IR (KBr):: 1645 cm⁻¹ (Amid-C=O)
1550, 1275 cm⁻¹ (N-NO₂)
- ESI-MS:: (M+H)⁺ = 519
(M+NH₄)⁺ = 536
(M+Na)⁺ = 541
(M+K)⁺ = 557

### b) N²-(Diphenylacetyl)-N-[(3-hydroxyphenyl)methyl]-argininamid-acetat

Hergestellt analog Beispiel 1c) aus N⁵-[Amino(nitroimino)methyl]-N²-(diphenylacetyl)-N-[(3-hydroxyphenyl)methyl]ornithinamid durch katalytische Hydrierung in Gegenwart von Palladiummohr und 80%iger wässeriger Essigsäure in einer Ausbeute von 79 % der Theorie.
Farblose, amorphe Substanz vom R_{f} 0,75.
IR (KBr): 1635-1670 cm⁻¹ (C=O)
ESI-MS: (M+H)⁺ = 474

### Beispiel 92

### (R)-N-[[3-[(4,5-Dihydro-2,4(3H)-dioxo-5,5-diphenyl-1H-imidazol-3-yl)methyl]phenyl]methyl]-N²-(diphenylacetyl)-argininamid-acetat

### a) 3-[(3-Cyanphenyl)methyl]-4,5-dihydro-5,5-diphenyl-1H-imidazol-2,4(3H)-dion

Hergestellt durch Umsetzung von 25,5 g (0,101 Mol) 5,5-Diphenylhydantoin mit 21,15 g (0,108 Mol) 3-(Brommethyl)-benzonitril in Gegenwart von 12,0 g (0,107 Mol) Kalium-tert.-butylat und 200 ml wasserfreiem Dimethylformamid. Nach üblicher Aufarbeitung erhielt man 34,0 g (92 % der Theorie) an farblosen Kristallen vom Fp. 160-164°C.
- IR (CH₂Cl₂):: 3330 cm⁻¹ (N-H)
2220 cm⁻¹ (C≡N)
1780, 1720 cm⁻¹ (Imidazolidindion-C=O)
MS: M⁺ = 367

### b) 3-[(4,5-Dihydro-2,4(3H)-dioxo-5,5-diphenyl-1H-imidazol-3-yl)methyl]benzenmethanamin

Hergestellt analog Beispiel 89f) aus 3-[(3-Cyanphenyl)methyl]-4,5-dihydro-5,5-diphenyl-1H-imidazol-2,4(3H)-dion durch katalytische Hydrierung in Gegenwart von Raney-Nickel und Ammoniak in einer Ausbeute von 100 % der Theorie.
Farblose Kristalle.
- IR (CH₂Cl₂):: 3430 cm⁻¹ (NH)
1780, 1720 cm⁻¹ (Imidazolidindion-C=O)
MS: M⁺ = 371

### c) (R)-N⁵-[Amino(nitroimino)methyl]-N²-[(tert.-butyloxy)carbonyl]-N-[[3-[(4,5-dihydro-2,4(3H)-dioxo-5,5-diphenyl-1H-imidazol-3-yl)methyl]phenyl]methyl]-ornithinamid

Hergestellt analog Beispiel 8a) aus Boc-D-Arg(NO₂)-OH, 3-[(4,5-Dihydro-2,4(3H)-dioxo-5,5-diphenyl-1H-imidazol-3-yl)-methyl]-benzenmethanamin und TBTU in einer Ausbeute von 65 % der Theorie.
Farblose Kristalle, die ohne Reinigung weiterverarbeitet wurden.

### d) (R)-N⁵-[Amino(nitroimino)methyl]-N-[[3-[(4,5-dihydro-2,4(3H)-dioxo-5,5-diphenyl-1H-imidazol-3-yl)methyl]phenyl]methyl]-ornithinamid-trifluoracetat

Hergestellt analog Beispiel 5e) aus (R)-N⁵-[Amino(nitroimino)methyl]-N²-[(tert.-butyloxy)carbonyl]-N-[[3-[(4,5-dihydro-2,4(3H)-dioxo-5,5-diphenyl-1H-imidazol-3-yl)methyl]phenyl]methyl]-ornithinamid durch Einwirkung von Trifluoressigsäure in einer Ausbeute von 100 %.
Farblose, amorphe Substanz, die ohne Reinigung weiterverarbeitet wurde.

### e) (R)-N⁵-[Amino(nitroimino)methyl]-N-[[3-[(4,5-dihydro-2,4(3H)-dioxo-5,5-diphenyl-1H-imidazol-3-yl)methyl]phenyl]methyl]-N²-(diphenylacetyl)-ornithinamid

Hergestellt analog Beispiel 69c) aus (R)-N⁵-[Amino(nitroimino)methyl]-N-[[3-[(4,5-dihydro-2,4(3H)-dioxo-5,5-diphenyl-1H-imidazol-3-yl)methyl]phenyl]methyl]-ornithinamid-trifluoracetat und Diphenylacetylchlorid in einer Ausbeute von 55 % der Theorie.
Farblose Kristalle vom Fp. 218-220°C (Diisopropylether/ Ethylacetat = 1/1 (v/v)).
- IR (KBr):: 3430, 3310, 3240 cm⁻¹ (NH)
1770, 1710 cm⁻¹ (Imidazolidindion-C=O)
1670, 1660, 1615 cm⁻¹ (C=O, C=N)
- ESI-MS:: (M+H)⁺ = 767
(M-H)⁻ = 765

### f) R-N-[[3-[(4,5-Dihydro-2,4(3H)-dioxo-5,5-diphenyl-1H-imidazol-3-yl)methyl]phenyl]methyl]-N²-(diphenylacetyl)-argininamid-acetat

Hergestellt analog Beispiel 1c) aus (R)-N⁵-[Amino(nitroimino)methyl]-N-[[3-[(4,5-dihydro-2,4(3H)-dioxo-5,5-diphenyl-1H-imidazol-3-yl)methyl]phenyl]methyl]-N²-(diphenylacetyl)ornithinamid durch katalytische Hydrierung in Gegenwart von Palladiummohr und 80%iger wässeriger Essigsäure in einer Ausbeute von 93 % der Theorie.
Farblose, amorphe Substanz.
- IR (CH₂Cl₂):: 1770, 1715 cm⁻¹ (Imidazolidindion-C=O)
1660 cm⁻¹ (Amid-C=O)
ESI-MS: (M+H)⁺ = 722

### Beispiel 93

### (R)-N²-(Diphenylacetyl)-N-[(4-hydroxyphenyl)methyl]argininamid-acetat

Hergestellt analog Beispiel 1c) aus (R)-N⁵-[Amino(nitroimino)methyl]-N²-(diphenylacetyl)-N-[(4-hydroxyphenyl)methyl]-ornithinamid durch katalytische Hydrierung in Gegenwart von Palladiummohr und 80%iger wässeriger Essigsäure in einer Ausbeute von 81 % der Theorie.
Farblose Kristalle vom Fp. 223-225°C (Essigsäuremethylester/ Methanol = 98/2 (v/v)).
R_{f} 0,73.
IR (KBr): 1680, 1665 cm⁻¹ (Amid-C=O)
ESI-MS: (M+H)⁺ = 474
Die Base schmilzt bei Fp.: 152-154°C (Aceton).

### Beispiel 94

### (R,S)-3-(3-Cyanphenyl)-N-[[3-[(4,5-dihydro-2,4(3H)-dioxo-5,5-diphenyl-1H-imidazol-3-yl)methyl]phenyl]methyl]-N²-(diphenylacetyl)alaninamid

### a) (R,S)-N²-[(tert.-Butyloxy)carbonyl]-3-(3-cyanphenyl)alanin

Hergestellt analog Beispiel 52b) aus 3-(3-Cyanphenyl)-alaninhydrochlorid, Di-tert.-butyl-dicarbonat und Natronlauge in einer Ausbeute von 28 % der Theorie.
Farblose Kristalle.
- IR (CH₂Cl₂):: 3430 cm⁻¹ (NH, zum Teil assoziiert)
2230 cm⁻¹ (C≡N)
ESI-MS: (M-H)⁻ = 289

### b) (R,S)-N²-[(tert.-Butyloxy)carbonyl]-3-(3-cyanphenyl)-N-[[3-[(4,5-dihydro-2,4(3H)dioxo-5,5-diphenyl-1H-imidazol-3-yl)methyl]phenyl]methyl]-alaninamid

Hergestellt analog Beispiel 8a) aus (R,S)-N²-[(tert.-Butyloxy)carbonyl]-3-(3-cyanphenyl)-alanin, 3-[(4,5-Dihydro-2,4(3H)-dioxo-5,5-diphenyl-1H-imidazol-3-yl)methyl]benzenmethanamin und TBTU in einer Ausbeute von 49 % der Theorie.
Farblose Kristalle vom Fp. 185-187°C (Z.).
- IR (KBr):: 2235 cm⁻¹ (C≡N)
1780, 1725, 1715 cm⁻¹ (Ester-C=O, Imidazolidindion-C=O)
1655 cm⁻¹ (Amid-C=O)

### c) (R,S)-3-(3-Cyanphenyl)-N-[[3-[(4,5-dihydro-2,4(3H)-dioxo-5,5-diphenyl-1H-imidazol-3-yl)methyl]phenyl]methyl]alaninamid-trifluoracetat

Hergestellt analog Beispiel 5e) aus (R,S)-N²-[(tert.-Butyloxy)carbonyl]-3-(3-cyanphenyl)-N-[[3-[(4,5-dihydro-2,4(3H)-di oxo-5,5-diphenyl-1H-imidazol-3-yl)methyl]phenyl]methyl]alaninamid durch Einwirkung von Trifluoressigsäure in einer Ausbeute von 100 %.
Farblose Kristalle, die ohne Reinigung weiterverarbeitet wurden.
- IR (CH₂Cl₂):: 2230 cm⁻¹ (C≡N)
1775, 1715 cm⁻¹ (Imidazolidindion-C=O)
1675 cm⁻¹ (Amid-C=O)

### d) (R,S)-3-(3-Cyanphenyl)-N-[[3-[(4,5-dihydro-2,4(3H)-dioxo-5,5-diphenyl-1H-imidazol-3-yl)methyl]phenyl]methyl]-N²-(diphenylacetyl)-alaninamid

Hergestellt analog Beispiel 69c) aus (R,S)-3-(3-Cyanphenyl)-N-[[3-[(4,5-dihydro-2,4(3H)-dioxo-5,5-diphenyl-1H-imidazol-3-yl)-methyl]phenyl]methyl]-alaninamid-trifluoracetat und Diphenylacetylchlorid in einer Ausbeute von 45 % der Theorie. Farblose Kristalle vom Fp. 211-213°C (Essigsäureethylester).
- IR (KBr):: 3290 cm⁻¹ (NH)
2230 cm⁻¹ (C≡N)
1770, 1715 cm⁻¹ (Imidazolidindion-C=O)
1645 cm⁻¹ (Amid-C=O)
MS: M⁺ = 737

### Beispiel 95

### N²-(Diphenylacetyl)-N-[2-(4-hydroxyphenyl)ethyl]-argininamid-acetat

### a) N⁵-[Amino(nitroimino)methyl]-N²-(diphenylacetyl)-N-[2-(4-hydroxyphenyl)ethyl]-ornithinamid

Hergestellt analog Beispiel 5d) aus (R)-N⁵-[Amino(nitroimino)methyl]-N²-(diphenylacetyl)-ornithin, 2-(4-Hydroxyphenyl)ethanamin und TBTU in einer Ausbeute von 44 % der Theorie.
Farblose Kristalle vom Fp. 144-145°C.
IR (KBr): 1645 cm⁻¹ (Amid-C=O)
   - ESI-MS:: (M+H)⁺ = 533
   (M+Na)⁺ = 555

### b) N²-(Diphenylacetyl)-N-[2-(4-hydroxyphenyl)ethyl]-argininamid-acetat

Hergestellt analog Beispiel 1c) aus N⁵-[Amino(nitroimino)methyl]-N²-(diphenylacetyl)-N-[2-(4-hydroxyphenyl)ethyl]-ornithinamid durch katalytische Hydrierung in Gegenwart von Palladiummohr und 80%iger wässeriger Essigsäure in einer Ausbeute von 51 % der Theorie.
Farblose, amorphe Substanz vom R_{f} 0,75.
IR (KBr): 1655 cm⁻¹ (Amid-C=O)
ESI-MS: (M+H)⁺ = 488

### Beispiel 96

### N²-(Diphenylacetyl)-N-[(4'-hydroxy)-[1,1'-biphenyl]-4-yl)-methyl]-argininamid-acetat

### a) N⁵-[Amino(nitroimino)methyl]-N²-(diphenylacetyl)-N-[(4'-hydroxy-[1,1'-biphenyl]-4-yl)methyl]-ornithinamid

Hergestellt analog Beispiel 5d) aus (R)-N⁵-[Amino(nitroimino)methyl]-N²-(diphenylacetyl)-ornithin, 4'-Hydroxy-[1,1'-biphenyl]-4-methanamin und TBTU in einer Ausbeute von 48 % der Theorie.
Farblose, amorphe Substanz, die ohne Reinigung weiterverarbeitet wurde.
- IR (KBr):: 1640 cm⁻¹ (Amid-C=O)
1575, 1265 cm⁻¹ (N-NO₂)
1505 cm⁻¹ (Amid-II)
- ESI-MS:: (M-H)⁻ = 593
(M+Na)⁺ = 617

### b) N²-(Diphenylacetyl)-N-[(4'-hydroxy-[1,1'-biphenyl]-4-yl)methyl]-argininamid-acetat

Hergestellt analog Beispiel 1c) aus N⁵-[Amino(nitroimino)methyl]-N²-(diphenylacetyl)-N-[(4'-hydroxy-[1,1'-biphenyl]-4-yl)methyl]-ornithinamid durch katalytische Hydrierung in Gegenwart von Palladiummohr und 80%iger wässeriger Essigsäure in einer Ausbeute von 48 % der Theorie.
Farblose, amorphe Substanz vom R_{f} 0,76.
- IR (KBr):: 1655 cm⁻¹ (Amid-C=O)
1505 cm⁻¹ (Amid-II)
ESI-MS: (M+H)⁺ = 550

### Beispiel 97

### N-[[4-[(1,2-Dihydro-3,5(4H)-dioxo-1,2-diphenyl-3H-1,2,4-triazol-4-yl)methyl]phenyl]methyl]-N²-(diphenylacetyl)-argininamid-acetat

### a) N⁵-[Amino(nitroimino)methyl]-H-[[4-[(1,2-dihydro-3,5(4H)-dioxo-1,2-diphenyl-3H-1,2,4-triazol-4-yl)methyl]-phenyl]methyl]-N²-(diphenylacetyl)-ornithinamid

Hergestellt analog Beispiel 5d) aus (R)-N⁵-[Amino(nitroimino)methyl]-N²-(diphenylacetyl)-ornithin, 4-[(1,2-Dihydro-3,5(4H)-dioxo-1,2-diphenyl-3H-1,2,4-triazol-4-yl)methyl]benzenmethanamin und TBTU in einer Ausbeute von 28 % der Theorie.
Farblose, amorphe Substanz, die ohne Reinigung weiterverarbeitet wurde.
- IR (KBr):: 1755, 1725 cm⁻¹ (Triazolidindion-C=O)
1645 cm⁻¹ (Amid-C=O)
1505 cm⁻¹ (Amid-II)
- ESI-MS:: (M+H)⁺ = 768
(M+H)⁺ = 790

### b) N-[[4-[(1,2-Dihydro-3,5(4H)-dioxo-1,2-diphenyl-3H-1,2,4-triazol-4-yl)methyl]phenyl]methyl]-N²-(diphenylacetyl)argininamid-acetat

Hergestellt analog Beispiel 1c) aus N⁵-[Amino(nitroimino)methyl]-N-[[4-[(1,2-dihydro-3,5(4H)-dioxo-1,2-diphenyl-3H-1,2,4-triazol-4-yl)methyl]phenyl]methyl]-N²-(diphenylacetyl) -ornithinamid durch katalytische Hydrierung in Gegenwart von Palladiummohr und 80%iger wässeriger Essigsäure in einer Ausbeute von 21 % der Theorie.
Farblose, amorphe Substanz vom R_{f} 0,73.
- IR (KBr):: 1780, 1730 cm⁻¹ (Triazolidindion-C=O)
1660 cm⁻¹ (Amid-C=O)
ESI-MS: (M+H)⁺ = 723

### Beispiel 98

### (R,S)-N⁵-(Aminocarbonyl)-N²-(diphenylacetyl)-N-[(4-hydroxyphenyl)methyl]-ornithinamid

Hergestellt analog Beispiel 5d) aus (R,S)-N⁵-(Aminocarbonyl)-N²-(diphenylacetyl)-ornithin (erhalten entsprechend Beispiel 1a) aus Diphenylacetylchlorid und (R,S)-Citrullin) und 4-Hydroxybenzenmethanamin sowie TBTU in einer Ausbeute von 34 % der Theorie.
Farblose Kristalle vom Fp. 217-220°C und R_{f} 0,89.
IR (KBr): 1640 cm⁻¹ (Amid-C=O)
   - ESI-MS:: (M+H)⁺ = 475
   (M+Na)⁺ = 497
   (M-H)⁻ = 473

### Beispiel 99

### N²-(Diphenylacetyl)-N-[(4-methoxyphenyl)methyl]-argininamid-acetat

### a) N⁵-[Amino(nitroimino)methyl]-N²-(diphenylacetyl)-N-[(4-methoxyphenyl)methyl]-ornithinamid

Hergestellt analog Beispiel 8a) aus (R)-N⁵-[Amino(nitroimino)methyl]-N²-(diphenylacetyl)-ornithin, 4-Methoxybenzenmethanamin und TBTU in einer Ausbeute von 37 % der Theorie.
- IR (KBr):: 1635 cm⁻¹ (Amid-C=O)
1515 cm⁻¹ (Amid-II)
- ESI-MS:: (M+H)⁺ = 533
(M+Na)⁺ = 555
(M+K)⁺ = 571

### b) N²-(Diphenylacetyl)-N-[(4-methoxyphenyl)methyl]-argininamid-acetat

Hergestellt analog Beispiel 1c) aus N⁵-[Amino(nitroimino)methyl]-N²-(diphenylacetyl)-N-[(4-methoxyphenyl)methyl]ornithinamid durch katalytische Hydrierung in Gegenwart von Palladiummohr und 80%iger Essigsäure in einer Ausbeute von 85 % der Theorie.
Farblose, amorphe Substanz vom R_{f} 0,72.
IR (KBr): 1660 cm⁻¹ (Amid-C=O)
   - EI-MS:: (M+H)⁺ = 488
   (2M+H)⁺ = 975

### Beispiel 100

### N²-(Diphenylacetyl)-N-[2-(4-methoxyphenyl)ethyl]-argininamid-acetat

### a) N⁵-[Amino(nitroimino)methyl]-N²-(diphenylacetyl)-N-[2-(4-methoxyphenyl)ethyl]-ornithinamid

Hergestellt analog Beispiel 8a) aus (R)-N⁵-[Amino(nitroimino)methyl]-N²-(diphenylacetyl)-ornithin, 4-Methoxybenzenethanamin und TBTU in einer Ausbeute von 47 % der Theorie.
Farblose Kristalle, die ohne Reinigung weiterverarbeitet wurden.
IR (KBr): 1670, 1655, 1630 cm⁻¹ (C=O, C=N)
   - EI-MS:: (M+H)⁺ = 547
   (M+Na)⁺ = 569

### b) N²-(Diphenylacetyl)-N-[2-(4-methoxyphenyl)ethyl]argininamid-acetat

Hergestellt analog Beispiel 1c) aus N⁵-[Amino(nitroimino)methyl]-N²-(diphenylacetyl)-N-[2-(4-methoxyphenyl)ethyl]ornithinamid durch katalytische Hydrierung in Gegenwart von Palladiummohr und 80%iger wässeriger Essigsäure in einer Ausbeute von 43 % der Theorie.
Farblose, amorphe Substanz vom R_{f} 0,72.
IR (KBr): 1655, 1630 cm⁻¹ (Amid-C=O)
EI-MS: (M+H)⁺ = 502

### Beispiel 101

### N²-(Diphenylacetyl)-N-[(2-methoxyphenyl)methyl]-argininamid-acetat

### a) N⁵-[Amino(nitroimino)methyl]-N²-(diphenylacetyl)-N-[2-methoxyphenyl)methyl]-ornithinamid

Hergestellt analog Beispiel 8a) aus (R)-N⁵-[Amino(nitroimino)methyl]-N²-(diphenylacetyl)-ornithin, 2-Methoxybenzenmethanamin und TBTU in einer Ausbeute von 29 % der Theorie.
Farblose Kristalle vom Fp. 182-185°C.
- EI-MS:: (M+H)⁺ = 533
(M+Na)⁺ = 555
(M+K)⁺ = 571

### b) N²-(Diphenylacetyl)-N-[(2-methoxyphenyl)methyl]-argininamid-acetat

Hergestellt analog Beispiel 1c) aus N⁵-[Amino(nitroimino)methyl]-N²-(diphenylacetyl)-N-[(2-methoxyphenyl)methyl]ornithinamid durch katalytische Hydrierung in Gegenwart von Palladiummohr und 80%iger Essigsäure in einer Ausbeute von 65 % der Theorie.
Farblose, amorphe Substanz vom R_{f} 0,70.
IR (KBr): 1650 cm⁻¹ (Amid-C=O)
EI-MS: (M+H)⁺ = 488

### Beispiel 102

### N²-(Diphenylacetyl)-N-[2-(3-methoxyphenyl)ethyl]-argininamid-acetat

### a) N⁵-[Amino(nitroimino)methyl]-N²-(diphenylacetyl)-N-[2-(3-methoxyphenyl)ethyl]-ornithinamid

Hergestellt analog Beispiel 8a) aus (R)-N⁵-[Amino(nitroimino)methyl]-N²-(diphenylacetyl)-ornithin, 3-Methoxybenzenethanamin und TBTU in einer Ausbeute von 28 % der Theorie.
Farblose Kristalle vom Fp. 200-203°C (Essigsäureethylester).
IR (KBr): 1670, 1655, 1635 cm⁻¹ (C=O, C=N)
   - ESI-MS:: (M+H)⁺ = 547
   (M+Na)⁺ = 569
   (M+K)⁺ = 585

### b) N²-(Diphenylacetyl)-N-[2-(3-methoxyphenyl)ethyl]-argininamid-acetat

Hergestellt analog Beispiel 1c) aus N⁵-[Amino(nitroimino)methyl]-N²-(diphenylacetyl)-N-[2-(3-methoxyphenyl)ethyl]ornithinamid durch katalytische Hydrierung in Gegenwart von Palladiummohr und 80%iger wässeriger Essigsäure in einer Ausbeute von 63 % der Theorie.
Farblose, amorphe Substanz vom R_{f} 0,76.
IR (CH₂Cl₂): 1660 cm⁻¹ (Amid-C=O)
ESI-MS: (M+H)⁺ = 502

### Beispiel 103

### N²-[N²-(Diphenylacetyl)-D-arginyl]-L-tyrosinamid

### a) N²-[N⁵-[Amino(nitroimino)methyl]-N²-(diphenylacetyl)-D-ornithyl]-L-tyrosinamid

Hergestellt analog Beispiel 8a) aus (R)-N⁵-[Amino(nitroimino)methyl]-N²-(diphenylacetyl)-ornithin, L-Tyrosinamid und TBTU in einer Ausbeute von 47 % der Theorie.
Farblose, amorphe Substanz.
- IR (KBr):: 1660 cm⁻¹ (Amid-C=O)
1515 cm⁻¹ (Amid-II)

### b) N²-[N²-(Diphenylacetyl)-D-arginyl]-L-tyrosinamid

Hergestellt analog Beispiel 1c) aus N²-[N⁵-[Amino(nitroimino)methyl]-N²-(diphenylacetyl)-D-ornithyl]-L-tyrosinamid durch katalytische Hydrierung in Gegenwart von Palladiummohr und 80%iger wässeriger Essigsäure.
Farblose, amorphe Substanz vom R_{f} 0,70.
- IR (KBr):: 1660 cm⁻¹ (Amid-C=O)
1515 cm⁻¹ (Amid-II)
ESI-MS: (M+H)⁺ = 531

### Beispiel 104

### N²-(Diphenylacetyl)-N-[(3-methoxyphenyl)methyl]-argininamid-acetat

### a) N⁵-[Amino(nitroimino)methyl]-N²-(diphenylacetyl)-N-[(3-methoxyphenyl)methyl]-ornithinamid

Hergestellt analog Beispiel 8a) aus (R)-N⁵-[Amino(nitroimino)methyl]-N²-(diphenylacetyl)-ornithin, 3-Methoxybenzenmethanamin und TBTU in einer Ausbeute von 55 % der Theorie.
- IR (KBr):: 1670, 1655, 1635 cm⁻¹ (Amid-C=O, C=N)
1495 cm⁻¹ (Amid-II)
- ESI-MS:: (M+H)⁺ = 533
(M+Na)⁺ = 555

### b) N²-(Diphenylacetyl)-N-[(3-methoxyphenyl)methyl]-argininamid-acetat

Hergestellt analog Beispiel 1c) aus N⁵-[Amino(nitroimino)methyl]-N²-(diphenylacetyl)-N-[(3-methoxyphenyl)methyl]ornithinamid durch katalytische Hydrierung in Gegenwart von Palladiummohr und 80%iger wässeriger Essigsäure in einer Ausbeute von 93 % der Theorie.
Farblose, amorphe Substanz vom R_{f} 0,73.
- IR (CH₂Cl₂):: 1660 cm⁻¹ (C=O)
1495 cm⁻¹ (Amid-II)
ESI-MS: (M+H)⁺ = 488

### Beispiel 105

### (R,S)-3-[4-(Aminoiminomethyl)phenyl]-N²-(diphenylacetyl)-N-[(4-methoxyphenyl)methyl]-alaninamid-hydrochlorid

### a) (R,S)-3-(4-Cyanphenyl)-alanin-hydrochlorid

Die Mischung aus 235 g (0,707 Mol) α-(Acetamido)-α-[(4-cyanphenyl)methyl]-malonsäurediethylester (Fp.: 163-165°C; hergestellt aus α-Acetamido-malonsäurediethylester und 4-(Brommethyl)-benzonitril in Gegenwart von Natriumethylat), 1,28 1 (3,84 Mol) 3N wässeriger Salzsäure und 0,64 1 Eisessig wurde 7 Stunden unter Rückfluß gekocht. Die auf +5°C gekühlte Mischung wurde filtriert, das Filtrat im Vakuum eingedampft. Der Rückstand wurde intensiv mit Isopropanol gewaschen und anschließend im Vakuum getrocknet. Man erhielt 92,9 g (58 % der Theorie) an farblosen Kristallen vom Fp. 219°C (Z.).

### b) (R,S)-3-(4-Cyanphenyl)-N²-(diphenylacetyl)-alanin

Hergestellt analog Beispiel 1a) aus (R,S)-3-(4-Cyanphenyl)alanin-hydrochlorid und Diphenylacetylchlorid in Gegenwart von Natronlauge in einer Ausbeute von 82 % der Theorie. Farblose Kristalle vom Fp. 110°C (Z.).
- IR (CH₂Cl₂):: 2225 cm⁻¹ (C≡N)
1655 cm⁻¹ (Amid-C=O)

### c) (R,S)-3-(4-Cyanphenyl)-N²-(diphenylacetyl)-N-[(4-methoxyphenyl)methyl]-alaninamid

Hergestellt analog Beispiel 8a) aus (R,S)-3-(4-Cyanphenyl)-N²-(diphenylacetyl)-alanin, 4-Methoxybenzenmethanamin und TBTU in einer Ausbeute von 61 % der Theorie.
Farblose Kristalle vom Fp. 213-215°C (Isopropanol).
- IR (KBr):: 1645 cm⁻¹ (Amid-C=O)
2230 cm⁻¹ (C≡N)
3270 cm⁻¹ (N-H)
2840 cm⁻¹ (OCH₃)
1515 cm⁻¹ (Amid-II)
ESI-MS: M⁺ = 503

### d) (R,S)-3-[4-(Aminoiminomethyl)phenyl]-N²-(diphenylacetyl)-N-[(4-methoxyphenyl)methyl]-alaninamid-hydrochlorid

Hergestellt analog Beispiel 79f), jedoch unter Verwendung von Methanol anstelle von Ethanol, aus (R,S)-3-(4-Cyanphenyl)-N²-(diphenylacetyl)-N-[(4-methoxyphenyl)methyl]-alaninamid und Chlorwasserstoff, später Ammoniumcarbonat, in einer Ausbeute von 82 % der Theorie.
Farblose Kristalle vom Fp. 161-166°C und R_{f} 0,77.
IR (KBr): 1665, 1645 cm⁻¹ (C=O, C=N)
ESI-MS: (M+H)⁺ = 521

### Beispiel 106

### (R,S)-3-[4-(Aminoiminomethyl)phenyl]-N²-(diphenylacetyl)-N-[(4-hydroxyphenyl)methyl]-alaninamid-hydrochlorid

### a) (R,S)-3-(4-Cyanphenyl)-N²-(diphenylacetyl)-N-[(4-hydroxyphenyl)methyl]-alaninamid

Hergestellt analog Beispiel 8a) aus (R,S)-3-(4-Cyanphenyl)-N²-(diphenylacetyl)-alanin, 4-Hydroxybenzenmethanamin und TBTU in einer Ausbeute von 58 % der Theorie.
Farblose Kristalle vom Fp. 190-194°C (Z.).
- IR (KBr):: 2230 cm⁻¹ (C≡N)
1645 cm⁻¹ (Amid-C=O)
1515 cm⁻¹ (Amid-II)
MS: M⁺ = 489

### b) (R,S)-3-(4-Aminoiminomethyl)phenyl]-N²-(diphenylacetyl)-N-[(4-hydroxyphenyl)methyl]-alaninamid-hydrochlorid

Hergestellt analog Beispiel 105d) aus (R,S)-3-(4-Cyanphenyl]-N²-(diphenylacetyl)-N-[(4-hydroxyphenyl)methyl]alaninamid unter Verwendung von Chlorwasserstoff und anschließend Ammoniumcarbonat in einer Ausbeute von 67 % der Theorie.
Farblose Kristalle vom Fp. 136°C (Z.) und R_{f} 0,78.
- IR (KBr):: 1655 cm⁻¹ (Amid-C=O)
1515 cm⁻¹ (Amid-II)
ESI-MS: (M+H)⁺ = 507

### Beispiel 107

### (R,S)-3-(3-Cyanphenyl]-N²-(diphenylacetyl)-N-[(4-hydroxyphenyl)methyl]-alaninamid

### a) (R,S)-3-(3-Cyanphenyl)-alanin-hydrochlorid

Hergestellt analog Beispiel 105a) aus α-(Acetamido)-α-[(3-cyanphenyl)methyl]malonsäurediethylester (Fp.: 139-141°C), Salzsäure und Eisessig in einer Ausbeute von 69 % der Theorie.
Farblose Kristalle vom Fp. 206°C (Z.).

### b) (R,S)-3-(3-Cyanphenyl)-N²-(diphenylacetyl)-alanin

Hergestellt analog Beispiel 1a) aus (R,S)-3-(3-Cyanphenyl)alanin-hydrochlorid und Diphenylacetylchlorid in Gegenwart von Natronlauge in einer Ausbeute von 58 % der Theorie.
Farblose Kristalle vom Fp. 145-147°C (Essigsäureethylester).
- IR (KBr):: 3380 cm⁻¹ (N-H)
2230 cm⁻¹ (C≡N)
1725 cm⁻¹ (Carbonsäure-C=O)
1665 cm⁻¹ (Amid-C=O)
1515 cm⁻¹ (Amid-II)

### c) (R,S)-3-(3-Cyanphenyl)-N²-(diphenylacetyl)-N-[(4-hydroxyphenyl)methyl]-alaninamid

Hergestellt analog Beispiel 8a) aus (R,S)-3-(3-Cyanphenyl)-N²-(diphenylacetyl)-alanin, 4-Hydroxybenzenmethanamin und TBTU in einer Ausbeute von 58 % der Theorie.
Farblose Kristalle vom Fp. 115-118°C (Z.) (Isopropanol) und R_{f} 0,96.
- IR (KBr):: 2230 cm⁻¹ (C≡N)
1645 cm⁻¹ (Amid-C=O)
MS: M⁺ = 489

### Beispiel 108

### (R)-N,N-Diethyl-N²-(diphenylacetyl)-argininamid-acetat

### a) (R)-N⁵-[Amino(nitroimino)methyl]-N,N-diethyl-N²-(diphenylacetyl)-ornithinamid

Hergestellt analog Beispiel 1b) aus (R)-N⁵-[Amino(nitroimino)methyl]-N²-(diphenylacetyl)-ornithin, N,N-Diethylamin und Chlorkohlensäureisobutylester in einer Ausbeute von 37 % der Theorie.
Farblose Kristalle vom Fp. 141-144°C (Essigsäureethylester/ Isopropanol = 1/1( v/v)).
IR (CH₂Cl₂): 1635, 1660 cm⁻¹ (Amid-C=O)
   - ESI-MS:: (M+H)⁺ = 469
   (M+Na)⁺ = 491
   (M+K)⁺ = 507
   (M+NH₄)⁺ = 486

### b) (R)-N,N-Diethyl-N²-(diphenylacetyl)-argininamid-acetat

Hergestellt analog Beispiel 1c) aus (R)-N⁵-[Amino(nitroimino)methyl]-N,N-diethyl-N²-(diphenylacetyl)-ornithinamid durch katalytische Hydrierung in Gegenwart von Palladiummohr und 80%iger wässeriger Essigsäure in einer Ausbeute von 64 % der Theorie.
Farblose amorphe Substanz vom R_{f} 0,63.
IR (KBr): 1680, 1620 cm⁻¹ (Amid-C=O)
ESI-MS: (M+H)⁺ = 424

### Beispiel 109

### (R)-N²-(Diphenylacetyl)-N-[[4-[[[(2,2-diphenylethyl)amino]carbonyl]amino]phenyl]methyl]-argininamid-acetat

### a) (R)-N⁵-[Amino(nitroimino)methyl]-N²-(diphenylacetyl)-N-[[4-[[[(2,2-diphenylethyl)amino]carbonyl]amino]phenyl]methyl]-ornithinamid

Hergestellt analog Beispiel 13a), jedoch unter Verwendung von Dichlormethan als Lösemittel anstelle von Tetrahydrofuran und ohne Verwendung von Triethylamin, aus 4-[[[(2,2-Diphenylethyl)amino]carbonyl]amino]benzenmethanamin (Fp.: 161-163°C; erhalten aus 4-Cyanphenylisocyanat und 2,2-Diphenylethanamin über N-(4-Cyanphenyl-N'-(2,2-diphenylethyl)-harnstoff, Fp.: 235-237°C; zuletzt durch katalytische Hydrierung in Gegenwart von Raney-Nickel und Ammoniak), (R)-N⁵-[Amino(nitroimino)methyl]-N²-(diphenylacetyl)-ornithin, DCC und HOBt in einer Ausbeute von 48 % der Theorie.
Farblose Kristalle vom Fp. 274-276°C (Isopropanol).
IR (KBr): 1640 cm⁻¹ (Carboxamid-C=O)
   - ESI-MS:: (M+H)⁺ = 741
   (M+Na)⁺ = 763
   (M-H)⁻ = 739

### b) (R)-N²-(Diphenylacetyl)-N-[[4-[[[(2,2-diphenylethyl)amino]carbonyl]amino]phenyl]methyl]-argininamid-acetat

Hergestellt analog Beispiel 1c) aus (R)-N⁵-[Amino(nitroimino)methyl]-N²-(diphenylacetyl)-N-[[4-[[[(2,2-diphenylethyl)amino]carbonyl]amino]phenyl]methyl]-ornithinamid durch katalytische Hydrierung in Gegenwart von Palladiummohr und 80%iger wässeriger Essigsäure in einer Ausbeute von 90 % der Theorie.
Farblose Kristalle vom Fp. 125-128°C und R_{f} 0,80.
IR (KBr): 1655 cm⁻¹ (C=O)
   - ESI-MS:: (M+H)⁺ = 696
   (2M+H)⁺ = 1391

### Beispiel 110

### (R)-N-(Diphenylmethyl)-N²-[(4-methoxyphenyl)acetyl)-argininamid-acetat

### a) (R)-N⁵-[Amino(nitroimino)methyl]-N²-[(tert.-butyloxy)carbonyl]-N-(diphenylmethyl)-ornithinamid

Hergestellt analog Beispiel 5d), jedoch unter Verwendung von Dichlormethan als Lösemittel anstelle von Acetonitril, aus Boc-D-Arg(NO₂)-OH, 1,1-Diphenylmethanamin und TBTU in einer Ausbeute von 83 % der Theorie.
Farblose Kristalle vom Fp. 125-127°C (Ethylacetat/Diisopropylether = 1/9 (v/v)).

### b) (R)-N⁵-[Amino(nitroimino)methyl]-N-(diphenylmethyl)ornithinamid-trifluoracetat

Hergestellt analog Beispiel 5e) aus (R)-N⁵-[Amino(nitroimino)methyl]-N²-[(tert.-butyloxy)carbonyl]-N-(diphenylmethyl)-ornithinamid durch Einwirkung von Trifluoressigsäure in einer Ausbeute von 95 % der Theorie.
Farblose Kristalle vom Fp. 130-133°C.

### c) (R)-N⁵-[Amino(nitroimino)methyl]-N-(diphenylmethyl)-N²-[(4-methoxyphenyl)acetyl]-ornithinamid

Hergestellt analog Beispiel 110a) aus (R)-N⁵-[Amino(nitroimino)methyl]-N-(diphenylmethyl)-ornithinamid-trifluoracetat, 4-Methoxybenzenethansäure und TBTU in einer Ausbeute von 47 % der Theorie.
Farblose Kristalle vom Fp. 185-187°C (Methanol).
- IR (KBr):: 1675, 1655, 1630 cm⁻¹ (C=O, C=N)
1515 cm⁻¹ (Amid-II)
- ESI-MS:: (M+H)⁺ = 533
(M+Na)⁺ = 555

### d) (R)-N-(Diphenylmethyl)-N²-[(4-methoxyphenyl)acetyl]argininamid-acetat

Hergestellt analog Beispiel 1c) aus (R)-N⁵-[Amino(nitroimino)methyl]-N-(diphenylmethyl)-N²-[(4-methoxyphenyl)acetyl]-ornithinamid durch katalytische Hydrierung in Gegenwart von Palladium/Tierkohle und 80%iger wässeriger Essigsäure.
Farblose Kristalle vom Fp. 150-160°C (Z.) und R_{f} 0,71.
- IR (CH₂Cl₂):: 1660 cm⁻¹ (Amid-C=O)
1515 cm⁻¹ (Amid-II)
ESI-MS: (M+H)⁺ = 488

### Beispiel 111

### (R)-N-(Diphenylmethyl)-N²-(phenylacetyl)-argininamid-acetat

### a) (R)-N⁵-[Amino(nitroimino)methyl]-N-(diphenylmethyl)-N²-(phenylacetyl)-ornithinamid

Hergestellt analog Beispiel 110a) aus Benzenethansäure, R-N⁵-[Amino(nitroimino)methyl]-N-(diphenylmethyl)-ornithinamid-trifluoracetat und TBTU in einer Ausbeute von 45 % der Theorie.
Farblose Kristalle vom Fp. 207-209°C (Methanol).
- IR (KBr):: 1640 cm⁻¹ (Amid-C=O)
3430, 3290 cm⁻¹ (NH)
- ESI-MS:: (M+H)⁺ = 503
(M+Na)⁺ = 525
(M+K)⁺ = 541

### b) (R)-N-(Diphenylmethyl)-N²-(phenylacetyl)-argininamidacetat

Hergestellt analog Beispiel 1c) aus (R)-N⁵-[Amino(nitroimino)methyl]-N-(diphenylmethyl)-N²-(phenylacetyl)-orni thinamid durch katalytische Hydrierung in Gegenwart von Palladiummohr und 80%iger wässeriger Essigsäure.
Farblose Kristalle vom Fp. 150-160°C (Z.) und R_{f} 0,71.
IR (KBr): 1645 cm⁻¹ (C=O)
ESI-MS: (M+H)⁺ = 458

### Beispiel 112

### (R)-N-(Diphenylmethyl)-N²-[(4-hydroxyphenyl)acetyl]-argininamid-acetat

### a) (R)-N⁵-[Amino(nitroimino)methyl]-N-(diphenylmethyl)-N²-[(4-hydroxyphenyl)acetyl]-ornithinamid

Hergestellt analog Beispiel 110a) aus 4-Hydroxybenzenethansäure und (R)-N⁵-[Amino(nitroimino)methyl]-N-(diphenylmethyl)-ornithinamid-trifluoracerat sowie TBTU in einer Ausbeute von 48 % der Theorie.
Farblose Kristalle vom Fp. 253-255°C (Methanol).
- IR (KBr):: 1675, 1635, 1615 cm⁻¹ (C=O, C=N)
1515 cm⁻¹ (Amid-II)
- ESI-MS:: (M+Na)⁺ = 541
(M+K)⁺ = 557

### b) (R)-N-(Diphenylmethyl)-N²-[(4-hydroxyphenyl)acetyl]argininamid-acetat

Hergestellt analog Beispiel 1c) aus (R)-N⁵-[Amino(nitroimino)methyl]-N-(diphenylmethyl)-N²-[(4-hydroxyphenyl)acetyl]-ornithinamid durch katalytische Hydrierung in Gegenwart von Palladiummohr und 80%iger wässeriger Essigsäure in einer Ausbeute von 75 % der Theorie.
Farblose Kristalle vom Fp. 150-160°C (Z.) und R_{f} 0,72.
- IR (KBr):: 1650 cm⁻¹ (Amid-C=O)
1515 cm⁻¹ (Amid-II)
ESI-MS: (M+H)⁺ = 474

### Beispiel 113

### N²-(Diphenylacetyl)-N-[4-(4-methoxyphenyl)butyl]-argininamid-acetat

### a) N⁵-[Amino(nitroimino)methyl]-N²-(diphenylacetyl)-N-[(4-methoxyphenyl)butyl]-ornithinamid

Hergestellt analog Beispiel 8a) aus (R)-N⁵-[Amino(nitroimino)methyl]-N²-(diphenylacetyl)-ornithin, 4-(4-Methoxyphenyl)-butanamin und TBTU in einer Ausbeute von 34 % der Theorie.
Farblose Kristalle vom Fp. 161-164°C (Methanol).
- IR (KBr):: 1670, 1655, 1635 cm⁻¹ (C=O, C=N)
1515 cm⁻¹ (Amid-II)
- ESI-MS:: (M+H)⁺ = 575
(M+Na)⁺ = 597
(M+K)⁺ = 613

### b) N²-(Diphenylacetyl)-N-[4-(4-methoxyphenyl)butyl]-argininamid-acetat

Hergestellt analog Beispiel 1c) aus N⁵-[Amino(nitroimino)methyl]-N²-(diphenylacetyl)-N-[4-(4-methoxyphenyl)butyl]ornithinamid durch katalytische Hydrierung in Gegenwart von Palladiummohr und 80%iger wässeriger Essigsäure.
Farblose, amorphe Substanz vom R_{f} 0,74.
IR (CH₂Cl₂): 1660 cm⁻¹ (Amid-C=O)
ESI-MS: (M+H)⁺ = 530

### Beispiel 114

### (R,S)-3-[3-(Aminoiminomethyl)phenyl]-N²-(diphenylacetyl)-N-[(4-methoxyphenyl)methyl]-alaninamid-hydrochlorid

### a) (R,S)-3-(3-Cyanphenyl)-N²-(diphenylacetyl)-N-[(4-methoxyphenyl)methyl]-alaninamid

Hergestellt analog Beispiel 8a) aus (R,S)-3-(3-Cyanphenyl)-N²-(diphenylacetyl)-alanin, 4-Methoxybenzenmethanamin und TBTU in einer Ausbeute von 46 % der Theorie.
Farblose Kristalle vom Fp. 208-210°C (Isopropanol).
- IR (KBr):: 3270 cm⁻¹ (NH)
2830 cm⁻¹ (C≡N)
1675, 1655, 1640 cm⁻¹ (C=O, C=N)
ESI-MS: M⁺ = 503

### b) (R,S)-3-[3-(Aminoiminomethyl)phenyl]-N²-(diphenylacetyl)-N-[(4-methoxyphenyl)methyl]-alaninamid-hydrochlorid

Hergestellt analog Beispiel 105d) aus (R,S)-3-(3-Cyanphenyl)-N²-(diphenylacetyl)-N-[(4-methoxyphenyl)methyl]-alaninamid durch Einwirkung von zunächst Chlorwasserstoff und später Ammoniumcarbonat in einer Ausbeute von 98 % der Theorie.
Farblose, amorphe Substanz vom R_{f} 0,78.
IR (KBr): 1655 cm⁻¹ (C=O)
ESI-MS: M⁺ = 521

### Beispiel 115

### (R,S)-3-[3-(Aminoiminomethyl)phenyl]-N²-(diphenylacetyl)-N-[(4-hydroxyphenyl)methyl]-alaninamid-hydrochlorid

### a) (R,S)-3-(3-Cyanphenyl)-N²-(diphenylacetyl)-N-[(4-hydroxyphenyl)methyl]-alaninamid

Hergestellt analog Beispiel 8a) aus (R,S)-3-(3-Cyanphenyl)-N²-(diphenylacetyl)-alanin, 4-Hydroxybenzenmethanamin und TBTU in einer Ausbeute von 58 % der Theorie.
Farblose Kristalle vom Fp. 115-118°C (Z.) (Isopropanol).
- IR (KBr):: 2230 cm⁻¹ (C≡N)
1645 cm⁻¹ (Amid-C=O)
1520 cm⁻¹ (Amid-II)
M⁺ = 489

### b) (R,S)-3-[3-(Aminoiminomethyl)phenyl]-N²-(diphenylacetyl)-N-[(4-hydroxyphenyl)methyl]-alaninamid-hydrochlorid

Hergestellt analog Beispiel 105d) aus (R,S)-3-(3-Cyanphenyl)-N²-(diphenylacetyl)-N-[(4-hydroxyphenyl)methyl]-alaninamid durch Einwirkung von zunächst Chlorwasserstoff und später Ammoniumcarbonat in einer Ausbeute von 97 % der Theorie.
Farblose Kristalle vom Fp. 205°C (Z.) (Ethanol/Diisopropylether = 1/9 (v/v)).
- IR (KBr):: 1655 cm⁻¹ (C=O)
1518 cm⁻¹ (Amid-II)
ESI-MS: (M+H)⁺ = 507

### Beispiel 116

### (R,S)-3-[3-(Aminoiminomethyl)phenyl]-N²-(diphenylacetyl)-)-N-[[3-[(1,2-dihydro-3,5(4H)-dioxo-1,2-diphenyl-3H-1,2,4-triazol-4-yl)methyl)phenyl]methyl]-alaninamid-hydrochlorid

### a) (R,S)-N²-[(tert.-Butyloxy)carbonyl]-3-(3-cyanphenyl)-N-[[3-[(1,2-dihydro-3,5(4H)-dioxo-1,2-diphenyl-3H-1,2,4-triazol-4-yl)methyl]phenyl]methyl]-alaninamid

Hergestellt analog Beispiel 8a) aus (R,S)-N²-[(tert.-Butyloxy)carbonyl]-3-(3-cyanphenyl)-alanin, 3-[(1,2-Dihydro-3,5(4H)-dioxo-1,2-diphenyl-3H-1,2,4-triazol-4-yl)methyl]-benzenmethanamin und TBTU in einer Ausbeute von 70 % der Theorie.
Farblose Kristalle vom Fp. 148-151°C (Methanol).
- IR (CH₂Cl₂):: 3430 cm⁻¹ (NH)
2230 cm⁻¹ (C≡N)
1780, 1730 cm⁻¹ (Triazolidindion-C=O)
1685 cm⁻¹ (Amid-C=O)
- ESI-MS:: (M+H)⁺ = 645
(M+NH₄)⁺ = 662
(M+Na)⁺ = 667
(M+K)⁺ = 683
(2M+H)⁺ = 1289
(M-H)⁻ = 643

### b) (R,S)-3-(3-Cyanphenyl)-N-[[3-[(1,2-dihydro-3,5(4H)-dioxo-1,2-diphenyl-3H-1,2,4-triazol-4-yl)methyl]phenyl]methyl]alaninamid-trifluoracetat

Hergestellt analog Beispiel 5e) aus (R,S)-N²-[(tert.-Butyloxy)carbonyl]-3-(3-cyanphenyl)-N-[[3-[(1,2-dihydro-3,5(4H)-dioxo-1,2-diphenyl-3H-1,2,4-triazol-4-yl)methyl]phenyl]methyl]-alaninamid durch Einwirkung von Trifluoressigsäure in quantitativer Ausbeute.
- IR (CH₂Cl₂):: 2230 cm⁻¹ (C≡N)
1780, 1725 cm⁻¹ (Triazolidindion-C=O)
1685 cm⁻¹ (Amid-C=O)
MS: M⁺ = 544

### c) (R,S)-3-(3-Cyanphenyl)-N²-(diphenylacetyl)-N-[[3-[(1,2-dihydro-3,5(4H)-dioxo-1,2-diphenyl-3H-1,2,4-triazol-4-yl)methyl]phenyl]methyl]-alaninamid

Hergestellt analog Beispiel 69c), jedoch unter Verwendung von ausschließlich Dimethylformamid als Lösemittel, aus (R,S)-3-(3-Cyanphenyl)-N-[[3-[(1,2-dihydro-3,5(4H)-dioxo-1,2-diphenyl-3H-1,2,4-triazol-4-yl)methyl]phenyl]methyl]-alaninamid-trifluoracetat und Diphenylacetylchlorid in einer Ausbeute von 78 % der Theorie.
Farblose Kristalle vom Fp. 186-190°C (Essigsäureethylester/ Diisopropylether = 1/1 (v/v)).
- IR (CH₂Cl₂):: 3420 cm⁻¹ (NH)
2230 cm⁻¹ (C≡N)
1780, 1725 cm⁻¹ (Triazolidindion-C=O)
1675 cm⁻¹ (Amid-C=O)
MS: M⁺ = 738

### d) (R,S)-3-[3-(Aminoiminomethyl)phenyl]-N²-(diphenylacetyl)-N-[[3-[(1,2-dihydro-3,5(4H)-dioxo-1,2-diphenyl-3H-1,2,4-triazol-4-yl)methyl]phenyl]methyl]-alaninamid-hydrochlorid

Hergestellt analog Beispiel 105d) aus (R,S)-3-(3-Cyanphenyl)-N²-(diphenylacetyl)-N-[[3-[(1,2-dihydro-3,5(4H)-dioxo-1,2-diphenyl-3H-1,2,4-triazol-4-yl)methyl]phenyl]methyl]alaninamid durch Einwirkung von zunächst Chlorwasserstoff und später Ammoniumcarbonat in einer Ausbeute von 98 % der Theorie.
Farblose Kristalle vom Fp. 225°C (Z.) und R_{f} 0,85.
- IR (CH₂Cl₂):: 1780, 1725 cm⁻¹ (Triazolidindion-C=O)
1675 cm⁻¹ (Amidinium, Amid-C=O)
ESI-MS: (M+H)⁺ = 756

### Beispiel 117

### (R)-N⁵-[Amino(nitroimino)methyl]-N²-[[bis-(4-bromphenyl)]acetyl]-N-[(4-hydroxyphenyl)methyl]-ornithinamid

Zu der Lösung von 0,9 g (2,775 mMol) (R)-N⁵-[Amino(nitroimino)methyl]-N-[(4-hydroxyphenyl)methyl]-ornithinamid in 50 ml Tetrahydrofuran gab man zunächst die Lösung von 0,8 g (7,55 mMol) Natriumcarbonat in 10 ml Wasser, dann tropfenweise die Lösung von 1,259 g (3,32 Mol) [Bis-(4-bromphenyl)]acetylchlorid in 50 ml Tetrahydrofuran und rührte anschließend noch 60 Minuten bei einer Reaktionstemperatur von 30°C. Das Lösemittel wurde im Vakuum abdestilliert, der Rückstand in Wasser aufgenommen und mit Essigsäure schwach sauer gestellt. Man extrahierte erschöpfend mit Essigsäureethylester, trocknete die vereinigten Essigesterextrakte über Natriumsulfat und dampfte sie ein. Der Rückstand wurde an Kieselgel (Baker, Kieselgel für die Flash-Chromatographie, 30-60 µm) unter Verwendung von Ethylacetat als mobiler Phase säulenchromatographisch gereinigt. Man erhielt 1,0 g (53 % der Theorie) an farblosen Kristallen vom Fp. 163-167°C (Aceton/ Diethylether) und R_{f} 0,96.
IR (KBr): 1660, 1630 cm⁻¹ (Amid-C=O)
EI-MS: (M-H)⁻ = 673/675/677 (Br₂)

### Beispiel 118

### (R)-N²-[[Bis-(4-bromphenyl)]acetyl]-N-[(4-hydroxyphenyl)methyl]-argininamid-formiat

Hergestellt analog Beispiel 14b) aus (R)-N⁵-[Amino(nitroimino)methyl]-N²-[[bis(4-bromphenyl)]acetyl)-N-[(4-hydroxyphenyl)methyl]-ornithinamid durch Reduktion mit Zinn(II)-chlorid-dihydrat in Gegenwart von 60%iger wässeriger Ameisensäure.
Farblose, amorphe Substanz vom R_{f} 0,80.
IR (KBr): 1655 cm⁻¹ (C=O)
ESI-MS: (M+H)⁺ = 630/632/634 (Br₂)

### Beispiel 119

### (R,S)-3-[[(Aminoiminomethyl)amino]methyl]-N²-(diphenylacetyl)-N-[(4-methoxyphenyl)methyl]-alaninamid-hydrochlorid

### a) (R,S)-N²-(Diphenylacetyl)-serinmethylester

Hergestellt analog Beispiel 1a), jedoch unter Verwendung von Natriumcarbonat anstelle von Natriumhydroxid, aus Diphenylacetylchlorid und Serinmethylester-hydrochlorid in einer Ausbeute von 85 % der Theorie.
Farblose Kristalle vom Fp. 156-158°C.
- IR (KBr):: 1745 cm⁻¹ (Ester-C=O)
1655 cm⁻¹ (Amid-C=O)
2850 cm⁻¹ (OCH₃)

### b) (R,S)-N²-(Diphenylacetyl)-O-(methylsulfonyl)serinmethylester

Zu der Mischung aus 12,11 g (0,0386 Mol) (R,S)-N²-(Diphenylacetyl)-serinmethylester, 3,32 ml (0,0429 Mol) Methansulfonsäurechlorid und 250 ml trockenem Tetrahydrofuran tropfte man die Lösung von 6,0 ml (0,043 Mol) Triethylamin in 50 ml trockenem Tetrahydrofuran, wobei man darauf achtete, daß die Reaktionstemperatur +40°C nicht überschritt. Man rührte noch 1 Stunde bei Zimmertemperatur, filtrierte die Mischung und engte das Filtrat im Vakuum ein. Der Rückstand wurde durch Verreiben mit Diisopropylether zur Kristallisation gebracht und noch einmal aus heißem Ethylacetat/Diisopropylether (3/7 (v/v)) umkristallisiert.
Fp.: 117-119°C.
Ausbeute: 11,87 g (79 % der Theorie).

### c) (R,S)-3-Cyan-N²-(diphenylacetyl)-alaninmethylester

Zu der Lösung von 1,91 g (0,039 Mol) Natriumcyanid in 150 ml Dimethylsulfoxid gab man portionsweise 15,5 g (0,0396 Mol) (R,S)-N²-(diphenylacetyl)-O-(methylsulfonyl)-serinmethylester und erwärmte anschließend 90 Minuten auf 60°C. Die Mischung wurde nach dem Erkalten in 1 Liter Wasser eingerührt und mit insgesamt 1 Liter einer Mischung aus Ethylacetat/Petrolether (1/1 (v/v)) erschöpfend extrahiert. Die organischen Auszüge wurden vereinigt, über Natriumsulfat getrocknet und vom Lösemittel befreit. Der Rückstand wurde mit Diisopropylether verrieben und zur Kristallisation gebracht. Man erhielt 10,11 g (80 % der Theorie) an farblosen Kristallen vom Fp. 126-129°C.

### d) (R,S)-3-Cyan-N²-(diphenylacetyl)-alanin

Hergestellt analog Beispiel 79d) aus (R,S)-3-Cyan-N²-(diphenylacetyl)-alaninmethylester und Lithiumhydroxid-hydrat in einer Ausbeute von 83 % der Theorie.
Farblose Kristalle vom Fp. 149-154°C (Essigsäureethylester und Diisopropylether).

### e) (R,S)-3-Cyan-N²-(diphenylacetyl)-N-[(4-methoxyphenyl)methyl]-alaninamid

Hergestellt analog Beispiel 8a) aus (R,S)-3-Cyan-N²-(diphenylacetyl)-alanin und 4-Methoxybenzenmethanamin sowie TBTU in einer Ausbeute von 59 % der Theorie.
Farblose Kristalle vom Fp. 183-187°C (Ethanol).
- IR (KBr):: 3310, 3210 cm⁻¹ (NH)
2840 cm⁻¹ (OCH₃)
2250 cm⁻¹ (C≡N, schwach)
1675, 1670, 1660, 1645 (Amid-C=O) cm⁻¹
1515 cm⁻¹ (Amid-II)

MS: M⁺ = 427

### f) (R,S)-3-(Aminomethyl)-N²-(diphenylacetyl)-N-[(4-methoxyphenyl)methyl]-alaninamid-hydrochlorid

Hergestellt analog Beispiel 29c) aus (R,S)-3-Cyan-N²-(diphenylacetyl)-N-[(4-methoxyphenyl)methyl]-alaninamid durch katalytische Hydrierung in Gegenwart von Palladium/Tierkohle und Salzsäure in einer Ausbeute von 30 % der Theorie.
Farblose, amorphe Substanz.
- IR (CH₂Cl₂):: 1680, 1655 cm⁻¹ (Amid-C=O)
2840 cm⁻¹ (OCH₃)
1515 cm⁻¹ (Amid II)

### g) (R,S)-3-[[(Aminoiminomethyl)amino]methyl]-N²-(diphenylacetyl)-N-[(4-methoxyphenyl)methyl]-alaninamid-hydrochlorid

Hergestellt analog Beispiel 42g) aus (R,S)-3-(Aminomethyl)-N²-(diphenylacetyl)-N-[(4-methoxyphenyl)methyl]-alaninamid-hydrochlorid, 3,5-Dimethylpyrazol-1-carbonsäureamidiniumnitrat und Triethylamin in einer Ausbeute von 20 % der Theorie.
Farblose, amorphe Substanz vom R_{f} 0,73.
- IR (KBr):: 2840 cm⁻¹ (OCH₃)
1695, 1675, 1655, 1630 cm⁻¹ (C=O, Amidinium)
1515 cm⁻¹ (Amid II)
- EI-MS:: (M+H)⁺ = 474
(2M+H)⁺ = 947

### Beispiel 120

### (R)-N²-(Diphenylacetyl)-N-[(4-ethoxyphenyl)methyl]-argininamid-acetat

### a) (R)-N⁵-[Amino(nitroimino)methyl]-N²-[(tert.-butyloxy)carbonyl]-N-[(4-ethoxyphenyl)methyl]-ornithinamid

Hergestellt analog Beispiel 8a) aus Boc-D-Arg-(NO₂)-OH, 4-Ethoxybenzenmethanamin-hydrochlorid (Fp.: 262-264°C; aus 4-Ethoxybenzonitril durch katalytische Hydrierung in Gegenwart von Palladium/Tierkohle und wässeriger Salzsäure), Triethylamin und TBTU in einer Ausbeute von 77 % der Theorie.
- IR (CH₂Cl₂):: 1730 cm⁻¹ (Ester-C=O)
1675 cm⁻¹ (Amid C=O)
1510 cm⁻¹ (Amid II)

### b) (R)-N⁵-[Amino(nitroimino)methyl]-N-[(4-ethoxyphenyl)methyl]-ornithinamid-trifluoracetat

Hergestellt analog Beispiel 5e) aus (R)-N⁵-[Amino(nitroimino)methyl]-N²-[(tert.-butyloxy)carbonyl]-N-[(4-ethoxyphenyl)methyl]-ornithinamid durch Einwirkung von Trifluoressigsäure in einer Ausbeute von 70 % der Theorie. Die aus dem Salz durch Behandlung mit 1N Natronlauge freigesetzte Base schmolz bei 182-184°C (Essigsäureethylester).
- ESI-MS:: (M+H)⁺ = 547
(M+Na)⁺ = 569
(M+K)⁺ = 585

### c) (R)-N⁵-[Amino(nitroimino)methyl]-N²-(diphenylacetyl)-N-[(4-ethoxyphenyl)methyl]-ornithinamid

Hergestellt analog Beispiel 69c) aus (R)-N⁵-[Amino(nitroimino)methyl]-N-[(4-ethoxyphenyl)methyl]-ornithinamid und Diphenylacetylchlorid in einer Ausbeute von 62 % der Theorie.
Farblose Kristalle vom Fp. 182-184°C (Ethylacetat/Methanol = 1/1 (v/v)).
- ESI-MS:: (M+H)⁺ = 547
(M+Na)⁺ = 569
(M+K)⁺ = 585

### d) (R)-N²-(Diphenylacetyl)-N-[(4-ethoxyphenyl)methyl]arginamid-acetat

Hergestellt analog Beispiel 1c) aus (R)-N⁵-[Amino(nitroimino)methyl]-N²-(diphenylacetyl)-N-[(4-ethoxyphenyl)-methyl]-ornithinamid durch katalytische Hydrierung in Gegenwart von Palladiummohr und 80%iger wässeriger Essigsäure in einer Ausbeute von 97 % der Theorie.
Farblose Kristalle vom Fp. 97-100°C und R_{f} 0,75.
- IR (CH₂Cl₂):: 1665 cm⁻¹ (C=O)
1515 cm⁻¹ (Amid-II)
- ESI-MS:: (M+H)⁺ = 502

### Beispiel 121

### (R)-N²-(Diphenylacetyl)-N-[(4-hydroxyphenyl)methyl]-N-methyl-argininamid

### a) (R)-N⁵-[Amino(nitroimino)methyl]-N²-[(tert.-butyloxy)carbonyl]-N-[(4-hydroxyphenyl)methyl]-N-methyl-ornithinamid

Hergestellt analog Beispiel 8a) aus Boc-D-Arg(NO₂)-OH, N-Methyl-4-hydroxybenzenmethanamin (das Hydrochlorid schmilzt bei 190-192°C [Ethanol]) und TBTU in einer Ausbeute von 80 % der Theorie.
Farblose Kristalle vom Fp. 78-81°C.
- IR (KBr):: 1705 cm⁻¹ (Ester-C=O)
1635 cm⁻¹ (C=O, C=N)
1520 cm⁻¹ (Amid-II)
- ESI-MS:: (M+Na)⁺ = 461
(M+K)⁺ = 477
(2M+Na)⁺ = 899

### b) (R)-N⁵-[Amino(nitroimino)methyl]-N-[(4-hydroxyphenyl)methyl]-N-methyl-ornithinamid-trifluoracetat

Hergestellt analog Beispiel 5e) aus (R)-N⁵-[Amino(nitroimino)methyl]-N²-[(tert.-butyloxy)carbonyl]-N-[(4-hydroxyphenyl)methyl]-N-methyl-ornithinamid durch Einwirkung von Trifluoressigsäure in einer Ausbeute von 93 % der Theorie.
Farblose Kristalle vom Fp. 114°C.
- IR (KBr):: 1650 cm⁻¹ (C=O)
- ESI-MS:: (M+H)⁺ = 339
(M+Na)⁺ = 361
(2M+H)⁺ = 677

### c) (R)-N⁵-[Amino(nitroimino)methyl]-N²-(diphenylacetyl)-N-[(4-hydroxyphenyl)methyl]-N-methyl-ornithinamid

Hergestellt analog Beispiel 69c) aus (R)-N⁵-[Amino(nitroimino)methyl]-N-[(4-hydroxyphenyl)methyl]-N-methyl-ornithinamid und Diphenylacetylchlorid in einer Ausbeute von 85 % der Theorie.
Farblose, amorphe Substanz, die ohne Reinigung weiterverarbeitet wurde.

### d) (R)-N²-(Diphenylacetyl)-N-[(4-hydroxyphenyl)methyl]-N-methyl-argininamid

Hergestellt analog Beispiel 1c) aus (R)-N⁵-[Amino(nitroimino)methyl]-N²-(diphenylacetyl)-N-[(4-hydroxyphenyl)methyl]-N-methyl-ornithinamid durch katalytische Hydrierung in Gegenwart von Palladiummohr und 80%iger wässeriger Essigsäure in einer Ausbeute von 84 % der Theorie.
Farblose, amorphe Substanz vom R_{f} 0,73.
IR (KBr): 1635.5 cm⁻¹ (Amid-C=O)
ESI-MS: (M+H)⁺ = 488

### Beispiel 122

### (R,S)-3-[3-Aminomethyl)phenyl]-N²-(diphenylacetyl)-N-[(4-hydroxyphenyl)methyl]-alaninamid

0,48 g (0,98 mMol) (R,S)-3-(3-Cyanphenyl)-N²-(diphenylacetyl)-N-[(4-hydroxyphenyl)methyl]-alaninamid wurden in einem Gemisch aus 200 ml Methanol und 5 ml Eisessig gelöst und nach Zugabe von 2,0 g Palladium/Tierkohle (10%ig) bei Zimmertemperatur und einem Druck von 5 bar bis zur Beendigung der Wasserstoffaufnahme hydriert. Der Katalysator wurde abgetrennt, das Filtrat im Vakuum vom Lösemittel befreit, der Rückstand in 50 ml Dichlormethan aufgenommen und nacheinander mit Wasser, gesättigter Natriumcarbonat-Lösung und Wasser gewaschen. Der aus der Dichlormethanlösung nach Abdampfen des Lösemittels hinterbleibende Rückstand ließ sich mit einer Mischung aus Diethylether/Diisopropylether/Methanol (49/49/2 (v/v/v)) durch Anreiben zur Kristallisation bringen. Man erhielt in einer Ausbeute von 0,47 g (97 % der Theorie) farblose Kristalle vom Fp. 208°C und R_{f} 0,78.
IR (KBr): 1643.3 cm⁻¹ (Amid-C=O)
MS: M⁺ = 493

### Beispiel 123

### (R,S)-N-[(4-Amino-3,5-dibromphenyl)methyl]-3-[3-(aminoiminomethyl)phenyl]-N²-(diphenylacetyl)-alaninamid-hydrochlorid

### a) (R,S)-3-(3-Cyanphenyl)-N²-(diphenylacetyl)-alanin

Hergestellt analog Beispiel 1a) aus Diphenylacetylchlorid, 3-(3-Cyanphenyl)-alanin-hydrochlorid und Natronlauge in einer Ausbeute von 58 % der Theorie.
Farblose Kristalle vom Fp. 145-147°C (Essigsäureethylester).

### b) (R,S)-N-[(4-Amino-3,5-dibromphenyl)methyl]-3-(3-cyanphenyl)-N²-(diphenylacetyl)-alaninamid

Hergestellt analog Beispiel 5d) aus (R,S)-3-(3-Cyanphenyl-N²-(diphenylacetyl)-alanin, 4-Amino-3,5-dibrombenzenmethanamin und TBTU in einer Ausbeute von 38 % der Theorie.
Farblose, amorphe Substanz.
- IR (KBr):: 2230 cm⁻¹ (C≡N)
1640 cm⁻¹ (Amid-C=O)

### c) (R,S)-N-[(4-Amino-3,5-dibromphenyl)methyl]-3-[3-(aminoiminomethyl)phenyl]-N²-(diphenylacetyl)-alaninamidhydrochlorid

Hergestellt analog Beispiel 105d) aus (R,S)-N-[(4-Amino-3,5-dibromphenyl)methyl]-3-(3-cyanphenyl)-N²-(diphenylacetyl)alaninamid durch Einwirkung von zunächst Chlorwasserstoff und dann Ammoniumcarbonat in einer Ausbeute von 57 % der Theorie.
Farblose, amorphe Substanz vom R_{f} 0,84.
- IR (KBr):: 1654.8 cm⁻¹ (Amidinium, Amid-C=O)
1520 cm⁻¹ (Amid-II).

ESI-MS: (M+H)⁺ = 662/664/666 (Br₂)

### Beispiel 124

### (R)-N²-[(rac.-5,11-Dihydro-6-oxo-6H-dibenz[b,e]azepin-11-yl)carbonyl]-N-[(4-ethoxyphenyl)methyl]-argininamidacetat

### a) (R)-N⁵-[Amino(nitroimino)methyl]-N²-[(rac.-5,11-dihydro-6-oxo-6H-dibenz[b,e]azepin-11-yl)carbonyl]-N-[(4-ethoxyphenyl)methyl]-ornithinamid

Hergestellt analog Beispiel 69c), jedoch unter Verwendung von N,N-Diisopropylethylamin anstelle von Triethylamin, aus rac. 11-(Chlorcarbonyl)-5,11-dihydro-6H-dibenz[b,e]azepin-6-on und (R)-N⁵-[Amino-(nitroimino)methyl]-N-[(4-ethoxyphenyl)methyl]-ornithinamid-trifluoracetat in einer Ausbeute von 85 % der Theorie.
Farblose, kristalline, sich bei ca. 140°C zersetzende Substanz.
- IR (KBr):: 1654.8 cm⁻¹ (C=O)
- ESI-MS:: (M+H)⁺ = 588
(M+Na)⁺ = 610
(M+NH₄)⁺ = 605
(M+K)⁺ = 626

### b) (R)-N²-[(rac.-5,11-Dihydro-6-oxo-6H-dibenz[b,e]azepin-11-yl)carbonyl]-N-[(4-ethoxyphenyl)methyl]-argininamidacetat

Hergestellt analog Beispiel 1c) aus (R)-N⁵-[Amino(nitroimino)methyl]-N²-[(rac.-5,11-dihydro-6-oxo-6H-dibenz[b,e]-azepin-11-yl)carbonyl]-N-[(4-ethoxyphenyl)methyl]-ornithinamid durch katalytische Hydrierung in Gegenwart von Palladiummohr und 80%iger wässeriger Essigsäure in einer Ausbeute von 90 % der Theorie.
Farblose Kristalle vom Fp. 135°C (Diisopropylether/Isopropanol 95/5 (v/v)) und R_{f} 0,66.
- IR (KBr):: 1654.8 cm⁻¹ (Amidinium, Amid-C=O)
1510 cm⁻¹ (Amid-II)

ESI-MS: (M+H)⁺ = 543

### Beispiel 125

### (R)-N-[[4-[(Dimethylamino)sulfonyloxy]phenyl]methyl]-N²-(diphenylacetyl)-argininamid-acetat

### a) (R)-N⁵-[Amino(nitroimino)methyl]-N²-[(tert.-butyloxy)carbonyl]-N-[[4-[(dimethylamino)sulfonyloxy]phenyl]methyl]-ornithinamid

Hergestellt analog Beispiel 8a) aus Boc-D-Arg(NO₂)-OH, 4-[(Dimethylamino)sulfonyloxyl-benzenmethanamin-hydrochlorid (Fp.: 215-218°C; erhalten aus käuflichem 4-[(Dimethylamino)sulfonyloxy]-benzonitril durch katalytische Hydrierung in Gegenwart von Palladium/Tierkohle und 1N wässeriger Salzsäure), Triethylamin und TBTU in einer Ausbeute von 48 % der Theorie.
Farblose Kristalle vom Fp. 82-84°C (Z.).
- IR (CH₂Cl₂):: 1715 cm⁻¹ (Ester-C=O)
1675, 1630 cm⁻¹ (Amid-C=O; C=N)
1505 cm⁻¹ (Amid-II)
1370, 1150 cm⁻¹ (SO₂)
- ESI-MS:: (M+H)⁺ = 532
(M+Na)⁺ = 554
(M+K)⁺ = 570

### b) (R)-N⁵-[Amino(nitroimino)methyl]-N-[[4-[(dimethylamino)sulfonyloxy]phenyl]methyl]-ornithinamid-trifluoracetat

Hergestellt analog Beispiel 5e), jedoch unter Verwendung von Tetrahydrofuran als Lösemittel anstelle von Dichlormethan, aus (R)-N⁵-[Amino(nitroimino)methyl]-N²-[(tert.-butyloxy)carbonyl]-N-[[4-[(dimethylamino)sulfonyloxy]phenyl]methyl]-ornithinamid durch Einwirkung von Trifluoressigsäure in einer Aubeute von 92 % der Theorie.
Farblose Kristalle vom Fp. 150-160°C.
- IR (KBr):: 1678 cm⁻¹ (C=O, C=N)
1369.4, 1149.5 cm⁻¹ (SO₂)

### c) (R)-N⁵-[Amino(nitroimino)methyl]-N²-(diphenylacetyl)-N-[[4-[(dimethylamino)sulfonyloxy]phenyl]methyl]-ornithinamid

Hergestellt analog Beispiel 5f), jedoch unter Verwendung von Dichlormethan als Lösemittel anstelle von Acetonitril, aus (R)-N⁵-[Amino(nitroimino)methyl]-N-[[4-[(dimethylamino)sulfonyloxy]phenyl]methyl]-ornithinamid-trifluoracetat, Diphenyl-essigsäure und TBTU in einer Ausbeute von 80 % der Theorie.
Farblose Kristalle vom Fp. 150-160°C.
- IR (KBr):: 1651.0 cm⁻¹ (C=O, C=N)
1510 cm⁻¹ (Amid-II)
1371.3, 1149.5 cm⁻¹ (SO₂)

### d) (R)-N-[[4-[(Dimethylamino)sulfonyloxy]phenyl]methyl]-N²-(diphenylacetyl)-argininamid-acetat

Hergestellt analog Beispiel 1c) aus (R)-N⁵-[Amino(nitroimino)methyl]-N²-(diphenylacetyl)-N-[[4-(dimethylamino) sulfonyloxy]phenyl]methyl]-ornithinamid durch katalytische Hydrierung in Gegenwart von Palladiummohr und 80%iger wässeriger Essigsäure in einer Ausbeute von 66 % der Theorie.
Farblose Kristalle vom Fp. 97-105°C und R_{f} 0,67.
- IR (KBr):: 1656.8 cm⁻¹ (Amidinium, Amid-C=O)
1369.4, 1149.5 cm⁻¹ (SO₂)

ESI-MS: (M+H)⁺ = 581

### Beispiel 126

### (R)-N²-(Diphenylacetyl)-N-[(2-hydroxyphenyl)methyl]-argininamid-acetat

### a) (R)-N⁵-[Amino(nitroimino)methyl]-N²-(diphenylacetyl)N-[(2-hydroxyphenyl)methyl]-ornithinamid

Hergestellt analog Beispiel 12a) aus (R)-N⁵-[Amino(nitroimino)methyl]-N²-(diphenylacetyl)-ornithin, 2-Hydroxybenzenmethanamin und Chlorkohlensäureisobutylester in einer Ausbeute von 40 % der Theorie.
Farblose Kristalle.
IR (KBr): 1635 cm⁻¹ (C=O, C=N)
EI-MS: (M-H)⁻ = 517

### b) (R)-N²-(Diphenylacetyl)-N-[(2-hydroxyphenyl)methyl]argininamid-acetat

Hergestellt analog Beispiel 1c) aus (R)-N⁵-[Amino(nitroimino)methyl]-N²-diphenylacetyl-N-[(2-hydroxyphenyl)methyl]-ornithinamid durch katalytische Hydrierung in Gegenwart von Palladiummohr und 80%iger wässeriger Essigsäure in einer Ausbeute von 77 % der Theorie.
Farblose, amorphe Substanz vom R_{f} 0,78.
IR (KBr): 1652.9 cm⁻¹ (Amid-C=O)
EI-MS: (M+H)⁺ = 474

### Beispiel 127

### (R)-N-[(4-Hydroxyphenyl)methyl]-N²-[(2-naphthyl)sulfonyl]argininamid-acetat

### a) (R)-N⁵-[Amino(nitroimino)methyl]-N-[(4-hydroxyphenyl)methyl]-N²-[(2-naphthyl)sulfonyl]-ornithinamid

Hergestellt analog Beispiel 88c), jedoch unter Verwendung von N,N-Diisopropylethylamin an Stelle von 4-Methylmorpholin, aus 2-Naphthalinsulfonsäurechlorid und (R)-N⁵-[Amino-(nitroimino)methyl]-N-[(4-hydroxyphenyl)methyl]-ornithinamid in einer Ausbeute von 26 % der Theorie.
Farbloses, hochviskoses Öl vom R_{f} 0,19 (Polygram^{(R)} SIL G/UV₂₅₄, Fertigfolien für die DC; Macherey-Nagel, Art. 805021; Fließmittel: Dichlormethan/Essigsäureethylester/Methanol/Cyclohexan/konz. wässeriges Ammoniak = 59/25/7,5/7,5/1 (v/v/v/v/v)).
- IR (KBr):: 1639.4 cm⁻¹ (Amid-C=O, N-NO₂)
1330.8; 1159,2 cm⁻¹ (N-SO₂)

### b) (R)-N-[(4-Hydroxyphenyl)methyl]-N²-[(2-naphthyl)sulfonyl]-argininamid-acetat

Hergestellt analog Beispiel 1c) aus (R)-N⁵-[Amino(nitroimino)methyl]-N-[(4-hydroxyphenyl)methyl]-N²-[(2-naphthyl)sulfonyl]-ornithinamid durch katalytische Hydrierung in einer Ausbeute von 63 % der Theorie. Farblose, amorphe Substanz vom R_{f} 0,70.
- IR (KBr):: 1663.2 cm⁻¹ (Amid-C=O, Amidinium)
1323.1; 1159.2 cm⁻¹ (N-SO₂)

ESI-MS: (M+H)⁺ = 470

### Beispiel 128

### (R)-N-[[4-(Aminomethyl)phenyl]methyl]-N²-(diphenylacetyl)argininamid-bis-(trifluoracetat)

### a) (R)-N²-(9-Fluorenylmethoxycarbonyl)-N⁵-[[(2,2,5,7,8-pentamethylchroman-6-sulfonyl)amino](imino)methyl]-N-[[4-[[(phenylmethoxycarbonyl)amino]methyl]phenyl]methyl]ornithinamid

Hergestellt analog Beispiel 109a), jedoch unter Verwendung von Tetrahydrofuran als Lösemittel an Stelle von Dichlormethan, aus Fmoc-D-Arg(Pmc)-OH und 4-[[(Phenylmethoxycarbonyl)amino]methyl]-benzenmethanamin (R. Epton et al., Polymer 21, 481-482 (1980); C.A. 93, 168654k (1980)) in einer Ausbeute von 88 % der Theorie.
Farblose Kristalle vom Fp. 132-136°C.
- IR (KBr):: 3323.2 cm⁻¹ (N-H)
1693.4 cm⁻¹ (Carboxyl-C=O, Ester C=O)
- ESI-MS:: (M+H)⁺ = 915
(M+Na)⁺ = 937

### b) (R)-N⁵-[[(-2,2,5,7,8-Pentamethylchroman-6-sulfonyl)amino](imino)methyl]-N-[[4-[[(phenylmethoxycarbonyl)amino] methyl]phenyl]methyl]-ornithinamid

Die Lösung von 14,41 g (15,75 mMol) (R)-N²-(9-Fluorenylmethoxycarbonyl)-N⁵-[[(2,2,5,7,8-pentamethylchroman-6-sulfonyl)amino](imino)methyl]-N-[[4-[[(phenylmethoxycarbonyl)amino]methyl]phenyl]methyl]-ornithinamid in 86 ml Dimethylformamid wurde mit 19 ml (13,5 g; 184,6 mMol) Diethylamin versetzt und über Nacht bei Raumtemperatur gerührt. Das Lösemittel wurde im Vakuum abdestilliert, der verbliebene Rückstand in 200 ml Essigsäureethylester aufgenommen und über Glasfaserfilter No. 8 (Schleicher & Schüll) filtriert. Das Filtrat wurde mit 50 ml Wasser gewaschen, über Natriumsulfat getrocknet und im Vakuum eingedampft. Die so erhaltene glasartige Phase wurde an Kieselgel (Macherey-Nagel, 70-230 mesh ASTM; Dichlormethan/Methanol/konz. wässeriges Ammoniak (90/10/0,25)) chromatographisch gereinigt und ergab 9,8 g (90 % der Theorie) einer einheitlichen, hochviskosen, nicht kristallisierenden Substanz.
IR (KBr): 1714.6; 1620.1 cm⁻¹ (C=O)

### c) (R)-N²-(Diphenylacetyl)-N⁵-[[(2,2,5,7,8-pentamethylchroman-6-sulfonyl)amino](imino)methyl]-N-[[4-[[(phenylmethoxycarbonyl)amino]methyl]phenyl]methyl]-ornithinamid

Hergestellt analog Beispiel 128a) aus Diphenylessigsäure, (R)-N⁵-[[(2,2,5,7,8-Pentamethylchroman-6-sulfonyl)amino]-(imino)methyl]-N-[[4-[[(phenylmethoxycarbonyl)amino]-methyl]phenyl]methyl]-ornithinamid und Dicyclohexylcarbodiimid in einer Ausbeute von 96 % der Theorie.
Farblose Kristalle vom Fp. 118-121°C.
- IR (KBr):: 3442.7; 3307.7 cm⁻¹ (N-H)
1693.4; 1643.3 cm⁻¹ (C=O)

### d) (R)-N-[[4-(Aminomethyl)phenyl]methyl]-N²-(diphenylacetyl)-N⁵-[[(2,2,5,7,8-pentamethylchroman-6-sulfonyl)amino](imino)methyl]-ornithinamid

Hergestellt analog Beispiel 27c), jedoch unter Verwendung von Methanol als Lösemittel, durch katalytische Hydrierung von (R)-N²-(Diphenylacetyl)-N⁵-[[(2,2,5,7,8-pentamethylchroman-6-sulfonyl)amino](imino)methyl]-N-[[4-[[(phenylmethoxycarbonyl)amino]methyl]phenyl]methyl]-ornithinamid in Gegenwart von Palladium/Tierkohle (10%ig) in einer Ausbeute von 79 % der Theorie.
Farblose, amorphe Substanz.
- IR (KBr):: 1652.9 cm⁻¹ (Amid-C=O)
1298.0; 1166.9 cm⁻¹ (N-SO₂)

### e) (R)-[[4-(Aminomethyl)phenyl]methyl]-N²-(diphenylacetyl)argininamid-bis-(trifluoracetat)

Unter äußerer Kühlung mit einer Mischung aus qestoßenem Eis und Methanol trug man in eine aus 9,3 ml Trifluoressigsäure 0,3 ml Anisol und 0,2 ml 1,2-Ethandithiol bestehende Lösung unter Rühren 1,0 g (1,328 mMol) (R)-N-[[4-(Aminomethyl)phenyl]methyl]-N²-(diphenylacetyl)-N⁵-[[(2,2,5,7,8-pentamethylchroman-6-sulfonyl)amino](imino)methyl]-ornithinamid ein und hielt nach Fortnahme der Kühlung noch 14 Stunden bei Raumtemperatur. Vom entstandenen Niederschlag wurde abfiltriert, das Filtrat nach Zusatz von 5 ml Diethylether erneut filtriert. Das erhaltene Filtrat wurde im Vakuum und bei Raumtemperatur eingedampft, der verbliebene viskose Rückstand mit 50 ml Diethylether verrieben. Man nutschte ab und erhielt 0,50 g (53 % der Theorie) an farblosen Kristallen vom Fp. 98-103°C und R_{f} 0,32, leicht löslich in Wasser, Methanol, Dimethylsulfoxid und Dichlormethan.
- IR (KBr):: 1670.3 cm⁻¹ (Amid-C=O, Guanidinium)
- ESI-MS:: (M+H)⁺ = 487
(2M+H)⁺ = 973

### Beispiel 129

### (R,S)-N²-(Diphenylacetyl)-N-[(4-hydroxyphenyl)methyl]-N⁵-methyl-ornithinamid

Hergestellt analog Beispiel 128d) aus (R,S)-N²-(Diphenylacetyl)-N-[(4-hydroxyphenyl)methyl]-N⁵-methyl-N⁵-(phenylme thyl)-ornithinamid durch katalytische Hydrierung in Gegenwart von Palladiumhydroxid/Aktivkohle (Pearlman's catalyst) in einer Ausbeute von 75 % der Theorie.
Farbloses Kristalle vom Fp. 118-130°C (Dichlormethan) und R_{f} 0,52.
- IR (KBr):: 3290 cm⁻¹ (N-H, O-H)
1635.5 cm⁻¹ (Amid-C=O)
- MS:: M⁺ = 445

### Beispiel 130

### (R)-N-[[4-(Aminosulfonylamino)phenyl]methyl]-N²-(diphenylacetyl)-argininamid

### a) (R)-N⁵-[Amino(nitroimino)methyl]-N-[[4-(aminosulfonylamino)phenyl]methyl]-N²-(diphenylacetyl)-ornithinamid

Hergestellt analog Beispiel 36a) aus (R)-N⁵-[Amino(nitroimino)methyl]-N²-(diphenylacetyl)-ornithin, 4-(Aminosulfonylamino)-benzenmethanamin (Fp. >250°C; hergestellt aus 4-Aminobenzonitril über N-(tert.Butyl)-N'-(4-cyanphenyl)-sulfamid (Fp. 162-163°C) [N-(tert.Butyl)-sulfamoylchlorid]; 4-(Aminosulfonylamino)-benzonitril vom Fp. 163-165°C [Trifluoressigsäure] und abschließende katalytische Hydrierung in Gegenwart von Raney-Nickel und Ammoniak) und TBTU in einer Ausbeute von 29 % der Theorie. Farblose Kristalle.
- IR (KBr):: 3379.1; 3307.9; 3263.4 cm⁻¹ (N-H; NH₂)
1641.3 cm⁻¹ (Amid-C=O)
- ESI-MS:: (M+Na)⁺ = 619
(M-H)⁻ = 595

### b) (R)-N-[[4-(Aminosulfonylamino)phenyl]methyl]-N²-(diphenylacetyl)-argininamid

Hergestellt analog Beispiel 1c) aus (R)-N⁵-[Amino(nitroimino)methyl]-N-[[4-(aminosulfonylamino)phenyl]methyl]-N²-(diphenylacetyl)-ornithinamid durch katalytische Hydrierung in Gegenwart von Palladiummohr und 80%iger wässeriger Essigsäure in einer Ausbeute von 83 % der Theorie.
Farblose Kristalle vom Fp. 165-167°C und R_{f} 0,62.
- IR (KBr):: 1639.4 cm⁻¹ (Amid-C=O)
- ES-MS:: (M+H)⁺ = 552

### Beispiel 131

### (R)-N-[(4-Aminophenyl)methyl]-N²-(diphenylacetyl)-argininamid-dihydrochlorid

### a) (R)-N⁵-[Amino(nitroimino)methyl]-N²-(diphenylacetyl)-N-[(4-nitrophenyl)methyl]-ornithinamid

Hergestellt analog Beispiel 74a), jedoch unter Verwendung von N,N-Diisopropylethylamin als Base an Stelle von Triethylamin, aus (R)-N⁵-[Amino(nitroimino)methyl]-N²-(diphenylacetyl)ornithin, 4-Nitrobenzenmethanamin-hydrochlorid und TBTU in einer Ausbeute von 61 % der Theorie.
Farblose Kristalle vom Fp. 110-112°C
- IR (KBr):: 3290.4 cm⁻¹ (N-H)
1641.3 cm⁻¹ (Amid-C=O)
1517.9; 1346.2 cm⁻¹ (NO₂)
- ESI-MS:: (M+Na)⁺ = 570
(2M+Na)⁺ = 1117

### b) (R)-N-[(4-Aminophenyl)methyl]-N²-(diphenylacetyl)-argininamid-dihydrochlorid

Hergestellt analog Beispiel 29c) aus (R)-N⁵-[Amino(nitroimino)methyl]-N²-(diphenylacetyl)-N-[(4-nitrophenyl)methyl]ornithinamid durch katalytische Hydrierung in Gegenwart von Palladiummohr in einer Ausbeute von 81 % der Theorie.
Farblose, amorphe Substanz vom R_{f} 0,48.
- IR (KBr):: 1649.0 cm⁻¹ (Amid-C=O)
- ESI-MS:: (M+H)⁺ = 473
(2M+H)⁺ = 945

### Beispiel 132

### (R)-N-[(6-Chinolinyl)methyl]-N²-(diphenylacetyl)-argininamid-acetat

### a) (R)-N⁵-[Amino(nitroimino)methyl]-N-[(6-chinolinyl)methyl]-N²-(diphenylacetyl)-ornithinamid

Hergestellt analog Beispiel 87a aus (R)-N⁵-[Amino(nitroimino)methyl]-N²-(diphenylacetyl)-ornithin, 6-Chinolinmethanamin (hergestellt aus 6-Methylchinolin über 6-Chinolincarboxaldehyd [Selendioxid] und dessen reduktive Aminierung [Ammoniumacetat/Natriumcyanoborhydrid) und TBTU in einer Ausbeute von 40 % der Theorie.
Farblose Kristalle vom Fp. 222-224°C.
IR (KBr): 1637.5 cm⁻¹ (Amid-C=O)

### b) (R)-N-[(6-Chinolinyl)methyl]-N²-(diphenylacetyl)-argininamid-acetat

Hergestellt analog Beispiel 14b) aus (R)-N⁵-[Amino(nitroimino)methyl]-N-[(6-chinolinyl)methyl]-N²-(diphenylacetyl)ornithinamid durch Reduktion mit Zinn(II)-chlorid-dihydrat in Gegenwart von 60%iger wässeriger Ameisensäure in einer Ausbeute von 29 % der Theorie.
Farblose, amorphe Substanz vom R_{f} 0,42.
- IR (KBr):: 1645.2 cm⁻¹ (Amid-C=O)
- ESI-MS:: (M+H)⁺ = 509
(M+2H)⁺⁺ = 255

### Beispiel 133

### (R,S)-N⁵-(5-Amino-1H-1,2,4-triazol-3-yl)-N²-(diphenylacetyl)-N-[(4-hydroxyphenyl)methyl]-ornithinamid

### a) (R,S)-N²-(Diphenylacetyl)-N-[(4-hydroxyphenyl)methyl]-N⁵-[phenoxy(cyanimino)methyl]-ornithinamid

Die Mischung aus 0,75 g (1,74 mMol) (R,S)-N²-(Diphenylacetyl)-N-[(4-hydroxyphenyl)methyl]-ornithinamid, 0,41 g (1,74 mMol) N-Cyandiphenoxyimidocarbonat und 36 ml Isopropanol wurde 15 Stunden bei Raumtemperatur gerührt. Der entstandene Kristallbrei wurde abgenutscht, mit zweimal je 5 ml Isopropanol gewaschen und im Vakuum getrocknet. Man erhielt 0,7 g (70 % der Theorie) farblose Kristalle vom Fp. 140-142°C.
- IR (KBr):: 2189,1 cm⁻¹ (C=N-CN)
1639,4 cm⁻¹ (Amid-C=O)

### b) (R,S)-N⁵-(5-Amino-1H-1,2,4-triazol-3-yl)-N²-(diphenylacetyl)-N-[(4-hydroxyphenyl)methyl]-ornithinamid

Zu der Aufschlämmung von 0,4 g (0,695 mMol) (R,S)-N²-(Diphenylacetyl)-N-[(4-hydroxyphenyl)methyl]-N⁵-[phenoxy(cyanimino)methyl]-ornithinamid in 16 ml Methanol gab man 0,19 ml (3,13 mMol) 80%iges Hydrazinhydrat und rührte anschließend 3 Stunden bei Raumtemperatur. Die erhaltene Mischung wurde im Vakuum zur Trockene gebracht, der Rückstand in wasserfreiem Ethanol aufgenommen und über Nacht im Kühlschrank (ca. +7°C) aufbewahrt. Die erhaltenen farblosen Kristalle wurden abgenutscht, mit 3 ml eiskaltem Ethanol gründlich gewaschen und im Vakuum getrocknet. Man erhielt 0,2 g (56 % der Theorie) an farblosen Kristallen vom Fp. 133-136°C und R_{f} 0,83.
- IR (KBr):: 1641.3 cm⁻¹ (Amid-C=O)
- MS:: M⁺ = 513

### Beispiel 134

### (R)-N²-[2-(3,4-Dichlorphenyl)-1-oxoethyl]-N-[(4-hydroxyphenyl)methyl]-argininamid-acetat

### a) (R)-N⁵-[Amino(nitroimino)methyl]-N²-[2-(3,4-dichlorphenyl)-1-oxoethyl]-N-[(4-hydroxyphenyl)methyl]-ornithinamid

Hergestellt analog Beispiel 12a) aus 3,4-Dichlorbenzenessigsäure, (R)-N⁵-[Amino(nitroimino)methyl]-N-[(4-hydroxyphenyl)methyl]-ornithinamid und TBTU in einer Ausbeute von 39 % der Theorie. Farblose amorphe Substanz vom R_{f} 0,59 (Polygram^{(R)} SIL G/UV₂₅₄, Fertigfolien für die DC; Macherey-Nagel, Art. 805021; Fließmittel: Dichlormethan/Methanol/Cyclohexan/konz. wässeriges Ammoniak = 68/15/15/2 (v/v/v/v)).
IR (KBr): 1652.9 cm⁻¹ (Amid-C=O)

### b) (R)-N²-[2-(3,4-Dichlorphenyl)-1-oxoethyl]-N-[(4-hydroxyphenyl)methyl]-argininamid-acetat

Hergestellt analog Beispiel 1c) aus (R)-N⁵-[Amino(nitroimino)methyl]-N²-[2-(3,4-dichlorphenyl)-1-oxoethyl]-N-[(4-hydroxyphenyl)methyl]-ornithinamid durch katalytische Hydrierung in Gegenwart von Palladium/Tierkohle und 80%iger wässeriger Essigsäure in einer Ausbeute von 88 % der Theorie.
Farblose amorphe Substanz vom R_{f} 0,65.
- IR (KBr):: 1652.9 cm⁻¹ (Amid-C=O)
- ESI-MS:: (M+H)⁺ = 466/468/470 (Cl₂)

### Beispiel 135

### (R)-N²-(Diphenylacetyl)-N-[[4-(2-hydroxyethyl)phenyl]methyl]-argininamid

### a) (R)-N⁵-[Amino(nitroimino)methyl]-N²-(diphenylacetyl)-N-[[4-(2-hydroxyethyl)phenyl]methyl]-ornithinamid

Hergestellt analog Beispiel 87a) aus (R)-N⁵-[Amino(nitroimino)methyl]-N²-(diphenylacetyl)-ornithin, 4-(2-Hydroxyethyl)-benzenmethanamin (hergestellt aus 4-Cyanphenylacetonitril über 4-Cyanphenylessigsäure, Fp. 152-154°C [konz. Salzsäure], und abschließende Reduktion mit Lithiumaluminiumhydrid) und TBTU in einer Ausbeute von 52 % der Theorie.
Farblose Kristalle vom Fp. 168-170°C (Aceton).
IR (KBr): 1641.3 cm⁻¹ (Amid-C=O)

### b) (R)-N²-(Diphenylacetyl)-N-[[4-(2-hydroxyethyl)phenyl]methyl]-argininamid

Hergestellt analog Beispiel 1c) aus (R)-N⁵-[Amino(nitroimino)methyl]-N²-(diphenylacetyl)-N-[[4-(2-hydroxyethyl)phenyl]methyl]-ornithinamid durch katalytische Hydrierung in Gegenwart von Palladiummohr und 80%iger wässeriger Essigsäure.
Farblose, amorphe Substanz vom R_{f} 0,65.
ESI-MS: (M+H)⁺ = 502

### Beispiel 136

### (R,S)-N⁵-(3-Amino-1,2,4-oxadiazol-5-yl)-N²-(diphenylacetyl)-N-[(4-hydroxyphenyl)methyl]-ornithinamid und (R,S)-N⁵-(5-Amino-1,2,4-oxadiazol-3-yl)-N²-(diphenylacetyl)-N-[(4-hydroxyphenyl)methyl]-ornithinamid

Zu der Aufschlämmung von 0,3 g (0,521 mMol) (R,S)-N²-(Diphenylacetyl)-N-[(4-hydroxyphenyl)methyl]-N⁵-[phenoxy(cyan imino)methyl]-ornithinamid in 14 ml Methanol gab man die Lösung von 46,2 mg (1,4 mMol) Hydroxylamin in 0,5 ml Wasser und rührte 24 Stunden bei Raumtemperatur. Vom entstandenen Niederschlag wurde abfiltriert, das Filtrat im Vakuum zur Trockene gebracht, der Rückstand mit 15 ml absolutem Ethanol verrührt und erneut filtriert. Das so erhaltene Filtrat wurde chromatographisch (Kieselgel Baker, 30-60 µm; Dichlormethan/Essigsäureethylester/Methanol/Cyclohexan/konz. wässeriges Ammoniak = 59/25/7,5/7,5/1 (v/v/v/v/v)) in zwei strukturisomere Produkte zerlegt:
A.: Farblose Kristalle vom Fp. 92-96°C und R_{f} 0,95 bzw. R_{f} 020 (Polygram^{(R)} SIL G/UV₂₅₄, Fertigfolien für die DC, Macherey-Nagel, Art. 805021; Fließmittel: Dichlormethan/ Essigsäureethylester/Methanol/Cyclohexan/ konz. wässeriges Ammoniak = 59/25/7,5/7,5/1 (v/v/v/v/v)).
   Ausbeute: 30 mg (11 % der Theorie).

   - ESI-MS:: (M+H)⁺ = 515
   (M+Na)⁺ = 537
   (M+Na)⁺ = 1051
B: Farblose Kristalle vom Fp. 70-73°C und R_{f} 0,95 bzw. R_{f} 0,17 (Untersuchungsbedingungen wie bei A).
   Ausbeute: 30 mg (11 % der Theorie).

   - ESI-MS:: (M+H)⁺ = 515
   (M+Na)⁺ = 537
   (2M+H)⁺ = 1029
   (2M+Na)⁺ = 1051

Wir ordnen provisorisch der Verbindung A die Struktur des (R,S)-N⁵-(5-Amino-1,2,4-oxadiazol-3-yl)-N²-(diphenylacetyl)-N-[(4-hydroxyphenyl)methyl]-ornithinamid, der Verbindung B die Struktur des (R,S)-N⁵-(3-Amino-1,2,4-oxadiazol-5-yl)-N²-(diphenylacetyl)-N-[(4-hydroxyphenyl)methyl]-ornithinamid zu; ob die Zuordnung nicht evtl. umgekehrt sein muß, läßt sich an Hand der bislang vorliegenden spektroskopischen Daten nicht zweifelsfrei sagen.

### Beispiel 137

### (R)-N-[(4-Hydroxyphenyl)methyl]-N²-(1-naphthoyl)-argininamid-acetat

### a) (R)-N⁵-[Amino(nitroimino)methyl]-N-[(4-hydroxyphenyl)methyl]-N²-(1-naphthoyl)-ornithinamid

Hergestellt analog Beispiel 116c) aus 1-Naphthoylchlorid und (R)-N⁵-[Amino(nitroimino)methyl]-N-[(4-hydroxyphenyl)methyl]-ornithinamid-hydrochlorid in einer Ausbeute von 49 % der Theorie.
Farblose Kristalle.
IR (KBr): 1668.3; 1622.0 cm⁻¹ (C=O, C=N)

### b) (R)-N-[(4-Hydroxyphenyl)methyl]-N²-(1-naphthoyl)-argininamid-acetat

Hergestellt analog Beispiel 1c) aus (R)-N⁵-[Amino(nitroimino)methyl]-N-[(4-hydroxyphenyl)methyl]-N²-(1-naphthoyl)ornithinamid durch katalytische Hydrierung in Gegenwart von Palladiummohr und 80%iger wässeriger Essigsäure in einer Ausbeute von 30 % der Theorie.
Farblose, amorphe Substanz vom R_{f} 0,70.
IR (KBr): 1637.5 cm⁻¹ (Amid-C=O)

- ESI-MS:: (M+H)⁺ = 434
(2M+H)⁺ = 867

### Beispiel 138

### (R)-N-[(4-Hydroxyphenyl)methyl]-N²-(2-naphthoyl)-argininamid-acetat

### a) (R)-N⁵-[Amino(nitroimino)methyl]-N-[(4-hydroxyphenyl)methyl]-N²-(2-naphthoyl)-ornithinamid

Hergestellt analog Beispiel 36a) aus (R)-N⁵-[Amino(nitroimino)methyl]-N²-(2-naphthoyl)-ornithin (erhalten analog Beispiel 1a) aus 2-Naphthoylchlorid und H-D-Arg(NO₂)-OH), 4-Hydroxybenzenmethanamin und TBTU in einer Ausbeute von 36 % der Theorie.
Farblose, amorphe Substanz.
IR (KBr): 1639.4; 1626.7 cm⁻¹ (C=O, C=N)

- ESI-MS:: (M+Na)⁺ = 501
(M-H)⁻ = 477

### b) (R)-N-[(4-Hydroxyphenyl)methyl]-N²-(2-naphthoyl)-argininamid-acetat

Hergestellt analog Beispiel 1c) aus (R)-N⁵-[Amino(nitroimino)methyl]-N-[(4-hydroxyphenyl)methyl]-N²-(2-naphthoyl)ornithinamid durch katalytische Hydrierung in Gegenwart von Palladiummohr und 80%iger wässeriger Essigäsure in einer Ausheute von 63 % der Theorie.
Farblose, amorphe Substanz vom R_{f} 0,68.
IR (KBr): 1652.9 cm⁻¹ (Amid-C=O)

- ESI-MS:: (M+H)⁺ = 434
(2M+H)⁺ = 867

### Beispiel 139

### (R)-N²-(2,2-Diphenyl-2-hydroxy-1-oxoethyl)-N-[(4-hydroxyphenyl)methyl]-argininamid-acetat

### a) (R)-N⁵-[Amino(nitroimino)methyl]-N²-(2,2-diphenyl-2-hydroxy-1-oxoethyl)-N-[(4-hydroxyphenyl)methyl]-ornithinamid

0,01 Mol des aus 2-Chlor-2,2-diphenyl-acetylchlorid und (R)-N⁵-[Amino(nitroimino)methyl]-N-[(4-hydroxyphenyl)methyl]-ornithinamid-hydrochlorid analog Beispiel 124a) erhaltenen rohen Reaktionsprodukts wurden in 50 ml 80%iger wässeriger Essigsäure gelöst und nach Zugabe von 5 g Natriumacetat 30 Minuten lang auf 80°C erhitzt. Das Reaktionsprodukt wurde zwischen Wasser und Essigsäureethylester verteilt, die Essigester-Extrakte wurden über Magnesiumsulfat getrocknet und im Vakuum eingedampft. Der verbliebene Rückstand wurde an Aluminiumoxid (Aktivitätsstufe IV) unter Verwendung von Essigsäureethylester/Methanol = 99/1 (v/v) zum Eluieren chromatographisch gereinigt. Nach Aufarbeitung der geeigneten Fraktionen erhielt man 2,4 g (45 % der Theorie) einer amorphen Substanz, die ohne weitere Reinigung in der nächsten Stufe verwendet wurde.
IR (KBr): 1649.0 cm⁻¹ (Amid-C=0)

- ESI-MS:: (M+H)⁺ = 535
(M+Na)⁺ = 557
(2M+Na)⁺ = 1091

### b) (R)-N²-(2,2-Diphenyl-2-hydroxy-1-oxoethyl)-N-[(4-hydroxyphenyl)methyl]-argininamid-acetat

Hergestellt analog Beispiel 1c) auf (R)-N⁵-[Amino(nitroimino)methyl]]-N²-(2,2-diphenyl-2-hydroxy-1-oxoethyl)N-[(-4-hydroxyphenyl)methyl]-ornithinamid durch katalytische Hydrierung in Gegenwart von Palladiummohr und 80%iger wässeriger Essigsäure in einer Ausbeute von 80 % der Theorie.
Farblose, amorphe Substanz vom F_{F} 0,60.
IR (KBr): 1652,9 cm⁻¹ (Amid-C=0, Guanidinium)

- ESI-MS:: (M+H)⁺ = 490
(2M+H)⁺ = 979

### Beispiel 140

### (R,S)-N²-(Diphenylacetyl)-N⁵-(1H-imidazol-2-yl)-N-[(4-methoxyphenyl)methyl]-ornithinamid

Hergestellt analog Beispiel 85 aus (R,S)-N²-(Diphenylacetyl)-N-[(4-methoxyphenyl)methyl]-ornithinamid und N-[(2,2-Diethoxy)ethyl]-S-methyl-thiuroniumiodid in einer Ausbeute von 12 % der Theorie.
Farblose Kristalle vom Fp. 200°C (Z.) und R_{f} 0,74.
- MS:: M⁺ = 511

### Beispiel 141

### (R,S)-3-[3-(Aminoiminomethyl)phenyl]-N²-[[(diphenylmethyl)amino]carbonyl]-N-[(4-hydroxyphenyl)methyl]-alaninamid-acetat

### a) (R,S)-3-(3-Cyanphenyl)-N²-[[(diphenylmethyl)amino]carbonyl]-alaninmethylester

Hergestellt analog Beispiel 79c) aus (R,S)-2-(3-Cyanphenyl)-1-(methoxycarbonyl)-ethylisocyanat und α-Phenyl-benzenmethanamin in einer Ausbeute von 61 % der Theorie.
Farblose Kristalle vom Fp. 192-193°C.
- IR (KBr):: 2229.6 cm⁻¹ (C≡N)
1732.0 cm⁻¹ (Ester-C=O)
1624.6 cm⁻¹ (Harnstoff-C=O)

### b) (R,S)-3-(3-Cyanphenyl)-N²-[[(diphenylmethyl)amino]carbonyl]-alanin

Hergestellt analog Beispiel 79d) aus (R,S)-3-(3-Cyanphenyl)-N²-[[(diphenylmethyl)amino]carbonyl]-alaninmethylester und Lithiumhydroxid-hydrat in einer Ausbeute von 41 % der Theorie.
Farblose Kristalle.
- IR (KBr):: 3377.2 cm⁻¹ (N-H)
2229.6 cm⁻¹ (C≡N)
1714.6 cm⁻¹ (Carbonsäure-C=O)
1633.6 cm⁻¹ (Harnstoff-C=O)
- ESI-MS:: (M+H)⁺ = 400
(2M+H)⁺ = 799
(M+Na)⁺ = 422
(2M+Na)⁺ = 821

### c) (R,S)-3-(3-Cyanphenyl)-N²-[[(diphenylmethyl)amino]carbonyl]-N-[(4-hydroxyphenyl)methyl]-alaninamid

Hergestellt analog Beispiel 36a) aus (R,S)-3-(3-Cyanphenyl)-N²-[[(diphenylmethyl)amino]carbonyl]-alanin, 4-Hydroxybenzenmethanamin und TBTU in einer Ausbeute von 82 % der Theorie.
Farblose, amorphe Substanz.
IR (KBr): 1629.8 cm⁻¹ (C=O)

### d) (R,S)-3-[3-[Amino(hydroxyimino)methyl]phenyl]-N²-[[(diphenylmethyl)amino]carbonyl]-N-[(hydroxyphenyl)methyl]-alaninamid

Das Gemisch aus 2,6 g (5,15 mMol) (R,S)-3-(3-Cyanphenyl)-N²-[[(diphenylmethyl)amino]carbonyl]-N-[(4-hydroxyphenyl)methyl]-alaninamid, 1,0 g (14,4 mMol) Hydroxylamin-hydrochlorid, 1,53 g (14,4 mMol) Natriumcarbonat, 30 ml Methanol und 5 ml Wasser wurde 1,5 Stunden unter Rückfluß gekocht. Nach dem Erkalten wurde mit 100 ml Wasser verdünnt, dann mit 100 ml Diethylether überschichtet, schließlich der entstandene farblose Niederschlag abgenutscht. Das Produkt wurde nacheinander mit je 5 ml Methanol, Acetonitril und Diethylether gewaschen und im Vakuum getrocknet. Man erhielt in einer Ausbeute von 0,7 g (25 % der Theorie) farblose Kristalle vom Fp. 175-177°C und R_{f} 0,41 (Kieselgel 60 F₂₅₄ für DC, Merck (Darmstadt); Dichlormethan/Cyclohexan/Methanol/konz. wässeriges Ammoniak 68/15/15/2 (v/v/v/v)).

### e) (R,S)-3-[3-(Aminoiminomethyl)phenyl]-N²-[[(diphenylmethyl)amino]carbonyl]-N-[(4-hydroxyphenyl)methyl]-alaninamid-acetat

Hergestellt analog Beispiel 27c) aus (R,S)-3-[3-[Amino(hydroxyimino)methyl)phenyl]-N²-[[(diphenylmethyl)amino]carbonyl]-N-[(4-hydroxypheny)methyl]-alaninamid durch katalytische Hydrierung in Gegenwart von Palladium/Tierkohle und Eisessig in einer Ausbeute von 20 % der Theorie.
Farblose, amorphe Substanz vom R_{f} 0,80.
IR (KBr): 1658.8 cm⁻¹ (Amid-C=O, Amidinium)

- ESI-MS:: (M+H)⁺ = 522
(2M+H)⁺ = 1043

### Beispiel 142

### (R,S)-N²-(Diphenylacetyl)-N⁵-(1H-imidazol-2-yl)-N-(phenylmethyl)-ornithinamid

### a) (R,S)-N⁵-(Phenylmethoxycarbonyl)-N-(phenylmethyl)-ornithinamid

Unter äußerer Kühlung und Einhaltung einer Reaktionstemperatur von 5-10°C wurde zu einer Lösung von 5,0 g (17,1 mMol) N²-Carboxy-N⁵-(phenylmethoxycarbonyl)-ornithin-anhydrid (M. Bodanszky, Principles of Peptide Synthesis, Springer-Verlag, 1984, S. 124) in 100 ml Tetrahydrofuran die Lösung von 12,2 ml (11,99 g; 0,112 Mol) Benzenmethanamin in 50 ml Tetrahydrofuran zugetropft. Man hielt noch 1 Stunde bei der angegebenen Temperatur und dampfte anschließend die erhaltene Suspension im Vakuum und bei einer Badtemperatur von +50°C ein. Das so erhaltene Produkt wurde mit 10 ml Diisopropylether bei +40°C verrührt. Der entstandene Kristallbrei wurde nach dem völligen Erkalten abgenutscht, das Kristallisat nochmals mit 10 ml Diisopropylether gewaschen und bei 60°C im Vakuum getrocknet.
Ausbeute: 4,90 g (81 % der Theorie).
Farblose Kristalle vom Fp. 87°C.
- IR (KBr):: 3330.9 cm⁻¹ (N-H)
1693.4 cm⁻¹ (Urethan-C=O)
1643.3 cm⁻¹ (Amid-C=O)
- MS:: M⁺ = 355

### b) (R,S)-N²-(Diphenylacetyl)-N⁵-(phenylmethoxycarbonyl)-N-(phenylmethyl)-ornithinamid

Hergestellt analog Beispiel 32d) aus (R,S)-N⁵-(Phenylmethoxycarbonyl)-N-(phenylmethyl)-ornithinamid und Diphenylacetylchlorid in einer Ausbeute von 75 % der Theorie.
Farblose Kristalle vom Fp. 167-170°C.

### c) (R,S)-N²-(Diphenylacetyl)-N-(phenylmethyl)-ornithinamidacetat

Hergestellt analog Beispiel 27c), jedoch unter Verwendung von Methanol/Wasser/Eisessig = 7/3/1 (v/v/v) als Lösemittel, durch katalytische Hydrierung von (R,S)-N²-(Diphenylacetyl)-N⁵-(phenylmethoxycarbonyl)-N-(phenylmethyl)-ornithinamid in Gegenwart von Palladium/Tierkohle als Katalysator in einer Ausbeute von 70 % der Theorie.
Farblose Kristalle vom Fp. 159-160°C.

### d) (R,S)-N²-(Diphenylacetyl)-N⁵-(1H-imidazol-2-yl)-N-(phenylmethyl)-ornithinamid

Hergestellt analog Beispiel 85) aus (R,S)-N²-(Diphenylacetyl)-N-(phenylmethyl)-ornithinamid-acetat und N-[(2,2-Diethoxy)ethyl]-S-methylthiuroniumiodid in einer Ausbeute von 25 % der Theorie.
Farblose Kristalle vom Fp. 195°C (Z.) und R_{f} 074.
- IR (KBr):: 3309.7; 3240.2 cm⁻1 (N-H)
1639.4; 1666.4 cm⁻¹ (Amid-C=O)
- MS:: M⁺ = 481

### Beispiel 143

### (R)-N²-(2,2-Diphenylethyl)-N-[(4-hydroxyphenyl)methyl]argininamid-hydrochlorid

### a) (R)-N⁵-[Amino(nitroimino)methyl]-N²-(2,2-diphenylethyl)-N-[(4-hydroxyphenyl)methyl]-ornithinamid-hydrochlorid

Die Mischung aus 1,8 g (5,0 mMol) (R)-N⁵-[Amino(nitroimino)methyl]-N-[(4-hydroxyphenyl)methyl]-ornithinamid-hydrochlorid, 1,0 g (5,096 mMol) Diphenylacetaldehyd, 0,75 g (11,94 mMol) Natriumcyanoborhydrid und 100 ml wasserfreiem Methanol wurde 24 Stunden bei Raumtemperatur gerührt. Das Lösemittel wurde im Vakuum abdestilliert, der Rückstand zwischen Wasser und Essigsäureethylester verteilt, die Essigesterphase mit 5 ml 1N-Salzsäure versetzt, der dabei entstandene Niederschlag abgenutscht und nacheinander mit Wasser und Essigsäureethylester gewaschen, dann im Vakuum getrocknet. Ausbeute: 1,4 g (52 % der Theorie).
Farblose Kristalle vom Fp. 244-247°C.
- IR (KBr):: 3394.5 cm⁻¹ (N-H)
1681.8; 1672.2; 1639.4 cm⁻¹ (C=O, C=N)
- ESI-MS:: (M+H)⁺ = 505
(M+Na)⁺ = 527
(2M+H)⁺ = 1009

### b) (R)-N²-(2,2-Diphenylethyl)-N-[(4-hydroxyphenyl)methyl]argininamid-hydrochlorid

Hergestellt analog Beispiel 1c) aus (R)-N⁵-[Amino(nitroimino)methyl]-N²-(2,2-diphenylethyl)-N-[(4-hydroxyphenyl)methyl]-ornithinamid-hydrochlorid durch katalytische Hydrierung in Gegenwart von Palladiummohr und 80%iger wässeriger Essigsäure in einer Ausbeute von 78 % der Theorie.
Farblose, amorphe Substanz vom R_{f} 0,55.
IR (KBr): 1652.9 cm⁻¹ (Amid-C=O, Guanidinium)
ESI-MS: (M+H)⁺ = 460

### Beispiel 144

### (R,S)-N-[(4-Amino-3,5-dichlorphenyl)methyl]-N²-(diphenylacetyl)-N⁵-(1H-imidazol-2-yl)-ornithinamid

### a) (R,S)-N²-(Diphenylacetyl)-N⁵-(phenylmethoxycarbonyl)ornithin

Hergestellt analog Beispiel 1a) aus Diphenylacetylchlorid und (R,S)-N⁵-(Phenylmethoxycarbonyl)-ornithin in Gegenwart von Natronlauge in einer Ausbeute von 100 % der Theorie.
Farblose Kristalle vom Fp. 128-130°C.

### b) (R,S)-N²-(Diphenylacetyl)-ornithin

Hergestellt analog Beispiel 142c) aus (R,S)-N²-(Diphenylacetyl)-N⁵-(phenylmethoxycarbonyl)-ornithin durch katalytische Hydrierung in Gegenwart von Palladium/Tierkohle in einer Ausbeute von 81 % der Theorie.
Farblose Kristalle vom Fp. 170-172°C.

### c) (R,S)-N⁵-(tert.Butyloxycarbonyl)-N²-(diphenylacetyl)ornithin

Hergestellt analog Beispiel 52b) aus (R,S)-N²-(Diphenylacetyl)-ornithin und Di-tert.-butyldicarbonat in Gegenwart von Natronlauge in einer Ausbeute von 99 % der Theorie.
Farblose, kristalline Substanz, die ohne weitere Reinigung verwendet wurde.

### d) (R,S)-N-[(4-Amino-3,5-dichlorphenyl)methyl]-N⁵-(tert.-butyloxycarbonyl)-N²-(diphenylacetyl)-ornithinamid

Hergestellt analog Beispiel 67a) aus (R,S)-N⁵-(tert.Butyloxycarbonyl)-N²-(diphenylacetyl)-ornithin, 4-Amino-3,5-dichlorbenzenmethanamin und TBTU in einer Ausbeute von 97 % der Theorie.
Farblose Kristalle vom Fp. 123°C.
- IR (KBr):: 3444.7; 3280.7 cm⁻¹ (N-H)
1685.7 cm⁻¹ (Urethan-C=O)
1639.4 cm⁻¹ (Amid-C=O)

### e) (R,S)-N-[(4-Amino-3,5-dichlorphenyl)methyl]-N²-(diphenylacetyl)-N⁵-(1H-imidazol-2-yl)-ornithinamid

Das analog Beispiel 36b) aus (R,S)-N-[(4-Amino-3,5-dichlorphenyl)methyl]-N⁵-(tert.-butyloxycarbonyl)-N²-(diphenylacetyl)-ornithinamid durch Behandlung mit methanolischer Chlorwasserstoff-Lösung erhaltene (R,S)-N-[(4-Amino-3,5-dichlorphenyl)methyl]-N²⁻(diphenylacetyl)-ornithinamid wurde als Rohprodukt direkt entsprechend Beispiel 85 mit N-[(2,2-Diethoxy)ethyl]-S-methylthiuroniumiodid, dann mit Salzsäure umgesetzt.
Ausbeute: 4 % der Theorie.
Farblose Kristalle vom Fp. 184-186°C und R_{f} 0,78.
- IR (KBr):: 3384.9; 3307.7 cm⁻¹ (N-H)
1651.0; 1635.5 cm⁻¹ (Amid-C=O)
- MS:: M⁺ = 564/566 (Cl₂)

### Beispiel 145

### (R,S)-N-[(4-Amino-3-fluorphenyl)methyl]-N²-(diphenylacetyl)-N⁵-(1H-imidazol-2-yl)-ornithinamid

### a) (R,S)-N-[(4-Amino-3-fluorphenyl)methyl]-N⁵-(tert.-butyloxycarbonyl)-N²-(diphenylacetyl)-ornithinamid

Hergestellt analog Beispiel 67a) aus (R,S)-N⁵-(tert.Butyloxycarbonyl)-N²-(diphenylacetyl)-ornithin, 4-Amino-3-fluorbenzenmethanamin und TBTU in einer Ausbeute von 60 % der Theorie.
Farblose Kristalle vom Fp. 168-170°C.
- IR (KBr):: 3446.6; 3555.9; 3261.4 cm⁻¹ (N-H)
1695.3 cm⁻¹ (Urethan-C=O)
1645.2 cm⁻¹ (Amid-C=O)

### b) (R,S)-N-[(4-Amino-3-fluorphenyl)methyl]-N²-(diphenylacetyl)-N⁵-(1H-imidazol-2-yl)-ornithinamid

Hergestellt analog Beispiel 144e) aus (R,S)-N-[(4-Amino-3-fluorphenyl)methyl]-N⁵-(tert.-butyloxycarbonyl)-N²-(diphenylacetyl)-ornithinamid durch aufeinanderfolgende Behandlung mit methanolischer Chlorwasserstoff-Lösung, dann N-[(2,2-Diethoxy)ethyl]-S-methylthiuroniumiodid, zuletzt mit Salzsäure in einer Ausbeute von 23 % der Theorie.
Farblose Kristalle vom Fp. 162°C und R_{f} 0,69.
- IR (KBr):: 1639.4 cm⁻¹ (Amid-C=O)
- MS:: M⁺ = 514

### Beispiel 146

### (R,S)-N-[(4-Hydroxyphenyl)methyl]-N⁵-(1H-imidazol-2-yl)-N²-(2-naphthoyl)-ornithinamid-hydrochlorid

### a) (R,S)-N-[(4-Hydroxyphenyl)methyl]-N⁵-(phenylmethoxycarbonyl)-ornithinamid

Hergestellt analog Beispiel 142a) aus (R,S)-4-[3-[(Phenylmethoxycarbonyl)amino]propyl]-4,5-dihydro-1,3-oxazol-2,5-dion und 4-Hydroxybenzenmethanamin in einer Ausbeute von 86 % der Theorie.
Farblose Kristalle vom Fp. 162°C (Ethylacetat).
- IR (KBr):: 1689.5 cm⁻¹ (Urethan-C=0)
- ESI-MS:: (M+H)⁺ = 372
(2M+H)⁺ = 743
(M+Na)⁺ = 394
(2M+Na)⁺ = 765

### b) (R,S)-N-[(4-Hydroxyphenyl)methyl]-N²-(2-naphthoyl)-N⁵-(phenylmethoxycarbonyl)-ornithinamid

Hergestellt analog Beispiel 36a) aus 2-Naphthoesäure, (R,S)N-[(4-Hydroxyphenyl)methyl]-N⁵-(phenylmethoxycarbonyl)-or-nithinamid und TBTU in einer Ausbeute von 100 % der Theorie.
Farblose Kristalle vom Fp. 168-170°C.
IR (KBr): 1687.6 cm⁻¹ (Urethan-C=0)

### c) (R,S)-N-[(4-Hydroxyphenyl)methyl]-N²-(2-naphthoyl)ornithinamid-acetat

Hergestellt analog Beispiel 142c) aus (R,S)-N-[(4-Hydroxyphenyl)methyl]-N²-(2-naphthoyl)-N⁵-(phenylmethoxycarbonyl)-ornithinamid durch katalytische Hydrierung in Gegenwart von Palladium/Tierkohle (10%ig) in einer Ausbeute von 100 % der Theorie.
Farblose Kristalle.
- IR (KBr):: 1635.5 cm⁻¹ (Amid-C=0)
1517.9 cm⁻¹ (Amid-II)

### d) (R,S)-N-[(4-Hydroxyphenyl)methyl]-N⁵-(1H-imidazol-2-yl)-N²-(2-naphthoyl)-ornithinamid-hydrochlorid

Hergestellt analog Beispiel 85 aus (R,S)-N-[(4-Hydroxyphenyl)methyl]-N²-(2-naphthoyl)-ornithinamid-acetat und N-[(2,2-Diethoxy)ethyl]-S-methylthiuroniumchlorid in einer Ausbeute von 44 % der Theorie.
Farblose Kristalle vom Fp. 197°C und R_{f} 0,76.
IR (KBr): 1670.3 cm⁻¹ (Amid-C=0)
ESI-MS: (M+H)⁺ = 458

### Beispiel 147

### (R,S)-N²-[[(Diphenylmethyl)amino]carbonyl]-N-[(4-hydroxyphenyl)methyl]-N⁵-(1H-imidazol-2-yl)-ornithinamid

### a) (R,S)-2-Isocyanato-5-[[(phenylmethoxy)carbonyl]amino]pentansäuremethylester

Als Dichlormethan-Lösung hergestellt analog Beispiel 79b) aus N⁵-[(Phenylmethoxy)carbonyl]-ornithinmethylester-hydrochlorid und Phosgen. Aliquote Teile davon wurden ohne Reinigung in den folgenden Reaktionen verwendet.

### b) (R,S)-N²-[[(Diphenylmethyl)amino]carbonyl]-N⁵-[(phenylmethoxy)carbonyl]-ornithinmethylester

Hergestellt analog Beispiel 79c) aus (R,S)-2-Isocyanato-4-[[(phenylmethoxy)carbonyl]amino]-pentansäuremethylester und α-Phenyl-benzenmethanamin in einer Ausbeute von 69 % der Theorie.
Farblose Kristalle vom Fp. 104-105°C.
- IR (KBr):: 3398.4 cm⁻¹ (N-H)
1749.3 cm⁻¹ (Ester-C=O)
1689.5 cm⁻¹ (Urethan-C=O)
1627.8 cm⁻¹ (Harnstoff-C=O)

### c) (R,S)-N²-[[(Diphenylmethyl)amino]carbonyl]-N⁵-[(phenylmethoxy)carbonyl]-ornithin

Hergestellt analog Beispiel 79d) aus (R,S)-N²-[[(Diphenylmethyl)amino]carbonyl]-N⁵-[(phenylmethoxy)carbonyl]-ornithinmethylester durch Verseifung mit Lithiumhydroxid-hydrat in einer Ausbeute von 54 % der Theorie.
Farblose Kristalle vom Fp. 172-173°C.
- IR (KBr):: 1718.5 cm⁻¹ (Carboxyl-C=O)
1674.1 cm⁻¹ (Urethan-C=O)
- ESI-MS:: (M+Na)⁺ = 498
(M-H)⁻ = 474

### d) (R,S)-N²-[[(Diphenylmethyl)amino]carbonyl]-N-[(4-hydroxyphenyl)methyl]-N⁵-[(phenylmethoxy)carbonyl]-ornithinamid

Hergestellt analog Beispiel 67a) aus (R,S)-N²-[[(Diphenylmethyl)amino]carbonyl]-N⁵-[(phenylmethoxy)carbonyl]-ornithin, 4-Hydroxybenzenmethanamin und TBTU in einer Ausbeute von 54 % der Theorie.
Farblose Kristalle vom Fp. 128-130°C.
- IR (KBr):: 1687.6 cm⁻¹ (Urethan-C=O)
1625.9 cm⁻¹ (Harnstoff-C=O)
- ESI-MS:: (M+H)⁺ = 581
(M+Na)⁺ = 603
(2M+Na)⁺ = 1183

### e) (R,S)-N²-[[(Diphenylmethyl)amino]carbonyl]-N-[(4-hydroxyphenyl)methyl]-ornithinamid-acetat

Hergestellt analog Beispiel 142c) aus (R,S)-N²-[[(Diphenylmethyl)amino]carbonyl]-N-[(4-hydroxyphenyl)methyl]-N⁵⁻[(phe nylmethoxy)carbonyl]-ornithinamid durch katalytische Hydrierung in Gegenwart von Palladium/Aktivkohle (10%ig) in quantitativer Ausbeute.
Farblose Kristalle.
IR (KBr): 1649.0; 1554.5 cm⁻¹ (C=O)

### f) (R,S)-N²-[[(Diphenylmethyl)amino]carbonyl]-N-[(4-hydroxyphenyl)methyl]-N⁵-(1H-imidazol-1-yl)-ornithinamid

Hergestellt analog Beispiel 85 aus (R,S)-N²-[[(Diphenylmethyl)amino]carbonyl]-N-[(4-hydroxyphenyl)methyl]-ornithinamid-acetat und N-[(2,2-Diethoxy)ethyl]-S-methylthiuroniumchlorid in einer Ausbeute von 8 % der Theorie.
Farblose Kristalle vom R_{f} 0.77
ESI-MS: (M+H)⁺ = 513

### Beispiel 148

### (R,S)-N-[(4-Hydroxyphenyl)methyl]-N⁵-(1H-imidazol-2-yl)-N²-[2-(2-naphthyl)-1-oxoethyl]-ornithinamid

### a) (R,S)-N-[(4-Hydroxyphenyl)methyl]-N⁵-[(phenylmethoxy)carbonyl]-ornithinamid

Hergestellt analog Beispiel 142a) aus (R,S)-3,4-Dihydro-4-[3-[[(phenylmethoxy)carbonyl]amino]propyl]-1,3-oxazol-2,5-dion und 4-Hydroxybenzenmethanamin in einer Ausbeute von 87 % der Theorie.
Farblose Kristalle vom Fp. 162-163°C.
IR (KBr): 1691.5 (Urethan-C=O)

### b) (R,S)-N-[(4-Hydroxyphenyl)methyl]-N²-[2-(2-naphthyl)-1-oxoethyl]-N⁵-[(phenylmethoxy)carbonyl]-ornithinamid

Hergestellt analog Beispiel 67a), jedoch unter Verwendung von Triethylamin als Base an Stelle von N,N-Diisopropyl-ethylamin und von Tetrahydrofuran/Dimethylformamid = 5/2 (v/v) als Lösemittel an Stelle von Dimethylformamid, aus (R,S)-N-[(4-Hydroxyphenyl)-methyl]-N⁵-[(phenylmethoxy)carbonyl]-ornithinamid, 4-Hydroxybenzenmethanamin und TBTU in einer Ausbeute von 75 % der Theorie.
Farblose Kristalle vom Fp. 120-125°C.
MS: M⁺ = 539

### c) (R,S)-N-[(4-Hydroxyphenyl)methyl]-N²-[2-(2-naphthyl)-1-oxoethyl]-ornithinamid-acetat

Hergestellt analog Beispiel 142c) aus (R,S)-N-[(4-Hydroxyphenyl)methyl]-N²-[2-(2-naphthyl)-1-oxoethyl]-N⁵-[(phenylmethoxy)carbonyl]-ornithinamid durch katalytische Hydrierung in Gegenwart von Palladium/Aktivkohle (10%ig) in einer Ausbeute von 86 % der Theorie.
Farblose Kristalle vom Fp. 160-162°C.
MS: M⁺ = 405

### d) (R,S)-N-[(4-Hydroxyphenyl)methyl]-N⁵-(1H-imidazol-2-yl)-N²-[2-(2-naphthyl)-1-oxoethyl]-ornithinamid

Hergestellt analog Beispiel 85 aus (R,S)-N-[(4-Hydroxyphenyl)methyl]-N²-[2-(2-naphthyl)-1-oxoethyl]-ornithinamidacetat und N-[(2,2-Diethoxy)ethyl]-S-methylthiuroniumchlorid in einer Ausbeute von 33 % der Theorie.
Farblose Kristalle vom R_{f} 0,70.
- IR (KBr):: 1645.2 cm⁻¹ (Amid-C=O)
- ESI-MS:: (M+H)⁺ = 472

### Beispiel 149

### (R,S)-N-[(4-Hydroxyphenyl)methyl]-N⁵-(1H-imidazol-2-yl)-N²-[[(2-naphthyl)amino]carbonyl]-ornithinamid

### a) (R,S)-N²-[[(2-Naphthyl)amino]carbonyl]-N⁵-[(phenylmethoxy)carbonyl]-ornithinmethylester

Hergestellt analog Beispiel 79c) aus (R,S)-2-Isocyanato-5-[(phenylmethoxy)carbonyl]amino]-pentansäuremethylester und 2-Naphthylamin in einer Ausbeute von 93 % der Theorie.
Farblose Kristalle.
IR (KBr): 1720.4 cm⁻¹ (Ester-C=O)

### b) (R,S)-N²-[[(2-Naphthyl)amino]carbonyl]-N⁵-[(phenylmethoxy)carbonyl]-ornithin

Das Gemisch aus 4,7 g (10,46 mMol) (R,S)-N²-[[(2-Naphthyl)amino]carbonyl]-N⁵-[(phenylmethoxy)carbonyl]-ornithinmethylester, 0,42 g (10,5 mMol) Natriumhydroxid, 50 ml Ethanol und 10 ml Wasser wurde eine Stunde unter Rückfluß gekocht. Nach dem Erkalten wurde mit 200 ml Wasser verdünnt und mit 5%iger wässeriger Salzsäure bis pH 3 angesäuert. Man extrahierte erschöpfend mit Essigsäureethylester, trocknete die vereinigten Essigester-Extrakte über Natriumsulfat, klärte sie mit Aktivkohle und dampfte sie im Vakuum ein. Das erhaltene Kristallisat wurde ohne weitere Reinigung weiterverarbeitet.
Ausbeute: 3,8 g (83 % der Theorie).

- IR (KBr):: 1687.6 cm⁻¹ (Urethan-C=O)
1677.1 cm⁻¹ (C=O)

### c) (R,S)-N-[(4-Hydroxyphenyl)methyl]-N²-[[(2-naphthyl)amino]carbonyl]-N⁵-[(phenylmethoxy)carbonyl]-ornithinamid

Hergestellt analog Beispiel 67a) aus (R,S)-N²-[[(2-Naphthyl)amino]carbonyl]-N⁵-[(phenylmethoxy)carbonyl]-ornithin, 4-Hydroxybenzenmethanamin und TBTU in quantitativer Ausbeute. Farblose Kristalle vom Fp. 145-146°C (Essigsäureethylester).
- IR (KBr):: 1685.7 cm⁻¹ (Urethan-C=O)
1635.5 cm⁻¹ (C=O)

### d) (R,S)-N-[(4-Hydroxyphenyl)methyl]-N²-[[(2-naphthyl)amino]carbonyl]-ornithinamid-acetat

Hergestellt analog Beispiel 142c) aus (R,S)-N-[(4-Hydroxyphenyl)methyl]-N²-[[(2-naphthyl)amino]carbonyl]-N⁵-[(phenylmethoxy)carbonyl]-ornithinamid durch katalytische Hydrierung in Gegenwart von Palladium/Aktivkohle (10%ig) in einer Ausbeute von 70 % der Theorie.
Farblose Kristalle.
IR (KBr): 1637.5 cm⁻¹ (C=O)

### e) (R,S)-N-[(4-Hydroxyphenyl)methyl]-N⁵-(1H-imidazol-2-yl)-N²-[[(2-naphthyl)amino]carbonyl]-ornithinamid

Hergestellt analog Beispiel 85 aus (R,S)-N-[(4-Hydroxyphenyl)methyl]-N²-[[(2-naphthyl)amino]carbonyl]-ornithinamid-acetat und N-[(2,2-Diethoxy)ethyl]-S-methylthiuroniumiodid in einer Ausbeute von 6 % der Theorie.
Farblose Kristalle vom R_{f} 0,75.
- IR (KBr):: 1670.3 cm⁻¹ (Amid-C=O)
- ESI-MS:: (M+H)⁺ = 473

### Beispiel 150

### (R,S)-N²-(Diphenylacetyl)-N-[(4-hydroxyphenyl)methyl]-3-[(4-piperidinyl)methyl]-alaninamid-hydrochlorid

### a) (R,S)-2-(Acetylamino)-4-(4-pyridinyl)-butansäureethylester

Hergestellt analog Beispiel 31a), jedoch unter Verwendung von Kaliumethylat an Stelle von Natriumethylat und von wasserfreiem Ethanol als Lösemittel an Stelle von Dioxan, aus Acetamidomalonsäurediethylester und 4-Vinylpyridin in einer Ausbeute von 39 % der Theorie:
Farbloses, hochviskoses Öl.
- IR (KBr):: 1741.6 cm⁻¹ (Ester-C=O)
1658.7 cm⁻¹ (Amid-C=O)
- MS:: M⁺ = 250

### b) (R,S)-3-[(4-Pyridinyl)methyl]-alanin-hydrochlorid

Die Mischung aus 9,0 g (0,036 Mol) (R,S)-2-(Acetylamino)-4-(4-pyridinyl)-butansäureethylester und 50 ml konz. Salzsäure wurde 5 Stunden unter Rückfluß gekocht. Dann wurde das Reaktionsgemisch im Vakuum zur Trockene gebracht, der Rückstand in Methanol aufgenommen und erneut im Vakuum eingedampft. Beim Verreiben mit Isopropanol trat Kristallisation ein. Man nutschte ab, wusch den Nutscheninhalt gründlich mit Diethylether und trocknete anschließend an der Luft. Man erhielt 7,5 g (96 % der Theorie) an farblosen Kristallen vom Fp. 208-210°C
- IR (KBr):: 1741.6 cm⁻¹
- MS:: m/e = 135, m/e = 118, m/e = 93

### c) (R,S)-N²-(Diphenylacetyl)-3-[(4-pyridinyl)methyl]-alanin

Hergestellt anaolg Beispiel 1a) aus (R,S)-3-[(4-Pyridinyl)methyl]-alanin-hydrochlorid und Diphenylacetylchlorid in Gegenwart von Natronlauge in einer Ausbeute von 62 % der Theorie.
Farblose Kristalle.
- IR (KBr):: 3300.0 cm⁻¹ (N-H, O-H)
1703.0 cm⁻¹ (Carboxyl-C=O)
1635.5 cm⁻¹ (Amid-C=O)
- MS:: M⁺ = 374

### d) (R,S)-N²-(Diphenylacetyl)-N-[(4-hydroxyphenyl)methyl]-3-[(4-pyridinyl)methyl]-alaninamid-hydrochlorid

Hergestellt anaolg Beispiel 67a) aus (R,S)-N²-(Diphenylacetyl)-3-[(4-pyridinyl)methyl]-alanin, 4-Hydroxybenzenmethanamin und TBTU in einer Ausbeute von 26 % der Theorie. Farblose Kristalle vom Fp. >250°C und R_{f} 0,55 (Kieselgel 60 F₂₅₄ für DC, Merck Darmstadt; Dichlormethan/Methanol/ Cyclohexan/konz. wässeriges Ammoniak = 68/15/15/2 (v/v/v/v)).
- IR (KBr):: 1645.2 cm⁻¹ (Amid-C=O)
- MS:: M⁺ = 479

### e) (R,S)-N²-(Diphenylacetyl)-N-[(4-hydroxyphenyl)methyl]-3-[(4-piperidinyl)methyl]-alaninamid-hydrochlorid

Die Lösung von 1,4 g (2,713 mMol) (R,S)-N²-(Diphenylacetyl)-N-[(4-hydroxyphenyl)methyl]-3-[(4-pyridinyl)methyl]-alaninamid-hydrochlorid in 50 ml Methanol wurde nach Zugabe von 0,2 g Platin(IV)-oxid und 10 ml 1N Salzsäure bei Raumtemperatur und 3 bar Wasserstoffdruck bis zur Beendigung der Wasserstoffaufnahme hydriert. Man filtrierte vom Katalysator ab, engte das Filtrat im Vakuum ein und kristallisierte den Rückstand aus Isopropanol um. Nach dem Waschen mit tert.-Butylmethylether und Trocknen im Vakuum erhielt man farblose Kristalle vom Fp. 234-235°C und R_{f} 0,57 in einer Ausbeute von 0,7 g (49 % der Theorie).
- IR (KBr):: 1643.3 cm⁻¹ (Amid-C=O)
- MS:: M⁺ = 485

### Beispiel 151

### (R,S)-N²-[(2-Chinolinyl)carbonyl]-N-[(4-hydroxyphenyl)methyl]-N⁵-(1H-imidazol-2-yl)-ornithinamid und Diastereomere von N-[(4-Hydroxyphenyl)methyl]-N⁵-(1H-imidazol-2-yl)-N²-[(1,2,3,4-tetrahydro-2-chinolinyl)carbonyl]-ornithinamid

### a) (R,S)-N²-[(2-Chinolinyl)carbonyl]-N-[(4-hydroxyphenyl)methyl]-N⁵-[(phenylmethoxy)carbonyl]-ornithinamid

Hergestellt analog Beispiel 148b) aus Chinolin-2-carbonsäure, (R,S)-N-[(4-Hydroxyphenyl)methyl]-N⁵-[(phenylmethoxy)carbonyl]-ornithinamid und TBTU in einer Ausbeute von 88 % der Theorie.
Farblose Kristalle vom Fp. 189-192°C.
- ESI-MS:: (M+Na)⁺ = 549
(M+K)⁺ = 565

### b) Gemisch von (R,S)-N²-[(2-Chinolinyl)carbonyl]-N-[(4-hydroxyphenyl)methyl]-ornithinamid und Diastereomeren von N-[(4-Hydroxyphenyl)methyl]-N²-[(1,2,3,4-tetrahydro-2-chinolinyl)carbonyl]-ornithinamid

Hergestellt analog Beispiel 142c) aus (R,S)-N²-[(2-Chinolinyl)carbonyl]-N-[(4-hydroxyphenyl)methyl]-N⁵-[(phenylmethoxy)carbonyl]-ornithinamid durch katalytische Hydrierung in Gegenwart von Palladium/Aktivkohle (10%ig) in einer Ausbeute von 74 % der Theorie.
IR (KBr): 1643 cm⁻¹ (Amid-C=O)

- ESI-MS:: (M₁+H)⁺ = 393
(M₂+H)⁺ = 397

Das Gemisch wurde ohne Trennung für die folgende Stufe verwendet.

### c) (R,S)-N²-[(2-Chinolinyl)carbonyl]-N-[(4-hydroxyphenyl)methyl]-N⁵-(1H-imidazol-2-yl)-ornithinamid und Diastereomere von N-[(4-Hydroxyphenyl)methyl]-N⁵-(1H-imidazol-2-yl)-N²-[(1,2,3,4-tetrahydro-2-chinolinyl)carbonyl]-ornithinamid

Das analog Beispiel 85 aus einem Gemisch von (R,S)-N²-[(2-Chinolinyl)carbonyl]-N-[(4-hydroxyphenyl)methyl]-ornithinamid und den beiden Diastereomeren von N-[(4-Hydroxyphenyl)methyl]-N²-[(1,2,3,4-tetrahydro-2-chinolinyl)carbonyl]ornithinamid durch Umsetzung mit zunächst N-[(2,2-Diethoxy)ethyl]-S-methylthiuroniumiodid, dann mit Salzsäure erhaltene Produktgemisch wurde an Kieselgel (Baker, 30-60 µm) unter Verwendung von Essigsäureethylester/Cyclohexan/Methanol/konz. wäßriges Ammoniak = 10/2/1/0,1 (v/v/v/v) als mobile Phase chromatographisch getrennt und anschließend jeweils aus Diisopropylether umkristallisiert. Man erhielt:
I. N²-[(2-Chinolinyl)carbonyl]-N-[(4-hydroxyphenyl)methyl]-N⁵-(1H-imidazol-2-yl)-ornithinamid,
   Ausbeute: 1 % der Theorie.
   Farblose Kristalle vom R_{f} 0,70
   ESI-MS: (M+H)⁺ = 459
II. N-[(4-Hydroxyphenyl)methyl]-N⁵-(1H-imidazol-2-yl)-N²-[(1,2,3,4-tetrahydro-2-chinolinyl)carbonyl]-ornithinamid (Diastereomer A),
   Ausbeute: 3 % der Theorie.
   Farblose Kristalle vom R_{f} 0,69
   ESI-MS: (M+H)⁺ = 463
III. N-[(4-Hydroxyphenyl)methyl]-N⁵-(1H-imidazol-2-yl)-N²-[(1,2,3,4-tetrahydro-2-chinolinyl)carbonyl]-ornithinamid (Diastereomer B),
   Ausbeute: 1 % der Theorie.
   Farblose Kristalle vom R_{f} 0,62
   ESI-MS: (M+H)⁺ = 463

### Beispiel 152

### Diastereomere von N-[(4-Hydroxyphenyl)methyl]-N⁵-(1H-imidazol-2-yl)-N²-[(1,2,3,4-tetrahydro-3-chinolinyl)carbonyl]-ornithinamid

### a) (R,S)-N²-[(3-Chinolinyl)carbonyl]-N-[(4-hydroxyphenyl)-methyl]-N⁵-[(phenylmethoxy)carbonyl]-ornithinamid

Hergestellt analog Beispiel 148b) aus Chinolin-3-carbonsäure, (R,S)-N-[(4-Hydroxyphenyl)methyl]-N⁵-[(phenylmethoxy)carbonyl]-ornithinamid und TBTU in einer Ausbeute von 60 % der Theorie.
Farblose Kristalle vom Fp. 181-183°C
- ESI-MS:: (M+H)⁺ = 527
(M+Na)⁺ = 549
(M+K)⁺ = 565

### b) Gemisch der Diastereomeren von N-[(4-Hydroxyphenyl)methyl]-N²-[(1,2,3,4-tetrahydro-3-chinolinyl)carbonyl]ornithinamid-acetat

Hergestellt analog Beispiel 142c) aus (R,S)-N²-[(3-Chinolinyl)carbonyl]-N-[(4-hydroxyphenyl)methyl]-N⁵-[(phenylmethoxy)carbonyl]-ornithinamid durch katalytische Hydrierung in Gegenwart von Palladium/Aktivkohle (10%ig) in einer Ausbeute von 70 % der Theorie.
Farbloses, amorphes Produkt, das ohne Trennung in der nächsten Stufe eingesetzt wurde.
IR (KBr): 1649.0 cm⁻¹ (Amid-C=O)

### c) Diastereomere von N-[(4-Hydroxyphenyl)methyl]-N⁵-(1H-imidazol-2-yl)-N²-[(1,2,3,4-tetrahydro-3-chinolinyl)carbonyl]-ornithinamid

Das analog Beispiel 85 aus einem Gemisch der beiden Diastereomeren von N-[(4-Hydroxyphenyl)methyl]-N²-[(1,2,3,4-tetrahydro-3-chinolinyl)carbonyl]-ornithinamid-acetat durch Umsetzung mit zunächst N-[(2,2-Diethoxy)ethyl]-S-methylthiuroniumiodid, dann mit Salzsäure erhaltene Produktgemisch wurde an Kieselgel (Baker, 15-25 µm) unter Verwendung von Essigsäureethylester/Cyclohexan/Methanol/konz. wässeriges Ammoniak säulenchromatographisch getrennt und anschließend jeweils aus Diisopropylether umkristallisiert.
Man erhielt:
I. N-[(4-Hydroxyphenyl)methyl]-N⁵-(1H-imidazol-2-yl)-N²-[(1,2,3,4-tetrahydro-3-chinolinyl)carbonyl]-ornithinamid (Diastereomer A),
   Ausbeute: 2 % der Theorie.
   Farblose Kristalle vom R_{f} 0,62
   ESI-MS: (M+H)⁺ = 463
II. N-[(4-Hydroxyphenyl)methyl]-N⁵-(1H-imidazol-2-yl)-N²-[(1,2,3,4-tetrahydro-3-chinolinyl)carbonyl]-ornithinamid (Diastereomer B),
   Ausbeute: 3 % der Theorie.
   Farblose Kristalle vom R_{f} 0,60
   - ESI-MS:: (M+H)⁺ = 463
   (M+Na)⁺ = 485
   (M+K)⁺ = 501

### Beispiel 153

### (R,S)-3-[2-[(Aminoiminomethyl)amino]phenyl]-N²-(diphenylacetyl)-N-[(4-hydroxyphenyl)methyl]-alaninamid-hydrochlorid

### a) α-(Acetylamino)-α-[(2-nitrophenyl)methyl]-malonsäurediethylester

Hergestellt analog Beispiel 31a) aus Acetamidomalonsäurediethylester und 2-Nitrobenzylchlorid in einer Ausbeute von 86 % der Theorie.
Farblose Kristalle vom Fp. 108°C
- IR (KBr):: 1745.5 cm⁻¹ (Ester-C=O)
1643.3 cm⁻¹ (Amid-C=O)
1527.5 cm⁻¹ (Amid-II, NO₂)
1355.9 cm⁻¹ (NO₂)

### b) (R,S)-3-(2-Nitrophenyl)-alanin-hydrochlorid

Hergestellt analog Beispiel 31b) aus α-(Acetylamino)-α-[(2-nitrophenyl)methyl]-malonsäurediethylester und wässeriger Salzsäure in einer Ausbeute von 47 % der Theorie.
Farblose Kristalle
- IR (KBr):: 1730.0 cm⁻¹ (Carbonsäure-C=O)
1523.7 cm⁻¹ (Amid-II, NO₂)
1336.6 cm⁻¹ (NO₂)
- ESI-MS:: (M+H)⁺ = 211
(M-H)⁻ = 209
(M+Na)⁺ = 233
(2M+Na) = 443
(2M-2H+Na) = 441

### c) (R,S)-N²-(Diphenylacetyl)-3-(2-nitrophenyl)-alanin

Hergestellt analog Beispiel 1a) aus Diphenylacetylchlorid und (R,S)-3-(2-Nitrophenyl)-alanin-hydrochlorid in Gegenwart von Natronlauge in einer Ausbeute von 97 % der Theorie.
Farblose Kristalle vom Fp. 150-151°C
- IR (KBr):: 3320 cm⁻¹ (N-H, O-H)
1710.8 cm⁻¹ (Carbonsäure-C=O)
1652.9 cm⁻¹ (Amid-C=O)
1529.5 cm⁻¹ (Amid-II, NO₂)
1344.3 cm⁻¹ (NO₂)

### d) (R,S)-N²-(Diphenylacetyl)-N-[(4-hydroxyphenyl)methyl]-3-(2-nitrophenyl)-alaninamid

Hergestellt analog Beispiel 67a) aus (R,S)-N²-(Diphenylacetyl)-3-(2-nitrophenyl)-alanin, 4-Hydroxybenzenmethanamin und TBTU in einer Ausbeute von 100 % der Theorie.
Farblose Kristalle
- IR (KBr):: 3280 cm⁻¹ (N-H, O-H)
1639.4 cm⁻¹ (Amid-C=O)
- ESI-MS:: (M+H)⁺ = 510
(M+Na)⁺ = 532
(M+K)⁺ = 548

### e) (R,S)-3-(2-Aminophenyl)-N²-(diphenylacetyl)-N-[(4-hydroxyphenyl)methyl]-alaninamid

Hergestellt analog Beispiel 25b), jedoch unter Verwendung von Methanol/Tetrahydrofuran = 1/5 (v/v) als Lösemittel, aus (R,S)-N²-(Diphenylacetyl)-N-[(4-hydroxyphenyl)methyl]-3-(2-nitrophenyl)-alaninamid durch katalytische Hydrierung in Gegenwart von Palladium/Aktivkohle (10%ig) in einer Ausbeute von 83 % der Theorie.
Farblose Kristalle vom Fp. 194-195°C
- IR (KBr):: 3450.4; 3359.8; 3265,3 cm⁻¹ (O-H; N-H)
1649.0 cm⁻¹ (Amid-C=O)
1616.3 cm⁻¹ (Amid-II)
- MS:: M⁺ =479

### f) (R,S)-3-[2-[(Aminoiminomethyl)amino]phenyl]-N²-(diphenylacetyl)-N-[(4-hydroxyphenyl)methyl]-alaninamid-hydrochlorid

Hergestellt analog Beispiel 70d) aus (R,S)-3-(2-Aminophenyl)-N²-(diphenylacetyl)-N-[(4-hydroxyphenyl)methyl]-alaninamid und Cyanamid in einer Ausbeute von 9% der Theorie.
Farblose Kristalle vom Fp. 175-176°C und R_{f} 0,80.
- IR (KBr):: 1678.0 cm⁻¹ (Guanidinium)
1643.3 cm⁻¹ (Amid-C=O)
- ESI-MS:: (M+H)⁺ = 522
(2M+H)⁺ = 1043
(2M+Na)⁺ = 1065

### Beispiel 154

### (R)-N²-[2-(2,4-Dichlorphenoxy)-1-oxoethyl]-N-[(4-hydroxyphenyl)methyl]-argininamid

### a) (R)-N⁵-[Amino(nitroimino)methyl]-N²-[2-(2,4-dichlorphenoxy)-1-oxoethyl]-ornithin

Hergestellt analog Beispiel 1a) aus 2-(2,4-Dichlorphenoxy)acetylchlorid und H-D-Arg(NO₂)-OH in Gegenwart von Natronlauge in einer Ausbeute von 56 % der Theorie.
Farblose Kristalle vom Fp. 235-237°C (Methanol/Wasser = 1/1 (v/v))
- IR (KBr):: 3386.8 cm⁻¹ (N-H, O-H)
1732 cm⁻¹ (Carbonsäure-C=O)
1629.8 cm⁻¹ (Amid-C=O)

### b) (R)-N⁵-[Amino(nitroimino)methyl]-N²-[2-(2,4-dichlorphenoxy)-1-oxoethyl]-N-[(4-hydroxyphenyl)methyl]-ornithinamid

Hergestellt analog Beispiel 67a) aus (R)-N⁵-[Amino(nitroimino)methyl]-N²-[2-(2,4-dichlorphenoxy)-1-oxoethyl]-ornithin, 4-Hydroxybenzenmethanamin und TBTU in einer Ausbeute von 66 % der Theorie.
Farblose Kristalle vom Fp. 205-210°C und R_{f} 0,50 (Kieselgel 60 F₂₅₄ für Dünnschichtchromatographie, Merck-Darmstadt; Fließmittel: Essigsäureethylester/Methanol/Eisessig = 100/10/5 (v/v/v)).
IR (KBr): 1645.2 cm⁻¹ (Amid-C=O)

### c) (R)-N²-[2-(2,4-Dichlorphenoxy)-1-oxoethyl)-N-[(4-hydroxyphenyl)methyl]-argininamid

Hergestellt analog Beispiel 1c) aus (R)-N⁵-[Amino(nitroimino)methyl]-N²-[2-(2,4-dichlorphenoxy)-1-oxoethyl]-N-[(4-hydroxyphenyl)methyl]-ornithinamid durch katalytische Hydrierung in Gegenwart von Palladiummohr und 80%iger wässeriger Essigsäure in einer Ausbeute von 87 % der Theorie.
Farblose, amorphe Substanz vom R_{f} 0,51
- IR (KBr):: 1652.9 cm⁻¹ (Amid-C=O)
- ESI-MS:: (M+H)⁺ = 482/484/486 (Cl₂)

### Beispiel 155

### (R) N²-[(9-Fluorenyl)carbonyl]-N-[[(4-hydroxyphenyl)methyl]-argininamid-acetat

### a) (R)-N⁵-[Amino(nitroimino)methyl]-N²-[(9-fluorenyl)carbonyl]-N-[(4-hydroxyphenyl)methyl]-ornithinamid

Hergestellt analog Beispiel 67a) aus 9-Fluorencarbonsäure, (R)-N⁵-[Amino(nitroimino)methyl]-N-[(4-hydroxyphenyl)methyl]-ornithinamid-hydrochlorid und TBTU in einer Ausbeute von 9 % der Theorie.
Farblose Kristalle vom Fp. 230-234°C und R_{f} 0,36 (Kieselgel 60 F₂₅₄ für DC, Merck-Darmstadt; Fließmittel: Essigsäureethylester/Methanol/Eisessig = 100/2/1 (v/v/v)).
- IR (KBr):: 1637.5 cm⁻¹ (Amid-C=O)
- ESI-MS:: (M+H)⁺ = 517
(M+Na)⁺ = 539
(M+K)⁺ = 555
(M-H)⁻ = 515

### b) (R)-N²-[(9-Fluorenyl)carbonyl]-N-[(4-hydroxyphenyl)methyl]-argininamid-acetat

Hergestellt analog Beispiel 1c) aus (R)-N⁵-[Amino(nitroimino)methyl]-N²-[(9-fluorenyl)carbonyl]-N-[(4-hydroxyphenyl)methyl]-ornithinamid durch katalytische Hydrierung in Gegenwart von Palladiummohr und 80%iger wässeriger Essigsäure in einer Ausbeute von 40 % der Theorie.
Farblose Kristalle vom Fp. 93-96°C und R_{f} 0,52
ESI-MS: (M+H)⁺ = 472

### Beispiel 156

### (R)-N-[(4-Hydroxyphenyl)methyl]-N²-(2,2,2-triphenyl-1-oxoethyl)-argininamid

### a) (R)-N⁵-[Amino(nitroimino)methyl]-N-[(4-hydroxyphenyl)methyl]-N²-(2,2,2-triphenyl-1-oxoethyl)-ornithinamid

Hergestellt analog Beispiel 8a) aus 2,2,2-Triphenylessigsäure, (R)-N⁵-[Amino(nitroimino)methyl]-N-[(4-hydroxyphenyl)methyl]-ornithinamid und TBTU in einer Ausbeute von 26 % der Theorie.
Farblose Kristalle.
IR (KBr): 1645 cm⁻¹ (Amid-C=O)

### b) (R)-N-[(4-Hydroxyphenyl)methyl]-N²-(2,2,2-triphenyl-1-oxoethyl)-argininamid

Hergestellt analog Beispiel 1c) aus (R)-N⁵-[Amino(nitroimino)methyl]-N-[(4-hydroxyphenyl)methyl]-N²-(2,2,2-triphenyl-1-oxoethyl)-ornithinamid durch katalytische Hydrierung in Gegenwart von Palladiummohr und 80%iger wässeriger Essigsäure in einer Ausbeute von 50 % der Theorie.
Farblose Kristalle vom R_{f} 0,54
- IR (KBr):: 1664.5 cm⁻¹ (Amid-C=O, Amidinium)
- ESI-MS:: (M+H)⁺ = 550

### Beispiel 157

### (R,S)-3-(4-Aminobutyl)-N²-(diphenylacetyl)-N-[(4-hydroxyphenyl)methyl]-alaninamid-hydrochlorid

### a) (R,S)-N-(Diphenylmethylen)-2-(4-cyanbutyl)-glycinmethylester

Zu der Lösung von 35 g (0,138 Mol) N-(Diphenylmethylen)-glycinmethylester in 300 ml Acetonitril gab man nacheinander 26,9 g (0,166 Mol) 5-Bromvaleronitril, 4,45 g (0,0138 Mol) Tetrabutylammoniumbromid und 76,2 g (0,551 Mol) Kaliumcarbonat und kochte die Mischung 3 Stunden unter Rückfluß. Nach nochmaliger Zugabe von 5,0 g (0,031 Mol) 5-Bromvaleronitril wurde weitere 5 Stunden unter Rückfluß erhitzt. Man filtrierte vom Unlöslichen ab und dampfte das Filtrat im Vakuum ein. Der in einer Ausbeute von 46,1 g (100 % der Theorie) erhaltene viskose, ölige Rückstand wurde ohne Reinigung in der nächsten Stufe verwendet.

### b) (R,S)-2-(4-Cyanbutyl)-glycinmethylester-hydrochlorid

Die Mischung aus 46,1 g (0,138 Mol) N-(Diphenylmethylen)-2-(4-cyanbutyl)-glycinmethylester und 179,5 ml 1N wässeriger Salzsäure wurde 4 Stunden bei Zimmertemperatur gerührt. Man extrahierte zweimal mit je 100 ml Ether und dampfte die wässerige Phase bei vermindertem Druck ein. Der Rückstand wurde mit Methanol digeriert, dann erneut im Vakuum vom Lösemittel befreit. Dieser Vorgang wurde noch zweimal wiederholt. Dann wurde der erhaltene Feststoff mit Tetrahydrofuran verrieben und abgenutscht. Das Filtrat ergab nach dem Abdestillieren 23,5 g (100 % der Theorie) einer amorphen Substanz, die als Rohprodukt weiterverabeitet wurde.
- IR (KBr):: 2246.9 cm⁻¹ (C≡N)
1749.3 cm⁻¹ (Ester-C=O)
- ESI-MS:: (M+H)⁺ = 171
(2M+H)⁺ = 341

### c) (R,S)-2-(4-Cyanbutyl)-N²-(diphenylacetyl)-glycinmethylester

Hergestellt analog Beispiel 1a), jedoch unter Verwendung von Natriumcarbonat an Stelle von Natriumhydroxid, aus Diphenylacetylchlorid und (R,S)-2-(4-Cyanbutyl)-glycinmethylesterhydrochlorid in einer Ausbeute von 42 % der Theorie.
Farblose Kristalle vom Fp. 95-99°C
- IR (KBr):: 3284.6 cm⁻¹ (N-H)
2246.9 cm⁻¹ (C=N)
1749.3 cm⁻¹ (Ester-C=O)
1643.3 cm⁻¹ (Amid-C=O)

### d) (R,S)-2-(4-Cyanbutyl)-N²-(diphenylacetyl)-glycin

3,0 g (8,23 mMol) (R,S)-2-(4-Cyanbutyl)-N²-(diphenylacetyl)-glycinmethylester wurden in 50 ml Aceton gelöst und nach Zugabe von 7,5 ml (30 mMol) 4 N Natronlauge über Nacht bei Zimmrtemperatur gerührt. Die nach dem Entfernen des Acetons im Vakuum verbliebene wässerige Phase wurde mit 1N Salzsäure angesäuert und mit Essigsäureethylester erschöpfend extrahiert. Die vereinigten Essigesterextrakte wurden über Natriumsulfat getrocknet und im Vakuum eingedampft. Der Rückstand wurde mit Diisopropylether verrieben. Man nutschte ab und erhielt 2,7 g (94 % der Theorie) an farblosen Kristallen vom Fp. 131-133°C.
- IR (KBr):: 3305.8 cm⁻¹ (N-H)
2245.0 cm⁻¹ (C≡N)
1716.5 cm⁻¹ (Carbonsäure-C=O)
1649.0 cm⁻¹ (Amid-C=O)

### e) (R,S)-2-(4-Cyanbutyl)-N²-(diphenylacetyl)-N-[(4-hydroxyphenyl)methyl)]-glycinamid

Hergestellt analog Beispiel 67a), jedoch unter Verwendung von Tetrahydrofuran/Dimethylformamid = 3/1 (v/v/) als Lösemittel an Stelle von reinem Dimethylformamid, aus (R,S)-2-(4-Cyanbutyl)-N²-(diphenylacetyl)-glycin, 4-Hydroxybenzenmethanaminhydrochlorid und TBTU in einer Ausbeute von 91 % der Theorie.
Farblose Kristalle vom Fp. 158-160°C (Diisopropylether)
- IR (KBr):: 3388.7; 3253.7 cm⁻¹ (N-H; O-H)
2254.7 cm⁻¹ (C≡N)
1643.3 cm⁻¹ (Amid-C=O)

### f) (R,S)-3-(4-Aminobutyl)-N²-(diphenylacetyl)-N-[(4-hydroxyphenyl)methyl]-alaninamid-hydrochlorid

Die Lösung von 0,7 g (1,537 mMol) (R,S)-2-(4-Cyanbutyl)-N²-(diphenylacetyl)-N-[(4-hydroxyphenyl)methyl]-glycinamid in 200 ml Methanol wurde nach Zugabe von 1 ml konz. Salzsäure und 1,0 g 10%igem Palladium/Aktivkohle-Katalysator 2,5 Stunden bei Raumtemperatur und einem Wasserstoffdruck von 3 bar hydriert. Man gab nochmals 0,5 g des Katalysators zu und hydrierte weitere 2,5 Stunden unter den gleichen Bedingungen. Die vom Katalysator befreite Lösung wurde eingedampft und der Rückstand mit eiskaltem Essigsäureethylester verrieben.
Man erhält 0,58 g (76 % der Theorie) an farblosen Kristallen vom Fp. 210-211°C und R_{f} 0,52.
- IR (KBr):: 3300 cm⁻¹ (N-H, O-H)
1639.4 cm⁻¹ (Amid-C=O)
- MS:: M⁺ = 459

### Beispiel 158

### (R,S)-6-(Aminoiminomethyl)-N²-(diphenylacetyl)-N-[(4-hydroxyphenyl)methyl]-norleucinamid-hydrochlorid

Hergestellt analog Beispiel 79f) aus (R,S)-2-(4-Cyanbutyl)-N²-(diphenylacetyl)-N-[(4-hydroxyphenyl)methyl]-glycinamid durch Umsetzung zunächst mit trockenem Chlorwasserstoff in wasserfreiem Ethanol, dann mit Ammoniumcarbonat in einer Ausbeute von 60 % der Theorie.
Farblose Kristalle von Fp. 107°C (Z.) und R_{f} 0,54
- IR (KBr):: 1687.6 cm⁻¹ (Amidinium)
1651.0 cm⁻¹ (Amid-C=O)
- ESI-MS:: (M+H)⁺ = 473

### Beispiel 159

### (R,S)-3-[4-(4,5-Dihydro-1H-imidazol-2-yl)phenyl]-N²-(diphenylacetyl)-N-[(4-hydroxyphenyl)methyl]-alaninamid-hydrochlorid

Zu 60 ml wasserfreiem Ethanol, das mit trockenem Chlorwasserstoff gesättigt und mit 30 ml wasserfreiem Petrolether überschichtet war, gab man in kleinen Portionen und unter Einhaltung einer Reaktionstemperatur von 0 bis +5°C insgesamt 5,2 g (10,62 mMol) (R,S)-3-(4-Cyanphenyl)-N²-(diphenylacetyl)-N-[(4-hydroxyphenyl)methyl]-alaninamid und rührte anschließend noch 14 Stunden bei der gleichen Temperatur. Der nach dem Abdampfen des Lösemittels verbleibende Rückstand wurde noch zweimal in je 50 ml wasserfreiem Ethanol aufgenommen und jeweils wieder im Vakuum bei einer Badtemperatur von maximal +40°C zur Trockene gebracht. Der Kolbenrückstand wurde in 50 ml trockenem Ethanol suspendiert und bei Raumtemperatur und unter Rühren mit 3,18 g (52,9 mMol) 1,2-Ethandiamin versetzt. Nach 20 Stunden wurde das Lösemittel im Vakuum entfernt, der Rückstand sorgfältig mit Wasser digeriert und abgenutscht. Nach dem Trocknen im Vakuum erhielt man 4,6 g (76 % der Theorie) an farblosen Kristallen vom Fp. 227-229°C und R_{f} 0,41.
- IR (KBr):: 3533.4; 3274.9 cm⁻¹ (N-H; O-H; N⁺-H)
1622.0 cm⁻¹ (Amid-C=O)

MS: M⁺ = 532

### Beispiel 160

### (R)-N-[[4-[3-(Dimethylamino)propyloxy]phenyl]methyl]-N²-(diphenylacetyl)-argininamid

### a) (R)-N⁵-[Amino(nitroimino)methyl]-N-[[4-[3-(dimethylamino)propyloxy]phenyl]methyl]-N²-(diphenylacetyl)ornithinamid

Hergestellt analog Beispiel 67a) aus (R)-N⁵-[Amino(nitroimino)methyl]-N²-(diphenylacetyl)-ornithin, 4-[3-(Dimethylamino)propyloxy]-benzenmethanamin und TBTU in einer Ausbeute von 62 % der Theorie.
Farblose, amorphe Substanz.
- IR (KBr):: 3363.7; 3298.1 cm⁻¹ (N-H)
2815.9; 2767.7 cm⁻¹ (Phenolether; NCH₃)
1639.4 cm⁻¹ (Amid-I)
- ESI-MS:: (M+H)⁺ = 604
(M+Na)⁺ = 626
(M+K)⁺ = 642
(2M+Na)⁺ = 1229

### b) (R)-N-[[4-[3-(Dimethylamino)propyloxy]phenyl]methyl]-N²-(diphenylacetyl)-argininamid

Hergestellt analog Beispiel 1c) aus (R)-N⁵-[Amino(nitroimino)methyl]-N-[[4-[3-(dimethylamino)propyloxy]phenyl]methyl]-N²-(diphenylacetyl)-ornithinamid durch katalytische Hydrierung in Gegenwart von Palladiummohr und 80%iger wässeriger Essigsäure in einer Ausbeute von 54 % der Theorie.
Farblose, amorphe Substanz vom R_{f} 0,21.
- IR (KBr):: 1649 cm⁻¹ (Amid-C=O)
- ESI-MS:: (M+H)⁺ = 559
(2M+H)⁺ = 1117

### Beispiel 161

### (R,S)-3-[3-[Amino(hydroxyimino)methyl]phenyl]-N²-(diphenylacetyl)-N-[(4-hydroxyphenyl)methyl]-alaninamid

Zu der Lösung von 0,5 g (1,0213 mMol) (R,S)-3-(3-Cyanphenyl)-N²-(diphenylacetyl)-alaninamid in 10 ml heißem Ethanol gab man die Lösung von 0,09 g (1,295 mMol) Hydroxylamin-hydrochlorid und 0,08 g (0,755 mMol) Natriumcarbonat in 1,5 ml Wasser und kochte die Mischung 2 Stunden lang unter Rückfluß. Man filtrierte heiß, befreite das Filtrat vom Lösemittel und nahm den Rückstand in 20 ml Methanol auf. Man setzte dann Wasser bis zur Beendigung der Fällung zu. Die entstandenen Kristalle wurden abgenutscht und im Vakuum über Calciumchlorid getrocknet.
Ausbeute: 0,41 g (77 % der Theorie).
Farblose Kristalle vom Fp. 157-160°C und R_{f} 0,82.
- IR (KBr):: 1641.3 cm⁻¹ (Amid-I)
1514.0 cm⁻¹ (Amid-II)
- ESI-MS:: (M+H)⁺ = 523
(M+Na)⁺ = 545
(M+K)⁺ = 561

### Beispiel 162

### (R,S)-3-[3-(Aminocarbonyl)phenyl]-N²-(diphenylacetyl)-N-[(4-hydroxyphenyl)methyl]-alaninamid

Die Mischung aus 0,5 g (1,0213 mMol) (R,S)-3-(3-Cyanophenyl)-N²-(diphenylacetyl)-N-[(4-hydroxyphenyl)methyl]-alaninamid, 30 ml mit Chlorwasserstoff gesättigtem Methanol und 2 ml Wasser wurde 12 Stunden bei Raumtemperatur gerührt. Man dampfte im Vakuum ein und reinigte den Rückstand chromatographisch an Kieselgel (Baker, 30-60 µm) unter Verwendung von Dichlormethan/Cyclohexan/Methanol/konz. wässrigem Ammoniak = 350/75/75/10 (v/v/v/v) zum Eluieren. Man erhielt 56 mg (11 % der Theorie) an farblosen Kristallen vom R_{f} 0,87.
MS: m/e = 461

### Beispiel 163

### (R)-N-[(4-Hydroxyphenyl)methyl]-N²-[3-(1-piperidinyl)-1-oxopropyl]-argininamid-dihydrochlorid

### a) (R)-N⁵-[Amino(nitroimino)methyl]-N-[(4-hydroxyphenyl)methyl]-N²-[3-(1-piperidinyl)-1-oxopropyl]-ornithinamid-hydrochlorid

Hergestellt analog Beispiel 67a) aus 3-(1-Piperidinyl)propansäure, (R)-N⁵-[Amino(nitroimino)methyl]-N-[(4-hydroxyphenyl)methyl]-ornithinamid-hydrochlorid und TBTU in einer Ausbeute von 14 % der Theorie. Farblose, amorphe Substanz vom R_{f} 0,70 (Kieselgelplatten für Dünnschichtchromatographie 60 F₂₅₄, Merck-Darmstadt; Fließmittel: Dichlormethan/Methanol/konz. wässeriges Ammoniak = 30/10/1 (v/v/v)).
- IR (KBr):: 1649 cm⁻¹ (Amid-C=O)
- ESI-MS:: (M+H)⁺ = 464
(M+Na)⁺ = 486
(2M+H)⁺ = 949
(2M+Na)⁺ = 971

### b) (R)-N-[(4-Hydroxyphenyl)methyl]-N²-[3-(1-piperidinyl)-1-oxopropyl]-argininamid-dihydrochlorid

Hergestellt analog Beispiel 1c) aus (R)-N⁵-[Amino(nitroimino)methyl-N-[(4-hydroxyphenyl)methyl]-N²-[3-(1-piperidinyl)-1-oxopropyl]-ornithinamid-hydrochlorid durch katalytische Hydrierung in Gegenwart von Palladiummohr und 80%iger wässeriger Essigsäure in einer Ausbeute von 78 % der Theorie.
Farblose, amorphe Substanz vom R_{f} 0,08.
- IR (KBr):: 1658.7 cm⁻¹ (Amid-C=O)
- ESI-MS:: (M+H)⁺ = 419
(M+2H)⁺ = 210
(M+HCl+H)⁺ = 455/457 (Cl)

### Beispiel 164

### (R,S)-3-[3-(Aminoiminomethyl)phenyl]-N²-(diphenylacetyl)-N-[[4-(ethoxycarbonyloxy)phenyl]methyl]-alaninamid

0,38 g (0,7 mMol) (R,S)-3-[3-(Aminoiminomethyl)phenyl]-N²-(diphenylacetyl)-N-[(4-hydroxyphenyl)methyl]-alaninamid-hydrochlorid wurden in 500 ml Dichlormethan suspendiert und unter Rühren mit 0,2 ml (2,1 mMol) Triethylamin, anschließend tropfenweise mit der Lösung von 0,078 ml (0,082 mMol) Chlorkohlensäureethylester in 10 ml Dichlormethan versetzt. Nach 2 Stunden wurden nochmals die gleichen Mengen an Chlorkohlensäureethylester und Triethylamin zugegeben und weitere 2 Stunden bei Raumtemperatur gerührt. Die Lösung wurde nacheinander mit Wasser, gesättigter wässeriger Natriumhydrogencarbonat-Lösung, 5%iger wässeriger Zitronensäure-Lösung und gesättigter wässeriger Kochsalz-Lösung gewaschen, über Magnesiumsulfat getrocknet und im Vakuum vom Lösemittel befreit. Der Rückstand wurde mit einer Mischung aus Diisopropylether, Petrolether und Isopropanol = 50/50/1 (v/v/v) verrieben und ergab nach dem Abnutschen und Trocknen im Vakuum 390 mg (96 % der Theorie) an farblosen Kristallen vom Fp. 117-120°C und R_{f} 0,47.
- IR (KBr):: 1760.9 cm⁻¹ (Urethan-C=O)
1652.9 cm⁻¹ (Amid-C=O)
- ESI-MS:: (M+H)⁺ = 651
(M+Na)⁺ = 673

### Beispiel 165

### (R,S)-N-[2-(1,2-Dihydro-3,5(4H)-dioxo-1,2-diphenyl-3H-1,2,4-triazol-4-yl)ethyl]-N²-(diphenylacetyl)-argininamid-hydrochlorid

### a) (R,S)-N⁵-[Amino(nitroimino)methyl]-N-[2-(1,2-dihydro-3,5(4H)-dioxo-1,2-diphenyl-3H-1,2,4-triazol-4-yl)ethyl]-N²-(diphenylacetyl)-ornithinamid

Hergestellt analog Beispiel 157e) aus (R,S)-N⁵-[Amino(nitroimino)methyl]-N²-(diphenylacetyl)-ornithin, 2-(1,2-Dihydro-3,5(4H)-dioxo-1,2-diphenyl-3H-1,2,4-triazol-4-yl)ethanamin und TBTU in einer Ausbeute von 23 % der Theorie.
Farblose Kristalle vom Fp. 188-189°C.
- IR (KBr):: 3342.4 cm⁻¹ (N-H)
1780.2; 1724.3 cm⁻¹ (Triazolidindion-C=O)
1652.9 cm⁻¹ (Amid-C=O)

### b) (R,S)-N-[2-(1,2-Dihydro-3,5(4H)-dioxo-1,2-diphenyl-3H-1,2,4-triazol-4-yl)ethyl]-N²-(diphenylacetyl)-ornithinamid-hydrochlorid

Hergestellt analog Beispiel 1c) aus (R,S)-N⁵-[Amino(nitroimino)methyl]-N-[2-(1,2-dihydro-3,5(4H)-dioxo-1,2-diphenyl-3H-1,2,4-triazol-4-yl)ethyl]-N²-(diphenylacetyl)-ornithinamid durch katalytische Hydrierung im Gegensatz von Palladiummohr und 80%iger wässeriger Essigsäure in einer Ausbeute von 52 % der Theorie.
Farblose Kristalle vom Fp. 153°C und R_{f} 0.53.
- IR (KBr):: 1768.6 cm⁻¹ (Fünfring-C=O)
1676.0 cm⁻¹ (Amid-C=O, Guanidinium)
- ESI-MS:: (M+H)⁺ = 647

### Beispiel 166

### (R,S)-N²-(Diphenylacetyl)-N-[(4-hydroxyphenyl)methyl]-2-methyl-argininamid

### a) (R,S)-2-(2-Cyanethyl)-N²-(phenylmethylen)-alaninmethylester (cis-/trans-Gemisch)

Hergestellt analog Beispiel 157a) aus N²-(Phenylmethylen)alaninmethylester und Acrylnitril in einer Ausbeute von 78 % der Theorie. Farbloses Öl, das ohne Reinigung weiterverarbeitet wurde.

### b) (R,S)-2-(2-Cyanethyl)-alaninmethylester-hydrochlorid

Hergestellt analog Beispiel 157b) aus (R,S)-2-(2-Cyanethyl)-N²-(phenylmethylen)-alaninmethylester (cis-/trans-Gemisch) und 1N Salzsäure in einer Ausbeute von 92 % der Theorie.
Farblose, amorphe Substanz.
- IR (KBr):: 2252.7 cm⁻¹ (C≡N)
1747.4 cm⁻¹ (Ester-C=O)
- ESI-MS:: (M+H)⁺ = 157
(M+Na)⁺ = 179
(2M+H)⁺ = 313
(2M+Na)⁺ = 335

### c) (R,S)-2-(2-Cyanethyl)-N²-(diphenylacetyl)-alaninmethylester

Hergestellt analog Beispiel 89c) aus Diphenylacetylchlorid und (R,S)-2-(2-Cyanethyl)-alaninmethylester-hydrochlorid in Gegenwart von 4-Methylmorpholin in einer Ausbeute von 43 % der Theorie.
Farblose Kristalle vom Fp. 172-174°C.
- IR (KBr):: 3269.2 cm⁻¹ (N-H)
2248.9 cm⁻¹ (C≡N)
1743.5 cm⁻¹ (Ester-C=O)
1643.3 cm⁻¹ (Amid-C=O)

### d) (R,S)-2-(2-Cyanethyl)-N²-(diphenylacetyl)-alanin

Hergestellt analog Beispiel 79d) aus (R,S)-2-(2-Cyanethyl)-N²-(diphenylacetyl)-alaninmethylester durch Verseifung mit Lithiumhydroxid-hydrat und Wasser in einer Ausbeute von 86 % der Theorie.
Farblose Kristalle vom Fp. 168-170°C.
- IR (KBr):: 3269.2 cm⁻¹ (N-H)
2252.7 cm⁻¹ (C≡N)
1747.4 cm⁻¹ (Carbonsäure-C=O)
1643.3 cm⁻¹ (Amid-C=O)

### e) (R,S)-2-(2-Cyanethyl)-N²-(diphenylacetyl)-N-[(4-hydroxyphenyl)methyl]-alaninamid

Hergestellt analog Beispiel 67a) aus (R,S)-2-(2-Cyanethyl)-N²-(diphenylacetyl)-alanin, 4-Hydroxybenzenmethanamin-hydrochlorid und TBTU in einer Ausbeute von 85 % der Theorie.
Farblose, amorphe Substanz.
- IR (KBr):: 3334.7 cm⁻¹ (N-H, O-H)
2248.9 cm⁻¹ (C≡N)
1654.8 cm⁻¹ (Amid-I)
1516.0 cm⁻¹ (Amid-II)

### f) (R,S)-N²-(Diphenylacetyl)-N-[(4-hydroxyphenyl)methyl]-2-methyl-ornithinamid-hydrochlorid

Hergestellt analog Beispiel 157f) aus (R,S)-2-(2-Cyanethyl)-N²-(diphenylacetyl)-N-[(4-hydroxyphenyl)methyl]-alaninamid durch katalytische Hydrierung in Gegenwart von Salzsäure und Palladium/Aktivkohle (10%ig) in einer Ausbeute von 21 % der Theorie.
Farblose Kristalle.
- IR (KBr):: 1652.9 cm⁻¹ (Amid-C=O)
- MS:: M⁺ = 445

### g) (R,S)-N²-(Diphenylacetyl)-N-[(4-hydroxyphenyl)methyl]-2-methyl-argininamid

Hergestellt analog Beispiel 42g) aus (R,S)-N²-(Diphenylacetyl)-N-[(4-hydroxyphenyl)methyl]-2-methyl-ornithinamid-hydrochlorid und 3,5-Dimethylpyrazol-1-carbonsäureamidiniumnitrat in einer Ausbeute von 15 % der Theorie.
Farblose, amorphe Substanz vom R_{f} 0,50.
- IR (KBr):: 1652.9 cm⁻¹ (Amid-C=O)
- ESI-MS:: (M+H)⁺ = 488

### Beispiel 167

### (R)-N²-(Diphenylacetyl)-N-methyl-N-(2-phenylethyl)-argininamid

### a) (R)-N⁵-[Amino(nitroimino)methyl]-N²-(diphenylacetyl)N-methyl-N-(2-phenylethyl)-ornithinamid

Hergestellt analog Beispiel 148b) aus (R)-N⁵-[Amino(nitroimino)methyl]-N²-(diphenylacetyl)-ornithin, N-Methyl-2-phenylethanamin und TBTU in einer Ausbeute von 76 % der Theorie.
Farblose Kristalle vom Fp. 110°C.
- IR (KBr):: 1625.9 cm⁻¹ (Amid-C=O)
- ESI-MS:: (M+H)⁺ = 531
(M+Na)⁺ = 553

### b) (R)-N²-(Diphenylacetyl)-N-methyl-N-(2-phenylethyl)-argininamid-acetat

Hergestellt analog Beispiel 1c) aus (R)-N⁵-[Amino(nitroimino)methyl]-N²-(diphenylacetyl)-N-methyl-N-(2-phenylethyl)-ornithinamid durch katalytische Hydrierung in Gegenwart von Palladiummohr und 80%iger wässeriger Essigsäure in einer Ausbeute von 90 % der Theorie.
Farblose Kristalle vom R_{f} 0,52.
- IR (KBr):: 1627.8 cm⁻¹ (Amid-C=O)
- ESI-MS:: (M+H)⁺ = 486
(M+Na)⁺ = 508

### Beispiel 168

### (R)-N-[[4-[(4,5-Dihydro-5,5-dimethyl-2,4(3H)-dioxo-1H-imidazol-3-yl)methyl]phenyl]methyl]-N²-(diphenylacetyl)-argininamid-diacetat

### a) 3-[(4-Cyanphenyl)methyl]-4,5-dihydro-5,5-dimethyl-1H-imidazol-2,4(3H)-dion

Hergestellt analog Beispiel 92a) aus 5,5-Dimethylhydantoin und 4-(Brommethyl)-benzonitril in Gegenwart von Kalium-tert.-butylat in einer Ausbeute von 98 % der Theorie.
Farblose Kristalle vom Fp. 173-175°C.

### b) 4-[(4,5-Dihydro-5,5-dimethyl-2,4(3H)-dioxo-1H-imidazol-3-yl)methyl]-benzenmethanamin-hydrochlorid

Hergestellt analog Beispiel 89f) aus 3-[(4-Cyanphenyl)methyl]-4,5-dihydro-5,5-dimethyl-1H-imidazol-2,4(3H)-dion durch katalytische Hydrierung in Gegenwart von Raney-Nickel und Ammoniak sowie abschließende Behandlung mit etherischer Chlorwasserstoff-Lösung in einer Ausbeute von 73 % der Theorie.
Farblose Kristalle vom Fp. >250°C.

### c) (R)-N⁵-[Amino(nitroimino)methyl]-N-[[4-[(4,5-dihydro-5,5-dimethyl-2,4(3H)-dioxo-1H-imidazol-3-yl)methyl]phenyl]methyl]-N²-(diphenylacetyl)-ornithinamid

Hergestellt analog Beispiel 81a) aus (R)-N⁵-[Amino(nitroimino)methyl]-N²-(diphenylacetyl)-ornithin, 4-[(4,5-Dihydro-5,5-dimethyl-2,4(3H)-dioxo-1H-imidazol-3-yl)methyl]benzenmethanamin-hydrochlorid und TBTU in einer Ausbeute von 60 % der Theorie.
Farblose Kristalle vom Fp. 224-226°C.
- IR (KBr):: 1755.1; 1706.9 cm⁻¹ (Hydantoin-C=O)
1641.3 cm⁻¹ (Amid-C=O)

### d) (R)-N-[[4-[(4,5-Dihydro-5,5-dimethyl-2,4(3H)-dioxo-1H-imidazol-3-yl)methyl]phenyl]methyl]-N²-(diphenylacetyl)argininamid-diacetat

Hergestellt analog Beispiel 1c) aus (R)-N⁵-[Amino(nitroimino)methyl]-N-[[4-[(4,5-dihydro-5,5-dimethyl-2,4(3H)-dioxo-1H-imidazol-3-yl)methyl]phenyl]methyl]-N²-(diphenylacetyl)ornithinamid durch katalytische Hydrierung in Gegenwart von Palladiummohr und 80%iger wässeriger Essigsäure in einer Ausbeute von 99 % der Theorie.
Farblose, amorphe Substanz vom R_{f} 0,54.
- IR (KBr):: 1768.6; 1710.8 cm⁻¹ (Hydantoin-C=O)
1656.8 cm⁻¹ (Amid-C=O)
- ESI-MS:: (M+H)⁺ = 598
(M+Na)⁺ = 620

### Beispiel 169

### (R)-N-[(4-Hydroxyphenyl)methyl]-N²-(5-phenyl-1-oxopentyl)argininamid-diacetat

### a) (R)-N⁵-[Amino(nitroimino)methyl]-N-[(4-hydroxyphenyl)methyl]-N²-(5-phenyl-1-oxopentyl)-ornithinamid

Hergestellt analog Beispiel 67a) aus 5-Phenylpentansäure, (R)-N⁵-[Amino(nitroimino)methyl]-N-[(4-hydroxyphenyl)methyl]-ornithinamid-hydrochlorid und TBTU in einer Ausbeute von 52 % der Theorie.
Farblose, amorphe Substanz.
- IR (KBr):: 3305.8 cm⁻¹ (N-H, O-H)
1637.5 cm⁻¹ (Amid-C=O)

### b) (R)-N-[(4-Hydroxyphenyl)methyl]-N²-(5-phenyl-1-oxopentyl)-argininamid-diacetat

Hergestellt analog Beispiel 1c) aus (R)-N⁵-[Amino(nitroimino)methyl]-N-[(4-hydroxyphenyl)methyl]-N²-(5-phenyl-1-oxopentyl)-ornithinamid durch katalytische Hydrierung in Gegenwart von Palladiummohr und 80%iger wässeriger Essigsäure in einer Ausbeute von 66 % der Theorie.
Farblose, amorphe Substanz von R_{f} 0,58.
- IR (KBr):: 1652.9 cm⁻¹ (Amid-C=O)
- ESI-MS:: (M+H)⁺ = 440
(2M+H)⁺ = 879

### Beisiel 170

### (R)-N²-(Diphenylacetyl)-N-[[4-(3-hydroxypropy)phenyl]methyl]-argininamid-acetat

### a) (R)-N⁵-[Amino(nitroimino)methyl]-N²-(diphenylacetyl)-N-[[4-(3-hydroxypropyl)phenyl]methyl]-ornithinamid

Hergestellt analog Beispiel 157e) aus (R)-N⁵-[Amino(nitroimino)methyl]-N²-(diphenylacetyl)-ornithin, 4-(3-Hydroxypropyl)-benzenmethanamin (Fp. 79-83°C; hergestellt aus 4-[[[(tert.-Butyloxy)carbonyl]amino]methyl]-benzenpropansäure durch aufeinanderfolgende Umsetzung mit Chlorkohlensäureethylester, Natriumborhydrid und halbkonzentrierter Salzsäure) und TBTU in einer Ausbeute von 41 % der Theorie.
Farblose Kristalle vom Fp. 131-133°C.
- IR (KBr):: 1641.3 cm⁻¹ (Amid-C=O)
- ESI-MS:: (M+H)⁺ = 561
(M+Na)⁺ = 583
(M+K)⁺ = 599

### b) (R)-N²-(Diphenylacetyl)-N-[[4-(3-hydroxypropyl)phenyl]methyl]-argininamid-acetat

Hergestellt analog Beispiel 1c) aus (R)-N⁵-[Amino(nitroimino)methyl]-N²-(diphenylacetyl)-N-[[4-(3-hydroxypropyl)phenyl]methyl]-ornithinamid durch katalytische Hydrierung in Gegenwart von Palladiummohr und 80%iger wässeriger Essigsäure in einer Ausbeute von 100 % der Theorie.
Farblose, amorphe Substanz vom R_{f} 0,57.
- IR (KBr):: 1652.9 cm⁻¹ (Amid-C=O)
- ESI-MS:: (M+H)⁺ = 516
(M+Na)⁺ = 538

### Beispiel 171

### (R)-N-[(4-Hydroxyphenyl)methyl]-N²-(4-phenyl-1-oxobutyl)argininamid-diacetat

### a) (R)-N⁵-[Amino(nitroimino)methyl]-N-[(4-hydroxyphenyl)methyl]-N²-(4-phenyl-1-oxobutyl)-ornithinamid

Hergestellt analog Beispiel 67a) aus 4-Phenylbutansäure, (R)-N⁵-[Amino(nitroimino)methyl]-N-[(4-hydroxyphenyl)methyl]-ornithinamid-hydrochlorid und TBTU in einer Ausbeute von 36 % der Theorie.
Farblose, amorphe Substanz.
- IR (KBr):: 1639.4 cm⁻¹ (Amid-C=O)
- ESI-MS:: (M+H)⁺ = 471
(M+Na)⁺ = 493
(M+K)⁺ = 509

### b) (R)-N-[(4-Hydroxyphenyl)methyl]-N²-(4-phenyl-1-oxobutyl)-argininamid-diacetat

Hergestellt analog Beispiel 1c) aus (R)-N⁵-[Amino(nitroimino)methyl]-N-[(4-hydroxyphenyl)methyl]-N²-(4-phenyl-1-oxobutyl)-ornithinamid durch katalytische Hydrierung in Gegenwart von Palladiummohr und 80%iger wässeriger Essigsäure in einer Ausbeute von 89 % der Theorie.
Farblose, amorphe Substanz vom R_{f} 0,57.
- IR (KBr):: 1652.9 cm⁻¹ (Amid-C=O)
- ESI-MS:: (M+H)⁺ = 426
(M+Na)⁺ = 448
(2M+H)⁺ = 851

### Beispiel 172

### (R)-N-[(4-Hydroxyphenyl)methyl]-N²-(6-phenyl-1-oxohexyl)argininamid-diacetat

### a) (R)-N⁵-[Amino(nitroimino)methyl]-N-[(4-hydroxyphenyl)methyl]-N²-(6-phenyl-1-oxohexyl)-ornithinamid

Hergestellt analog Beispiel 67a) aus 6-Phenylhexansäure, (R)-N⁵-[Amino(nitroimino)methyl]-N-[(4-hydroxyphenyl)methyl-ornithinamid-hydrochlorid und TBTU in einer Ausbeute von 36 % der Theorie.
Farblose, amorphe Substanz.
IR (KBr): 1639.4 cm⁻¹ (Amid-C=O)

### b) (R)-N-[(4-Hydroxyphenyl)methyl]-N²-(6-phenyl-1-oxohexyl)-argininamid-diacetat

Hergestellt analog Beispiel 1c) aus (R)-N⁵-[Amino(nitroimino)methyl]-N-[(4-hydroxyphenyl)methyl]-N²-(6-phenyl-1-oxohexyl)-ornithinamid durch katalytische Hydrierung in Gegenwart von Palladiummohr und 80%iger wässeriger Essigsäure in einer Ausbeute von 82 % der Theorie.
Farblose, amorphe Substanz vom R_{f} 0,59.
- IR (KBr):: 1652.9 cm⁻¹ (Amid-C=O)
- ESI-MS:: (M+H)⁺ = 454
(M+Na)⁺ = 476

### Beispiel 173

### (R,S)-3-[(3-(Aminoiminomethyl)phenyl]-N²-(2,2-diphenylethyl)-N-[(4-hydroxyphenyl)methyl]-alaninamid-hydrochlorid

### a) (R,S)-N²-[(tert.-Butyloxy)carbonyl]-3-(3-cyanphenyl)alanin

Hergestellt analog Beispiel 52b), jedoch unter Verwendung von tert.-Butanol an Stelle von Tetrahydrofuran als Cosolvens, aus 3-(3-Cyanphenyl)-alanin und Di-tert.-butyldicarbonat in einer Ausbeute von 78 % der Theorie.
- IR (KBr):: 2231.5 cm⁻¹ (C≡N)
1716.5 cm⁻¹ (Urethan-C=O)

### b) (R,S)-N²-[(tert.-Butyloxy)carbonyl]-3-(3-cyanphenyl)-N-[(4-hydroxyphenyl)methyl]-alaninamid

Hergestellt analog Beispiel 67a) aus (R,S)-N²-[(tert.-Butyloxy)carbonyl]-3-(3-cyanphenyl)-alanin und 4-Hydroxybenzenmethanamin und TBTU in einer Ausbeute von 77 % der Theorie.
Farblose Kristalle vom Fp. 141-144°C (Z.)
- IR (KBr):: 2233.4 cm⁻¹ (C≡N)
1685.7 cm⁻¹ (Urethan-C=O)
1631.7 cm⁻¹ (Amid-C=O)

### c) (R,S)-3-(3-Cyanphenyl)-N-[(4-hydroxyphenyl)methyl]-alaninamid

Hergestellt analog Beispiel 5e) aus (R,S)-N²-[(tert.-Butyloxy)carbonyl]-3-(3-cyanphenyl)-N-[(4-hydroxyphenyl)methyl]alaninamid durch Einwirkung von Trifluoressigsäure in einer Ausbeute von 95 % der Theorie.
- IR (KBr):: 2229 cm⁻¹ (C≡N)
1649.0 cm⁻¹ (Amid-I)

### d) (R,S)-3-(3-Cyanphenyl)-N²-(2,2-diphenylethyl)-N-[(4-hydroxyphenyl)methyl]-alaninamid

Hergestellt analog Beispiel 143a) aus Diphenylacetaldehyd, (R,S)-3-(3-Cyanphenyl)-N-[(4-hydroxyphenyl)methyl]-alaninamid und Natriumcyanoborhydrid in einer Ausbeute von 60 % der Theorie.
Farblose, amorphe Substanz.
- IR (KBr):: 2229.6 cm⁻¹ (C≡N)
1647.1 cm⁻¹ (Amid-CO)

### e) (R,S)-3-[3-(Aminoiminomethyl)phenyl]-N²-(2,2-diphenylethyl)-N-[(4-hydroxyphenyl)methyl]-alaninamid-hydrochlorid

Hergestellt analog Beispiel 105d) aus (R,S)-3-(Cyanphenyl)-N²-(2,2-diphenylethyl)-N-[(4-hydroxyphenyl)methyl]-alaninamid durch Umsetzung zunächst mit Chlorwasserstoff in Methanol, dann mit Ammoniumcarbonat in quantitativer Ausbeute.
Farblose Kristalle vom R_{f} 0,58.
- IR (KBr):: 1674.1 cm⁻¹ (Amidinium-C=N)
1652.9 cm⁻¹ (Amid-CO)
- ESI-MS:: (M+H)⁺ = 493
(M+Na)⁺ = 515

## Patentansprüche

1. Aminosäurederivate der allgemeinen Formel
T - Z - NR¹ - (R²CR³) - CO - Y - (CH₂)ₙ - R , (I)
in der
die R-(CH₂)ₙ-Gruppe eine Alkylgruppe mit 1 bis 3 Kohlenstoffatomen, die in ω-Stellung durch 2 Phenylgruppen substituiert sein kann,
eine geradkettige Alkylengruppe mit 2 bis 5 Kohlenstoffatomen, die endständig durch eine Hydroxygruppe substituiert ist,
eine Phenyl- oder Phenylalkylyruppe mit 1 bis 4 Kohlenstoffatomen im Alkylteil, wobei die Phenylgruppe jeweils durch ein Fluor-, Chlor- oder Bromatom, durch eine Alkylaminocarbonylgruppe mit 1 bis 4 Kohlenstoffatomen im Alkylteil, durch eine Ethylaminocarbonylaminogruppe, in der der Ethylteil durch ein oder zwei Phenylreste substituiert ist, durch eine Methyl-, Hydroxymethyl -, Phenyl -, Hydroxyphenyl -, Hydroxy-, Methoxy-, Ethoxy-, Dimethylaminosulfonyloxy-, Cyano-, Carboxy-, Methoxycarbonyl-, Acetyl-, Aminocarbonyl-, Dimethylaminocarbonyl-, Amino-, Methylamino-, Dimethylamino-, Acetylamino-, Methoxycarbonylamino-, Ethoxycarbonylamino-, Aminocarbonylamino-, Methylaminocarbonylamino-, Dimethylaminocarbonylamino-, Methylsulfonylamino-, Aminosulfonyl-, [Amino(imino)- methyl]-, [Amino(imino)methyl]amino-, [1,2-Dihydro-3,5(4H)-dioxo-1,2-diphenyl-3H-1,2,4-triazol-4-yl]methyl-, [1,5-Dihydro-2,4(3H)-dioxo-5,5-diphenyl-imidazol-3-yl]methyl-, Aminomethyl-, Aminosulfonylamino-, Hydroxyethyl-, Hydroxypropyl-, Hydroxybutyl-, 3-(Dimethylamino)propoxy- oder Ethoxycarbonyloxy-Gruppe substituiert sein kann,
eine Methylgruppe, die durch eine Hydroxycyclohexyl-, 4-Hydroxy-3-methyl-phenyl-, 3,4-Dimethoxyphenyl-, 3-Hydroxy-4-methoxyphenyl-, 3-Methoxy-4-hydroxyphenyl-, 4-Amino-3,5-dibromphenyl-, 4-Amino-3,5-dichlorphenyl-, 4-Hydroxy-3,5-dibromphenyl-, 4-Hydroxy-3,5-dichlorphenyl-, 4-Amino-3-fluorphenyl-, 4-Hydroxy-3,5-dimethylphenyl-, Thienyl-, Pyridinyl-, Indolyl-, Benzimidazolyl-, Chinolinyl- oder 2-(Diphenylaminocarbonyl)-2,3-dihydro-1H-isoindolyl-Gruppe substituiert ist,
eine Ethylgruppe, die durch eine Indolyl-, 5-Methoxyindolyl-, 1,2-Diphenyl-3,5(4H)-dioxo-1,2,4-triazol-4-yl- oder Imidazolylgruppe substituiert ist,
eine 2-(2-Hydroxyphenyl)ethylgruppe, die in α-Stellung durch eine Aminocarbonylgruppe substituiert ist,
eine 3-[1-[2-[5,11-Dihydro-6(6H)-oxopyrido[2,3-b][1,4]benzodiazepin-5-yl]-2-oxoethyl]-4-piperidinyl]propyl- oder 3-Hydroxy-1-propin-1-yl-Gruppe,
die T-Z-Gruppe ein Wasserstoffatom,
eine Carbonylgruppe, die durch eine Alkylgruppe mit 1 bis 4 Kohlenstoffatomen, welche in α- oder β-Stellung durch eine Alkylgruppe mit 1 bis 3 Kohlenstoffatomen, durch ein oder zwei gegebenenfalls durch Methoxygruppen, Hydroxygruppen, Chlor- oder Bromatome mono- oder disubstituierte Phenylreste substituiert sein kann, substituiert ist,
eine Carbonylgruppe, die durch eine durch eine Benzyloxy- oder Phenylethoxygruppe substituierte Indolylgruppe, durch eine Methyl-, Tricyclo[3.3.1.1^{3.7}]dec-1-yl-, Benzyl-, (Hydroxyphenyl)methyl-, (Methylphenyl)methyl, (Biphenylyl)methyl-, (Dichlorphenyl)methyl-, Phenyl-, 3,5-Dichlorphenyl-, 3,4-Dichlorphenyl-, Naphthyl-, Naphthylmethyl-, α-Cyclopentyl-benzyl-, Diphenylamino-, Naphthylamino-, Hexamethylenimino-, 5-Phenylethoxy-indolyl-, 1,2,3,4-Tetrahydro-2-chinolinyl-, 2-Chinolinyl-, 1,2,3,4-Tetrahydro-3-chinolinyl-, (Dichlorphenoxy)methyl-, 9-Fluorenyl-, Triphenylmethyl-, 1-Piperidinyl-, (Diphenylmethyl)amino- oder 5,10-Dihydro-11(11H)-oxo-dibenzo[b,e][1,4]diazepin-5-yl-Gruppe substituiert ist, oder
eine 4-Amino-3,5-dichlor-phenylsulfonyl- oder Naphthylsulfonylgruppe,
R¹ das Wasserstoffatom,
R² einen unverzweigten Alkylrest mit 1 bis 5 Kohlenstoffatomen, der in ω-Stellung durch eine Guanidinogruppe, in der die Wasserstoffatome an den Stickstoffatomen jeweils durch Alkylgruppen mit 1 bis 3 Kohlenstoffatomen ersetzt sein können, durch eine Amino-, tert.Butoxycarbonylamino-, Dimethylamino-, N-Methyl-benzylamino-, Methylamino-, Aminocarbonyl-, Aminocarbonylamino-, Aminomethylidenimino-, Methylaminomethylidenimino-, [Amino(nitroimino)methyl]amino-, 1H-Imidazol-2-yl-amino-, 4,5-Dihydro-1H-imidazol-2-yl-amino-, (5-Amino-4H-1,2,4-triazol-3-yl)amino-, (3-Amino-1,2,4-oxadiazol-5-yl)amino- oder (5-Amino-1,2,4-oxadiazol-3-yl)amino- Gruppe substituiert ist,
eine Methylgruppe, die durch eine Phenyl-, Cyanophenyl-, Aminomethylphenyl-, Amidinophenyl-, Methylaminomethylideniminophenyl-, Cyaniminomethylaminophenyl-, Methyliminomethylaminophenyl-, (4,5-Dihydro-1H-imidazol-2-yl)phenyl- oder Imidazolylgruppe substituiert ist,
R³ das Wasserstoffatom oder die Methylgruppe und
Y eine gegebenenfalls durch eine Methyl- oder Ethylgruppe substituierte Iminogruppe mit den Maßgaben bedeuten, daß
(i)
R¹ kein Wasserstoffatom darstellt, falls
die Gruppe R-(CH₂)ₙ- eine Alkylgruppe mit 1 bis 3 Kohlenstoffatomen oder eine Phenylgruppe,
R² eine Amidinophenylmethylgruppe,
R³ ein Wasserstoffatom,
T-Z eine 4-Amino-3,5-dichlorphenylsulfonylgruppe oder die Naphthylsulfonylgruppe und
Y eine Iminogruppe bedeuten, sowie
(ii)
R² keinen unverzweigten Alkylrest mit 3 oder 4 Kohlenstoffatomen, der in ω-Stellung durch eine Guanidinogruppe, in der die Wasserstoffatome an den Stickstoffatomen jeweils durch eine Alkylgruppe mit 1 bis 3 Kohlenstoffatomen ersetzt sein können, durch eine Amino-, tert. Butoxycarbonylamino-, Dimethylamino-, N-Methyl-benzylamino-, Methylamino-, Aminocarbonyl- oder Aminocarbonyl-aminogruppe substituiert ist, darstellt,
falls
R¹, R³ und T-Z jeweils ein Wasserstoffatom und
Y eine gegebenenfalls durch eine Methyl- oder Ethylgruppe substituierte Iminogruppe bedeuten, sowie
(iii)
R² keine Phenylmethylgruppe darstellt, falls
R¹, R³ und T-Z jeweils ein Wasserstoffatom,
Y die Iminogruppe und
die Gruppe R-(CH₂)ₙ- eine Alkylgruppe mit 1 bis 3 Kohlenstoffatomen oder eine Phenylalkylgruppe mit 1 bis 4 Kohlenstoffatomen im Alkylteil bedeuten,
und folgende Verbindungen:
(1) (R)-N-[[4-[(4,5-Dihydro-5,5-dimethyl-2,4(3H)-dioxa-1H-imidazol-3-yl)methyl]phenyl]methyl]-N²-(diphenylacetyl)-argininamid,
(2) (R,S)-N²-(Diphenylacetyl)-N-[(4-hydroxyphenyl)methyl]-3-[(4-piperidinyl)methyl]-alaninamid,
(3) (R,S)-3-[3-[Amino(hydroxyimino)methyl]phenyl]-N²-(diphenylacetyl)-N-[(4-hydroxyphenyl)methyl]-alaninamid,
(4) (R,S)-3-[3-(Aminocarbonyl)phenyl]-N²-(diphenylacetyl)-N-[(4-hydroxyphenyl)methyl]-alaninamid,
(5) (R)-N²-(2,2-Diphenylechyl)-N-[(4-hydroxyphenyl)methyl]-argininamid,
(6) (R,S)-3-[(3-(Aminoiminomethyl)phenyl)-N²-(2,2-diphenylethyl)-N-[(4-hydroxyphenyl)methyl]-alaninamid,
(7) (R)-N-[(4-Hydroxyphenyl)methyl]-N²-(5-phenyl-1-oxopentyl)-argininamid
(8) (R)-N-[(4-Hydroxyphenyl)methyl]-N²-(4-phenyl-1-oxobutyl)argininamid,
(9) (R)-N-[(4-Hydroxyphenyl)methyl]-N²-(6-phenyl-1-oxohexyl)argininamid,
(10) (R,S)-N²-(2,2-Diphenyl-2-hydroxy-1-oxoethyl)-N-[(4-hydroxyphenyl)methyl]-argininamid,
(11) N²-(Diphenylacetyl)-N-[(4-(methylamino)cyclohexyl)methyl]argininamid,
(12) (R)-N-[(4-Hydroxyphenyl)methyl]-N²-(tricyclo[3.3.1.1^{3.7}]-dec-1-ylacetyl)-argininamid,
(13) (R)-N-[(4-Hydroxyphenyl)methyl]-N²-[3-(1-piperidinyl)-1-oxopropyl]-argininamid,
(14) N²-(Diphenylacetyl)-N-[3-[1-[2-[5,11-dihydro-6(6H)-oxopyrido[2,3-b][1,4]benzodiazepin-5-yl]-2-oxoethyl]-4-piperidinyl]propyl]-argininamid,
(15) (R)-N²-[(rac.-5,11-Dihydro-6-oxo-6H-dibenz[b,e]azepin-11-yl)carbonyl]-N-[(4-ethoxyphenyl)methyl]-argininamid und
(16) N²-[N²-(Diphenylacetyl)-D-arginyl]-L-tyrosinamid,
deren Tautomere, deren Diastereomere, deren Enantiomere, deren Gemische und deren Salze.

2. Aminosäurederivate der allgemeinen Formel I gemäß Anspruch 1, in der
die R(CH₂)ₙ-Gruppe eine Phenylmethylgruppe, wobei die Phenylgruppe jeweils durch ein Fluor-, Chlor- oder Bromatom, durch eine Methyl-, Hydroxymethyl-, Hydroxyethyl-, Hydroxy-propyl-, Hydroxy-, Methoxy-, Ethoxy-, Dimethylaminosulfonyloxy-, Ethoxycarbonyloxy-, Acetyl-, Methoxycarbonyl-, Aminocarbonyl-, Aminocarbonylamino-, Methylaminocarbonylamino-, Aminosulfonyl-, Dimethylamino-, [1,2-Dihydro-3,5(4H)-dioxo-1,2-diphenyl-3H-1,2,4-triazol-4-yl]methyl- oder [1,5-Dihydro-2,4(3H)-dioxo-5,5-diphenyl-imidazol-3-yl]methyl-Gruppe substituiert ist,
eine Methylgruppe, die durch eine 3,4-Dimethoxyphenyl-, 3-Hydroxy-4-methoxyphenyl-, 3-Methoxy-4-hydroxyphenyl-, 4-Amino-3,5-dibromphenyl-, 4-Amino-3,5-dichlorphenyl-, 4-Hydroxy-3,5-dimethylphenyl-, 4'-Hydroxy-4-biphenylyl-, Thienyl-, Pyridinyl-, Benzimidazolyl- oder 1-[2-[5,11-Dihydro-6(6H)-oxopyrido[2,3-b][1,4]benzodiazepin-5-yl]-2-oxoethyl]-4-piperidinyl]- oder 4-Amino-3-fluorphenyl-Gruppe substituiert ist,
die T-Z-Gruppe eine Diphenylacetylgruppe, in der der Phenylkern jeweils durch ein Chlor- oder Bromatom substituiert sein kann,
eine Carbonylgruppe, die durch eine durch eine Benzyloxy- oder Phenylethoxygruppe substituierte Indolylgruppe, durch eine Benzyl-, (Dichlorphenyl)methyl-, Dichlorphenyl-, Naphthyl-, Naphthylmethyl-, α-Cyclopentylbenzyl-, 9-Fluorenyl- oder (Diphenylmethyl)amino-Gruppe substituiert ist,
R¹ das Wasserstoffatom,
R² eine geradkettige Alkylenkette mit 2 bis 5 Kohlenstoffatomen, welche endständig durch eine Amino-, Amidino-, Guanidino-, Aminocarbonyl-, Aminocarbonylamino- oder (1H-Imidazol-2-yl)amino-Gruppe substituiert ist, oder
eine Methylgruppe, die durch eine (Aminomethyl)phenyl-, Amidinophenyl- oder (4,5-Dihydro-1H-imidazol-2-yl)phenyl-Gruppe substituiert ist,
R³ das Wasserstoffatom oder die Methylgruppe und
Y eine gegebenenfalls durch eine Methylgruppe substituierte Iminogruppe bedeuten,
deren Tautomere, deren Diastereomere, deren Enantiomere, deren Gemische und deren Salze.

3. Folgende Aminosäurederivate der allgemeinen Formel I gemäß Anspruch 1:
(1) (R)-N²-(Diphenylacetyl)-N-(phenylmethyl)-argininamid,
(2) (R)-N²-(Diphenylacetyl)-N-[(4-methylphenyl)methyl]argininamid,
(3) (R)-N-[2-(4-Hydroxyphenyl)ethyl]-N²-[[5-(2-phenylethoxy)-1H-indol-2-yl)carbonyl]-argininamid,
(4) N-[(4-Aminocarbonylaminophenyl)methyl]-N²-(diphenylacetyl)-argininamid,
(5) (R)-N-[(4-Hydroxyphenyl)methyl]-N²-[[5-(2-phenylethoxy)-1H-indol-2-yl]carbonyl]-argininamid,
(6) (R)-N²-(Diphenylacetyl)-N-[(4-fluorphenyl)methyl]-argininamid,
(7) (R)-N-[(4-Bromphenyl)methyl]-N²-(diphenylacetyl)-argininamid,
(8) (R)-N²-(Diphenylacetyl)-N-(2-phenylethyl)-argininamid,
(9) Optisch aktives Diastereomerengemisch von N²-(α- Cyclopentyl-phenylacetyl)-N-[(4-hydroxyphenyl)methyl]-D-argininamid,
(10) N-[[4-(Dimethylamino)phenyl]methyl]-N²-(diphenylacetyl)argininamid,
(11) (R)-N²-(Diphenylacetyl)-N-[[4-(hydroxymethyl)phenyl]methyl]-argininamid,
(12) (R)-N²-(Diphenylacetyl)-N-[[4-(1-oxoethyl)phenyl]methyl]-argininamid,
(13) (R)-N-[(4-Chlorphenyl)methyl]-N²-(diphenylacetyl)-argininamid,
(14) (R)-N²-(Diphenylacetyl)-N-[[4-[(methylaminocarbonyl)-amino]-phenyl]methyl]-argininamid,
(15) (R)-N-[(4-Amino-3,5-dichlorphenyl)methyl]-N²-(diphenylacetyl)-argininamid,
(16) (R)-N-[(4-Amino-3,5-dichlorphenyl)methyl]-N²-(3,3-diphenyl-1-oxopropyl)-argininamid,
(17) (R)-N-[(4-Amino-3,5-dichlorphenyl)methyl]-N²-(3,4-dichlorbenzoyl)-argininamid,
(18) N²-(Diphenylacetyl)-N-[3-[1-[2-[5,11-dihydro-6(6H)oxopyrido[2,3-b][1,4]benzodiazepin-5-yl]-2-oxoethyl]-4-piperidinyl]propyl]-argininamid,
(19) N²-(Diphenylacetyl)-N-[(4-hydroxy-3-methoxyphenyl)methyl]-argininamid,
(20) N²-(Diphenylacetyl)-N-[(3-hydroxy-4-methoxyphenyl)methyl]-argininamid,
(21) (R,S)-N-[(4-Amino-3,5-dibromphenyl)methyl]-N⁶-(aminoiminomethyl)-N²-(diphenylacetyl)-lysinamid,
(22) N-[(3,5-Dimethyl-4-hydroxyphenyl)methyl]-N²-(diphenylacetyl)-argininamid,
(23) N-[(1H-Benzimidazol-5-yl)methyl]-N²-(diphenylacetyl)argininamid,
(24) N²-(Diphenylacetyl)-N-[(4-hydroxy-3-methylphenyl)methyl]-argininamid,
(25) N-[[4-(Aminocarbonyl)phenyl]methyl]-N²-(diphenylacetyl)-argininamid,
(26) (R,S)-N⁶-(Aminoiminomethyl)-N²-(diphenylacetyl)-N-[(4-hydroxyphenyl)methyl]-lysinamid,
(27) (R)-N-[(4-Hydroxyphenyl)methyl]-N²-(phenylacetyl)-argininamid,
(28) N²-(Diphenylacetyl)-N-[(1H-indol-5-yl)methyl]-argininamid,
(29) (R)-N-[[4-(Aminosulfonyl)phenyl]methyl]-N²-(diphenylacetyl)-argininamid,
(30) (R)-N²-(Diphenylacetyl)-N-[[4-(methoxycarbonyl)phenyl]methyl]-argininamid,
(31) (R)-N²-(Diphenylacetyl)-N-[(4-pyridinyl)methyl]-argininamid,
(32) (R)-N-[(4-Amino-3,5-dibromphenyl)methyl]-N²-(diphenylacetyl)-argininamid,
(33) (R)-N²-(Diphenylacetyl)-N-[(2-thienyl)methyl]-argininamid,
(34) (R)-N-[(4-Amino-3,5-dichlorphenyl)methyl]-N²-(2-naphthoyl)-argininamid,
(35) (R,S)-N⁵-(4,5-Dihydro-1H-imidazol-2-yl)-N²-(diphenylacetyl)-N-[(4-hydroxyphenyl)methyl]-ornithinamid,
(36) (R,S)-N²-(Diphenylacetyl)-N-[(4-hydroxyphenyl)methyl]-N⁵-(1H-imidazol-2-yl)-ornithinamid,
(37) (R)-N-[[3-[(1,2-Dihydro-3,5(4H)-dioxo-1,2-diphenyl-3H-1,2,4-triazol-4-yl)methyl]phenyl]methyl]-N²-(diphenylacetyl)argininamid,
(38) N²-(Diphenylacetyl)-N-[(3-hydroxyphenyl)methyl]-argininamid,
(39) (R)-N-[[3-[(4,5-Dihydro-2,4(3H)-diaxo-5,5-diphenyl-1H-imidazol-3-yl)methyl]phenyl]methyl]-N²-(diphenylacetyl)-argininamid,
(40) (R)-N²-(Diphenylacetyl)-N-[(4-hydroxyphenyl)methyl]-argininamid,
(41) N²-(Diphenylacetyl)-N-[2-(4-hydroxyphenyl)ethyl]-argininamid,
(42) N²-(Diphenylacetyl)-N-[(4'-hydroxy-[1,1'-biphenyl]-4-yl)methyl]-argininamid,
(43) N-[[4-[(1,2-Dihydro-3,5(4H)-dioxo-1,2-diphenyl-3H-1,2,4-triazol-4-yl)methyl]phenyl]methyl]-N²-(diphenylacetyl)-argininamid,
(44) N²-(Diphenylacetyl)-N-[(4-methoxyphenyl)methyl]-argininamid,
(45) N²-(Diphenylacetyl)-N-[2-(4-methoxyphenyl)ethyl]-argininamid,
(46) N²-(Diphenylacetyl)-N-[2-(3-methoxyphenyl)ethyl]-argininamid,
(47) N²-(Diphenylacetyl)-N-[(3-methoxyphenyl)methyl]-argininamid,
(48) (R,S)-3-[4-(Aminoiminomethyl)phenyl]-N²-(diphenylacetyl)-N-[(4-hydroxyphenyl)methyl]-alaninamid,
(49) (R,S)-3-[3-(Aminoiminomethyl)phenyl]-N²-(diphenylacetyl)-N-[(4-methoxyphenyl)methyl]-alaninamid,
(50) (R,S)-3-[3-(Aminoiminomethyl)phenyl]-N²-(diphenylacetyl)-N-[(4-hydroxyphenyl)methyl]-alaninamid,
(51) (R)-N²-[Bis-(4-bromphenyl)acetyl]-N-[(4-hydroxyphenyl)methyl]-argininamid,
(52) (R)-N²-(Diphenylacetyl)-N-[(4-ethoxyphenyl)methyl]-argininamid,
(53) (R)-N²-(Diphenylacetyl)-N-[(4-hydroxyphenyl)methyl]-N-methyl-argininamid,
(54) (R,S)-N-[(4-Amino-3,5-dibromphenyl)methyl]-3-[3-(aminoiminomethyl)phenyl]-N²-(diphenylacetyl)-alaninamind,
(55) (R)-N-[[4-[(Dimethylamino)sulfonyloxy]phenyl]methyl]-N²-(diphenylacetyl)-argininamid,
(56) (R)-N-[(4-Hydroxyphenyl)methyl]-N²-(1-naphthoyl)-arqininamid,
(57) (R)-N-[(4-Hydroxyphenyl)methyl]-N²-(2-naphthoyl)-argininamid,
(57) (R)-N²-(2,2-Diphenyl-2-hydroxyacetyl)-N-[(4-hydroxyphenyl)methyl]-argininamid,
(58) (R,S)-N²-(Diphenylacetyl)-N⁵-(1H-imidazol-2-yl)-N-[(4-methoxyphenyl)methyl]-ornithinamid,
(59) (R,S)-3-[3-(Aminoiminomethyl)phenyl]-N²-[[(diphenylmethyl)amino]carbonyl]-N-[(4-hydroxyphenyl)methyl]-alaninamid,
(60) (R,S)-N²-(Diphenylacetyl)-N⁵-(1H-imidazol-2-yl)-N-(phenylmethyl)-ornithinamid,
(61) (R,S)-N-[(4-Amino-3,5-dichlorphenyl)methyl]-N²-(diphenylacetyl)-N⁵-(1H-imidazol-2-yl)-ornithinamid,
(62) (R,S)-N-[(4-Hydroxyphenyl)methyl]-N⁵-(1H-imidazol-2-yl)-N²-(2-naphthoyl)-ornithinamid,
(63) (R,S)-N²-[[(Diphenylmethyl)amino]carbonyl]-N-[(4-hydroxyphenyl)methyl]-N⁵-(1H-imidazol-2-yl)-ornithinamid,
(64) (R,S)-N-[(4-Hydroxyphenyl)methyl]-N⁵-(1H-imidazol-2-yl)-N²-[(2-naphthyl)acetyl]-ornithinamid,
(65) (R,S)-N-[(4-Hydroxyphenyl)methyl]-N⁵-(1H-imidazol-2-yl)-N²-[[(2-naphthyl)amino]carbonyl]-ornithinamid,
(66) (R,S)-N-[(4-Hydroxyphenyl)methyl]-N⁵-(1H-imidazol-2-yl)-N²-[(1,2,3,4-tetrahydrochinolin-3-yl)carbonyl]-ornithinamid,
(67) (R,S)-N-[(4-Hydroxyphenyl)methyl]-N⁵-(1H-imidazol-2-yl)-N²-[(1,2,3,4-tetrahydrochinolin-2-yl)carbonyl]-ornithinamid,
(68) (R)-N-[(4-Aminosulfonylaminophenyl)methyl]-N²-(diphenylacetyl)-argininamid,
(69) (R)-N-[(4-Aminophenyl)methyl]-N²-(diphenylacetyl)-argininamid,
(70) (R)-N-[(6-Chinolinyl)methyl]-N²-(diphenylacetyl)-argininamid,
(71) (R)-N²-[(3,4-Dichlorphenyl)acetyl]-N-[(4-hydroxyphenyl)methyl]-argininamid,
(72) (R)-N²-(Diphenylacetyl)-N-[[4-(2-hydroxyethyl)phenyl]methyl]-argininamid,
(73) (R,S)-N⁵-(3-Amino-1,2,4-oxadiazol-5-yl)-N²-(diphenylacetyl)-N-[(4-hydroxyphenyl)methyl]-ornithinamid,
(74) (R)-N²-[(9-Fluorenyl)carbonyl]-N-[(4-hydroxyphenyl)methyl]-argininamid,
(75) (R,S)-6-(Aminoiminomethyl)-N²-(diphenylacetyl)-N-[(4-hydroxyphenyl)methyl]-norleucinamid,
(76) (R,S)-3-[4-(4,5-Dihydro-1H-imidazol-2-yl)phenyl]-N²-(diphenylacetyl)-N-[(4-hydroxyphenyl)methyl]-alaninamid,
(77) (R,S)-3-[3-(Aminoiminomethyl)phenyl]-N²-(diphenylacetyl)-N-[(4-ethoxycarbonyloxyphenyl)methyl]-alaninamid,
(78) (R,S)-N-[2-(1,2-Dihydro-1,2-diphenyl-3,5(4H)-dioxo-1,2,4-triazol-4-yl)ethyl]-N²-(diphenylacetyl)-argininamid,
(79) (R,S)-N²-(Diphenylacetyl)-N-[(4-hydroxyphenyl)methyl]-2-methyl-argininamid,
(80) (R)-N²-(Diphenylacetyl)-N-[[4-(3-hydroxypropyl)phenyl]methyl]-argininamid,
(81) (R)-N-[[4-[(4,5-Dihydro-5,5-dimethyl-2,4(3H)-dioxo-1H-imidazol-3-yl)methyl]phenyl]methyl]-N²-(diphenylacetyl)-argininamid
und deren Salze.

4. Physiologisch verträgliche Salze der Verbindungen der allgemeinen Formel I gemäß den Ansprüchen 1 bis 3 mit Ausnahme derjenigen Verbindungen, in denen T-Z ein Wasserstoffatom darstellt, mit anorganischen oder organischen Säuren oder Basen.

5. Arzneimittel, enthaltend als Wirkstoff eine Verbindung der allgemeinen Formel I gemäß den Ansprüchen 1 bis 3 mit Ausnahme derjenigen Verbindungen, in denen T-Z ein Wasserstoffatom darstellt, oder dessen physiologisch verträgliches Salz gemäß Anspruch 4 neben gegebenenfalls einem oder mehreren inerten Trägerstoffen und/oder Verdünnungsmitteln.

6. Verwendung einer Verbindung der allgemeinen Formel I gemäß den Ansprüchen 1 bis 4 sowie von Verbindungen der Formel I, in denen
die Gruppe R-(CH₂)ₙ- eine Alkylgruppe mit 1 bis 3 Kohlenstoffatomen oder eine Phenylgruppe,
R¹ und R³ jeweils ein Wasserstoffatom,
R² eine Amidinophenylmethylgruppe,
T-Z eine 4-Amino-3,5-dichlorphenylsulfonylgruppe oder die Naphthylsulfonylgruppe und
Y eine Iminogruppe bedeuten,
mit Ausnahme derjenigen Verbindungen, in denen T-Z ein Wasserstoffatom darstellt, zur Herstellung eines Arzneimittels, welches zur Behandlung cardiovasculärer Erkrankungen, der chronischen Herzinsuffizienz, von coronaren Herzerkrankungen, von subarachnoidalen Blutungen, des chronischen Nierenversagens, von Tumorerkrankungen, von Hyperthyreoidismus, der Obesitas und des Diabetes geeignet ist.

7. Verfahren zur Herstellung eines Arzneimittels gemäß Anspruch 5, dadurch gekennzeichnet, daß auf nichtchemischem Wege eine Verbindung der allgemeinen Formel I gemäß den Ansprüchen 1 bis 4 mit Ausnahme derjenigen Verbindungen, in denen T-Z ein Wasserstoffatom darstellt, in einen oder in mehrere inerte übliche Trägerstoffe und/oder Verdünnungsmittel eingearbeitet wird.

8. Verfahren zur Herstellung der neuen Aminosäurederivate der allgemeinen Formel I gemäß den Ansprüchen 1 bis 4 und 9, dadurch gekennzeichnet, daß
a) zur Herstellung von Verbindungen der allgemeinen Formel I, in der T-Z die in den Ansprüchen 1 bis 3 erwähnten Bedeutungen mit Ausnahme der eines Wasserstoffatoms besitzt, eine Verbindung der allgemeinen Formel II,
T^{A} - Z - X , (II)
in der
T^{A}-Z die in den Ansprüchen 1 bis 3 für T-Z erwähnten Bedeutungen mit Ausnahme der eines Wasserstoffatoms besitzt und X die Hydroxygruppe, ein Halogenatom, eine Alkylsulfonyloxygruppe mit 1 bis 10 Kohlenstoffatomen, eine gegebenenfalls durch Chlor- oder Bromatome, durch Methyl- oder Nitrogruppen mono-, di- oder trisubstituierte Phenylsulfonyloxy- oder Naphthylsulfonyloxygruppe bedeutet, wobei die Substituenten gleich oder verschieden sein können,
mit einem α-Aminosäurederivat der allgemeinen Formel III,
H - NR¹ - (R^{2a}CR³) - CO - Y - (CH₂)ₙ-R , (III)
in der
R-(CH₂)ₙ, R¹, R³ und Y wie in den Ansprüchen 1 bis 3 definiert sind und
R^{2a} die für R² in den Ansprüchen 1 bis 3 erwähnten Bedeutungen besitzt oder einen durch Schutzreste substituierten Rest R² oder einen Präcursorrest für den Rest R² darstellt,
gekuppelt und, falls nötig, anschließend eine verwendete Schutzgruppe abgespalten oder eine verwendete Präcursor-Funktion abgewandelt wird oder
b) zur Herstellung von Verbindungen der allgemeinen Formel I, in der T-Z die in den Ansprüchen 1 bis 3 erwähnten Bedeutungen mit Ausnahme der eines Wasserstoffatoms besitzt, eine Verbindung der allgemeinen Formel IV,
T^{A} - Z - NR¹- (R^{2a}CR³) - COOH , (IV)
in der
R¹ und R³ wie in den Ansprüchen 1 bis 3 erwähnt definiert sind,
T^{A}-Z mit Ausnahme der eines Wasserstoffatoms die in den Ansprüchen 1 bis 3 für T-Z erwähnten Bedeutungen besitzt und R^{2a} die für R² in den Ansprüchen 1 bis 3 erwähnten Bedeutungen besitzt oder einen durch Schutzreste substituierten Rest R² oder einen Präcursorrest für den Rest R² darstellt,
mit einer Verbindung der allgemeinen Formel V,
H - Y - (CH₂)ₙ - R , (V)
in der
R-(CH₂)ₙ und Y die in den Ansprüchen 1 bis 3 erwähnten Bedeutungen besitzen, gekuppelt und, falls nötig, anschließend eine verwendete Schutzgruppe abgespalten oder eine verwendete Präcursor-Funktion abgewandelt wird oder
c) zur Herstellung von Verbindungen der allgemeinen Formel I, in der T-Z mit Ausnahme des Wasserstoffatoms die für T-Z in den Ansprüchen 1 bis 3 erwähnten Bedeutungen besitzt und R² eine geradkettige Alkylgruppe mit 1 bis 5 Kohlenstoffatomen, die in ω-Stellung durch eine 4,5-Dihydro-1H-imidazol-2-yl-aminogruppe oder eine Guanidinogruppe, in der die Wasserstoffatome an den Stickstoffatomen jeweils durch Alkylgruppen mit 1 bis 3 Kohlenstoffatomen ersetzt sein können, substituiert ist, bedeutet,
eine Verbindung der allgemeinen Formel VI,
T^{A} - Z - NR¹ - (R^{2b}CR³) - CO - Y - (CH₂)ₙ - R , (VI)
in der
R-(CH₂)ₙ, R¹, R³ und Y wie in den Ansprüchen 1 bis 3 definiert sind, T^{A}-Z mit Ausnahme des Wasserstoffatoms die für T-Z in den Ansprüchen 1 bis 3 erwähnten Bedeutungen besitzt und R^{2b} eine geradkettige Alkylgruppe mit 1 bis 5 Kohlenstoffatomen bedeutet, wobei die Alkylgruppe in ω-Stellung durch eine R⁶NH-Gruppe substituiert ist und R⁶ ein Wasserstoffatom oder eine Alkylgruppe mit 1 bis 3 Kohlenstoffatomen darstellt,
mit einem Kohlensäurederivat der allgemeinen Formel VII,
X¹ - (C=NR⁷) - (R⁸NR⁹) , (VII)
in der
R⁷, R⁸ und R⁹, die gleich oder verschieden sein können, Wasserstoffatome, Alkylgruppen mit 1 bis 3 Kohlenstoffatomen oder
R⁷ und R⁸ zusammen auch eine n-Alkylengruppe mit 2 Kohlenstoffatomen und
X¹ eine Austrittsgruppe bedeuten, umgesetzt wird oder
d) zur Herstellung von Verbindungen der allgemeinen Formel I, in der T-Z mit Ausnahme des Wasserstoffatoms die für T-Z in den Ansprüchen 1 bis 3 erwähnten Bedeutungen besitzt und R² eine geradkettige Alkylgruppe mit 1 bis 5 Kohlenstoffatomen darstellt, wobei die Alkylgruppe in ω-Stellung durch eine R^{8a}R⁹N-C(=NH)-NR⁶-Gruppe substituiert ist und R⁶, R^{8a} und R⁹, die gleich oder verschieden sein können, Wasserstoffatome oder Alkylgruppen mit 1 bis 3 Kohlenstoffatomen darstellen, eine Verbindung der allgemeinen Formel VI,
T^{A} - Z - NR¹ - (R^{2b}CR³) - CO - Y - (CH₂)ₙ - R , (VI)
in der
R-(CH₂)ₙ, R¹, R³ und Y wie in den Ansprüchen 1 bis 3 definiert sind, T^{A}-Z mit Ausnahme des Wasserstoffatoms die für T-Z in den Ansprüchen 1 bis 3 erwähnten Bedeutungen besitzt und R^{2b} eine geradkettige Alkylgruppe mit 1 bis 5 Kohlenstoffatomen bedeutet, wobei die Alkylgruppe in ω-Stellung durch eine R⁶NH-Gruppe substituiert ist und R⁶ ein Wasserstoffatom oder eine Alkylgruppe mit 1 bis 3 Kohlenstoffatomen darstellt,
mit einem Cyanamid der allgemeinen Formel VIII,
R^{8a}R⁹N - CN , (VIII)
in der
R^{8a} und R⁹, die gleich oder verschieden sein können, jeweils Wasserstoffatome oder Alkylgruppen mit jeweils 1 bis 3 Kohlenstoffatomen bedeuten, umgesetzt wird oder
e) zur Herstellung von Verbindungen der allgemeinen Formel I, in der T-Z mit Ausnahme des Wasserstoffatoms die für T-Z in den Ansprüchen 1 bis 3 erwähnten Bedeutungen besitzt und R² eine geradkettige Alkylgruppe mit 1 bis 5 Kohlenstoffatomen darstellt, wobei die Alkylgruppe in ω-Stellung durch eine HR⁸N-C(=NR⁷)-NR⁶-Gruppe substituiert ist und R⁶, R⁷ und R⁸, die gleich oder verschieden sein können, Wasserstoffatome oder Alkylgruppen mit 1 bis 3 Kohlenstoffatomen darstellen, eine Verbindung der allgemeinen Formel VI,
T^{A} - Z - NR¹ - (R^{2b}CR³) - CO - Y - (CH₂)ₙ - R , (VI)
in der
R-(CH₂)ₙ, R¹, R³ und Y wie in den Ansprüchen 1 bis 3 definiert sind, T^{A}-Z mit Ausnahme des Wasserstoffatoms die für T-Z in den Ansprüchen 1 bis 3 erwähnten Bedeutungen besitzt und R^{2b} eine geradkettige Alkylgruppe mit 1 bis 5 Kohlenstoffatomen bedeutet, die in ω-Stellung durch eine R⁶NH-Gruppe substituiert ist, wobei R⁶ ein Wasserstoffatom oder eine Alkylgruppe mit 1 bis 3 Kohlenstoffatomen darstellt,
mit einem Carbodiimid der allgemeinen Formel IX,
R⁷ - N = C = N - R⁸ , (IX)
in der
R⁷ und R⁸ , die gleich oder verschieden sein können, Wasserstoffatome oder Alkylgruppen mit 1 bis 3 Kohlenstoffatomen darstellen, umgesetzt wird oder
f) zur Herstellung von Verbindungen der allgemeinen Formel I, in der T-Z mit Ausnahme des Wasserstoffatoms die für T-Z in den Ansprüchen 1 bis 3 erwähnten Bedeutungen besitzt und R² eine Amidinophenylmethylgruppe darstellt, eine Verbindung der allgemeinen Formel X,
T^{A} - Z - NR¹ - (R^{2c}CR³) - CO - Y - (CH₂)ₙ - R , (X)
in der
R-(CH₂)ₙ, R¹, R³ und Y wie in den Ansprüchen 1 bis 3 definiert sind, T^{A}-Z mit Ausnahme des Wasserstoffatoms die für T-Z in den Ansprüchen 1 bis 3 erwähnten Bedeutungen besitzt und R^{2c} eine Cyanophenylmethylgruppe bedeutet,
mit einem Alkohol der allgemeinen Formel XI,
R⁵ - OH , (XI)
in der
R⁵ eine Alkylgruppe mit 1 bis 3 Kohlenstoffatomen bedeutet, umgesetzt und anschließend mit Ammoniak behandelt wird oder
g) zur Herstellung von Verbindungen der allgemeinen Formel I, in der T-Z mit Ausnahme des Wasserstoffatoms die für T-Z in den Ansprüchen 1 bis 3 erwähnten Bedeutungen besitzt und R² Amidinophenylmethylgruppe darstellt, eine Verbindung der allgemeinen Formel XII,
T^{A} - Z - NR¹ - (R^{2d}CR³) - CO - Y - (CH₂)ₙ - R , (XII)
in der
R-(CH₂)ₙ, R¹, R³ und Y wie in den Ansprüchen 1 bis 3 definiert sind, T^{A}-Z mit Ausnahme des Wasserstoffatoms die für T-Z in den Ansprüchen 1 bis 3 erwähnten Bedeutungen besitzt und R^{2d} eine durch eine H₂N-C(=NOH)-Gruppe substituierte Phenylmethylgruppe darstellt, hydrogenolysiert wird oder
h) zur Herstellung von Verbindungen der allgemeinen Formel I, in der T-Z mit Ausnahme des Wasserstoffatoms die für T-Z in den Ansprüchen 1 bis 3 erwähnten Bedeutungen besitzt und R² eine Amidinophenylmethylgruppe darstellt, ein Nitril der allgemeinen Formel X,
T^{A} - Z - NR¹ - (R^{2c}CR³) - CO - Y - (CH₂)ₙ - R , (X)
in der
R-(CH₂)n, R¹, R³, und Y wie in den Ansprüchen 1 bis 3 definiert sind, T^{A}-Z mit Ausnahme der Bedeutung eines Wasserstoffatoms wie in den Ansprüchen 1 bis 3 definiert ist und R^{2c} eine Cyanophenylmethylgruppe darstellt,
in ein Thioamid der allgemeinen Formel XIII,
T^{A} - Z - NR¹ - (R^{2c}'CR³) - CO - Y - (CH₂)ₙ - R , (XIII)
in der
R-(CH₂)ₙ, R¹, R³, Y und T^{A}-Z wie oben angegeben definiert sind und R^{2c}' eine durch eine Aminothiocarbonylgruppe substituierte Phenylmethylgruppe darstellt, übergeführt und anschließend mit einer Verbindung der allgemeinen Formel XIV,
R⁵-X² , (XIV)
in der
R⁵ eine Alkylgruppe mit 1 bis 3 Kohlenstoffatomen und X² eine Austrittsgruppe darstellt, oder mit einem Trialkyloxoniumtetrafluoroborat der allgemeinen Formel XV,
(R⁵)₃OBF₄ , (XV)
in der
R⁵ eine Alkylgruppe mit 1 bis 3 Kohlenstoffatomen darstellt, alkyliert und anschließend mit Ammoniak umgesetzt wird oder
i) zur Herstellung von Verbindungen der allgemeinen Formel I, in der T-Z mit Ausnahme des Wasserstoffatoms die für T-Z in den Ansprüchen 1 bis 3 erwähnten Bedeutungen besitzt und R² Cyaniminomethylaminophenylmethylgruppe darstellt, eine Verbindung der allgemeinen Formel VI,
T^{A} - Z - NR¹ - (R^{2b}CR³) - CO - Y - (CH₂)ₙ - R , (VI)
in der
R-(CH₂)ₙ, R¹, R³, und Y wie in den Ansprüchen 1 bis 3 definiert sind, T^{A}-Z mit Ausnahme der Bedeutung eines Wasserstoffatoms wie in den Ansprüchen 1 bis 3 definiert ist und R^{2b} Aminophenylmethylgruppe darstellt, mit einem N-Cyan-formimidsäureester der allgemeinen Formel XVI,
R¹³O-CH=N-CN , (XVI)
in der
R¹³ eine Alkylgruppe mit 1 bis 10 Kohlenstoffatomen darstellt, umgesetzt wird oder
j) zur Herstellung von Verbindungen der allgemeinen Formel I, in der T-Z mit Ausnahme des Wasserstoffatoms die für T-Z in den Ansprüchen 1 bis 3 erwähnten Bedeutungen besitzt und R² eine in ω-Stellung durch eine Aminomethylidenimino- oder Methylaminomethylideniminogruppe substituierte geradkettige Alkylgruppe mit 1 bis 5 Kohlenstoffatomen oder eine Methylaminomethylideniminophenylmethylgruppe darstellt, eine Verbindung der allgemeinen Formel XVII,
T^{A} - Z - NR¹ - (R^{2d'}CR³) - CO - Y - (CH₂)ₙ - R , (XVII)
in der
R-(CH₂)ₙ, R¹, R³ und Y wie in den Ansprüchen 1 bis 3 definiert sind, T^{A}-Z mit Ausnahme der Bedeutung eines Wasserstoffatoms die in den Ansprüchen 1 bis 3 für T-Z erwähnten Bedeutungen besitzt und R^{2d'} eine geradkettige Alkylgruppe mit 1 bis 5 Kohlenstoffatomen oder eine Phenylmethylgruppe darstellt, wobei die Alkylgruppe in ω-Stellung und der vorstehend erwähnte Aromat jeweils durch eine NC-N=CH-NH-Gruppe substituiert sind,
mit einem Amin der allgemeinen Formel XVIII,
H₂NR^{8a} , (XVIII)
in der
R^{8a} ein Wasserstoffatom oder eine Methylgruppe darstellt, umgesetzt wird oder
k) zur Herstellung von Verbindungen der allgemeinen Formel I, in der T-Z eine Diphenylaminocarbonyl-, Naphthylaminocarbonyl-, Hexamethylenaminocarbonyl-, 1-Piperidinyl- oder (Diphenylmethyl)aminocarbonyl-Gruppe darstellt, ein Isocyanat der allgemeinen Formel XIX,
O=C=N - (R^{2a}CR³)-CO-NR⁴-(CH₂)ₙ-R , (XIX)
in der
R-(CH₂)ₙ, R³ und R⁴ wie in den Ansprüchen 1 bis 3 definiert sind und R^{2a} die für R² in den Ansprüchen 1 bis 3 erwähnten Bedeutungen besitzt oder einen durch Schutzreste substituierten Rest R² oder einen Präcursorrest für den Rest R² darstellt,
mit einem Amin der allgemeinen Formel XX,
T¹T²N-H , (XX)
in der
T¹ und T² jeweils eine Phenylgruppe oder
T¹ ein Wasserstoffatom und T² eine Naphthyl- oder Diphenylmethylgruppe oder
T¹ und T² zusammen eine n-Alkylengruppe mit 5 oder 6 Kohlenstoffatomen bedeuten,
umgesetzt wird oder
l) zur Herstellung von Verbindungen der allgemeinen Formel I, in der T-Z ein Wasserstoffatom darstellt, von einer Verbindung der allgemeinen Formel XXI,
R¹⁴-NR¹ - (R²CR³) - CO - Y - (CH₂)ₙ-R , (XXI)
in der
R-(CH₂)ₙ, R¹, R², R³ und Y wie in den Ansprüchen 1 bis 3 definiert sind und R¹⁴ eine tert.-Butoxycarbonyl- oder eine 9-Fluorenylmethoxycarbonylgruppe darstellt, der Rest R¹⁴ abgespalten wird oder
m) zur Herstellung von Verbindungen der allgemeinen Formel I, in der ein Wasserstoffatom einer im Rest R² vorhandenen HN<, HN= oder H₂N-Gruppe durch eine tert.-Butoxycarbonylgruppe ersetzt ist, eine Verbindung der allgemeinen Formel I,
T - Z - NR¹ - (R²CR³) - CO - Y - (CH₂)ₙ - R , (I)
in der R-(CH₂)ₙ, R¹ bis R³, T-Z und Y mit der Maßgabe wie in den Ansprüchen 1 bis 3 definiert sind, daß R² mindestens eine freie HN<, HN= oder H₂N-Gruppe enthalten muß, mit einer Verbindung der allgemeinen Formel XXII,
X³ - W , (XXII)
in der
W eine tert.-Butoxycarbonylgruppe darstellt und
X³ eine Austrittsgruppe bedeutet, umgesetzt wird oder
n) zur Herstellung von Verbindungen der allgemeinen Formel I, in der T-Z eine Naphthylaminocarbonyl- oder (Diphenylmethyl)aminocarbonyl-Gruppe darstellt, ein Isocyanat der allgemeinem Formel XXIII,
T'N=C=O , (XXIII)
in der
T' eine Naphthyl- oder Diphenylmethylgruppe bedeutet,
mit einer Verbindung der allgemeinen Formel III,
H - NR¹ - (R^{2a}CR³) - CO - Y - (CH₂)ₙ-R , (III)
in der
R-(CH₂)ₙ, R¹, R³ und Y wie in den Ansprüchen 1 bis 3 definiert sind und R^{2a} die für R² in den Ansprüchen 1 bis 3 erwähnten Bedeutungen besitzt oder einen durch Schutzreste substituierten Rest R² oder einen Präcursorrest für den Rest R² darstellt, umgesetzt wird und
gewünschtenfalls anschließend eine so erhaltene Verbindung der allgemeinen Formel I in seine Diastereomeren aufgetrennt wird und/oder
ein so erhaltenes Racemat einer Verbindung der allgemeinen Formel I in seine Enantiomeren aufgetrennt wird und/oder
eine so erhaltene Verbindung der allgemeinen Formel I in ihre Salze mit anorganischen Säuren oder Basen, insbesondere zur pharmazeutischen Anwendung in ihre physiologisch verträglichen Salze, übergeführt wird.

9. D- und (R)-Enantiomeren der Verbindungen gemäß den Ansprüchen 1 bis 3.

## Claims

1. Amino acid derivatives of general formula
T - Z - NR¹ - (R²CR³) - CO - Y - (CH₂)ₙ - R (I)
wherein
the R-(CH₂)ₙ- group denotes a C₁₋₃-alkyl group which may be substituted by 2 phenyl groups in the ω-position,
a straight-chained C₂₋₅-alkylene group terminally substituted by a hydroxy group,
a phenyl or phenylalkyl group having 1 to 4 carbon atoms in the alkyl moiety in which the phenyl group is optionally substituted by a fluorine, chlorine or bromine atom, by an alkylaminocarbonyl group having 1 to 4 carbon atoms in the alkyl moiety, by an ethylaminocarbonylamino group (in which the ethyl moiety is substituted by one or two phenyl groups), or by a methyl, hydroxymethyl, phenyl, hydroxyphenyl, hydroxy, methoxy, ethoxy, dimethylaminosulphonyloxy, cyano, carboxy, methoxycarbonyl, acetyl, aminocarbonyl, dimethylaminocarbonyl, amino, methylamino, dimethylamino, acetylamino, methoxycarbonylamino, ethoxycarbonylamino, aminocarbonylamino, methylaminocarbonylamino, dimethylaminocarbonylamino, methylsulphonylamino, aminosulphonyl, [amino(imino)-methyl]-, [amino(imino)methyl]amino, [1,2-dihydro-3,5(4H)-dioxo-1,2-diphenyl-3H-1,2,4-triazol-4-yl]methyl, [1,5-dihydro-2,4(3H)-dioxo-5,5-diphenyl-imidazol-3-yl]methyl, aminomethyl, aminosulphonylamino, hydroxyethyl, hydroxypropyl, hydroxybutyl, 3-(dimethylamino)propoxy or ethoxycarbonyloxy group, a methyl group which is substituted by a hydroxycyclohexyl, 4-hydroxy-3-methyl-phenyl, 3,4-dimethoxyphenyl, 3-hydroxy-4-methoxyphenyl, 3-methoxy-4-hydroxyphenyl, 4-amino-3,5-dibromophenyl, 4-amino-3,5-dichlorophenyl, 4-hydroxy-3,5-dibromophenyl, 4-hydroxy3,5-dichlorophenyl, 4-amino-3-fluorophenyl, 4-hydroxy-3,5-dimethylphenyl, thienyl, pyridinyl, indolyl, benzimidazolyl, quinolinyl or 2-(diphenylamino-carbonyl)-2,3-dihydro-1H-isoindolyl group,
an ethyl group substituted by an indolyl, 5-methoxyindolyl, 1,2-diphenyl-3,5(4H)-dioxo-1,2,4-triazol-4-yl or imidazolyl group,
a 2-(2-hydroxyphenyl)ethyl group substituted in the α-position by an aminocarbonyl group,
a 3-[1-[2-[5,11-dihydro-6(6H)-oxopyrido[2,3-b][1,4]-benzodiazepin-5-yl]-2-oxoethyl]-4-piperidinyl]propyl or 3-hydroxy-1-propyn-1-yl group,
the T-Z- group denotes a hydrogen atom,
a carbonyl group which may be substituted by a C₁₋₄-alkyl group which is itself substituted in the α- or β-position by a C₁₋₃-alkyl group or by one or two phenyl groups optionally mono- or disubstituted by methoxy groups, hydroxy groups or chlorine or bromine atoms,
a carbonyl group which is substituted by an indolyl group (itself substituted by a benzyloxy or phenylethoxy group), or by a methyl, tricyclo[3.3.1.1^{3.7}]dec-1-yl, benzyl, (hydroxyphenyl)methyl, (methylphenyl)methyl, (biphenylyl)methyl, (dichlorophenyl)methyl, phenyl, 3,5-dichlorophenyl, 3,4-dichlorophenyl, naphthyl, naphthylmethyl, α-cyclopentyl-benzyl, diphenylamino, naphthylamino, hexamethyleneimino, 5-phenylethoxyindolyl, 1,2,3,4-tetrahydro-2-quinolinyl, 2-quinolinyl, 1,2,3,4-tetrahydro-3-quinolinyl, (dichlorophenoxy)-methyl, 9-fluorenyl, triphenylmethyl, 1-piperidinyl, (diphenylmethyl)amino or 5,10-dihydro-11(11H)-oxodibenzo[b,e][1,4]diazepin-5-yl group,
or
a 4-amino-3,5-dichloro-phenylsulphonyl or naphthylsulphonylamino group,
R¹ denotes a hydrogen atom,
R² denotes an unbranched C₁₋₅-alkyl group which is substituted in the ω-position by a guanidino group (wherein the hydrogen atoms at the nitrogen atoms may each be replaced by C₁₋₃-alkyl groups), by an amino, tert.butoxycarbonylamino, dimethylamino, N-methylbenzylamino, methylamino, aminocarbonyl, aminocarbonylamino, aminomethylideneimino, methylaminomethylideneimino, [amino(nitroimino)methyl]-amino, 1H-imidazol-2-yl-amino, 4,5-dihydro-1N-imidazol-2-yl-amino, (5-amino-4H-1,2,4-triazol-3-yl)amino, (3-amino-1,2,4-oxadiazol-5-yl)amino or (5-amino-1,2,4-oxadiazol-3-yl)amino group,
a methyl group which is substituted by a phenyl, cyanophenyl, aminomethylphenyl, amidinophenyl, methylaminomethylideneiminophenyl, cyanoiminomethylaminophenyl, methyliminomethylaminophenyl, (4,5-dihydro1H-imidazol-2-yl)phenyl or imidazolyl group,
R³ denotes a hydrogen atom or a methyl group and
Y denotes an imino group optionally substituted by a methyl or ethyl group;
with the proviso that
(i)
R¹ does not denote a hydrogen atom when
the group R-(CH₂)ₙ- is an alkyl group with 1-3 carbon atoms or a phenyl group,
R² is an amidinophenylmethyl group,
R³ is a hydrogen atom,
T-Z is a 4-amino-3,5-dichlorophenyl-sulphonyl group or a naphthylsulphonyl group and
Y is an imino group; and
(ii)
R² does not denote an unbranched C₃₋₄-alkyl group which may be replaced in the ω position by a guanidino group wherein the hydrogen atoms at the nitrogen atoms may each be replaced by a C₁₋₃-alkyl group, by an amino, tert. butoxycarbonylamino, dimethylamino, N-methylbenzylamino, methylamino, aminocarbonyl or aminocarbonylamino group,
when
R¹, R³ and T-Z each represent a hydrogen atom, and
Y denotes an imino group optionally substituted by a methyl or ethyl group, and
(iii)
R² does not denote a phenylmethyl group if
R¹, R³ and T-Z each represent a hydrogen atom,
Y denotes an imino group and
the R-(CH₂)ₙ- group denotes a C₁₋₃-alkyl group or a phenylalkyl having 1 to 4 carbon atoms in the alkyl moiety,
and the following compounds:
(1) (R)-N-[[4-[(4,5-dihydro-5,5-dimethyl-2,4(3H)-dioxo-1H-imidazol-3-yl)methyl]phenyl]methyl]-N²-(diphenylacetyl)-argininamide,
(2) (R,S)-N²-(diphenylacetyl)-N-[(4-hydroxyphenyl)methyl]-3-[(4-piperidinyl)methyl]alaninamide,
(3) (R,S)-3-[3-[amino(hydroxyimino)methyl]phenyl]-N²-(diphenylacetyl)-N-[(4-hydroxyphenyl)methyl]alaninamide,
(4) (R,S)-3-[3-(aminocarbonyl)phenyl]-N²-(diphenylacetyl)-N-[(4-hydroxyphenyl)methyl]alaninamide,
(5) (R)-N²-(2,2-diphenylethyl)-N-[(4-hydroxyphenyl)methyl]-argininamide,
(6) (R,S)-3-[3-(aminoiminomethyl)phenyl]-N²-(2,2-diphenylethyl)-N-[(4-hydroxyphenyl)methyl]-alaninamide,
(7) (R)-N-[(4-hydroxyphenyl)methyl]-N²-(5-phenyl-1-oxopentyl)-argininamide,
(8) (R)-N-[(4-hydroxyphenyl)methyl]-N²-(4-phenyl-1-oxobutyl)-argininamide,
(9) (R)-N-[(4-hydroxyphenyl)methyl]-N²-(6-phenyl-1-oxohexyl)-argininamide,
(10) (R,S)-N²-(2,2-diphenyl-2-hydroxy-1-oxoethyl)-N-[(4-hydroxyphenyl)methyl]-argininamide,
(11) N²-(diphenylacetyl)-N-[(4-(methylamino)cyclohexyl)methyl]-argininamide,
(12) (R)-N-[(4-hydroxyphenyl)methyl]-N²-(tricyclo[3.3.1.1^{3.7}]-dec-1-ylacetyl)argininamide,
(13) (R)-N-[(4-hydroxyphenyl)methyl]-N²-[3-(1-piperidinyl)-1-oxopropyl]-argininamide,
(14) N²-(diphenylacetyl)-N-[3-[1-[2-[5,11-dihydro-6(6H)-oxo-pyrido[2,3-b][1,4]benzodiazepin-5-yl]-2-oxoethyl]-4-piperidinyl]propyl]-argininamide,
(15) (R)-N²-[(rac.-5,11-dihydro-6-oxo-6H-dibenz[b,e]azepin-11-yl)-carbonyl]-N-[(4-ethoxyphenyl)methyl]-argininamide and
(16) N²-[N²-(diphenylacetyl)-D-arginyl]-L-tyrosinamide,
the tautomers, the diastereomers and enantiomers thereof, mixtures thereof and the salts thereof.

2. Amino acid derivatives of general formula I according to claim 1 wherein
the R(CH₂)ₙ group denotes a phenylmethyl group, wherein the phenyl group is substituted by a fluorine, chlorine or bromine atom, or by a methyl, hydroxymethyl, hydroxyethyl, hydroxypropyl, hydroxy, methoxy, ethoxy, dimethylaminosulphonyloxy, ethoxycarbonyloxy, acetyl, methoxycarbonyl, aminocarbonyl, aminocarbonylamino, methylaminocarbonylamino, aminosulphonyl, dimethylamino, [1,2-dihydro-3,5(4H)-dioxo-1,2-diphenyl-3H-1,2,4-triazol-4-yl]methyl or [1,5-dihydro-2,4(3H)-dioxo-5,5-diphenyl-imidazol-3-yl]methyl group,
a methyl group which is substituted by a 3,4-dimethoxyphenyl, 3-hydroxy-4-methoxyphenyl, 3-methoxy-4-hydroxyphenyl, 4-amino-3,5-dibromophenyl, 4-amino-3,5-dichlorophenyl, 4-hydroxy-3,5-dimethylphenyl, 4'-hydroxy-4-biphenylyl, thienyl, pyridinyl, benzimidazolyl, 1-[2-[5,11-dihydro-6(6H)-oxopyrido[2,3-b][1,4]-benzodiazepin-5-yl]-2-oxoethyl]-4-piperidinyl or 4-amino-3-fluorophenyl group,
the T-Z- group denotes a diphenylacetyl group in which the phenyl nucleus may in each case be substituted by a chlorine or bromine atom,
a carbonyl group which is substituted by an indolyl group (itself substituted by a benzyloxy or phenylethoxy group) or by a benzyl, (dichlorophenyl)methyl, dichlorophenyl, naphthyl, naphthylmethyl, α-cyclopentylbenzyl, 9-fluorenyl or (diphenylmethyl)amino group,
R¹ denotes a hydrogen atom,
R² denotes a straight-chained C₂₋₅-alkylene chain which is terminally substituted by an amino, amidino, guanidino, aminocarbonyl, aminocarbonylamino or (1H-imidazol-2-yl)amino group or
a methyl group which is substituted by an (aminomethyl)phenyl, amidinophenyl or (4,5-dihydro-1H-imidazol-2-yl)phenyl group,
R³ denotes a hydrogen atom or a methyl group and
Y denotes an optionally methyl-substituted imino group,
the tautomers, the diastereomers, and the enantiomers thereof, mixtures thereof and the salts thereof.

3. The following amino acid derivatives of general formula I according to claim 1:
(1) (R)-N²-(diphenylacetyl)-N-(phenylmethyl)argininamide,
(2) (R)-N²-(diphenylacetyl)-N-[(4-methylphenyl)methyl]argininamide,
(3) (R)-N-[2-(4-hydroxyphenyl)ethyl]-N²-[[5-(2-phenylethoxy)-1H-indol-2-yl)carbonyl]-argininamide,
(4) N-[(4-aminocarbonylaminophenyl)methyl]-N²-(diphenylacetyl)-argininamide,
(5) (R)-N-[(4-hydroxyphenyl)methyl]-N²-[[5-(2-phenylethoxy)-1H-indol-2-yl]carbonyl]-argininamide,
(6) (R)-N²-(diphenylacetyl)-N-[(4-fluorophenyl)methyl]argininamide,
(7) (R)-N-[(4-bromophenyl)methyl]-N²-(diphenylacetyl)argininamide,
(8) (R)-N²-(diphenylacetyl)-N-(2-phenylethyl)argininamide,
(9) optically active diastereomer mixture of N²-(α-cyclopentyl-phenylacetyl)-N-[(4-hydroxyphenyl)methyl]-D-argininamide,
(10) N-[[4-(dimethylamino)phenyl]methyl]-N²-(diphenylacetyl)-argininamide,
(11) (R)-N²-(diphenylacetyl)-N-[[4-(hydroxymethyl)phenyl]-methyl]argininamide,
(12) (R)-N²-(diphenylacetyl)-N-[[4-(1-oxoethyl)phenyl]methyl]-argininamide,
(13) (R)-N-[(4-chlorophenyl)methyl]-N²-(diphenylacetyl)argininamide,
(14) (R)-N²-(diphenylacetyl)-N-[[4-[(methylaminocarbonyl)-amino]phenyl]methyl]argininamide,
(15) (R)-N-[(4-amino-3,5-dichlorophenyl)methyl]-N²-(diphenylacetyl)-argininamide,
(16) (R)-N-[(4-amino-3,5-dichlorcphenyl)methyl]-N²-(3,3-diphenyl-1-oxopropyl)-argininamide,
(17) (R)-N-[(4-amino-3,5-dichlorophenyl)methyl]-N²-(3,4-dichlorobenzoyl)-argininamide,
(18) N²-(diphenylacetyl)-N-[3-[1-[2-[5,11-dihydro-6(6H)-oxopyrido[2,3-b][1,4]benzodiazepin-5-yl]-2-oxoethyl]-4-piperidinyl]-propyl]-argininamide,
(19) N²-(diphenylacetyl)-N-[(4-hydroxy-3-methoxyphenyl)methyl]-argininamide,
(20) N²-(diphenylacetyl)-N-[(3-hydroxy-4-methoxyphenyl)methyl]-argininamide,
(21) (R,S)-N-[(4-amino-3,5-dibromophenyl)methyl]-N⁶-(aminoiminomethyl)-N²-(diphenylacetyl)-lysinamide,
(22) N-[(3,5-dimethyl-4-hydroxyphenyl)methyl]-N²-(diphenylacetyl)-argininamide,
(23) N-[(1H-benzimidazol-5-yl)methyl]-N²-(diphenylacetyl)-argininamide
(24) N²-(diphenylacetyl)-N-[(4-hydroxy-3-methylphenyl)methyl]-argininamide
(25) N-[[4-(aminocarbonyl)phenyl]methyl]-N²-(diphenylacetyl)-argininamide
(26) (R,S)-N⁶-(aminoiminomethyl)-N²-(diphenylacetyl)-N-[(4-hydroxyphenyl)methyl]-lysinamide,
(27) (R)-N-[(4-hydroxyphenyl)methyl]-N²-(phenylacetyl)argininamide
(28) N²-(diphenylacetyl)-N-[(1H-indol-5-yl)methyl]argininamide,
(29) (R)-N-[[4-(aminosulphonyl)phenyl]methyl]-N²-(diphenylacetyl)-argininamide,
(30) (R)-N²-(diphenylacetyl)-N-[[4-(methoxycarbonyl)phenyl]-methyl]-argininamide,
(31) (R)-N²-(diphenylacetyl)-N-[(4-pyridinyl)methyl]argininamide,
(32) (R)-N-[(4-amino-3,5-dibromophenyl)methyl]-N²-(diphenylacetyl)-argininamide,
(33) (R)-N²-(diphenylacetyl)-N-[(2-thienyl)methyl]argininamide,
(34) (R)-N-[(4-amino-3,5-dichlorophenyl)methyl]-N²-(2-naphthoyl)-argininamide,
(35) (R,S)-N⁵-(4,5-dihydro-1H-imidazol-2-yl)-N²-(diphenylacetyl)-N-[(4-hydroxyphenyl)methyl]ornithinamide,
(36) (R,S)-N²-(diphenylacetyl)-N-[(4-hydroxyphenyl)methyl]-N⁵-(1H-imidazol-2-yl)ornithinamide,
(37) (R)-N-[[3-[(1 2-dihydro-3,5(4H)-dioxo-1,2-diphenyl-3H-1,2,4-triazol-4-yl)methyl]phenyl]methyl]-N²-(diphenylacetyl)-argininamide,
(38) N²-(diphenylacetyl)-N-[(3-hydroxyphenyl)methyl]argininamide,
(39) (R)-N-[[3-[(4,5-dihydro-2,4(3H)-dioxo-5,5-diphenyl-1H-imidazol-3-yl)methyl]phenyl]methyl]-N²-(diphenylacetyl)-argininamide,
(40) (R)-N²-(diphenylacetyl)-N-[(4-hydroxyphenyl)methyl]argininamide,
(41) N²-(diphenylacetyl)-N-[2-(4-hydroxyphenyl)ethyl]argininamide,
(42) N²-(diphenylacetyl)-N-[(4'-hydroxy-[1,1'-biphenyl]4-yl) methyl]-argininamide,
(43) N-[[4-[(1,2-dihydro-3,5(4H)-dioxo-1,2-diphenyl-3H-1,2,4-triazol-4-yl)methyl]phenyl]methyl]-N²-(diphenylacetyl)-argininamide,
(44) N²-(diphenylacetyl)-N-[(4-methoxyphenyl)methyl]argininamide,
(45) N²-(diphenylacetyl)-N-[2-(4-methoxyphenyl)ethyl]argininamide,
(46) N²-(diphenylacetyl)-N-[2-(3-methoxyphenyl)ethyl]argininamide,
(47) N²-(diphenylacetyl)-N-[(3-methoxyphenyl)methyl]argininamide,
(48) (R,S)-3-[4-(aminoiminomethyl)phenyl]-N²-(diphenylacetyl)-N-[(4-hydroxyphenyl)methyl]alaninamide,
(49) (R,S)-3-[3-(aminoiminomethyl)phenyl]-N²-(diphenylacetyl)-N-[(4-methoxyphenyl)methyl]alaninamide,
(50) (R,S)-3-[3-(aminoiminomethyl)phenyl]-N²-(diphenylacetyl)-N-[(4-hydroxyphenyl)methyl]alaninamide,
(51) (R)-N²-[bis-(4-bromophenyl)acetyl]-N-[(4-hydroxyphenyl)methyl]-argininamide,
(52) (R)-N²-(diphenylacetyl)-N-[(4-ethoxyphenyl)methyl]-argininamide,
(53) (R)-N²-(diphenylacetyl)-N-[(4-hydroxyphenyl)methyl]N-methyl-argininamide,
(54) (R,S)-N-[(4-amino-3,5-dibromophenyl)methyl]-3-[3-(aminoiminomethyl)phenyl]-N²-(diphenylacetyl)alaninamide,
(55) (R)-N-[4-[(dimethylamino)sulphonyloxy]phenyl]methyl]-N²-(diphenylacetyl)-argininamide,
(56) (R)-N-[(4-hydroxyphenyl)methyl]-N²-(1-naphthoyl)argininamide,
(57) (R)-N-[(4-hydroxyphenyl)methyl]-N²-(2-naphthoyl)argininamide,
(57) (R)-N²-(2,2-diphenyl-2-hydroxyacetyl)-N-[(4-hydroxyphenyl)-methyl]-argininamide,
(58) (R,S)-N²-(diphenylacetyl)-N⁵-(1H-imidazol-2-yl)-N-[(4-methoxyphenyl)methyl]-ornithinamide,
(59) (R,S)-3-[3-(aminoiminomethyl)phenyl]-N²-[[(diphenylmethyl)-amino]carbonyl]-N-[(4-hydroxyphenyl)methyl]-alaninamide,
(60) (R,S)-N²-(diphenylacetyl)-N⁵-(1H-imidazol-2-yl)-N-(phenylmethyl)-ornithinamide,
(61) (R,S)-N-[(4-amino-3,5-dichlorophenyl)methyl]-N²-(diphenylacetyl)-N⁵-(1H-imidazol-2-yl)-ornithinamide,
(62) (R,S)-N-[(4-hydroxyphenyl)methyl]-N⁵-(1H-imidazol-2-yl)-N²-(2-naphthoyl)-ornithinamide,
(63) (R,S)-N²-[[(diphenylmethyl)amino]carbonyl]-N-[(4-hydroxyphenyl)methyl]-N⁵-(1H-imidazol-2-yl)ornithinamide,
(64) (R,S)-N-[(4-hydroxyphenyl)methyl]-N⁵-(1H-imidazol-2-yl)-N²-[(2-naphthyl)acetyl]-ornithinamide,
(65) (R,S)-N-[(4-hydroxyphenyl)methyl]-N⁵-(1H-imidazol-2-yl)-N²-[[(2-naphthyl)amino]carbonyl]-ornithinamide,
(66) (R,S)-N-[(4-hydroxyphenyl)methyl]-N⁵-(1H-imidazol-2-yl)-N²-[(1,2,3,4-tetrahydroquinolin-3-yl)carbonyl]ornithinamide,
(67) (R,S)-N-[(4-hydroxyphenyl)methyl]-N⁵-(1H-imidazol-2-yl)-N²-[(1,2,3,4-tetrahydroquinolin-2-yl)carbonyl]ornithinamide,
(68) (R)-N-[(4-aminosulphonylaminophenyl)methyl]-N²-(diphenylacetyl)-argininamide,
(69) (R)-N-[(4-aminophenyl)methyl]-N²-(diphenylacetyl)argininamide,
(70) (R)-N-[(6-quinolinyl)methyl]-N²-(diphenylacetyl)argininamide,
(71) (R)-N²-[(3,4-dichlorophenyl)acetyl]-N-[(4-hydroxyphenyl)methyl]-argininamide,
(72) (R)-N²-(diphenylacetyl)-N-[[4-(2-hydroxyethyl)phenyl]-methyl]-argininamide,
(73) (R,S)-N⁵-(3-amino-1,2,4-oxadiazol-5-yl)-N²-(diphenylacetyl)-N-[(4-hydroxyphenyl)methyl]ornithinamide,
(74) (R)-N²-[(9-fluorenyl)carbonyl]-N-[(4-hydroxyphenyl)methyl]-argininamide,
(75) (R,S)-6-(aminoiminomethyl)-N²-(diphenylacetyl)-N-[(4-hydroxyphenyl)methyl]-norleucinamide
(76) (R,S)-3-[4-(4,5-dihydro-1H-imidazol-2-yl)phenyl]-N²-(diphenylacetyl)-N-[(4-hydroxyphenyl)methyl]alaninamide,
(77) (R,S)-3-[3-(aminoiminomethyl)phenyl]-N²-(diphenylacetyl)-N-[(4-ethoxycarbonyloxyphenyl)methyl]-alaninamide,
(78) (R,S)-N-[2-(1,2-dihydro-1,2-diphenyl-3,5(4H)-dioxo-1,2,4-triazol-4-yl)ethyl]-N²-(diphenylacetyl)argininamide,
(79) (R,S)-N²-(diphenylacetyl)-N-[(4-hydroxyphenyl)methyl]-2-methyl-argininamide,
(80) (R)-N²-(diphenylacetyl)-N-[[4-(3-hydroxypropyl)phenyl]-methyl]-argininamide
(81) (R)-N-[[4-[(4,5-dihydro-5,5-dimethyl-2,4(3H)-dioxo-1H-imidazol-3-yl)methyl]phenyl]methyl]-N²-(diphenylacetyl)-argininamide
and the salts thereof.

4. Physiologically acceptable salts of the compounds of general formula I according to claims 1 to 6 with the exclusion of those compounds in which T-Z denotes a hydrogen atom, with inorganic or organic acids or bases.

5. Pharmaceutical compositions containing as active substance a compound of general formula I according to claims 1 to 3 with the exclusion of those compounds in which T-Z denotes a hydrogen atom, or a physiologically acceptable salt thereof according to claim 4, optionally together with one or more inert carriers and/or diluents.

6. Use of a compound of general formula I according to claims 1 to 4 or compounds of formula I in which
the R-(CH₂)ₙ- group denotes a C₁₋₃-alkyl group or a phenyl group,
R¹ and R³ each denote a hydrogen atom,
R² denotes an amidinophenylmethyl group,
T-Z denotes a 4-amino-3,5-dichlorophenylsulphonyl group or a naphthylsulphonyl group and
Y denotes an imino group,
with the exception of those compounds wherein T-Z denotes a hydrogen atom, for preparing a pharmaceutical composition which is suitable for treating cardiovascular diseases, chronic heart insufficiency, coronary heart diseases, sub-arachnoidal bleeding, chronic kidney failure, tumour diseases, over-active thyroid, obesity and diabetes.

7. Process for preparing a pharmaceutical composition according to claim 5, characterised in that a compound of general formula I according to claims 1 to 4 with the exclusion of those compounds in which T-Z denotes a hydrogen atom, is incorporated in one or more inert conventional carriers and/or diluents by a non-chemical method.

8. Process for preparing the new amino acid derivatives of general formula I according to claims 1 to 4 and 9, characterised in that:
a) in order to prepare compounds of general formula I wherein T-Z has the meanings given in claims 1 to 3 with the exception of a hydrogen atom:
a compound of general formula II
T^{A} - Z - X (II)
(wherein
T^{A}-Z has the meanings given for T-Z in claims 1 to 3 with the exception of a hydrogen atom and
X denotes a hydroxy group, a halogen atom, a C₁₋₁₀-alkylsulphonyloxy group, a phenylsulphonyloxy or naphthylsulphonyloxy group optionally mono-, di- or trisubstituted by chlorine or bromine atoms or by methyl or nitro groups, wherein the substituents may be identical or different)
is coupled with an α-amino acid derivative of general formula III
H - NR¹ - (R^{2a}CR³) - CO - Y - (CH₂)ₙ-R (III)
wherein
R-(CH₂)ₙ, R¹, R³ and Y are as defined in claims 1 to 3 and R^{2a} has the meanings given for R² in claims 1 to 3 or denotes a group R² substituted by a protecting group, or denotes a precursor group for the group R²,
and, if necessary, any protecting group used is subsequently cleaved or any precursor function used is modified, or
b) in order to prepare compounds of general formula I wherein T-Z has the meanings given in claims 1 to 3 with the exception of a hydrogen atom:
a compound of general formula IV
T^{A} - Z - NR¹ - (R^{2a}CR³) - COOH (IV)
(wherein
R¹ and R³ are defined as in claims 1 to 3, T^{A}-Z has the meanings given for T-Z in claims 1 to 3 with the exception of a hydrogen atom and
R^{2a} has the meanings given for R² in claims 1 to 3 or denotes a group R² substituted by a protecting group, or denotes a precursor group for the group R²,
is coupled with a compound of general formula V
H - Y - (CH₂)ₙ - R (V)
wherein
R-(CH₂)ₙ and Y have the meanings given in claims 1 to 3, and subsequently, if necessary, any protecting group used is cleaved or any precursor function used is modified, or
c) in order to prepare compounds of general formula I wherein T-Z has the meanings given for T-Z in claims 1 to 3 with the exception of the hydrogen atom and R² denotes a straight-chained C₁₋₅-alkyl group which is substituted in the ω position by a 4,5-dihydro-1H-imidazol-2-ylamino group or a guanidino group wherein the hydrogen atoms at the nitrogen atoms may each be replaced by C₁₋₃-alkyl groups,
a compound of general formula VI
T^{A} - Z - NR¹ - (R²CR³) - CO - Y- (CH₂)ₙ - R (VI)
(wherein
R-(CH₂)ₙ, R¹ to R³ and Y are defined as in claims 1 to 3, T^{A}-Z has the meanings given for T-Z in claims 1 to 3 with the exception of the hydrogen atom and R^{2b} denotes a straight-chained C₁₋₅-alkyl group, wherein the alkyl group is substituted in the ω position by an R⁶NH group and R⁶ denotes a hydrogen atom or a C₁₋₃-alkyl group,
is reacted with a carbonic acid derivative of general formula VII
X¹ - (C=NR⁷) - (R⁸NR⁹) (VII)
wherein
R⁷, R⁸ and R⁹, which may be identical or different, denote hydrogen atoms or C₁₋₃-alkyl groups or R⁷ and R⁸ together may also denote an n-alkylene group having 2 carbon atoms and
X¹ denotes a leaving group, or
d) in order to prepare compounds of general formula I wherein T-Z has the meanings given for T-Z in claims 1 to 3 with the exception of the hydrogen atom and R² denotes a straight-chained C₁₋₅-alkyl group, wherein the alkyl group in the ω-position is substituted by an R^{8a}R⁹N-C(=NH)-NR⁶- group and R⁶, R^{8a} and R⁹, which may be identical or different, denote hydrogen atoms or C₁₋₃-alkyl groups:
a compound of general formula VI
T^{A} - Z - NR¹ - (R^{2b}CR³) - CO - Y - (CH₂)ₙ - R (VI)
(wherein
R-(CH₂)ₙ, R¹, R³ and Y are defined as in claims 1 to 3, T^{A}-Z has the meanings given for T-Z in claims 1 to 3, with the exception of the hydrogen atom, and R^{2b} denotes a straight-chained C₁₋₅-alkyl group, wherein the alkyl group in the ω-position is substituted by an R⁶NH- group and R⁶ denotes a hydrogen atom or a C₁₋₃-alkyl group)
is reacted with a cyanamide of general formula VIII
R^{8a}R⁹N - CN (VIII)
wherein
R^{8a} and R⁹, which may be identical or different, each denote hydrogen atoms or C₁₋₃-alkyl groups, or
e) in order to prepare compounds of general formula I wherein T-Z has the meanings given for T-Z in claims 1 to 3 with the exception of the hydrogen atom and R² denotes a straight-chained C₁₋₅-alkyl group, wherein the alkyl group in the ω-position is substituted by an HR⁸N-C(=NR⁷)-NR⁶- group and R⁶, R⁷ and R⁸, which may be identical or different, denote hydrogen atoms or C₁₋₃-alkyl groups:
a compound of general formula VI
T^{A} - Z - NR¹ - (R^{2b}CR³) CO - Y - (CH₂)ₙ - R (VI)
(wherein
R-(CH₂)ₙ, R¹, R³and Y are defined as in claims 1 to 3, T^{A}-Z has the meanings given for T-Z in claims 1 to 3, with the exception of the hydrogen atom, and R^{2b} denotes a straight-chained C₁₋₅-alkyl group, wherein the alkyl group in the ω-position is substituted by an R⁶NH- group and R⁶ denotes a hydrogen atom or a C₁₋₃-alkyl group)
is reacted with a carbodiimide of general formula IX
R⁷ - N = C = N - R⁸ (IX)
wherein
R⁷ and R⁸, which may be identical or different, denote hydrogen atoms or C₁₋₃-alkyl groups, or
f) in order to prepare compounds of general formula I wherein T-Z has the meanings given for T-Z in claims 1 to 3 with the exception of the hydrogen atom and R² denotes an amidinophenylmethyl group:
a compound of general formula X
T^{A} - Z - NR¹ - (R^{2c}CR³) - CO - Y - (CH₂)ₙ - R (X)
(wherein
R-(CH₂)ₙ, R¹, R³ and Y are defined as in claims 1 to 3, T^{A}-Z has the meanings given for T-Z in claims 1 to 3, with the exception of the hydrogen atom, and R^{2c} denotes a cyanophenylmethyl group,
is reacted with an alcohol of general formula XI
R⁵ - OH (XI)
wherein R⁵ denotes a C₁₋₃-alkyl group and is then treated with ammonia, or
g) in order to prepare compounds of general formula I wherein T-Z has the meanings given for T-Z in claims 1 to 3 and R² denotes an amidinophenylmethyl group,
a compound of general formula XII
T^{A} - Z - NR¹ - (R^{2d}CR³) - CO - Y - (CH₂)ₙ - R (XII)
(wherein
R-(CH₂)ₙ, R¹, R³ and Y are defined as in claims 1 to 3, T^{A}-Z has the meanings given for T-Z in claims 1 to 3, with the exception of the hydrogen atom, and R^{2d} denotes a phenylmethyl group substituted by an H₂N-C(=NOH)-group) is hydrogenolysed, or
h) in order to prepare compounds of general formula I wherein T-Z has the meanings given for T-Z in claims 1 to 3 and R² denotes an amidinophenylmethyl group,
a nitrile of general formula X
T^{A} - Z - NR¹ - (R^{2c}CR³) - CO - Y - (CH₂)ₙ - R (X)
(wherein
R-(CH₂)ₙ, R¹, R³ and Y are defined as in claims 1 to 3, T^{A}-Z has the meanings given in claims 1 to 3, with the exception of the hydrogen atom, and R^{2c} denotes a cyanophenylmethyl group,
is converted into a thioamide of general formula XIII
T^{A} - Z - NR¹ - (R^{2c'}CR³) - CO - Y - (CH₂)ₙ - R (XIII)
(wherein
R-(CH₂)ₙ, R¹, R³, Y and T^{A}-Z are as hereinbefore defined and R^{2c'} denotes a phenylmethyl group substituted by an aminothiocarbonyl group) and
subsequently alkylated with a compound of general formula XIV
R⁵-X² (XIV)
wherein
R⁵ denotes a C₁₋₃-alkyl group and
X² denotes a leaving group,
or with a trialkyloxoniumtetrafluoroborate of general formula XV
(R⁵)₃OBF₄ (XV)
(wherein
R⁵ denotes a C₁₋₃-alkyl group), and
subsequently reacted with ammonia, or
i) in order to prepare compounds of general formula I wherein T-Z has the meanings given for T-Z in claims 1 to 3 with the exception of the hydrogen atom and R² denotes a cyaniminomethylaminophenylmethyl group,
a compound of general formula VI
T^{A} - Z - NR¹ - (R^{2b}CR³) - CO - Y - (CH₂)ₙ - R (VI)
(wherein
R-(CH₂)ₙ, R¹, R³ and Y are defined as in claims 1 to 3, T^{A}-Z has the meanings given in claims 1 to 3 with the exception of the hydrogen atom and R^{2b} denotes an aminophenylmethyl group,
is reacted with an N-cyano-formimidic acid ester of general formula XVI
R¹³O-CH=N-CN (XVI)
wherein
R¹³ denotes a C₁₋₁₀-alkyl group, or
j) in order to prepare compounds of general formula I wherein T-Z has the meanings given for T-Z in claims 1 to 3 with the exception of the hydrogen atom and R² denotes a straight-chained C₁₋₅-alkyl group substituted in the ω-position by an aminomethylideneimino or methylaminomethylideneimino group, or a methylaminomethylideneiminophenylmethyl group,
a compound of general formula XVII
T^{A} - Z - NR¹ - (R^{2d'}CR³) - CO - Y - (CH₂)ₙ - R (XVII)
(wherein
R-(CH₂)ₙ, R¹, R³ and Y are defined as in claims 1 to 3, T^{A}-Z has the meanings given for T-Z in claims 1 to 3 with the exception of the hydrogen atom and R^{2d'} denotes a straight-chained C₁₋₅-alkyl group or a phenylmethyl group, wherein the alkyl group in the ω-position and the above-mentioned aromatic group are each substituted by an NC-N=CH-NH- group)
is reacted with an amine of general formula XVIII
H₂NR^{8a} (XVIII)
wherein
R^{8a} denotes a hydrogen atom or a methyl group, or
k) in order to prepare compounds of general formula I wherein T-Z denotes a diphenylaminocarbonyl, naphthylaminocarbonyl, hexamethyleneaminocarbonyl, 1-piperidinyl or (diphenylmethyl)aminocarbonyl group,
an isocyanate of general formula XIX
O=C=N - (R^{2a}CR³)-CO-NR⁴-(CH₂)ₙ-R (XIX)
(wherein
R-(CH₂)ₙ, R³ and R⁴ are defined as in claims 1 to 3 and R^{2a} has the meanings given for R² in claims 1 to 3 or denotes a group R² substituted by protecting groups or denotes a precursor group for the group R²,
is reacted with an amine of general formula XX
T¹T²N-H (XX)
wherein
T¹ and T² each denote a phenyl group or
T¹ denotes a hydrogen atom and T² denotes a naphthyl or diphenylmethyl group or
T¹ and T² together denote an n-alkylene group with 5 or 6 carbon atoms,
or
1) in order to prepare compounds of general formula I wherein T-Z denotes a hydrogen atom,
the group R¹⁴ is cleaved from a compound of general formula XXI,
R¹⁴ -NR¹ - (R²CR³) - CO - Y - (CH₂)ₙ-R (XXI)
(wherein
R-(CH₂)ₙ, R¹, R², R³ and Y are defined as in claims 1 to 3 and
R¹⁴ denotes a tert.butoxycarbonyl or 9-fluorenylmethoxycarbonyl group), or
m) in order to prepare compounds of general formula I wherein a hydrogen atom of an HN<, HN= or H₂N- group present in the group R² is replaced by a tert. butoxycarbonyl group,
a compound of general formula I
T - Z - NR¹ - (R²CR³) - CO - Y - (CH₂)ₙ - R (I)
(wherein
R-(CH₂)ₙ, R¹ to R³, T-Z and Y are defined as in claims 1 to 3, with the proviso that R² must contain at least one free HN<, HN= or H₂N- group) is reacted with a compound of general formula XXII
X³ - W (XXII)
wherein
W denotes a tert. butoxycarbonyl group and
X³ denotes a leaving group, or
n) in order to prepare compounds of general formula I wherein T-Z denotes a naphthylaminocarbonyl or (diphenylmethyl)aminocarbonyl group: an isocyanate of general formula XXIII
T'N=C=O (XXIII)
wherein
T' denotes a naphthyl or diphenylmethyl group,
is reacted with a compound of general formula III
H - NR¹ - (R^{2a}CR³) - CO - Y - (CH₂)ₙ-R (III)
wherein
R-(CH₂)ₙ, R¹, R³ and Y are defined as in claims 1 to 3 and R^{2a} has the meanings given for R² in claims 1 to 3 or denotes a group R² substituted by protecting groups or denotes a precursor group for the group R², and
subsequently, if desired, a compound of general formula I thus obtained is separated into its diastereomers and/or
a racemate of a compound of general formula I thus obtained is resolved into its enantiomers and/or
a compound of general formula I thus obtained is converted into the salts thereof with inorganic acids or bases, particularly for pharmaceutical use into the physiologically acceptable salts thereof.

9. D- and (R)-enantiomers of the compounds according to claims 1 to 3.

## Revendications

1. Dérivés d'aminoacides de formule générale
T - Z - NR¹ - (R²CR³) - CO - Y - (CH₂)ₙ - R (I)
où
le groupe R-(CH₂)ₙ- représente un groupe alkyle de 1 à 3 atomes de carbone qui peut être substitué en position ω par 2 groupes phényle,
un groupe alkylène linéaire de 2 à 5 atomes de carbone qui est substitué en position terminale par un groupe hydroxyle, un groupe phényle ou phénylalkyle de 1 à 4 atomes de carbone dans la partie alkyle où le groupe phényle peut être substitué dans chaque cas par un atome de fluor, de chlore ou de brome, par un groupe alkylaminocarbonyle de 1 à 4 atomes de carbone dans la partie alkyle, par un groupe éthylaminocarbonylamino dans lequel la partie éthyle est substituée par un ou deux restes phényle, par un groupe méthyle, hydroxyméthyle, phényle, hydroxyphényle, hydroxyle, méthoxy, éthoxy, diméthylaminosulfonyloxy, cyano, carboxyle, méthoxycarbonyle, acétyle, aminocarbonyle, diméthylaminocarbonyle, amino, méthylamino, diméthylamino, acétylamino, méthoxycarbonylamino, éthoxycarbonylamino, aminocarbonylamino, méthylaminocarbonylamino, diméthylaminocarbonylamino, méthylsulfonylamino, aminosulfonyle, [amino(imino)méthyle], [amino(imino)méthyl]amino, [1,2-dihydro-3,5(4H)-dioxo-1,2-diphényl-3H-1,2,4-triazol-4-yl]méthyle, [1,5-dihydro-2,4(3H)-dioxo-5,5-diphényl-imidazol-3-yl]-méthyle, aminométhyle, aminosulfonylamino, hydroxyéthyle, hydroxypropyle, hydroxybutyle, 3-(diméthylamino)propoxy ou éthoxycarbonyloxy,
un groupe méthyle qui est substitué par un groupe hydroxycyclohexyle, 4-hydroxy-3-méthylphényle, 3,4-diméthoxyphényle, 3-hydroxy-4-méthoxyphényle, 3-méthoxy-4-hydroxyphényle, 4-amino-3,5-dibromophényle, 4-amino-3,5-dichlorophényle, 4-hydroxy-3,5-dibromophényle, 4-hydroxy-3,5-dichlorophényle, 4-amino-3-fluorophényle, 4-hydroxy-3,5-diméthylphényle, thiényle, pyridinyle, indolyle, benzimadazolyle, quinolinyle ou 2-(diphénylaminocarbonyl)-2,3-dihydro-1H-isoindolyle, un groupe éthyle qui est substitué par un groupe indolyle, 5-méthoxyindolyle, 1,2-diphényl-3,5(4H)-dioxo-1,2,4-triazol-4-yle ou imidazolyle,
un groupe 2-(2-hydroxyphényl)éthyle qui est substitué en position α par un groupe aminocarbonyle,
un groupe 3-[1-[2-[5,11-dihydro-6(6H)-oxopyrido[2,3-b][1,4]-benzodiazépin-5-yl]-2-oxoéthyl]-4-pipéridinyl]propyle ou 3-hydroxy-1-propyn-1-yle,
le groupe T-Z représente un atome d'hydrogène, un groupe carbonyle qui est substitué par un groupe alkyle de 1 à 4 atomes de carbone qui peut être substitué en position α ou β par un groupe alkyle de 1 à 3 atomes de carbone, par un ou deux restes phényle éventuellement mono-ou disubstitués par des groupes méthoxy, des groupes hydroxyle, des atomes de chlore ou de brome,
un groupe carbonyle qui est substitué par un groupe indolyle substitué par un groupe benzyloxy ou phényléthoxy, par un groupe méthyle, tricyclo[3.3.1.1^{3.7}]déc-1-yle, benzyle, (hydroxyphényl)méthyle, (méthylphényl)méthyle, (biphénylyl)méthyle, (dichlorophényl)méthyle, phényle, 3,5-dichlorophényle, 3,4-dichlorophényle, naphtyle, naphtylméthyle, α-cyclopentylbenzyle, diphénylamino, naphtylamino, hexaméthylènimino, 5-phényléthoxy-indolyle, 1,2,3,4-tétrahydro-2-quinolinyle, 2-quinolinyle, 1,2,3,4-tétrahydro-3-quinolinyle, (dichlorophénoxy)méthyle, 9-fluorényle, triphénylméthyle, 1-pipéridinyle, (diphénylméthyl)amino ou 5,10-dihydro-11(11H)-oxo-dibenzo[b,e]-[1,4]diazépin-5-yle, ou
un groupe 4-amino-3,5-dichlorophénylsulfonyle OU naphtylsulfonyle,
R¹ représente l'atome d'hydrogène,
R² représente un reste alkyle non ramifié de 1 à 5 atomes de carbone qui est substitué en position ω par un groupe guanidino dans lequel les atomes d'hydrogène sur les atomes d'azote peuvent être remplacés à chaque fois par des groupes alkyle de 1 à 3 atomes de carbone, par un groupe amino, tert.butoxycarbonylamino, diméthylamino, N-méthylbenzylamino, méthylamino, aminocarbonyle, aminocarbonylamino, aminométhylidènimino, méthylaminométhylidènimino, [amino(nitroimino)méthyl]amino, 1H-imidazol-2-yl-amino, 4,5-dihydro-1H-imidazol-2-yl-amino, (5-amino-4H-1,2,4-triazol-3-yl)amino, (3-amino-1,2,4-oxadiazol-5-yl)amino ou (5-amino-1,2,4-oxadiazol-3-yl) amino,
un groupe méthyle qui est substitué par un groupe phényle, cyanophényle, aminométhylphényle, amidinophényle, méthylaminométhylidèniminophényle, cyaniminométhylaminophényle, méthyliminométhylaminophényle, (4,5-dihydro-1H-imidazol-2-yl)phényle ou imidazolyle,
R³ représente l'atome d'hydrogène ou le groupe méthyle et
Y représente un groupe imino éventuellement substitué par un groupe méthyle ou éthyle, avec les conditions que
(i)
R¹ ne représente pas un atome d'hydrogène quand le groupe R-(CH₂)ₙ- représente un groupe alkyle de 1 à 3 atomes de carbone ou un groupe phényle,
R² représente un groupe amidinophénylméthyle,
R³ représente un atome d'hydrogène,
T-Z représente un groupe 4-amino-3,5-dichlorophénylsulfonyle
ou le groupe naphtylsulfonyle et
Y représente un groupe imino, et
(ii)
R² ne représente pas un reste alkyle non ramifié de 3 ou 4 atomes de carbone qui est substitué en position ω par un groupe guanidino dans lequel les atomes d'hydrogène sur les atomes d'azote peuvent être remplacés à chaque fois par un groupe alkyle de 1 à 3 atomes de carbone, par un groupe amino, tert.butoxycarbonylamino, diméthylamino, N-méthylbenzylamino, méthylamino, aminocarbonyle ou aminocarbonylamino, quand
R¹, R³ et T-Z représentent chacun un atome d'hydrogène et Y représente un groupe imino éventuellement substitué par un groupe méthyle ou éthyle, et
(iii)
R² ne représente pas un groupe phénylméhyle quand R¹, R³ et T-Z représentent chacun un atome d'hydrogène,
Y représente le groupe imino et
le groupe R-(CH₂)ₙ- représente un groupe alkyle de 1 à 3 atomes de carbone ou un groupe phénylalkyle de 1 à 4 atomes de carbone dans la partie alkyle,
et les composés suivants:
(1) (R)-N-[[4-[(4,5-dihydro-5,5-diméthyl-2,4(3H)-dioxo-1H-imidazol-3-yl)méthyl]phényl)méthyl]-N²-(diphénylacétyl)argininamide,
(2) (R,S)-N²-(diphénylacétyl)-N-[(4-hydroxyphényl)méthyl]-3-[(4-pipéridinyl)méthyl]-alaninamide,
(3) (R,S)-3-[3-[amino(hydroxyimino)méthyl]phényl]-N²-(diphénylacétyl)-N-[(4-hydroxyphényl)méthyl]alaninamide,
(4) (R,S)-3-[3-(aminocarbonyl)phényl]-N²-(diphénylacétyl)-N-[(4-hydroxyphényl)méthyl]alaninamide,
(5) (R)-N²-(2,2-diphényléthyl)-N-[(4-hydroxyphényl)méthyl]argininamide,
(6) (R,S)-3-[(3-(aminoiminométhyl)phényl]-N²-(2,2-diphényléthyl)-N-[(4-hydroxyphényl)méthyl]-alaninamide,
(7) (R)-N-[(4-hydroxyphényl)méthyl]-N²-(5-phényl-1-oxopentyl)argininamide,
(8) (R)-N-[(4-hydroxyphényl)méthyl]-N²-(4-phényl-1-oxobutyl)argininamide,
(9) (R)-N-[(4-hydroxyphényl)méthyl]-N²-(6-phényl-1-oxohexyl)argininamide,
(10) (R,S)-N²-(2,2-diphényl-2-hydroxy-1-oxoéthyl)-N-[(4-hydroxyphényl)méthyl]-argininamide,
(11) N²-(diphénylacétyl)-N-[(4-(méthylamino)cyclohexyl)méthyl]argininamide,
(12) (R)-N-[(4-hydroxyphényl)méthyl]-N²-(tricyclo[3.3.1.1^{3.7}]déc-1-ylacéthyl)argininamide,
(13) (R)-N-[(4-hydroxyphényl)méthyl]-N²-[3-(1-pipéridinyl)-1-oxopropyl]argininamide,
(14) N²-(diphénylacétyl)-N-[3-[1-[2-[5,11-dihydro-6(6H)-oxopyrido[2,3-b][1,4]benzodiazépin-5-yl]-2-oxoéthyl]-4-pipéridinyl]propyl]argininamide,
(15) (R)-N²-[rac.-5,11-dihydro-6-oxo-6H-dibenz[b,e]azépin-11-yl)-carbonyl]-N-[(4-éthoxyphényl)méthyl]argininamide et
(16) N²-[N²-(diphénylacétyl)-D-arginyl]-L-tyrosinamide,
leurs tautomères, leurs diastéréoisomères, leurs énantiomères, leurs mélanges et leurs sels.

2. Dérivés d'aminoacides de formule générale I selon la revendication 1 où
le groupe R-(CH₂)ₙ- représente un groupe phénylméthyle où le groupe phényle est substitué dans chaque cas par un atome de fluor, de chlore ou de brome, par un groupe méthyle, hydroxyméthyle, hydroxyéthyle, hydroxypropyle, hydroxyle, méthoxy, éthoxy, diméthylaminosulfonyloxy, éthoxycarbonyloxy, acétyle, méthoxycarbonyle, aminocarbonyle, aminocarbonylamino, méthylaminocarbonylamino, aminosulfonyle, diméthylamino, [1,2-dihydro-3,5(4H)-dioxo1,2-diphényl-3H-1,2,4-triazol-4-yl]-méthyle ou [1,5-dihydro-2,4(3H)-dioxo-5,5-diphényl-imidazol-3-yl]-méthyle, un groupe méthyle qui est substitué par un groupe 3,4-diméthoxyphényle, 3-hydroxy-4-méthoxyphényle, 3-méthoxy-4-hydroxyphényle, 4-amino-3,5-dibromophényle, 4-amino-3,5-dichlorophényle, 4-hydroxy-3,5-diméthylphényle, 4'-hydroxy-4-biphénylyle, thiényle, pyridinyle, benzimidazolyle ou 1-[2-[5,11-dihydro-6(6H)-oxopyrido[2,3-b][1,4]benzodiazépin-5-yl]-2-oxoéthyl]-4-pipéridinyle] ou 4-amino-3-fluorophényle,
le groupe T-Z représente un groupe diphénylacétyle dans lequel le noyau phényle peut être substitué dans chaque cas par un atome de chlore ou de brome,
un groupe carbonyle qui est substitué par un groupe indolyle substitué par un groupe benzyloxy ou phényléthoxy, par un groupe benzyle, (dichlorophényl)méthyle, dichlorophényle, naphtyle, naphtylméthyle, α-cyclopentylbenzyle, 9-fluorényle ou (diphénylméthyl)amino,
R¹ représente l'atome d'hydrogène,
R² représente une chaîne alkylène linéaire de 2 à 5 atomes de carbone qui est substituée en position terminale par un groupe amino, amidino, guanidino, aminocarbonyle, aminocarbonylamino ou (1H-imidazol-2-yl)amino, ou
un groupe méthyle qui est substitué par un groupe (aminométhyl)phényle, amidinophényle ou (4,5-dihydro-1H-imidazol-2-yl)phényle,
R³ représente l'atome d'hydrogène ou le groupe méthyle et Y représente un groupe imino éventuellement substitué par un groupe méthyle,
leurs tautomères, leurs diastéréoisomères, leurs énantiomères, leurs mélanges et leurs sels.

3. Dérivés d'aminoacides de formule générale I selon la revendication 1 suivants:
(1) (R)-N²-(diphénylacétyl)-N-(phénylméthyl)argininamide,
(2) (R)-N²-(diphénylacétyl)-N-[(4-méthylphényl)méthyl]argininamide,
(3) (R)-N-[2-(4-hydroxyphényl)éthyl]-N²-[[5-(2-phényléthoxy)-1H-indol-2-yl)carbonyl]argininamide,
(4) N-[(4-aminocarbonylaminophényl)méthyl]-N²-(diphénylacétyl)argininamide,
(5) (R)-N-[(4-hydroxyphényl)méthyl]-N²-[[5-(2-phényléthoxy)-1H-indol-2-yl)carbonyl]argininamide,
(6) (R)-N²-(diphénylacétyl)-N-[4-fluorophényl)méthyl]argininamide,
(7) (R)-N-[(4-bromophényl)méthyl]-N²-(diphénylacétyl)argininamide,
(8) (R)-N²-(diphénylacétyl)-N-(2-phényléthyl)argininamide,
(9) mélange de diastéréoisomères optiquement actif de N²-(α-cyclopentyl-phénylacétyl)-N-[(4-hydroxyphényl)méthyl]-D-argininamide,
(10) N-[(4-(diméthylamino)phényl]méthyl]-N²-(diphénylacétyl)argininamide,
(11) (R)-N²-(diphénylacétyl)-N-[[4-(hydroxyméthyl)phényl]méthyl]argininamide,
(12) (R)-N²-(diphénylacétyl)-N-[[4-(1-oxoéthyl)phényl]méthyl]argininamide,
(13) (R)-N-[(4-chlorophényl)méthyl]-N²-(diphénylacétyl)argininamide,
(14) (R)-N²-(diphénylacétyl)-N-[[4-[(méthylaminocarbonyl)amino]phényl]méthyl]argininamide,
(15) (R)-N-[(4-amino-3,5-dichlorophényl)méthyl]-N²-(diphénylacétyl)argininamide,
(16) (R)-N-[(4-amino-3,5-dichlorophényl)méthyl]-N²-(3,3-diphényl-1-oxopropyl)argininamide,
(17) (R)-N-[(4-amino-3,5-dichlorophényl)méthyl]-N²-(3,4-dichlorobenzoyl)argininamide,
(18) N²-(diphénylacétyl)-N-[3-[1-[2-[5,11-dihydro-6(6H)-oxopyrido[2,3-b][1,4]benzodiazépin-5-yl]-2-oxoéthyl]-4-pipéridinyl]propyl]argininamide,
(19) N²-(diphénylacétyl)-N-[(4-hydroxy-3-méthoxyphényl)méthyl]argininamide,
(20) N²-(diphénylacétyl)-N-[(3-hydroxy-4-méthoxyphényl)méthyl]argininamide,
(21) (R,S)-N-[(4-amino-3,5-dibromophényl)méthyl]-N⁶-(aminoiminométhyl)-N²-(diphénylacétyl)lysinamide,
(22) N-[(3,5-diméthyl-4-hydroxyphényl)méthyl]-N²-(diphénylacétyl)argininamide,
(23) N-[(1H-benzimidazol-5-yl)méthyl]-N²-(diphénylacétyl)argininamide,
(24) N²-(diphénylacétyl)-N-[(4-hydroxy-3-méthylphényl)méthyl]argininamide,
(25) N-[[4-(aminocarbonyl)phényl]méthyl]-N²-(diphénylacétyl)argininamide,
(26) (R,S)-N⁶-(aminoiminométhyl)-N²-(diphénylacétyl)-N-[(4-hydroxyphényl)méthyl]lysinamide,
(27) (R)-N-[(4-hydroxyphényl)méthyl]-N²-(phénylacétyl)argininamide,
(28) N²-(diphénylacétyl)-N-[(1H-indol-5-yl)méthyl]argininamide,
(29) (R)-N-[[4-(aminosulfonyl)phényl]méthyl]-N²-(diphénylacétyl)-argininamide,
(30) (R)-N²-(diphénylacétyl)-N-[[4-(méthoxycarbonyl)phényl]méthyl]-argininamide,
(31) (R)-N²-(diphénylacétyl)-N-[(4-pyridinyl)méthyl]argininamide,
(32) (R)-N-[(4-amino-3,5-dibromophényl)méthyl]-N²-(diphénylacétyl)-argininamide,
(33) (R)-N²-(diphénylacétyl)-N-[(2-thiényl)méthyl]-argininamide,
(34) (R)-N-[(4-amino-3,5-dichlorophényl)méthyl]-N²-(2-naphtoyl)-argininamide,
(35) (R,S)-N⁵-(4,5-dihydro-1H-imidazol-2-yl)-N²-(diphénylacétyl)-N-[(4-hydroxyphényl)méthyl]-ornithinamide,
(36) (R,S)-N²-(diphénylacétyl)-N-[(4-hydroxyphényl)méthyl]-N⁵-(1H-imidazol-2-yl)-ornithinamide,
(37) (R)-N-[[3-[(1,2-dihydro-3,5(4H)-dioxo-1,2-diphényl-3H-1,2,4-triazol-4-yl)méthyl]phényl]méthyl]-N²-(diphénylacétyl)-argininamide,
(38) N²-(diphénylacétyl)-N-[(3-hydroxyphényl)méthyl]argininamide,
(39) (R)-N-[[3-[(4,5-dihydro-2,4(3H)-dioxo-5,5-diphényl-1H-imidazol-3-yl)méthyl]phényl]méthyl]-N²-(diphénylacétyl)argininamide,
(40) (R)-N²-(diphénylacétyl)-N-[(4-hydroxyphényl)méthyl]argininamide,
(41) N²-(diphénylacétyl)-N-[2-(4-hydroxyphényl)éthyl]argininamide,
(42) N²-(diphénylacétyl)-N-[(4'-hydroxy-[1,1'-biphényl]-4-yl)méthyl]-argininamide,
(43) N-[[4-[(1,2-dihydro-3,5(4H)-dioxo-1,2-diphényl-3H-1,2,4-triazol-4-yl)méthyl]phényl]méthyl]-N²-(diphénylacétyl)argininamide,
(44) N²-(diphénylacétyl)-N-[(4-méthoxyphényl)méthyl]-argininamide,
(45) N²-(diphénylacétyl)-N-[2-(4-méthoxyphényl)éthyl]-argininamide,
(46) N²-(diphénylacétyl)-N-[2-(3-méthoxyphényl)éthyl]argininamide,
(47) N²-(diphénylacétyl)-N-[(3-méthoxyphényl)méthyl]argininamide,
(48) (R,S)-3-[4-(aminoiminométhyl)phényl]-N²-(diphénylacétyl)-N-[(4-hydroxyphényl)méthyl]-alaninamide,
(49) (R,S)-3-[3-(aminoiminométhyl)phényl]-N²-(diphénylacétyl)-N-[(4-méthoxyphényl)méthyl]-alaninamide,
(50) (R,S)-3-[3-(aminoiminométhyl)phényl]-N²-(diphénylacétyl)-N-[(4-hydroxyphényl)méthyl]-alaninamide,
(51) (R)-N²-[bis-(4-bromophényl)acétyl]-N-[(4-hydroxyphényl)méthyl]-argininamide,
(52) (R)-N²-(diphénylacétyl)-N-[(4-éthoxyphényl)méthyl]argininamide,
(53) (R)-N²-(diphénylacétyl)-N-[(4-hydroxyphényl)méthyl]-N-méthyl-argininamide,
(54) (R,S)-N-[(4-amino-3,5-dibromophényl)méthyl]-3-[3-(aminoiminométhyl)phényl]-N²-(diphénylacétyl)-alaninamide,
(55) (R)-N-[[4-[(diméthylamino)sulfonyloxy]phényl]méthyl]-N²-(diphénylacétyl)-argininamide,
(56) (R)-N-[(4-hydroxyphényl)méthyl]-N²-(1-naphtoyl)argininamide,
(57) (R)-N-[(4-hydroxyphényl)méthyl]-N²-(2-naphtoyl)argininamide,
(57) (R)-N²-(2,2-diphényl-2-hydroxyacétyl)-N-[(4-hydroxyphényl)méthyl]argininamide,
(58) (R,S)-N²-(diphénylacétyl)-N⁵-(1H-imidazol-2-yl)-N-[(4-méthoxyphényl)méthyl]ornithinamide,
(59) (R,S)-3-[3-(aminoiminométhyl)phényl]-N²-[[(diphénylméthyl)amino]carbonyl]-N-[(4-hydroxyphényl)méthyl]alaninamide,
(60) (R,S)-N²-(diphénylacétyl)-N⁵-(1H-imidazol-2-yl)-N-(phénylméthyl)ornithinamide,
(61) (R,S)-N-[(4-amino-3,5-dichlorophényl)méthyl]-N²-(diphénylacétyl)-N⁵-(1H-imidazol-2-yl)-ornithinamide,
(62) (R,S)-N-[(4-hydroxyphényl)méthyl]-N⁵-(1H-imidazol-2-yl)-N²-(2-naphtoyl)-ornithinamide,
(63) (R,S)-N²-[[(diphénylméthyl)amino]carbonyl]-N-[(4-hydroxyphényl)méthyl]-N⁵-(1H-imidazol-2-yl)-ornithinamide,
(64) (R,S)-N-[(4-hydroxyphényl)méthyl]-N⁵-(1H-imidazol-2-yl)-N²-[(2-naphtyl)acétyl]-ornithinamide,
(65) (R,S)-N-[(4-hydroxyphényl)méthyl]-N⁵-(1H-imidazol-2-yl)-N²-[[(2-naphtyl)amino]carbonyl]-ornithinamide,
(66) (R,S)-N-[(4-hydroxyphényl)méthyl]-N⁵-(1H-imidazol-2-yl)-N²-[(1,2,3,4-tétrahydroquinolin-3-yl)carbonyl]ornithinamide,
(67) (R,S)-N-[(4-hydroxyphényl)méthyl]-N⁵-(1H-imidazol-2-yl)-N²-[(1,2,3,4-tétrahydroquinolin-2-yl)carbonyl]ornithinamide,
(68) (R)-N-[(4-aminosulfonylaminophényl)méthyl]-N²-(diphénylacétyl)-argininamide,
(69) (R)-N-[(4-aminophényl)méthyl]-N²-(diphénylacétyl)argininamide,
(70) (R)-N-[(6-quinolinyl)méthyl]-N²-(diphénylacétyl)argininamide,
(71) (R)-N²-[(3,4-dichlorophényl)acétyl]-N-[(4-hydroxyphényl)méthyl]-argininamide,
(72) (R)-N²-(diphénylacétyl)-N-[[4-(2-hydroxyéthyl)phényl]méthyl]-argininamide,
(73) (R,S)-N⁵-(3-amino-1,2,4-oxadiazol-5-yl)-N²-(diphénylacétyl)-N-[(4-hydroxyphényl)méthyl]-ornithinamide,
(74) (R)-N²-[(9-fluorényl)carbonyl]-N-[(4-hydroxyphényl)méthyl]-argininamide,
(75) (R,S)-6-(aminoiminométhyl)-N²-(diphénylacétyl)-N-[(4-hydroxyphényl)méthyl]-norleucinamide,
(76) (R,S)-3-[4-(4,5-dihydro-1H-imidazol-2-yl)phényl]-N²-(diphénylacétyl)-N-[(4-hydroxyphényl)méthyl]-alaninamide,
(77) (R,S)-3-[3-(aminoiminométhyl)phényl]-N²-(diphénylacétyl-N-[(4-éthoxycarbonyloxyphényl)méthyl]-alaninamide,
(78) (R,S)-N-[2-[1,2-dihydro-1,2-diphényl-3,5(4H)-dioxo-1,2,4-triazol-4-yl)éthyl]-N²-(diphénylacétyl)-argininamide,
(79) (R,S)-N²-(diphénylacétyl)-N-[(4-hydroxyphényl)méthyl]-2-méthyl-argininamide,
(80) (R)-N²-(diphénylacétyl)-N-[[4-(3-hydroxypropyl)phényl]méthyl]-argininamide,
(81) (R)-N-[[4-[(4,5-dihydro-5,5-diméthyl-2,4(3H)-dioxo-1H-imidazol-3-yl)méthyl]phényl]méthyl]-N²-(diphénylacétyl)argininamide
et leurs sels.

4. Sels physiologiquement acceptables des composés de formule générale I selon les revendications 1 à 3 à l'exception des composés dans lesquels T-Z représente un atome d'hydrogène, avec des acides ou des bases inorganiques ou organiques.

5. Médicament contenant comme principe actif un composé de formule générale I selon les revendications 1 à 3 à l'exception des composés dans lesquels T-Z représente un atome d'hydrogène, ou son sel physiologiquement acceptable selon la revendication 4 outre éventuellement un ou plusieurs supports et/ou diluants inertes.

6. Utilisation d'un composé de formule générale I selon les revendications 1 à 4 et de composés de formule I dans lesquels
le groupe R-(CH₂)ₙ- représente un groupe alkyle de 1 à 3 atomes de carbone ou un groupe phényle,
R¹ et R³ représentent chacun un atome d'hydrogène,
R² représente un groupe amidinophénylméthyle,
T-Z représente un groupe 4-amino-3,5-dichlorophénylsulfonyle ou le groupe naphtylsulfonyle et
Y représente un groupe imino,
à l'exception des composés dans lesquels T-Z représente un atome d'hydrogène, pour la préparation d'un médicament qui convient pour le traitement des maladies cardiovasculaires, de l'insuffisance cardiaque chronique, des maladies cardiaques coronariennes, des hémorragies sousarachnoïdiennes, de l'insuffisance rénale chronique, des maladies tumorales, de l'hyperthyroïdie, de l'obésité et du diabète.

7. Procédé de préparation d'un médicament selon la revendication 5 caractérisé en ce que, par voie non chimique, un composé de formule générale I selon les revendications 1 à 4 à l'exception des composés dans lesquels T-Z représente un atome d'hydrogène est incorporé dans un ou plusieurs supports et/ou diluants inertes courants.

8. Procédé de préparation des nouveaux dérivés d'aminoacides de formule générale I selon les revendications 1 à 4 et 9 caractérisé en ce que
a) pour la préparation de composés de formule générale I où T-Z possède les significations indiquées dans les revendications 1 à 3 à l'exception de celle d'un atome d'hydrogène, un composé de formule générale II
T^{A} - Z - X (II)
où
T^{A}-Z possède les significations indiquées dans les revendications 1 à 3 pour T-Z à l'exception de celle d'un atome d'hydrogène et X représente le groupe hydroxyle, un atome d'halogène, un groupe alkylsulfonyloxy de 1 à 10 atomes de carbone, un groupe phénylsulfonyloxy ou naphtylsulfonyloxy éventuellement mono-, di- ou trisubstitué par des atomes de chlore ou de brome, par des groupes méthyle ou nitro, les substituants pouvant être identiques ou différents,
est couplé avec un dérivé d'α-aminoacide de formule générale III
H - NR¹ - (R^{2a}CR³) - CO - Y - (CH₂)ₙ - R (III)
où
R-(CH₂)ₙ, R¹, R³ et Y sont définis comme dans les revendications 1 à 3 et
R^{2a} possède les significations indiquées pour R² dans les revendications 1 à 3 ou représente un reste R² substitué par un reste protecteur ou un reste précurseur pour le reste R², et, Si nécessaire, un groupe protecteur employé est ensuite clivé ou une fonction précurseur employée est ensuite convertie ou
b) pour la préparation de composés de formule générale I où T-Z possède les significations indiquées dans les revendications 1 à 3 à l'exception de celle d'un atome d'hydrogène, un composé de formule générale IV
T^{A} - Z - NR¹ - (R^{2a}CR³) - COOH (IV)
où
R¹ et R³ sont définis comme indiqué dans les revendications 1 à 3, T^{A}-Z possède les significations citées dans les revendications 1 à 3 pour T-Z à l'exception de celle d'un atome d'hydrogène et R^{2a} possède les significations indiquées pour R² dans les revendications 1 à 3 ou représente un reste R² substitué par un reste protecteur ou un reste précurseur pour le reste R²,
est couplé avec composé de formule générale V
H - Y - (CH₂)ₙ - R (V)
où
R-(CH₂)ₙ et Y possèdent les significations indiquées dans les revendications 1 à 3 et, si nécessaire, un groupe protecteur employé est ensuite clivé ou une fonction précurseur employée est ensuite convertie ou
c) pour la préparation de composés de formule générale I où T-Z possède les significations indiquées pour T-Z dans les revendications 1 à 3 à l'exception de l'atome d'hydrogène et R² représente un groupe alkyle linéaire de 1 à 5 atomes de carbone qui est substitué en position ω par un groupe 4,5-dihydro-1H-imidazol-2-yle ou un groupe guanidino dans lequel les atomes d'hydrogène sur les atomes d'azote peuvent être remplacés dans chaque cas par des groupes alkyle de 1 à 3 atomes de carbone,
un composé de formule générale VI
T^{A} - Z - NR¹ - (R^{2b}CR³) - CO - Y - (CH₂)ₙ - R (VI)
où
R-(CH₂)ₙ, R¹, R³ et Y sont définis comme dans les revendications 1 à 3, T^{A}-Z possède les significations indiquées pour T-Z dans les revendications 1 à 3 à l'exception de l'atome d'hydrogène et R^{2b} représente un groupe alkyle linéaire de 1 à 5 atomes de carbone, le groupe alkyle étant substitué en position ω par un groupe R⁶NH et R⁶ représentant un atome d'hydrogène ou un groupe alkyle de 1 à 3 atomes de carbone,
est mis à réagir avec un dérivé d'acide carbonique de formule générale VII
X¹ - (C=NR⁷) - (R⁸NR⁹) (VII)
où
R⁷, R⁸ et R⁹, qui peuvent être identiques ou différents, représentent des atomes d'hydrogène, des groupes alkyle de 1 à 3 atomes de carbone ou
R⁷ et R⁸ représentent ensemble aussi un groupe n-alkylène de 2 atomes de carbone et
X¹ représente un groupe partant, ou bien
d) pour la préparation de composés de formule générale I où T-Z possède les significations indiquées pour T-Z dans les revendications 1 à 3 à l'exception de l'atome d'hydrogène et R² représente un groupe alkyle linéaire de 1 à 5 atomes de carbone, le groupe alkyle étant substitué en position ω par un groupe R^{8a}R⁹N-C(=NH)-NR⁶ et R⁶, R^{8a} et R⁹, qui peuvent être identiques ou différents, représentent des atomes d'hydrogène ou des groupes alkyle de 1 à 3 atomes de carbone,
un composé de formule générale VI
T^{A} - Z - NR¹ - (R^{2b}CR³) - CO - Y - (CH₂)ₙ - R (VI)
où
R-(CH₂)ₙ, R¹, R³ et Y sont définis comme dans les revendications 1 à 3, T^{A}-Z possède les significations indiquées pour T-Z dans les revendications 1 à 3 à l'exception de l'atome d'hydrogène et R^{2b} représente un groupe alkyle linéaire de 1 à 5 atomes de carbone, le groupe alkyle étant substitué en position ω par un groupe R⁶NH et R⁶ représentant un atome d'hydrogène ou un groupe alkyle de 1 à 3 atomes de carbone,
est mis à réagir avec un cyanamide de formule générale VIII
R^{8a}R⁹N - CN (VIII)
où
R^{8a} et R⁹, qui peuvent être identiques ou différents, représentent à chaque fois des atomes d'hydrogène ou des groupe alkyle de 1 à 3 atomes de carbone à chaque fois ou e) pour la préparation de composés de formule générale I où T-Z possède les significations indiquées pour T-Z dans les revendications 1 à 3 à l'exception de l'atome d'hydrogène et R² représente un groupe alkyle linéaire de 1 à 5 atomes de carbone, le groupe alkyle étant substitué en position ω par un groupe HR⁸N-C(=NR⁷)-NR⁶ et R⁶, R⁷ et R⁸, qui peuvent être identiques ou différents, représentent des atomes d'hydrogène ou des groupes alkyle de 1 à 3 atomes de carbone,
un composé de formule générale VI
T^{A} - Z - NR¹ - (R^{2b}CR³) - CO - Y - (CH₂)ₙ - R (VI)
où
R-(CH₂)ₙ, R¹, R³ et Y sont définis comme dans les revendications 1 à 3, T^{A}-Z possède les significations indiquées pour T-Z dans les revendications 1 à 3 à l'exception de l'atome d'hydrogène et R^{2b} représente un groupe alkyle linéaire de 1 à 5 atomes de carbone, le groupe alkyle étant substitué en position ω par un groupe R⁶NH, R⁶ représentant un atome d'hydrogène ou un groupe alkyle de 1 à 3 atomes de carbone,
est mis à réagir avec un carbodiimide de formule générale IX
R⁷ - N = C = N - R⁸ (IX)
où
R⁷ et R⁸, qui peuvent être identiques ou différents, représentent des atomes d'hydrogène ou des groupe alkyle de 1 à 3 atomes de carbone ou
f) pour la préparation de composés de formule générale I où T-Z possède les significations indiquées pour T-Z dans les revendications 1 à 3 à l'exception de l'atome d'hydrogène et R² représente un groupe amidinophénylméthyle,
un composé de formule générale X
T^{A} - Z - NR¹ - (R^{2c}CR³) - CO - Y - (CH₂)ₙ - R (X)
où
R-(CH₂)ₙ, R¹, R³ et Y sont définis comme dans les revendications 1 à 3, T^{A}-Z possède les significations indiquées pour T-Z dans les revendications 1 à 3 à l'exception de l'atome d'hydrogène et R^{2c} représente un groupe cyanophénylméthyle,
est mis à réagir avec un alcool de formule générale XI
R⁵ - OH (XI)
où
R⁵ représente un groupe alkyle de 1 à 3 atomes de carbone, puis est traité avec l'ammoniac ou
g) pour la préparation de composés de formule générale I où T-Z possède les significations indiquées pour T-Z dans les revendications 1 à 3 à l'exception de l'atome d'hydrogène et R² représente un groupe amidinophénylméthyle,
un composé de formule générale XII
T^{A} - Z - NR¹ - (R^{2d}CR³) - CO - Y - (CH₂)ₙ - R (XII)
où
R-(CH₂)ₙ, R¹, R³ et Y sont définis comme dans les revendications 1 à 3, T^{A}-Z possède les significations indiquées pour T-Z dans les revendications 1 à 3 à l'exception de l'atome d'hydrogène et R^{2d} représente un groupe phénylméthyle substitué par un groupe H₂N-C(=NOH), est hydrogénolysé ou
h) pour la préparation de composés de formule générale I où T-Z possède les significations indiquées pour T-Z dans les revendications 1 à 3 à l'exception de l'atome d'hydrogène et R² représente un groupe amidinophénylméthyle,
un nitrile de formule générale X
T^{A} -Z- NR¹ - (R^{2c}CR³) - CO - Y - (CH₂)ₙ - R (X)
où
R-(CH₂)ₙ, R¹, R³ et Y sont définis comme dans les revendications 1 à 3, T^{A}-Z est défini comme dans les revendications 1 à 3 à l'exception de la signification d'un atome d'hydrogène et R^{2c} représente un groupe cyanophénylméthyle,
est converti en un thioamide de formule générale XIII
T^{A} - Z - NR¹ - (R^{2c'}CR³) - CO - Y - (CH₂)ₙ - R (XIII)
où
R-(CH₂)ₙ, R¹, R³, Y et T^{A}-Z sont définis comme indiqué ci-dessus et R^{2c'} représente un groupe phénylméthyle substitué par un groupe aminothiocarbonyle, puis est alkylé avec un composé de formule générale XIV
R⁵ - X² (XIV)
où
R⁵ représente un groupe alkyle de 1 à 3 atomes de carbone et X² représente un groupe partant, ou avec un tétrafluoroborate de trialkyloxonium de formule générale XV
(R⁵)₃OBF4 (XV)
où
R⁵ représente un groupe alkyle de 1 à 3 atomes de carbone, puis est mis à réagir avec l'ammoniac ou
i) pour la préparation de composés de formule générale I où T-Z possède les significations indiquées pour T-Z dans les revendications 1 à 3 à l'exception de l'atome d'hydrogène et R² représente un groupe cyaniminométhylaminophénylméthyle,
un composé de formule générale VI
T^{A} - Z - NR¹ - (R^{2b}CR³) - CO - Y - (CH₂)ₙ - R (VI)
où
R-(CH₂)ₙ, R¹, R³ et Y sont définis comme dans les revendications 1 à 3, T^{A}-Z est défini comme dans les revendications 1 à 3 à l'exception de la signification d'un atome d'hydrogène et R^{2b} représente un groupe aminophénylméthyle,
est mis à réagir avec un ester d'acide N-cyanoformimique de formule générale XVI
R¹³O-CH=N-CN (XVI)
où
R¹³ représente un groupe alkyle de 1 à 10 atomes de carbone, ou
j) pour la préparation de composés de formule générale I où T-Z possède les significations indiquées pour T-Z dans les revendications 1 à 3 à l'exception de l'atome d'hydrogène et R² représente un groupe alkyle linéaire de 1 à 5 atomes de carbone substitué en position ω par un groupe aminométhylidènimino ou méthylaminométhylidènimino ou un groupe méthylaminométhylidèniminophénylméthyle,
un composé de formule générale XVII
T^{A} - Z - NR¹ - (R^{2d'}CR³) - CO - Y - (CH₂)ₙ - R (XVII)
où
R-(CH₂)ₙ, R¹, R³ et Y sont définis comme dans les revendications 1 à 3, T^{A}-Z possède les significations indiquées pour T-Z dans les revendications 1 à 3 à l'exception de l'atome d'hydrogène et R^{2d'} représente un groupe alkyle linéaire de 1 à 5 atomes de carbone ou un groupe phénylméthyle, le groupe alkyle étant substitué en position ω par un groupe NC-N=CH-NH et le groupe aromatique cité précédemment étant substitué par un groupe NC-N=CH-NH, est mis à réagir avec une amine de formule générale XVIII
H₂NR^{8a} (XVIII)
où
R^{8a} représente un atome d'hydrogène ou un groupe méthyle, ou
k) pour la préparation de composés de formule générale I où T-Z représente un groupe diphénylaminocarbonyle, naphtylaminocarbonyle, hexaméthylènaminocarbonyle, 1-pipéridinyle ou (diphénylméthyl)aminocarbonyle,
un isocyanate de formule générale XIX
O=C=N - (R^{2a}CR³) - CO - NR⁴ - (CH₂)ₙ - R (XIX)
où
R-(CH₂)ₙ, R³ et R⁴ sont définis comme dans les revendications 1 à 3 et R^{2a} possède les significations indiquées pour R² dans les revendications 1 à 3 ou représente un reste R² substitué par des restes protecteurs ou un reste précurseur pour le reste R²,
est mis à réagir avec une amine de formule générale XX
T¹T²N-H (XX)
où
T¹ et T² représentent chacun un groupe phényle ou
T¹ représente un atome d'hydrogène et T² représente un groupe naphtyle ou diphénylméthyle ou
T¹ et T² représentent ensemble un groupe n-alkylène de 5 ou 6 atomes de carbone, ou
l) pour la préparation de composés de formule générale I où T-Z représente un atome d'hydrogène, le reste R¹⁴ d'un composé de formule générale XXI
R¹⁴ - NR¹ - (R²CR³) - CO - Y - (CH₂)ₙ - R (XXI)
où
R-(CH₂)ₙ, R¹, R², R³ et Y sont définis comme dans les revendications 1 à 3 et R¹⁴ représente un groupe tert.-butoxycarbonyle ou un groupe 9-fluorénylméthoxycarbonyle est clivé, ou
m) pour la préparation de composés de formule générale I où un atome d'hydrogène d^{'}un groupe HN<, HN= ou H₂N- présent dans le reste R² est remplacé par un groupe tert.-butoxycarbonyle, un composé de formule générale I
T - Z - NR¹ - (R²CR³) - CO - Y - (CH₂)ₙ - R (I)
où
R-(CH₂)ₙ, R¹ à R³, T-Z et Y sont définis comme dans les revendications 1 à 3 à condition que R² contienne au moins un groupe HN<, HN= ou H₂N- libre, est mis à réagir avec un composé de formule générale XXII
X³ - W (XXII)
où
W représente un groupe tert.-butoxycarbonyle et X³ représente un groupe partant, ou
n) pour la préparation de composés de formule générale I où T-Z représente un groupe naphtylaminocarbonyle ou (diphénylméthyl)aminocarbonyle, un isocyanate de formule générale XXIII
T'N=C=O (XXIII)
où
T' représente un groupe naphtyle ou diphénylméthyle, est mis à réagir avec un composé de formule générale III
H - NR¹ - (R^{2a}CR³) - CO - Y - (CH₂)ₙ - R (III)
où
R-(CH₂)ₙ, R¹, R³ et Y sont définis comme dans les revendications 1 à 3 et R^{2a} possède les significations indiquées pour R² dans les revendications 1 à 3 ou représente un reste R² substitué par des restes protecteurs ou un reste précurseur pour le reste R²,
et, si on le souhaite, un composé de formule générale I ainsi obtenu est ensuite résolu en ses diastéréoisomères et/ou
un racémate d'un composé de formule générale I ainsi obtenu est dédoublé en ses énantiomères et/ou
un composé de formule générale I ainsi obtenu est converti en ses sels avec des acides ou des bases inorganiques, en particulier pour l'utilisation pharmaceutique en ses sels physiologiquement acceptables.

9. Enantiomères (D) et (R) des composés selon les revendications 1 à 3.
